# EUROPEAN PATENT SPECIFICATION

(11) **EP 1 668 152 B1**
(45) Date of publication and mention of the grant of the patent: **06.08.2008**
(21) Application number: 04769358.5
(22) Date of filing: 27.08.2004
(51) Int. Cl.: C12Q 1/68

(54) **IDENTIFICATION OF AN ERBB2 GENE EXPRESSION SIGNATURE IN BREAST CANCER**
IDENTIFIZIERUNG EINER ERBB2-GENEXPRESSIONSSIGNATUR BEI BRUSTKREBS
IDENTIFICATION D'UNE SIGNATURE D'EXPRESSION DU GENE ERBB2 DANS LE CANCER DU SEIN

(30) Priority: 28.08.2003 US 498497 P; 27.08.2004 US 928465
(43) Date of publication of application: 14.06.2006
(60) Divisional application: 08007447.9
(73) Proprietor: Ipsogen, 13009 Marseille (FR); INSTITUT NATIONAL DE LA SANTE ET DE LA RECHERCHE MEDICALE (INSERM), 75654 Paris Cédex 13 (FR); Institut Paoli-Calmettes, 13009 Marseille (FR)
(72) Inventor: BIRNBAUM, Daniel, F-13009 Marseille (FR); BERTUCCI, François, Immeuble Le Sully, F-13008 Marseille (FR); JACQUEMIER, Jocelyne, F-13011 Marseille (FR); DEBONO, Stéphane, Rés. La Palmeraie-Borely Apt D22, F-13008 Marseille (FR); BORIE, Nathalie, F-13013 Marseille (FR); GINESTIER, Christophe, HLM de Luminy Bat D Appt 23, F-13009 Marseille (FR)
(74) Representative: Bredema
(86) International application number: PCT/IB2004/002968
(87) International publication number: WO 2005/021788

(56) References cited:
- WO-A2-02/10436
- WO-A2-02/059271
- WO-A2-02/068579
- WO-A2-02/103320
- WO-A2-03/060470
- US-A1- 2003 096 237
- "GPL91: Affymetrix GeneChip Human Genome U95 Set HG-U95A" NCBI GEO, 11 March 2002 (2002-03-11), XP002293987
- BERTUCCI FRANCOIS ET AL: "Breast cancer revisited using DNA array-based gene expression profiling." INTERNATIONAL JOURNAL OF CANCER, vol. 103, no. 5, 20 February 2003 (2003-02-20), pages 565-571, XP002316056 ISSN: 0020-7136 cited in the application
- OH JULIANA J ET AL: "Identification of differentially expressed genes associated with HER-2/neu overexpression in human breast cancer cells" NUCLEIC ACIDS RESEARCH, vol. 27, no. 20, 15 October 1999 (1999-10-15), pages 4008-4017, XP002937284 ISSN: 0305-1048 cited in the application
- WILSON KATHERINE S ET AL: "Differential gene expression patterns in HER2/neu-positive and negative breast cancer cell lines and tissues" AMERICAN JOURNAL OF PATHOLOGY, vol. 161, no. 4, October 2002 (2002-10), pages 1171-1185, XP002316057 ISSN: 0002-9440 cited in the application
- BERTUCCI FRANCOIS ET AL: "Identification and validation of an ERBB2 gene expression signature in breast cancers" ONCOGENE, vol. 23, no. 14, 1 April 2004 (2004-04-01), pages 2564-2575, XP008042086 ISSN: 0950-9232

### SEQUENCE LISTING

<110> IPSOGEN, INSTITUT NATIONAL DE LA SANTÉ ET DE LA RECHERCHE MÉDICALE -INSERM- & INSTITUT PAOLI-CALMETTES
<120> Identification of an ERBB2 gene expression signature in breast cancers
<130> 34389/PCT
<140> PCT/FR04/XXXXX
   <141> 2004-08-27
<150> US60/498,497
   <151> 2003-08-28
<160> 94
<170> Patent In Ver. 2.1
<210> 1
   <211> 405
   <212> DNA
   <213> Artificial Sequence
<220>
   <223> Description of Artificial Sequence:primer
<220>
   <221> misc_feature
   <222> (1) .. (405)
   <223> 3' terminal sequence. j domain containing protein 1 (JDP1) gene.
<400> 1
   ttttaagcac aaattcattg cctttattct gatgagcttc tcttcatggg atttacggac 60
   actgaattat atccagactc tgctaacttc ttggttttct ccctcctatc gcctaatgac 120
   tctttaagct actacatgag tctaatccat gggcatcctg agcttcacaa attcacgtcg 180
   cacccagcga gaaacccacc gtcttcactg agtcattcaa agcttcccac tgctggaatg 240
   gcatcgacat ctggctcctt cgccagtggt catagcgggc tcgactctct tcattggnca 300
   gaatctcctt tgccttctgc agtttctgaa aagtctccac agctttgggg gttttnagga 360
   tgcttgtctg gggtgacatt ccagagctct gactttaaat tctgc                 405
<210> 2
   <211> 409
   <212> DNA
   <213> Artificial Sequence
<220>
   <223> Description of Artificial Sequence:primer
<220>
   <221> misc_feature
   <222> (1)..(409)
   <223> 5' terminal sequence. j domain containing protein 1 (JDP1) gene.
<400> 2
   gattaagtca tctaaatgga tgcaatactg aattacaggt cagaagatac tgaagattac 60
   tacacattac tgggatgtga tgaactatct tcggttgaac aaatcctggc agaatttaaa 120
   gtcagagctc tggaatgtca cccagacaag catcctgaaa accccaaagc tgtggagact 180
   tttcagaaac tgcagaaggc aaaggagatt ctgaccaatg aagagagtcg agcccgctat 240
   gaccactggc gaaggagcca gatgtcgatg ccattccagc agtgggaagc tttgaatgac 300
   tcagtgaaga cggtgggttt ctcgctgggt gcgacgttga atttgttgaa gctcagggtt 360
   gcccntgggt ttaggactca tgttagttag gctttaaagn gttctttag             409
<210> 3
   <211> 1203
   <212> DNA/RNA
   <213> Artificial Sequence
<220>
   <223> Description of Artificial Sequence:primer
<220>
   <221> misc_feature
   <222> (1) .. (1203)
   <223> j domain containing protein 1 (JDP1) gene.
<400> 3
   catctggtgt attgactgtg gccagtctta aagctagttt ttgctatgtg gaacatgctg 60
   ctctaattca gatttaaaga gtttcttcct gttaattcga agctcactgt gcctcttgtt 120
   tccgagggaa gaaggactga ttaagtcatc taaatggatg caatactgaa ttacaggtca 180
   gaagatactg aagattacta cacattactg ggatgtgatg aactatcttc ggttgaacaa 240
   atcctggcag aatttaaagt cagagctctg gaatgtcacc cagacaagca tcctgaaaac 300
   cccaaagctg tggagacttt tcagaaactg cagaaggcaa aggagattct gaccaatgaa 360
   gagagtcgag cccgctatga ccactggcga aggagccaga tgtcgatgcc attccagcag 420
   tgggaagctt tgaatgactc agtgaagacg tcaatgcact gggttgtcag aggtaaaaaa 480
   gacctgatgc tggaagaatc tgacaagact cataccacca agatggaaaa tgaggaatgt 540
   aatgagcaaa gagaaagaaa gaaagaggag ctggcttcaa ccgcagagaa aacggagcag 600
   aaagaaccca agcccctaga gaagtcagtc tccccgcaaa attcagattc ttcaggtttt 660
   gcagatgtga atggttggca ccttcgtttc cgctggtcca aggatgctcc ctcagaactc 720
   ctgaggaagt tcagaaacta tgaaatatga aatatctctg cttcaaaaaa tgaggaagag 780
   caagactgtc ccctatgctg ccaacatgca gtctttgttt atgtcttaaa aatgtcatgt 840
   ttatgtcatg tctgtgaatt gctgagtact aattgattcc tccatccttg aatcagttct 900
   cataatgctt tttaaataag aaaaattcag aagatgaatt tcttccaata tttgaataaa 960
   ttaaagctct tagatacaga gtagattgta ttatatgctt tttcctatta atactactta 1020
   tagaaatcca ttaaaaagca atctctgtac agtgtattta aatatttcat tgacatactg 1080
   tgatctctat tagtgatgga tgtacaaaaa atgttttctt acccttgact tacaatgaaa 1140
   tgtgaaatta cttgtctgaa ccccgtgggg agaaataaat aattttccca aagttcaaaa 1200
   aaa                                                               1203
<210> 4
   <211> 440
   <212> DNA
   <213> Artificial Sequence
<220>
   <223> Description of Artificial Sequence:primer
<220>
   <221> misc_feature
<222> (1) .. (440)
   <223> 3' terminal sequence. proteasome (prosome, macropain) subunit, beta type, 3 (PSMB3) gene.
<400> 4
   aattttgaca ggctatttta ttncaaaaaa agaaaaaaaa gtgggctctg ggancagggt 60
   tagnccattc gggcctncag tntcctgggg gngattttgn ccttctcgat gacntggaca 120
   atgactccca tgcctgacac tgcatcccgg nccacagcat tcagcatggc ttgggagatg 180
   gtttcaaaca ggtganccgg anccatgttg ggntcccaga ggatctcaca cattccgtac 240
   atttgtncgg cgcaggtgcc actgaccaca aagtcatcag tcaccatggg ggcagccgat 300
   gaggtntagg agagcaaatg aagggnttaa aggtttttcg ggntccaacc cggcaatgac 360
   tgggctcagt ntaggtaagg gggccaaacc nttttttcat aacaggggtt tggncaccat 420
   ggcttcatga ggggtttaan                                             440
<210> 5
   <211> 467
   <212> DNA
   <213> Artificial Sequence
<220>
   <223> Description of Artificial Sequence:primer
<220>
   <221> misc_feature
   <222> (1) .. (467)
   <223> 5' terminal sequence. proteasome (prosome, macropain) subunit, beta type, 3 (PSMB3) gene.
<400> 5
   atccaggccc agttggtgac cacggacttc cagaagatct ttcccatggg tgaccggctg 60
   tacatcggtc tggccgggct cgccactgac gtccagacag ttgcccagcg cctcaagttc 120
   cggctgaacc tgtatgagtt gaaggaaggt cggcagatca aaccttatac cctcatgagc 180
   atggtngcca acctcttgta tgagaaacgg tttggccctt actacactga gccagtcatt 240
   gccgggttgg acccgaagac ctttaagccc ttcatttgct ctctaggacc tcatcggctg 300
   ccccatgggt gactgatgac tttgtgggtc agtgggcacc tgcggccgaa caaatgttac 360
   ggaatgtgtg gagtcctttg ggaggcccaa catgggttcc ggattcaact gttttgaaaa 420
   ccattttccc aagccnggtg gattgttttg gaacngggtg nagtttt 467
<210> 6
   <211> 784
   <212> DNA/RNA
   <213> Artificial Sequence
<220>
   <223> Description of Artificial Sequence:primer
<220>
   <221> misc_feature
   <222> (1) .. (784)
   <223> proteasome (prosome, macropain) subunit, beta type, 3 (PSMB3) gene.
<400> 6
   gagcggttgc gcagtgaagg ctagacccgg tttactggaa ttgctctggc gatcgagggg 60
   tcctagtaca ccgcaatcat gtctattatg tcctataacg gaggggccgt catggccatg 120
   aaggggaaga actgtgtggc catcgctgca gacaggcgct tcgggatcca ggcccagatg 180
   gtgaccacgg acttccagaa gatctttccc atgggtgacc ggctgtacat cggtctggcc 240
   gggctcgcca ctgacgtcca gacagttgcc cagcgcctca agttccggct gaacctgtat 300
   gagttgaagg aaggtcggca gatcaaacct tataccctca tgagcatggt ggccaacctc 360
   ttgtatgaga aacggtttgg cccttactac actgagccag tcattgccgg gttggacccg 420
   aagaccttta agcccttcat ttgctctcta gacctcatcg gctgccccat ggtgactgat 480
   gactttgtgg tcagtggcac ctgcgccgaa caaatgtacg gaatgtgtga gtccctctgg 540
   gagcccaaca tggatccgga tcacctgttt gaaaccatct cccaagccat gctgaatgct 600
   gtggaccggg atgcagtgtc aggcatggga gtcattgtcc acatcatcga gaaggacaaa 660
   atcaccacca ggacactgaa ggcccgaatg gactaaccct gttcccagag cccacttttt 720
   tttctttttt tgaaataaaa tagcctgtct ttcaaaaaaa aaaaaaaaaa aaaaaaaaaa 780
   aaaa 784
<210> 7
   <211> 273
   <212> DNA
   <213> Artificial Sequence
<220>
   <223> Description of Artificial Sequence:primer
<220>
   <221> misc feature
   <222> (1) .. (273)
   <223> 3' terminal sequence. n-acetyltransferase 1 (arylamine n-acetyltransferase) (NAT1) gene.
<400> 7
   acaatatttt atttactcat ctaccaataa aacttttcta ggaattcaac aataaaccaa 60
   cattaaaagc tttctagcat aaatcaccaa tttccaagat aaccacaggc catctttaaa 120
   atacattttt tattattatt attattatta tttgaaaagg tttgtggtta tgtttcttta 180
   aaaagctgtt taattatata tgatgacatt tttatagggt gaaatgattt gatgtctagg 240
   gnttttcttc aaaataaggg taaggggtac agg                               273
<210> 8
   <211> 425
   <212> DNA
   <213> Artificial Sequence
<220>
   <223> Description of Artificial Sequence:primer
<220>
   <221> misc feature
   <222> (1) .. (425)
   <223> 5' terminal sequence. n-acetyltransferase 1 (arylamine n-acetyltransferase) (NAT1) gene.
<400> 8
   naagattttg agtctatgaa tacatacctg cagacatctc catcatctgt gtttactagt 60
   aaatcatttt gttccttgca gaccccagat gggttncact gtttggtggg cttcaccctc 120
   acccatagga gattcaatta taaggacaat acagatctaa tagagttcaa gactctgagt 180
   gaggaagaaa tagaaaaagt gctgaaaaat atatttaata tttccttgca gagaaagctt 240
   gtgcccaaac atggtgatag attttttact atttagaata aggagtaaaa caatcttgtc 300
   tatttgtcat ccagctcacc agttatccaa ctgacggacc tattcatgta tcnttctgta 360
   cccttacctt tatttttgga aggaaaatcc taggacatcc aaatcctttt cacctattaa 420
   aaaat                                                             425
<210> 9
   <211> 1319
   <212> DNA/RNA
   <213> Artificial Sequence
<220>
   <223> Description of Artificial Sequence:primer
<220>
   <221> misc_feature
   <222> (1) .. (1319)
   <223> n-acetyltransferase 1 (arylamine n-acetyltransferase) (NAT1) gene.
<400> 9
   atacaatgaa agcactagaa ataattatta tacttataac cattgtattt ttacatgttt 60
   aaaatatagc cataattagc ctactcaaat ccaagtgtaa aagtaaaatg atttgctttc 120
   gttttgtttt ccttgcttag gggatcatgg acattgaagc atatcttgaa agaattggct 180
   ataagaagtc taggaacaaa ttggacttgg aaacattaac tgacattctt caacaccaga 240
   tccgagctgt tccctttgag aaccttaaca tccattgtgg ggatgccatg gacttaggct 300
   tagaggccat ttttgatcaa gttgtgagaa gaaatcgggg tggatggtgt ctccaggtca 360
   atcatcttct gtactgggct ctgaccacta ttggttttga gaccacgatg ttgggagggt 420
   atgtttacag cactccagcc aaaaaataca gcactggcat gattcacctt ctcctgcagg 480
   tgaccattga tggcaccaac tacattgtcg atgctgggtt tggacgctca taccagatgt 540
   ggcagcctct ggagttaatt tctgggaagg atcagcctca ggtgccttgt gtcttccgtt 600
   tgacggaaga gaatggattc tggtatctag accaaatcag aacccaacag tacattccaa 660
   atgaagaatt tcttcattct gatctcctag aagacagcaa ataccgaaaa atctactcct 720
   ttactcttaa gcctcgaaca attgaagatt ttgagtctat gaatacatac ctgcagacat 780
   ctccatcatc tgtgtttact agtaaatcat tttgttcctt gcagacccca gatggggttc 840
   actgtttggt gggcttcacc ctcacccata ggagattcaa ttataaggac aatacagatc 900
   taatagagtt caagactctg agtgaggaag aaatagaaaa agtgctgaaa aatatattta 960
   atatttcctt gcagagaaag cttgtgccca aacatggtga tagatttttt actatttaga 1020
   ataaggagtg aaacaatctt gtctatttgt catccagctc accagttatc aactgacgac 1080
   ctatcatgta tcttctgtac ccttacctta ttttgaagaa atcctagaca tcaaatcatt 1140
   tcacctataa aaatgtcatc atatataatt aaacagcttt ttaaagaaac ataaccacaa 1200
   accttttcaa ataataataa taataataat aataaatgtc ttttaaagag gcctgtggtt 1260
   atcttggaaa ttggtgattt atgctagaaa gcttttaatg ttggtttatt gttgaattc 1319
<210> 10
   <211> 336
   <212> DNA
   <213> Artificial Sequence
<220>
   <223> Description of Artificial Sequence:primer
<220>
   <221> misc_feature
   <222> (1) .. (336)
   <223> 3' terminal sequence. hypothetical protein loc148696 (LOC148696) gene.
<400> 10
   gggctcccaa aactttttat ttagagggaa gaatgctagg gagatgggta tgcagagggt 60
   tgaccaaatt ggaagaaaat atttattctg tagtttggtg ttggaaaagg gaattttcca 120
   atcagccaca cctcagtgtt gcggcaaaat aattcttggc tcccctggaa acgcatgggc 180
   aaggtagggc agagctgctg ctgctgatac tgccaccacc ctgggcttcc tgctgactct 240
   gggctactcc ctggggacaa cagatttgca ttgacgtccg gggctgtcca gaggccctca 300
   agagccagtt gtgagctgag cccagtatgg gaaaga 336
<210> 11
   <211> 356
   <212> DNA
   <213> Artificial Sequence
<220>
   <223> Description of Artificial Sequence:primer
<220>
   <221> misc_feature
   <222> (1) .. (356)
   <223> 3' terminal sequence. sapiens, clone image:4831215, mrna (EST AA878915) gene.
<400> 11
   tttgagaaat agggtttttt attatatatt catttttcca tatttctgga aaatgaaaca 60
   gacatggagt atttcaaagc tgggagagat cattatattg gagttacctt agaaagaggg 120
   ggctgtttct gccaccacaa caaagaatga aacaacacaa atatttacgt tttcttcttg 180
   tcctcataat gcttttactg cactcgtctt tcaaccttta acatctactg catctgtgac 240
   tgttgtttca gtaaaacacg gaagcttgaa tgtaagctca tttcattgcc tgctgcacaa 300
   cacacaaaac agcattttct tattcttgaa atatgatagc aacgtctcct tccatg     356
<210> 12
   <211> 369
   <212> DNA
   <213> Artificial Sequence
<220>
   <223> Description of Artificial Sequence:primer
<220>
   <221> misc_feature
   <222> (1) .. (369)
   <223> 3' terminal sequence. nucleolar protein 3 (apoptosis repressor with card domain) (NOL3) gene.
<400> 12
   cattgtcann nttgttttaa ttgtctggct tctctctgga ctgggagctc agtgaggatt 60
   ctgaccagtg acttacacaa aaggcgctct atacatatta taatatattc gcttactaaa 120
   tgantaagga ctttccaact gcgtttctga gttttacaga tgggaaaact aaggccaaag 180
   gataggagtg gggttcacac agctaagtct gaagggaaac tgggaagcag caaccacctc 240
   tgcacctcac ctgggtctaa ggagggggtt cagggacttg gggccaccaa actctagggg 300
   cctgtcccct cagggtgcaa ttncagctgc tccnagggct atggccaacc ctcttgccag 360
   agaggcagc                                                         369

<210> 13
   <211> 1411
   <212> DNA/RNA
   <213> Artificial Sequence
<220>
   <223> Description of Artificial Sequence:primer
<220>
   <221> misc_feature
   <222> (1) .. (1411)
   <223> nucleolar protein 3 (apoptosis repressor with card domain) (NOL3) gene.
<400> 13
   ggcacgaggg agaggcagga ggacaccgag ttccccgtgt tggcctccag gtcctgtgct 60
   tgcggagccg tccggcggct gggatcgagc cccgacaatg ggcaacgcgc aggagcggcc 120
   gtcagagact atcgaccgcg agcggaaacg cctggtcgag acgctgcagg cggactcggg 180
   actgctgttg gacgcgctgc tggcgcgggg cgtgctcacc gggccagagt acgaggcatt 240
   ggatgcactg cctgatgccg agcgcagggt gcgccgccta ctgctgctgg tgcagggcaa 300
   gggcgaggcc gcctgccagg agctgctacg ctgtgcccag cgtaccgcgg gcgcgccgga 360
   ccccgcttgg gactggcagc acgtgggtcc gggctaccgg gaccgcagct atgaccctcc 420
   atgcccaggc cactggacgc cggaggcacc cggctcgggg accacatgcc ccgggttgcc 480
   cagagcttca gaccctgacg aggccggggg ccctgagggc tccgaggcgg tgcaatccgg 540
   gaccccggag gagccagagc cagagctgga agctgaggcc tctaaagagg ctgaaccgga 600
   gccggagcca gagccagagc tggaacccga ggctgaagca gaaccagagc cggaactgga 660
   gccagaaccg gacccagagc ccgagcccga cttcgaggaa agggacgagt ccgaagattc 720
   ctgaaggcca gagctctgac aggcggtgcc ccgcccatgc tggataggac ctgggatgct 780
   gctggagctg aatcggatgc caccaaggct cggtccagcc cagtaccgct ggaagtgaat 840
   aaactccgga gggtcggacg ggacctgggc tctctccacg attctggctg tttgcccagg 900
   aacttagggt gggtacctct gagtcccagg gacctgggca ggcccaagcc caccacgagc 960
   atcatccagt cctcagccct aatctgccct taggagtcca ggctgcaccc tggagatccc 1020
   aaacctagcc ccctagtggg acaaggacct gaccctcctg cccgcataca caacccattt 1080
   cccctggtga gccacttggc agcatatgta ggtaccagct caaccccacg caagttcctg 1140
   agctgaacat ggagcaaggg gagggtgact tctctccaca tagggagggc ttagagctca 1200
   cagccttggg aagtgagact agaagagggg agcagaaagg gaccttgagt agacaaaggc 1260
   cacacacatc attgtcatta ctgttttaat tgtctggctt ctctctggac tgggagctca 1320
   gtgaggattc tgaccagtga cttacacaaa aggcgctcta tacatattat aatatattcg 1380
   cttactaaat gaaaaaaaaa aaaaaaaaaa a 1411
<210> 14
   <211> 545
   <212> DNA
   <213> Artificial Sequence
<220>
   <223> Description of Artificial Sequence:primer
<220>
   <221> misc_feature
   <222> (1) .. (545)
   <223> 3' terminal sequence. integrin, alpha 2b (platelet glycoprotein iib of iib/iiia complex, antigen cd41b) (ITGA2B) gene.
<400> 14
   gggggggtag cccagctctg ttgggaggga aacgacacca agaggaccca atggaacagc 60
   tccaacccaa agcttggagg caacttgttg gagaaggggc ggtgcaggta gcacgcccaa 120
   ccctcctgct agaatagtgt aggctgcacc atcactcccc ctcttcatca tcttcttcca 180
   ggggtggccg gttccgcttg aagaagccga ccttccacat ggccaggacc aggatggtga 240
   gcagcagcag gccacccagc acacccacca gcacccacca gattggaatg gccctctcct 300
   ccaaggcccg gagcagctgt gtccacacct gagcttcccc tcggggcagg ctgagcgggg 360
   gcaccgcata ggggagggag gacacgttga accatgcgtg cgactgcagc acaaactgat 420
   ccagaggcct ctggtagaag ctgggcacca caaggaaggc agcaccgtga acatggccgc 480
   tgccgcgcgc catctcctgc aggtcacctg caccaagtac aggcggcggg tcgcactttc 540
   gagaa 545
<210> 15
   <211> 3334
   <212> DNA/RNA
   <213> Artificial Sequence
<220>
   <223> Description of Artificial Sequence:primer
<220>
   <221> misc_feature
   <222> (1) .. (3334)
   <223> integrin, alpha 2b (platelet glycoprotein iib of iib/iiia complex, antigen cd41b) (ITGA2B) gene.
<400> 15
<210> 16
   <211> 639
   <212> DNA
   <213> Artificial Sequence
<220>
   <223> Description of Artificial Sequence:primer
<220>
   <221> misc_feature
   <222> (1) .. (639)
   <223> 3' terminal sequence. lymphotoxin alpha (tnf superfamily, member 1) (LTA) gene.
<400> 16
   tttttttttt cagagaaaag aggtttattg ggcttcatcg agggtgcaga tgcctccgtg 60
   tggggctctg gtcggcagct ggctttcaga gcctttccct gccttctggg gccctgtgat 120
   ccctcatgcc tacttctttc tctcttggtc agccttgtgc gcatgccctc tcactcttca 180
   tctcttgggc ctgtctctgt ttctccttgg atgttcttct attattcccc tctctccatc 240
   ctccataaat aaataattta attttttttg ccttcataaa tagtcccctc cctgcctcta 300
   gtcatccccc aagctcctcc atgtgcctgc tcttcctctg tgtgtggatc taggccccac 360
   ctagctggtg ggacagacca acagctttgg gctgggaatt cctaggcagg cttgaaatcc 420
   tcagccagac agacatcagg gatggttcag ggaggtgtgg tcccctggat gcctagaatt 480
   ccttctttga aagctccggt gacttgatca ggggagactt gagctgttgg aatggcaaag 540
   agaggtggtg acgaccctgc aatggtcaga atggaggcag aaatggggag aaggcttgaa 600
   atcattantt ttctttctgg attttccaag tctacagag                        639
<210> 17
   <211> 1386
   <212> DNA/RNA
   <213> Artificial Sequence
<220>
   <223> Description of Artificial Sequence:primer
<220>
   <221> misc_feature
   <222> (1) .. (1386)
   <223> lymphotoxin alpha (tnf superfamily, member 1) (LTA) gene.
<400> 17
   gccccatctc cttgggctgc ccgtgcttcg tgctttggac taccgcccag cagtgtcctg 60
   ccctctgcct gggcctcggt ccctcctgca cctgctgcct ggatccccgg cctgcctggg 120
   cctgggcctt ggttctcccc atgacaccac ctgaacgtct cttcctccca agggtgtgtg 180
   gcaccaccct acacctcctc cttctggggc tgctgctggt tctgctgcct ggggcccagg 240
   ggctccctgg tgttggcctc acaccttcag ctgcccagac tgcccgtcag caccccaaga 300
   tgcatcttgc ccacagcacc ctcaaacctg ctgctcacct cattggagac cccagcaagc 360
   agaactcact gctctggaga gcaaacacgg accgtgcctt cctccaggat ggtttctcct 420
   tgagcaacaa ttctctcctg gtccccacca gtggcatcta cttcgtctac tcccaggtgg 480
   tcttctctgg gaaagcctac tctcccaagg ccacctcctc cccactctac ctggcccatg 540
   aggtccagct cttctcctcc cagtacccct tccatgtgcc tctcctcagc tcccagaaga 600
   tggtgtatcc agggctgcag gaaccctggc tgcactcgat gtaccacggg gctgcgttcc 660
   agctcaccca gggagaccag ctatccaccc acacagatgg catcccccac ctagtcctca 720
   gccctagtac tgtcttcttt ggagccttcg ctctgtagaa cttggaaaaa tccagaaaga 780
   aaaaataatt gatttcaaga ccttctcccc attctgcctc cattctgacc atttcagggg 840
   tcgtcaccac ctctcctttg gccattccaa cagctcaagt cttccctgat caagtcaccg 900
   gagctttcaa agaaggaatt ctaggcatcc caggggacca cacctccctg aaccatccct 960
   gatgtctgtc tggctgagga tttcaagcct gcctaggaat tcccagccca aagctgttgg 1020
   tctgtcccac cagctaggtg gggcctagat ccacacacag aggaagagca ggcacatgga 1080
   ggagcttggg ggatgactag aggcagggag gggactattt atgaaggcaa aaaaattaaa 1140
   ttatttattt atggaggatg gagagagggg aataatagaa gaacatccaa ggagaaacag 1200
   agacaggccc aagagatgaa gagtgagagg gcatgcgcac aaggctgacc aagagagaaa 1260
   gaagtaggca tgagggatca cagggcccca gaaggcaggg aaaggctctg aaagccagct 1320
   gccgaccaga gccccacacg gaggcatctg caccctcgat gaagcccaat aaacctcttt 1380
   tctctg                                                            1386
<210> 18
   <211> 255
   <212> DNA
   <213> Artificial Sequence
<220>
   <223> Description of Artificial Sequence:primer
<220>
   <221> misc_feature
   <222> (1)..(255)
   <223> 3' terminal sequence. nuclear factor of kappa light polypeptide gene enhancer in b-cells inhibitor, epsilon (NFKBIE) gene.
<400> 18
   ctagaaggga aaaacttttt ttaacgtctc tgccatttta gtgtcatgtt actgaaactt 60
   cccttcccca gtggctcagt tcagagcctt ctgtgtgaag tctttaaaca acggtgtttc 120
   agggtcctca acagcaataa gatgggacac ctctcagggt tctgcttgct cctatttcag 180
   tctcttctca ctcaggagag tccatgcttc ctactggttg gcagtttcag gctgacccaa 240
   catgggtaaa acaag                                                  255
<210> 19
   <211> 2067
   <212> DNA/RNA
   <213> Artificial Sequence
<220>
   <223> Description of Artificial Sequence:primer
<220>
   <221> misc_feature
   <222> (1) .. (2067)
   <223> nuclear factor of kappa light polypeptide gene enhancer in b-cells inhibitor, epsilon (NFKBIE) gene.
<400> 19
   ggaattccaa tgaatgaatg aatgaatgag tgaatgaatc aacgaaggag tgagtcaagg 60
   cccgggaacc acagactcca agcctacgca gagcccggga agggggattc cggaggggcg 120
   gggcctcttt ccggaagcgc ccgccggggg cggggagggg gcggggccat ccgcgtgagg 180
   cgaccctgtt ggtccggagg ggcggggcga ggaggaggac ggccttgggc ggttcggctg 240
   cccacagtaa ccgctgggtg gacctggcca gcgctccgaa ccttgtcctc gctgcgcgcc 300
   ggcccctcgg agccccacag cccgggaagg aggcggccgc gggcggggcg cccgctctgc 360
   caagcggacc cgcaacccgg aaaggcggcg cggcggagcc tggagccgga tcctgctcag 420
   accgggcccc ggccggccag agccgcgggc atgtcggagg cgcggaaggg gccggacgag 480
   gcggaggaga gccagtacga ctctggcatt gagtctctgc gctctctgcg ctccctaccc 540
   gagtccacct cggctccagc ctccgggccc tcggacggca gcccccagcc ctgcacccat 600
   cctccgggac ccgtcaagga accacaggag aaggaagacg cggatgggga gcgggctgat 660
   tccacctatg gctcctcctc gctcacctac accctgtcct tgctgggggg ccccgaggct 720
   gaggacccgc ccccacgcct gccactcccc cacgtggggg cgctgagccc tcagcagctg 780
   gaagcactca cttacatctc cgaggacgga gacacgctgg tccacctggc agtgattcat 840
   gaggccccag cggtgctgct ctgttgcctg gctttgctgc cccaggaggt cctggacatt 900
   caaaataacc tttaccagac agcactccat ctggctgtac atctggacca accgggcgca 960
   gttcgggcac tggtgctgaa gggggccagc cgggcactac aggaccggca tggagacaca 1020
   gcccttcatg tggcctgcca gcgccagtct tggcctgtgc ccgctgcctg ctggaagggc 1080
   gggccagagc caggcagagg aacatctcac tctctggacc tccagctgca aaactggcaa 1140
   ggtctggctt gtctccacat tgccaccctt cagaagaacc aaccactcat ggaattgctg 1200
   cttcggaatg gagctgacat tgatgtgcag gagggctcca gtggtaagac agcgctgcac 1260
   ctggctgtgg aaacccaaga gcggggcctg gtacagttcc tgctccaggc tggtgcccag 1320
   gtagatgccc gcatgctgaa cgggtgcaca cccctgcacc tggcagctgg ccggggtctc 1380
   atgggcatct catccactct gtgcaaggcg ggtgctgact ccctgctgcg gaatgtggag 1440
   gatgagacgc cccaggacct gactgaggaa tcccttgtcc ttttgccctt tgatgacctg 1500
   aagatctcag ggaaactgct gctgtgtacc gactgaagcc aggcagggtc tgggatcctc 1560
   agggctccac ctctccatct ggaagccgga gccataactg ctgcagtttg ggcccaggct 1620
   atgtgctctt ctggtgccct agggactgct gtggccagag cctggggcca gccagtacag 1680
   tcctgagccg aggaggaggg actgcaagtg gaagagagcc agtctggaag gaagagcttt 1740
   ccaggtggac agggcttctt ggaagacccc caaagcccca ggtatcctgg gtgaagcctg 1800
   tttgcctctc ttgaaaatgg caggtgctct tgttttaccc atgttgggtc agcctgaaac 1860
   tgccaaccag taggaagcat ggactctcct gagtgagaag agactgaaat aggagcaagc 1920
   agaaccctga gaggtgtcca tcttcttgct gttgaggacc ctgaaacacc gttgtttaaa 1980
   gacttcacac agaaggctct gaactgagcc actggggaag ggaagtttca gtaacatgac 2040
   actaaaatgg cagagacgtt aaaaaaa                                       2067
<210> 20
   <211> 498
   <212> DNA
   <213> Artificial Sequence
<220>
   <223> Description of Artificial Sequence:primer
<220>
   <221> misc_feature
   <222> (1) .. (498)
   <223> 3' terminal sequence. cadherin, egf lag seven-pass g-type receptor 2 (flamingo homolog, drosophila) (CELSR2) gene.
<400> 20
   aagtcatgat taatttnaaa ccagttaaat ttttaaccct ttgcaggaat tgctgagggg 60
   agaagacagg gggagaatcc acggcagaaa agccagagnt gggcctcaca aatgagaaac 120
   aggagccttt ctttcctgcc cacagcctct ttttctagcc acctgctcca gaaggaaagt 180
   cagtgacaag tctggaatac atgcttggag taaatggtga ctcagatgaa aagcagtcgg 240
   caaaactgag gagaggggag gagaggtggg gagatgatgg cctggggcaa ggggaagggc 300
   gtcaagcccc aagccagggc tgctgggaac acccagcctg tgatggccat atcagacccc 360
   cgggactgga cacgaaccca tcccaacgca aaaagcaaat aaataacaaa ttttttaact 420
   tttttcttca caggggagct ggggttggan tcggggagaa agggggacag ggcctggncc 480
   ttttgaagga gagagcca                                               498
<210> 21
   <211> 10531
   <212> DNA/RNA
   <213> Artificial Sequence
<220>
   <223> Description of Artificial Sequence:primer
<220>
   <221> misc_feature
   <222> (1) .. (10531)
   <223> cadherin, egf lag seven-pass g-type receptor 2 (flamingo homolog, drosophila) (CELSR2) gene.
<400> 21
<210> 22
   <211> 459
   <212> DNA
   <213> Artificial Sequence
<220>
   <223> Description of Artificial Sequence:primer
<220>
   <221> misc_feature
   <222> (1) .. (459)
   <223> 3' terminal sequence. peptidyl arginine deiminase, type ii (PADI2) gene.
<400> 22
   ttgacttcct tatgatncca tttatttcat tgttctttag tcgagctctt ccctaaacat 60
   ctttagatct ccaccacagg ctcttttcca gaaatttgaa actgtgttct tcttgccatc 120
   ttcacgacat cccctgccct cttacataag atatttcaac atcaaggtgg aagcaggaac 180
   ttagctgagt tttgcaacag agaagcgtat tctaggccta catttataga aagtgggggt 240
   ggggaagagc catgagtcca cgggggtata tccacaccga gggttgtcac actgggtggg 300
   gcaagtgaga tggggaacgg gtntgtgagt ccnggggaac ttcagaaaca tcagaaatta 360
   cccgacatca ttggggaaag ccttaggaaa aatctntaaa ggcacacttg tctggcacat 420
   ggggagggcg ttcanttccc ccnaattgta ggcttaaaa                        459
<210> 23
   <211> 2348
   <212> DNA/RNA
   <213> Artificial Sequence
<220>
   <223> Description of Artificial Sequence:primer
<220>
   <221> misc_feature
   <222> (1) .. (2348)
   <223> peptidyl arginine deiminase, type ii (PADI2) gene.
<400> 23
   cgcacctgct gcaggtgctc ccggccgccc cggaccagcg agcgcgggca ctgcggcggg 60
   gaggatgctg cgcgagcgga ccgtgcggct gcagtacggg agccgcgtgg aggcggtgta 120
   cgtgctgggc acctacctct ggaccgatgt ctacagcgcg gccccagccg gggcccaaac 180
   cttcagcctg aagcactcgg aacacgtgtg ggtggaggtg gtgcgtgatg gggaggctga 240
   ggaggtggcc accaatggca agcagcgctg gcttctctcg cccagcacca ccctgcgggt 300
   caccatgagc caggcgagca ccgaggccag cagtgacaag gtcaccgtca actactatga 360
   cgaggaaggg agcattccca tcgaccaggc ggggctcttc ctcacagcca ttgagatctc 420
   cctggatgtg gacgcagacc gggatggtgt ggtggagaag aacaacccaa agaaggcatc 480
   ctggacctgg ggccccgagg gccagggggc catcctgctg gtgaactgtg accgagagac 540
   accctggttg cccaaggagg actgccgtga tgagaaggtc tacagcaagg aagatctcaa 600
   ggacatgtcc cagatgatcc tgcggaccaa aggccccgac cgcctccccg ccggatacga 660
   gatagttctg tacatttcca tgtcagactc agacaaagtg ggcgtgttct acgtggagaa 720
   cccgttcttc ggccaacgct atatccacat cctgggccgg cggaagctct accatgtggt 780
   caagtacacg ggtggctccg cggagctgct gttcttcgtg gaaggcctct gtttccccga 840
   cgagggcttc tcaggcctgg tctccatcca tgtcagcctg ctggagtaca tggcccagga 900
   cattcccctg actcccatct tcacggacac cgtgatattc cggattgctc cgtggatcat 960
   gacccccaac atcctgcctc ccgtgtcggt gtttgtgtgc tgcatgaagg ataattacct 1020
   gttcctgaaa gaggtgaaga accttgtgga gaaaaccaac tgtgagctga aggtctgctt 1080
   ccagtaccta aaccgaggcg atcgctggat ccaggatgaa attgagtttg gctacatcga 1140
   ggccccccat aaaggcttcc ccgtggtgct ggactctccc cgagatggaa acctaaagga 1200
   cttccctgtg aaggagctcc tgggcccaga ttttggctac gtgacccggg agcccctctt 1260
   tgagtctgtc accagccttg actcatttgg aaacctggag gtcagtcccc cagtgaccgt 1320
   gaacggcaag acatacccgc ttggccgcat cctcatcggg agcagctttc ctctgtctgg 1380
   tggtcggagg atgaccaagg tggtgcgtga cttcctgaag gcccagcagg tgcaggcacc 1440
   cgtggagctc tactcagact ggctgactgt gggccacgtg gatgagttca tgtcctttgt 1500
   ccccatcccc ggcacaaaga aattcctgct actcatggcc agcacctcgg cctgctacaa 1560
   gctcttccga gagaagcaga aggacggcca tggagaggcc atcatgttca aaggcttggg 1620
   tgggatgagc agcaagcgaa tcaccatcaa caagattctg tccaacgaga gccttgtgca 1680
   ggagaacctg tacttccagc gctgcctgga ctggaaccgt gacatcctca agaaggagct 1740
   gggactgaca gagcaggaca tcattgacct gcccgctctg ttcaagatgg acgaggacca 1800
   ccgtgccaga gccttcttcc caaacatggt gaacatgatc gtgctggaca aggacctggg 1860
   catccccaag ccattcgggc cacaggttga ggaggaatgc tgcctggaga tgcacgtgcg 1920
   tggcctcctg gagcccctgg gcctcgaatg caccttcatc gacgacattt ctgcctacca 1980
   caaatttctg ggggaagtcc actgtggcac caacgtccgc aggaagccct tcaccttcaa 2040
   gtggttgcac atggtgccct gacctgccag gggccctggc gtttgcctcc ttcgcttagt 2100
   tctccagacc ctccctcaca cgcccagagc cttctgctga catggactgg acagccccgc 2160
   tgggagacct ttgggacgtg gggtggaatt tggggtatct gtgccttgcc ctccctgaga 2220
   ggggcctcag tgtcctctga agccatcccc agtgagcctc gactctgtcc ctgctgaaaa 2280
   tagctgggcc agtgtctctg tagccctgac ataaggaaca gaacacaaca aaacacagca 2340
   aaccatgt 2348
<210> 24
   <211> 600
   <212> DNA
   <213> Artificial Sequence
<220>
   <223> Description of Artificial Sequence:primer
<220>
   <221> misc_feature
   <222> (1) .. (600)
   <223> 3' terminal sequence. signal transducer and activator of transcription 3 (acute-phase response factor) (STAT3) gene.
<400> 24
   tctcatttct ctatttttaa aagcgcccag attgctcaaa gatagcagaa gtaggagatt 60
   aaaaaaaatc tggaaccaca aagttagtag tttcagatga tctggggttt ggctgtgtga 120
   ggggtggcag aatgcaggta ggcgccttag tcgtatcttt ctgcagcttc cgttctcagc 180
   tcctcacatg ggggaggtag cgcactccga ggtcaactcc atgtcaaagg tgagggactc 240
   aaactgccct cctgctgagg gttcagcacc ttcaccatta tttccaaact gcatcaatga 300
   atctaaagtg cggggggaca tcggcaggtc aatggtattg ctgcaggtcg ttggtgtcac 360
   acagataaac ttggtcttca ggtatggggc agcgctacct gngtcagctt caggatgctc 420
   ctggctctct ggccgacaat actttccgaa tgcctcctcc ttgggaatgt caggatagag 480
   atagaccagt ggagacacca ggatattggt agcatccatg atcttatagc ccatgatgat 540
   ttcagcaaat gacatgttgt tcagctgctg ctttgtgtat ggntccacgg actggatctg 600
<210> 25
   <211> 3455
   <212> DNA/RNA
   <213> Artificial Sequence
<220>
   <223> Description of Artificial Sequence:primer
<220>
<221> misc_feature
   <222> (1) .. (3455)
   <223> signal transducer and activator of transcription 3 (acute-phase response factor) (STAT3) gene.
<400> 25
   ggtttccgga gctgcggcgg cgcagactgg gagggggagc cgggggttcc gacgtcgcag 60
   ccgagggaac aagccccaac cggatcctgg acaggcaccc cggcttggcg ctgtctctcc 120
   ccctcggctc ggagaggccc ttcggcctga gggagcctcg ccgcccgtcc ccggcacacg 180
   cgcagccccg gcctctcggc ctctgccgga gaaacagttg ggacccctga ttttagcagg 240
   atggcccaat ggaatcagct acagcagctt gacacacggt acctggagca gctccatcag 300
   ctctacagtg acagcttccc aatggagctg cggcagtttc tggccccttg gattgagagt 360
   caagattggg catatgcggc cagcaaagaa tcacatgcca ctttggtgtt tcataatctc 420
   ctgggagaga ttgaccagca gtatagccgc ttcctgcaag agtcgaatgt tctctatcag 480
   cacaatctac gaagaatcaa gcagtttctt cagagcaggt atcttgagaa gccaatggag 540
   attgcccgga ttgtggcccg gtgcctgtgg gaagaatcac gccttctaca gactgcagcc 600
   actgcggccc agcaaggggg ccaggccaac caccccacag cagccgtggt gacggagaag 660
   cagcagatgc tggagcagca ccttcaggat gtccggaaga gagtgcagga tctagaacag 720
   aaaatgaaag tggtagagaa tctccaggat gactttgatt tcaactataa aaccctcaag 780
   agtcaaggag acatgcaaga tctgaatgga aacaaccagt cagtgaccag gcagaagatg 840
   cagcagctgg aacagatgct cactgcgctg gaccagatgc ggagaagcat cgtgagtgag 900
   ctggcggggc ttttgtcagc gatggagtac gtgcagaaaa ctctcacgga cgaggagctg 960
   gctgactgga agaggcggca acagattgcc tgcattggag gcccgcccaa catctgccta 1020
   gatcggctag aaaactggat aacgtcatta gcagaatctc aacttcagac ccgtcaacaa 1080
   attaagaaac tggaggagtt gcagcaaaaa gtttcctaca aaggggaccc cattgtacag 1140
   caccggccga tgctggagga gagaatcgtg gagctgttta gaaacttaat gaaaagtgcc 1200
   tttgtggtgg agcggcagcc ctgcatgccc atgcatcctg accggcccct cgtcatcaag 1260
   accggcgtcc agttcactac taaagtcagg ttgctggtca aattccctga gttgaattat 1320
   cagcttaaaa ttaaagtgtg cattgacaaa gactctgggg acgttgcagc tctcagagga 1380
   tcccggaaat ttaacattct gggcacaaac acaaaagtga tgaacatgga agaatccaac 1440
   aacggcagcc tctctgcaga attcaaacac ttgaccctga gggagcagag atgtgggaat 1500
   gggggccgag ccaattgtga tgcttccctg attgtgactg aggagctgca cctgatcacc 1560
   tttgagaccg aggtgtatca ccaaggcctc aagattgacc tagagaccca ctccttgcca 1620
   gttgtggtga tctccaacat ctgtcagatg ccaaatgcct gggcgtccat cctgtggtac 1680
   aacatgctga ccaacaatcc caagaatgta aactttttta ccaagccccc aattggaacc 1740
   tgggatcaag tggccgaggt cctgagctgg cagttctcct ccaccaccaa gcgaggactg 1800
   agcatcgagc agctgactac actggcagag aaactcttgg gacctggtgt gaattattca 1860
   gggtgtcaga tcacatgggc taaattttgc aaagaaaaca tggctggcaa gggcttctcc 1920
   ttctgggtct ggctggacaa tatcattgac cttgtgaaaa agtacatcct ggccctttgg 1980
   aacgaagggt acatcatggg ctttatcagt aaggagcggg agcgggccat cttgagcact 2040
   aagcctccag gcaccttcct gctaagattc agtgaaagca gcaaagaagg aggcgtcact 2100
   ttcacttggg tggagaagga catcagcggt aagacccaga tccagtccgt ggaaccatac 2160
   acaaagcagc agctgaacaa catgtcattt gctgaaatca tcatgggcta taagatcatg 2220
   gatgctacca atatcctggt gtctccactg gtctatctct atcctgacat tcccaaggag 2280
   gaggcattcg gaaagtattg tcggccagag agccaggagc atcctgaagc tgacccaggt 2340
   agcgctgccc catacctgaa gaccaagttt atctgtgtga caccaacgac ctgcagcaat 2400
   accattgacc tgccgatgtc cccccgcact ttagattcat tgatgcagtt tggaaataat 2460
   ggtgaaggtg ctgaaccctc agcaggaggg cagtttgagt ccctcacctt tgacatggag 2520
   ttgacctcgg agtgcgctac ctcccccatg tgaggagctg agaacggaag ctgcagaaag 2580
   atacgactga ggcgcctacc tgcattctgc cacccctcac acagccaaac cccagatcat 2640
   ctgaaactac taactttgtg gttccagatt ttttttaatc tcctacttct gctatctttg 2700
   agcaatctgg gcacttttaa aaatagagaa atgagtgaat gtgggtgatc tgcttttatc 2760
   taaatgcaaa taaggatgtg ttctctgaga cccatgatca ggggatgtgg cggggggtgg 2820
   ctagagggag aaaaaggaaa tgtcttgtgt tgttttgttc ccctgccctc ctttctcagc 2880
   agctttttgt tattgttgtt gttgttctta gacaagtgcc tcctggtgcc tgcggcatcc 2940
   ttctgcctgt ttctgtaagc aaatgccaca ggccacctat agctacatac tcctggcatt 3000
   gcacttttta accttgctga catccaaata gaagatagga ctatctaagc cctaggtttc 3060
   tttttaaatt aagaaataat aacaattaaa gggcaaaaaa cactgtatca gcatagcctt 3120
   tctgtattta agaaacttaa gcagccgggc atggtggctc acgcctgtaa tcccagcact 3180
   ttgggaggcc gaggcggatc ataaggtcag gagatcaaga ccatcctggc taacacggtg 3240
   aaaccccgtc tctactaaaa gtacaaaaaa ttagctgggt gtggtggtgg gcgcctgtag 3300
   tcccagctac tcgggaggct gaggcaggag aatcgcttga acctgagagg cggaggttgc 3360
   agtgagccaa aattgcacca ctgcacactg cactccatcc tgggcgacag tctgagactc 3420
   tgtctcaaaa aaaaaaaaaa aaaaaaaaaa aaaaa                            3455
<210> 26
   <211> 658
   <212> DNA
   <213> Artificial Sequence
<220>
   <223> Description of Artificial Sequence:primer
<220>
   <221> misc_feature
   <222> (1) .. (658)
   <223> 5' terminal sequence. 2'-5'-oligoadenylate synthetase 2, 69/71kDa, transcript variant 2 (OAS2) gene.

<400> 26
   aaaagaagca aaggaatggt tatcctctcc ctgcttcaag gatgggactg gaaacccaat 60
   accaccttgg aaagtgccgg taaaagtcat ctaaaggagg cgttgtctgg aaatagccct 120
   gtaacaggct tgaatcaaag aacttctcct actgtagcaa cctgaaatta actcagacac 180
   aaataaagga aacccagctc acaggagctt aaacagctgg tcagccccct aagcccccac 240
   tacaagtgat cctcaggcag gtaaccccag attcatgcac tgtagggtgc tgcgcagcat 300
   ccctagtctc tacccagtag atgccactag ccctcctctc ccagtgacaa ccaaaagtct 360
   tcagacattg tcaaacgttc ccctgggttc acagatcttt ctgcctttgg cttttggctc 420
   caccctcttt agctgttaat ttgagtactt atggccctga aagcggccac ggtgcctcca 480
   gatggcaggt ttgcaatcca agcaggaaga aggaaaagat acccaaaggt caagaacaca 540
   gtgattttat tagaagtttc atccgcaaat tttcttccat ttcattgctc agaaatgtca 600
   tgtggttacc tgtaacttga aggtggctac aaagatgact gtggacgtgg gttgcact   658
<210> 27
   <211> 3068
   <212> DNA/RNA
   <213> Artificial Sequence
<220>
   <223> Description of Artificial Sequence:primer
<220>
   <221> misc_feature
   <222> (1) .. (3068)
   <223> 2',5'-oligoadenylate synthetase 2, 69/71kDa (OAS2) gene.
<400> 27
   cggcagccag ctgagagcaa tgggaaatgg ggagtcccag ctgtcctcgg tgcctgctca 60
   gaagctgggt tggtttatcc aggaatacct gaagccctac gaagaatgtc agacactgat 120
   cgacgagatg gtgaacacca tctgtgacgt ctgcaggaac cccgaacagt tccccctggt 180
   gcagggagtg gccataggtg gctcctatgg acggaaaaca gtcttaagag gcaactccga 240
   tggtaccctt gtccttttct tcagtgactt aaaacaattc caggatcaga agagaagcca 300
   acgtgacatc ctcgataaaa ctggggataa gctgaagttc tgtctgttca cgaagtggtt 360
   gaaaaacaat ttcgagatcc agaagtccct tgatgggtcc accatccagg tgttcacaaa 420
   aaatcagaga atctctttcg aggtgctggc cgccttcaac gctctgagct taaatgataa 480
   tcccagcccc tggatctatc gagagctcaa aagatccttg gataagacaa atgccagtcc 540
   tggtgagttt gcagtctgct tcactgaact ccagcagaag ttttttgaca accgtcctgg 600
   aaaactaaag gatttgatcc tcttgataaa gcactggcat caacagtgcc agaaaaaaat 660
   caaggattta ccctcgctgt ctccgtatgc cctggagctg cttacggtgt atgcctggga 720
   acaggggtgc agaaaagaca actttgacat tgctgaaggc gtcagaacgg ttctggagct 780
   gatcaaatgc caggagaagc tgtgtatcta ttggatggtc aactacaact ttgaagatga 840
   gaccatcagg aacatcctgc tgcaccagct ccaatcagcg aggccagtaa tcttggatcc 900
   agttgaccca accaataatg tgagtggaga taaaatatgc tggcaatggc tgaaaaaaga 960
   agctcaaacc tggttgactt ctcccaacct ggataatgag ttacctgcac catcttggaa 1020
   tgtcctgcct gcaccactct tcacgacccc aggccacctt ctggataagt tcatcaagga 1080
   gtttctccag cccaacaaat gcttcctaga gcagattgac agtgctgtta acatcatccg 1140
   tacattcctt aaagaaaact gcttccgaca atcaacagcc aagatccaga ttgtccgggg 1200
   aggatcaacc gccaaaggca cagctctgaa gactggctct gatgccgatc tcgtcgtgtt 1260
   ccataactca cttaaaagct acacctccca aaaaaacgag cggcacaaaa tcgtcaagga 1320
   aatccatgaa cagctgaaag ccttttggag ggagaaggag gaggagcttg aagtcagctt 1380
   tgagcctccc aagtggaagg ctcccagggt gctgagcttc tctctgaaat ccaaagtcct 1440
   caacgaaagt gtcagctttg atgtgcttcc tgcctttaat gcactgggtc agctgagttc 1500
   tggctccaca cccagccccg aggtttatgc agggctcatt gatctgtata aatcctcgga 1560
   cctcccggga ggagagtttt ctacctgttt cacagtcctg cagcgaaact tcattcgctc 1620
   ccggcccacc aaactaaagg atttaattcg cctggtgaag cactggtaca aagagtgtga 1680
   aaggaaactg aagccaaagg ggtctttgcc cccaaagtat gccttggagc tgctcaccat 1740
   ctatgcctgg gagcagggga gtggagtgcc ggattttgac actgcagaag gtttccggac 1800
   agtcctggag ctggtcacac aatatcagca gctcggcatc ttctggaagg tcaattacaa 1860
   ctttgaagat gagaccgtga ggaagtttct actgagccag ttgcagaaaa ccaggcctgt 1920
   gatcttggac ccaggcgaac ccacaggtga cgtgggtgga ggggaccgtt ggtgttggca 1980
   tcttctggac aaagaagcaa aggttaggtt atcctctccc tgcttcaagg atgggactgg 2040
   aaacccaata ccaccttgga aagtgccggt aaaagtcatc taaaggaggc gttgtctgga 2100
   aatagccctg taacaggctt gaatcaaaga acttctccta ctgtagcaac ctgaaattaa 2160
   ctcagacaca aataaaggaa acccagctca caggagctta aacagctggt cagcccccct 2220
   aagcccccac tacaagtgat cctcaggcag gtaaccccag attcatgcac tgtagggctg 2280
   ggcgcagcat ccctaggtct ctacccagta gatgccacta gccctcctct cccagtgaca 2340
   accaaaagtc ttcacatgtt caaacgttcc cctgggttca cagatctttc tgcctttggc 2400
   ttttggctcc accctcttta gctgttaatt tgagtactta tggccctgaa agcggccacg 2460
   gtgcctccag atggcaggtt tgcaatccaa gcaggaagaa ggaaaagata cccaaaggtc 2520
   aagaacacag tgattttatt agaagtttca tccgcaaatt ttcttccatt tcattgctca 2580
   gaatgtcatg tggttacctg taacttgaag gtggctacaa agatgactgt ggaggtggtt 2640
   gcacttgcca cccaaggatg tctgccacac ctctccaagc cctcctacct accaagatat 2700
   acctgatata tccaccagat atctcctcag atatacttgg ttctctccac caggttcttt 2760
   ctttaaagca ggattctcaa ctttgatact tactcacatt gggctagaca gttctttgtt 2820
   tggaggctct cttgtgcatg taggatgttg agcagcatgt gtggcctgta cccagtacat 2880
   gccacccagt tgtgacaatt aaaagtgtct tgagacttta tcatgtgtct tctgccctag 2940
   gtgagaaccc ttgcactaca ggaaccctac acccaacctg gggggaatgt agggaagagg 3000
   tgccaagcca accgtggggt tagctctaat tattaagtta tgcattataa ataaatacca 3060
   aaaaattg                                                          3068
<210> 28
   <211> 286
   <212> DNA
   <213> Artificial Sequence
<220>
   <223> Description of Artificial Sequence:primer
<220>
   <221> misc_feature
   <222> (1) .. (286)
   <223> 3' terminal sequence. growth factor receptor-bound protein 7 (GRB7) gene.
<400> 28
   aaaaaataat ctttattgnc actagtataa aacagagcag nncaactggc ctntcggnct 60
   gtacaaagtg tggggcgtga aaccgctggg ctgcccccac ttctcccana attccctgcc 120
   ctagagcagc acctccagag ctaggagaag gagagggggc cacccaaggn cttcccttga 180
   ggagaggggt caggagtgga ctggagtggg ggctnttttc tatctgaggg aggcaaagaa 240
   gcagaggaga aaactggagt gggcggaacc ctcccgccct cgtgcc                286
<210> 29
   <211> 253
   <212> DNA
   <213> Artificial Sequence
<220>
   <223> Description of Artificial Sequence:primer
<220>
   <221> misc_feature
   <222> (1) .. (253)
   <223> 5' terminal sequence. growth factor receptor-bound protein 7 (GRB7) gene.
<400> 29
   tgcctccctc agatagaaaa cagcccccac tccagtccac tcctgacccc tctcctcaag 60
   ggaaggcctt gggtggcccc ctctccttct cctagctctg gaggtgctgc tctagggcag 120
   ggaattatgg gagaagtngg ggcagcccag gcgntttcac gcccacactt tgtacagacc 180
   gagaggccag ttgatctgct ctgttttata ctagtgacaa taaagattat tttttgatac 240
   aaaaaaaaaa aaa                                                    253
<210> 30
   <211> 2205
   <212> DNA/RNA
   <213> Artificial Sequence
<220>
   <223> Description of Artificial Sequence:primer
<220>
   <221> misc_feature
   <222> (11)..(2205)
   <223> growth factor receptor-bound protein 7 (GRB7) gene.
<400> 30
   cacagggctc ccccccgcct ctgacttctc tgtccgaagt cgggacaccc tcctaccacc 60
   tgtagagaag cgggagtgga tctgaaataa aatccaggaa tctgggggtt cctagacgga 120
   gccagacttc ggaacgggtg tcctgctact cctgctgggg ctcctccagg acaagggcac 180
   acaactggtt ccgttaagcc cctctctcgc tcagacgcca tggagctgga tctgtctcca 240
   cctcatctta gcagctctcc ggaagacctt tggccagccc ctgggacccc tcctgggact 300
   ccccggcccc ctgatacccc tctgcctgag gaggtaaaga ggtcccagcc tctcctcatc 360
   ccaaccaccg gcaggaaact tcgagaggag gagaggcgtg ccacctccct cccctctatc 420
   cccaacccct tccctgagct ctgcagtcct ccctcacaga gcccaattct cgggggcccc 480
   tccagtgcaa gggggctgct cccccgcgat gccagccgcc cccatgtagt aaaggtgtac 540
   agtgaggatg gggcctgcag gtctgtggag gtggcagcag gtgccacagc tcgccacgtg 600
   tgtgaaatgc tggtgcagcg agctcacgcc ttgagcgacg agacctgggg gctggtggag 660
   tgccaccccc acctagcact ggagcggggt ttggaggacc acgagtccgt ggtggaagtg 720
   caggctgcct ggcccgtggg cggagatagc cgcttcgtct tccggaaaaa cttcgccaag 780
   tacgaactgt tcaagagctc cccacactcc ctgttcccag aaaaaatggt ctccagctgt 840
   ctcgatgcac acactggtat atcccatgaa gacctcatcc agaacttcct gaatgctggc 900
   agctttcctg agatccaggg ctttctgcag ctgcggggtt caggacggaa gctttggaaa 960
   cgctttttct gtttcttgcg ccgatctggc ctctattact ccaccaaggg cacctctaag 1020
   gatccgaggc acctgcagta cgtggcagat gtgaacgagt ccaacgtgta cgtggtgacg 1080
   cagggccgca agctctacgg gatgcccact gacttcggtt tctgtgtcaa gcccaacaag 1140
   cttcgaaatg gacacaaggg gcttcggatc ttctgcagtg aagatgagca gagccgcacc 1200
   tgctggctgg ctgccttccg cctcttcaag tacggggtgc agctgtacaa gaattaccag 1260
   caggcacagt ctcgccatct gcatccatct tgtttgggct ccccaccctt gagaagtgcc 1320
   tcagataata ccctggtggc catggacttc tctggccatg ctgggcgtgt cattgagaac 1380
   ccccgggagg ctctgagtgt ggccctggag gaggcccagg cctggaggaa gaagacaaac 1440
   caccgcctca gcctgcccat gccagcctcc ggcacgagcc tcagtgcagc catccaccgc 1500
   acccaactct ggttccacgg gcgcatttcc cgtgaggaga gccagcggct tattggacag 1560
   cagggcttgg tagacggcct gttcctggtc cgggagagtc agcggaaccc ccagggcttt 1620
   gtcctctctt tgtgccacct gcagaaagtg aagcattatc tcatcctgcc gagcgaggag 1680
   gagggtcgcc tgtacttcag catggatgat ggccagaccc gcttcactga cctgctgcag 1740
   ctcgtggagt tccaccagct gaaccgcggc atcctgccgt gcttgctgcg ccattgctgc 1800
   acgcgggtgg ccctctgacc aggccgtgga ctggctcatg cctcagcccg ccttcaggct 1860
   gcccgccgcc cctccaccca tccagtggac tctggggcgc ggccacaggg gacgggatga 1920
   ggagcgggag ggttccgcca ctccagtttt ctcctctgct tctttgcctc cctcagatag 1980
   aaaacagccc ccactccagt ccactcctga cccctctcct caagggaagg ccttgggtgg 2040
   ccccctctcc ttctcctagc tctggaggtg ctgctctagg gcagggaatt atgggagaag 2100
   tgggggcagc ccaggcggtt tcacgcccca cactttgtac agaccgagag gccagttgat 2160
   ctgctctgtt ttatactagt gacaataaag attatttttt gatac                 2205
<210> 31
   <211> 380
   <212> DNA
   <213> Artificial Sequence
<220>
   <223> Description of Artificial Sequence:primer
<220>
   <221> misc_feature
   <222> (1) .. (380)
   <223> 3' terminal sequence. sapiens cdna flj33383 fis, clone brace2006514. (EST N33243) gene.
<400> 31
   tttttttttt tggagcttgg taatgagcat ttttattaaa ttccccaagt acaagccccg 60
   gtgtacacaa taagcaattt cagttttgag taactgccag ggaaggaatg tccccctttc 120
   tgtttgttga ctgccttctg atttgtttga aagctttttt cttcttgcat tatcttgacc 180
   attcctggta tgaaggatgt tgtcttgttt ctcctcagtt tcatcttctg cagaagaaga 240
   aaccatgcca ggcggagtct cttactgttc atggcttcca tggctttacn ctgttccctt 300
   tttctctcac aagtcatcag ctcagatcta aaaatactct aagggtaaat ccactggnga 360
   ataaaatggt tccaatgtgc                                             380
<210> 32
   <211> 300
   <212> DNA
   <213> Artificial Sequence
<220>
   <223> Description of Artificial Sequence:primer
<220>
   <221> misc_feature
   <222> (1) .. (300)
   <223> 5' terminal sequence. sapiens cdna flj33383 fis, clone brace2006514. (EST N33243) gene.
<400> 32
   aatactcagc aaatgtgaaa ctttatttgc tcttacttca aaattagtcc aaaatgttgg 60
   aaataaaata taagacattg atctagatat gaggtttttc tccttcattc tcagctgtcg 120
   aagaaatcaa agtagcatat gcacaaggtt aaaaaccaca tatacaaata ctatagaaca 180
   gcttataatg aaaaccttgc ctgcctttat aaaaaatgtg attatcttct tctgttaatg 240
   tcaataaaag atggtttgtc ctagaaggtc ttataagtgg taaatggtan tatgntctgg 300
<210> 33
   <211> 466
   <212> DNA
   <213> Artificial Sequence
<220>
   <223> Description of Artificial Sequence:primer
<220>
   <221> misc_feature
   <222> (1) .. (466)
   <223> 3' terminal sequence. protein phosphatase 1, regulatory (inhibitor) subunit 1b (dopamine and camp regulated phosphoprotein, darpp-32) (PPP1R1B) gene.
<400> 33
   atttacaaag atttattaca gcacgggagg ggttcaggcc tggagttagg gaagaagggg 60
   aaaggggcag agcagctggg ggacaaggaa aacctggcgc cccccgctgt gtgccccacc 120
   gggacaataa actaggcggc attcctggca tcaaagcaca aaacgcaaca aagaggtctc 180
   tgccagtcca tcttccaggc acccaggagg agcaagggtg attaagggaa gattcccaaa 240
   atgttgaggc tatggagaaa aacgccttag tcctggaccc tggtagaagc cggtgagaga 300
   agtggtgact tggaatcctc cataggaaag tgggtagaaa aggatctaag ggtacctcaa 360
   ggttctcagg acctcctttc cccagatctt agggtcctgc cctgtgggtc tcctgtgtcc 420
   aggggagagg atctggggag tagaattgtg aagggcaatc ccgttc                466
<210> 34
   <211> 352
   <212> DNA
   <213> Artificial Sequence
<220>
   <223> Description of Artificial Sequence:primer
<220>
   <221> misc_feature
   <222> (1) .. (352)
   <223> 5' terminal sequence. protein phosphatase 1, regulatory (inhibitor) subunit 1b (dopamine and camp regulated phosphoprotein, darpp-32) (PPP1R1B) gene.
<400> 34
   ccacaggcna ggaccctaag atctggggaa aggaggtcct gagaaccttg aggtaccctt 60
   agatcctttt ctacncactt tcctatggag gattccaagt caccacttct ctcaccggct 120
   tctaccaggg tccaggacta aggcgttttt ctccatagcc tcaacatttt gggaatcttc 180
   ccttaatcac ccttgctcct cctgggtgcc tggaagatgg actggcagag acctctttgt 240
   tgcgttttgt gctttggatg ccaggaatgc cgcctagttt atgtccccgg tgggcacaca 300
   gcgggggggc gccaaggttt tccttggtcc cccaagctgg ctctgcncct tt         352
<210> 35
   <211> 1841
   <212> DNA/RNA
   <213> Artificial Sequence
<220>
   <223> Description of Artificial Sequence:primer
<220>
   <221> misc_feature
   <222> (1) .. (1841)
   <223> protein phosphatase 1, regulatory (inhibitor) subunit 1b (dopamine and camp regulated phosphoprotein, darpp-32) (PPP1R1B) gene.
<400> 35
   agctgggacc ggagggtgag cccggcagag gcagagacac acgcggagag gaggagaggc 60
   tgagggaggg aggtggagaa ggacgggaga ggcagagaga ggagacacgc agagacactc 120
   aggaggggag agacaccgag acgcagagac actcaggagg ggagagacac cgagacgcag 180
   agacacccag gccggggagc gcgagggagc gaggcacaga cctggctcag cgagcgcggg 240
   gggcgagccc cgagtcccga gagcctgggg gcgcgcccag cccgggcgcc gaccctcctc 300
   ccgctcccgc gccctcccct cggcgggcac ggtattttta tccgtgcgcg aacagccctc 360
   ctcctcctct cgccgcacag cccgccgcct gcgcggggga gcccagcaca gaccgccgcc 420
   gggaccccga gtcgcgcacc ccagccccac cgcccacccc gcgcgccatg gaccccaagg 480
   accgcaagaa gatccagttc tcggtgcccg cgccccctag ccagctcgac ccccgccagg 540
   tggagatgat ccggcgcagg agaccaacgc ctgccatgct gttccggctc tcagagcact 600
   cctcaccaga ggaggaagcc tccccccacc agagagcctc aggagagggg caccatctca 660
   agtcgaagag acccaacccc tgtgcctaca caccaccttc gctgaaagct gtgcagcgca 720
   ttgctgagtc tcacctgcag tctatcagca atttgaatga gaaccaggcc tcagaggagg 780
   aggatgagct gggggagctt cgggagctgg gttatccaag agaggaagat gaggaggaag 840
   aggaggatga tgaagaagag gaagaagaag aggacagcca ggctgaagtc ctgaaggtca 900
   tcaggcagtc tgctgggcaa aagacaacct gtggccaggg tctggaaggg ccctgggagc 960
   gcccaccccc tctggatgag tccgagagag atggaggctc tgaggaccaa gtggaagacc 1020
   cagcactaag tgagcctggg gaggaacctc agcgcccttc cccctctgag cctggcacat 1080
   aggcacccag cctgcatctc ccaggaggaa gtggagggga catcgctgtt ccccagaaac 1140
   ccactctatc ctcaccctgt tttgtgctct tcccctcgcc tgctagggct gcggcttctg 1200
   acttctagaa gactaaggct ggtctgtgtt tgcttgtttg cccacctttg gctgataccc 1260
   agagaacctg ggcacttgct gcctgatgcc cacccctgcc agtcattcct ccattcaccc 1320
   agcgggaggt gggatgtgag acagcccaca ttggaaaatc cagaaaaccg ggaacaggga 1380
   tttgcccttc acaattctac tccccagatc ctctcccctg gacacaggag acccacaggg 1440
   caggacccta agatctgggg aaaggaggtc ctgagaacct tgaggtaccc ttagatcctt 1500
   ttctacccac tttcctatgg aggattccaa gtcaccactt ctctcaccgg cttctaccag 1560
   ggtccaggac taaggcgttt ttctccatag cctcaacatt ttgggaatct tcccttaatc 1620
   acccttgctc ctcctgggtg cctggaagat ggactggcag agacctcttt gttgcgtttt 1680
   gtgctttgat gccaggaatg ccgcctagtt tatgtccccg gtggggcaca cagcgggggg 1740
   cgccaggttt tccttgtccc ccagctgctc tgcccctttc cccttcttcc ctgactccag 1800
   gcctgaaccc ctcccgtgct gtaataaatc tttgtaaata a                     1841
<210> 36
   <211> 430
   <212> DNA
   <213> Artificial Sequence
<220>
   <223> Description of Artificial Sequence:primer
<220>
   <221> misc_feature
   <222> (1) .. (430)
   <223> 3' terminal sequence. cyclin-dependent kinase-like 5 (CDKL5) gene.
<400> 36
   cattgacctg aaaaatcctt gcttctcttt ttccttgagt tgctctgaat gactaatatt 60
   ttcaggactt ttccatggat cgaatgctgg ttgtcttttt gagtggttgg ttccagaact 120
   gctctctgat ggtagagaag aaactctagt tgacacatta ttttcatgga aagaattgtc 180
   tggacgtgga gatggcactc tgtaaaagct accaactctc cttggcacag gatcattgta 240
   taggtgtcta ttttctttgg gggctgtgca atcatcagag tgtgggtcat gatacactcc 300
   accctcagac ttctgggcgt ggtatgggaa ggaagaggng ccttccctgg gagggtcncc 360
   ttttgaccga angatctttg ccacagtcat cccctnggaa ggggagctgt ttctccaggc 420
   tgggggtgac                                                        430
<210> 37
   <211> 520
   <212> DNA
   <213> Artificial Sequence
<220>
   <223> Description of Artificial Sequence:primer
<220>
   <221> misc_feature
   <222> (1) .. (520)
   <223> 5' terminal sequence. cyclin-dependent kinase-like 5 (CDKL5) gene.
<400> 37
   gacacagtga cacgagaact ttgctcagcc cttctggaag aaataaccga aatgagggaa 60
   cgctggactc acgtcgaacc acaaccagac attctaagac gatggaggaa ttgaagctgc 120
   cggagcacat ggacagtagc cattcccatt cactgtctgc acctcacgaa tctttttctt 180
   atggactggg ctacaccagc ccttttcttt cccagcaacg tcctcatagg cattctatgt 240
   atgtgacccg tgacaaagtg agagccaagg nttggatgga agcttgagca tagggcaagg 300
   gatggcagct agagccaaca gcctgcaact cttgtcaccc cagcctggag aacagctccc 360
   tccagagatg actgtggcaa gatcttcggt caaagagacc tccagagaag gcacctcttc 420
   cttccataca cgccagaagt ctgagggtgg agtgtatcat gacccacact ctgatgatgg 480
   cacagccccc aaagaaaata gacacctata caatgattct                       520
<210> 38
   <211> 3399
   <212> DNA/RNA
   <213> Artificial Sequence
<220>
   <223> Description of Artificial Sequence:primer
<220>
   <221> misc_feature
   <222> (1) .. (3399)
   <223> cyclin-dependent kinase-like 5 (CDKL5) gene.
<400> 38
   cggcgacggc gtcctcagga gctgtggggt cccctgctag aagtggggga ctcggcgggg 60
   gagtcattta atacttcatg attagaacaa atatgtgaaa gttcccacca accagtgaga 120
   atttcttcct tcagacggtt ttggatctta ctgcacagct ttctgagaag ttcttttggt 180
   gccatgtttt gtggcttgca tcaaaagagg agtttgtctt catgaagatt cctaacattg 240
   gtaatgtgat gaataaattt gagatccttg gggttgtagg tgaaggagcc tatggagttg 300
   tacttaaatg cagacacaag gaaacacatg aaattgtggc gatcaagaaa ttcaaggaca 360
   gtgaagaaaa tgaagaagtc aaagaaacga ctttacgaga gcttaaaatg cttcggactc 420
   tcaagcagga aaacattgtg gagttgaagg aagcatttcg tcggagggga aagttgtact 480
   tggtgtttga gtatgttgaa aaaaatatgc tcgaattgct ggaagaaatg ccaaatggag 540
   ttccacctga gaaagtaaaa agctacatct atcagctaat caaggctatt cactggtgcc 600
   ataagaatga tattgtccat cgagatataa aaccagaaaa tctcttaatc agccacaatg 660
   atgtcctaaa actgtgtgac tttggttttg ctcgtaatct gtcagaaggc aataatgcta 720
   attacacaga gtacgttgcc accagatggt atcggtcccc agaactctta cttggcgctc 780
   cctatggaaa gtccgtggac atgtggtcgg tgggctgtat tcttggggag cttagcgatg 840
   gacagccttt atttcctgga gaaagtgaaa ttgaccaact ttttactatt cagaaggtgc 900
   taggaccact tccatctgag cagatgaagc ttttctacag taatcctcgc ttccatgggc 960
   tccggtttcc agctgttaac catcctcagt ccttggaaag aagatacctt ggaattttga 1020
   atagtgttct acttgaccta atgaagaatt tactgaagtt ggacccagct gacagatact 1080
   tgacagaaca gtgtttgaat caccctacat ttcaaaccca gagacttctg gatcgttctc 1140
   cttcaaggtc agcaaaaaga aaaccttacc atgtggaaag cagcacattg tctaatagaa 1200
   accaagccgg caaaagtact gctttgcagt ctcaccacag atctaacagc aaggacatcc 1260
   agaacctgag tgtaggcctg ccccgggctg acgaaggtct ccctgccaat gaaagcttcc 1320
   taaatggaaa ccttgctgga gctagtctta gtccactgca caccaaaacc taccaagcaa 1380
   gcagccagcc tgggtctacc agcaaagatc tcaccaacaa caacatacca caccttctta 1440
   gcccaaaaga agccaagtca aaaacagagt ttgattttaa tattgaccca aagccttcag 1500
   aaggcccagg gacaaagtac ctcaagtcaa acagcagatc tcagcagaac cgccactcat 1560
   tcatggaaag ctctcaaagc aaagctggga cactgcagcc caatgaaaag cagagtcggc 1620
   atagctatat tgacacaatt ccccagtcct ctaggagtcc ctcctacagg accaaggcca 1680
   aaagccatgg ggcactgagt gactccaagt ctgtgagcaa cctttctgaa gccagggccc 1740
   aaattgcgga gcccagtacc agtaggtact tcccatctag ctgcttagac ttgaattctc 1800
   ccaccagccc aacccccacc agacacagtg acacgagaac tttgctcagc ccttctggaa 1860
   gaaataaccg aaatgaggga acgctggact cacgtcgaac cacaaccaga cattctaaga 1920
   cgatggagga attgaagctg ccggagcaca tggacagtag ccattcccat tcactgtctg 1980
   cacctcacga atctttttct tatggactgg gctacaccag ccccttttct tcccagcaac 2040
   gtcctcatag gcattctatg tatgtgaccc gtgacaaagt gagagccaag ggcttggatg 2100
   gaagcttgag catagggcaa gggatggcag ctagagccaa cagcctgcaa ctcttgtcac 2160
   cccagcctgg agaacagctc cctccagaga tgactgtggc aagatcttcg gtcaaagaga 2220
   cctccagaga aggcacctct tccttccata cacgccagaa gtctgagggt ggagtgtatc 2280
   atgacccaca ctctgatgat ggcacagccc ccaaagaaaa tagacaccta tacaatgatc 2340
   ctgtgccaag gagagttggt agcttttaca gagtgccatc tccacgtcca gacaattctt 2400
   tccatgaaaa taatgtgtca actagagttt cttctctacc atcagagagc agttctggaa 2460
   ccaaccactc aaaaagacaa ccagcattcg atccatggaa aagtcctgaa aatattagtc 2520
   attcagagca actcaaggaa aaagagaagc aaggattttt caggtcaatg aaaaagaaaa 2580
   agaagaaatc tcaaacagta cccaattccg acagccctga tcttctgacg ttgcagaaat 2640
   ccattcattc tgctagcact ccaagcagca gaccaaagga gtggcgcccc gagaagatct 2700
   cagatctgca gacccaaagc cagccattaa aatcactgcg caagttgtta catctctctt 2760
   cggcctcaaa tcacccggct tcctcagatc cccgcttcca gcccttaaca gctcaacaaa 2820
   ccaaaaattc cttctcagaa attcggattc accccctgag ccaggcctct ggcgggagca 2880
   gcaacatccg gcaggaaccc gcaccgaagg gcaggccagc cctccagctg ccagacggtg 2940
   gatgtgatgg cagaagacag agacaccatt ctggacccca agatagacgc ttcatgttaa 3000
   ggacgacaga acaacaagga gaatacttct gctgtggtga cccaaagaag cctcacactc 3060
   cgtgcgtccc aaaccgagcc cttcatcgtc caatctccag tcctgctccc tatccagtac 3120
   tccaggtccg aggcacttcc atgtgcccga cactccaggt ccgaggcact gatgctttca 3180
   gctgcccaac ccagcaatcc gggttctctt tcttcgtgag acacgttatg agggaagccc 3240
   tgattcacag ggcccaggta aaccaagctg cgctcctgac ataccatgag aatgcggcac 3300
   tgacgggcaa gtgacttctg caagcctgcg gctggtccca atgccctgaa tcacctctct 3360
   catggaagaa ccaattaaca ccaatgaatc aaccaaaac                       3399
<210> 39
   <211> 396
   <212> DNA
   <213> Artificial Sequence
<220>
   <223> Description of Artificial Sequence:primer
<220>
   <221> misc_feature
   <222> (1) .. (396) <223> 5' terminal sequence. ribosomal protein 119 (RPL19) gene.
<400> 39
   natttcgccc cctctctccc tcctcccctc gccctcggtg ctcagaagat accgtgaatc 60
   taagaagatc gatcgccaca tgtatcacag cctgtacctg aaggtgaagg ggaatgtgtt 120
   caaaaacaag cggattctca tggaacacat ccacaagctg aaggcagaca aggcccgcaa 180
   gaagctcctg gctgaccagg ctgaggccgc aggtctaaga ccaaggaagc acgcaagcgc 240
   cgtgaagagc nctccaggca agaaggagga gatcatcaag actttatcca aggaggaaga 300
   gaccaagaaa taaaacctcc cactttgtct gtacatactg gcctctgtga ttacatagat 360
   cagccattaa aataaaacaa gccttaatct gcaaaa                          396
<210> 40
   <211> 698
   <212> DNA/RNA
   <213> Artificial Sequence
<220>
   <223> Description of Artificial Sequence:primer
<220>
   <221> misc_feature
   <222> (1)..(698)
   <223> ribosomal protein 119 (RPL19) gene.
<400> 40
   ctttcctttc gctgctgcgg ccgcagccat gagtatgctc aggcttcaga agaggctcgc 60
   ctctagtgtc ctccgctgtg gcaagaagaa ggtctggtta gaccccaatg agaccaatga 120
   aatcgccaat gccaactccc gtcagcagat ccggaagctc atcaaagatg ggctgatcat 180
   ccgcaagcct gtgacggtcc attcccgggc tcgatgccgg aaaaacacct tggcccgccg 240
   gaagggcagg cacatgggca taggtaagcg gaagggtaca gccaatgccc gaatgccaga 300
   gaaggtcaca tggatgagga gaatgaggat tttgcgccgg ctgctcagaa gataccgtga 360
   atctaagaag atcgatcgcc acatgtatca cagcctgtac ctgaaggtga aggggaatgt 420
   gttcaaaaac aagcggattc tcatggaaca catccacaag ctgaaggcag acaaggcccg 480
   caagaagctc ctggctgacc aggctgaggc ccgcaggtct aagaccaagg aagcacgcaa 540
   gcgccgtgaa gagcgcctcc aggccaagaa ggaggagatc atcaagactt tatccaagga 600
   ggaagagacc aagaaataaa acctcccact ttgtctgtac atactggcct ctgtgattac 660
   atagatcagc cattaaaata aaacaagcct taatctgc                         698
<210> 41
   <211> 204
   <212> DNA
   <213> Artificial Sequence
<220>
   <223> Description of Artificial Sequence:primer
<220>
   <221> misc_feature
   <222> (1) .. (204)
   <223> 3' terminal sequence. cadherin 15, m-cadherin (myotubule) (CDH15) gene.
<400> 41
   tttttttttt tttttttttt tttttttttt ttttttttca ttcagattta cccaggaggt 60
   tgctgtcttt canacaaaga tgaggttcac tggnaggagg caaaggtggg actagggagg 120
   tgacccgcat gggccagatn ggagagaaac tcttcccacc ccggcagaag gggcctcttc 180
   ctggccgccc catccanact cagg                                        204
<210> 42
   <211> 457
   <212> DNA
   <213> Artificial Sequence
<220>
   <223> Description of Artificial Sequence:primer
<220>
   <221> misc_feature
   <222> (1) .. (457)
   <223> 5' terminal sequence. cadherin 15, m-cadherin (myotubule) (CDH15) gene.
<400> 42
   caggacgcct acgacatcag ccagctgcgt cacccgacag cgctgagcct gcctctggga 60
   ccgccgccac ttcgcagaga tgccccgcag ncagcctgca cccccagcca ccccgagtgc 120
   tgcccaccag ccccctggac atcgccgact tcatcaatga tggcttggag gctgcagata 180
   gtgaccccag tgtgccgcct tacgacacag ccctcatcta tgactacgag ggtgacggct 240
   cggtggcggg gacntgagct ccatcctgtc cagccagggc gatgaggacc aggactacga 300
   ctacctcaga gactgggggc cccgcttcgc ccggctggca gacatgtatg ggcacccgtg 360
   cgggttngga gttacggggc cagatgggac caccaggcca gggagggtct ttctcctggg 420
   gcactgctac ccagacacag aggccggaca gcctgan                          457
<210> 43
   <211> 2875
   <212> DNA/RNA
   <213> Artificial Sequence
<220>
   <223> Description of Artificial Sequence:primer
<220>
   <221> misc_feature
   <222> (1) .. (2875) <223> cadherin 15, m-cadherin (myotubule) (CDH15) gene.
<400> 43
   acttgcgctg tcactcagcc tggacgcgct tcttcgggtc gcgggtgcac tccggcccgg 60
   ctcccgcctc ggccccgatg gacgccgcgt tcctcctcgt cctcgggctg ttggcccaga 120
   gcctctgcct gtctttgggg gttcctggat ggaggaggcc caccaccctg tacccctggc 180
   gccgggcgcc tgccctgagc cgcgtgcgga gggcctgggt catccccccg atcagcgtat 240
   ccgagaacca caagcgtctc ccctaccccc tggttcagat caagtcggac aagcagcagc 300
   tgggcagcgt catctacagc atccagggac ccggcgtgga tgaggagccc cggggcgtct 360
   tctctatcga caagttcaca gggaaggtct tcctcaatgc catgctggac cgcgagaaga 420
   ctgatcgctt caggctaaga gcgtttgccc tggacctggg aggatccacc ctggaggacc 480
   ccacggacct ggagattgta gttgtggatc agaatgacaa ccggccagcc ttcctgcagg 540
   aggcgttcac tggccgcgtg ctggagggtg cagtcccagg cacctatgtg accagggcag 600
   aggccacaga tgccgacgac cccgagacgg acaacgcagc gctgcggttc tccatcctgc 660
   agcagggcag ccccgagctc ttcagcatcg acgagctcac aggagagatc cgcacagtgc 720
   aagtggggct ggaccgcgag gtggtcgcgg tgtacaatct gaccctgcag gtggcggaca 780
   tgtctggaga cggcctcaca gccactgcct cagccatcat cacccttgat gacatcaatg 840
   acaatgcccc cgagttcacc agggatgagt tcttcatgga ggccatagag gccgtcagcg 900
   gagtggatgt gggacgcctg gaagtggagg acagggacct gccaggctcc ccaaactggg 960
   tggccaggtt caccatcctg gaaggcgacc ccgatgggca gttcaccatc cgcacggacc 1020
   ccaagaccaa cgagggtgtt ctgtccattg tgaaggccct ggactatgag agctgtgaac 1080
   actacgaact caaagtgtcg gtgcagaatg aggccccgct gcaggcggct gcccttaggg 1140
   ctgagcgggg ccaggccaag gtccgcgtgc atgtgcagga caccaacgag ccccccgtgt 1200
   tccaggagaa cccacttcgg accagcctag cagagggggc acccccaggc actctggtgg 1260
   ccaccttctc tgcccgggac cctgacacag agcagctgca gaggctcagc tactccaagg 1320
   actacgaccc ggaagactgg ctgcaagtgg acgcagccac tggccggatc cagacccagc 1380
   acgtgctcag cccggcgtcc cccttcctca agggcggctg gtacagagcc atcgtcctgg 1440
   cccaggatga cgcctcccag ccccgcaccg ccaccggcac cctgtccatc gagatcctgg 1500
   aggtgaacga ccatgcacct gtgctggccc cgccgccgcc gggcagcctg tgcagcgagc 1560
   cacaccaagg cccaggcctc ctcctgggcg ccacggatga ggacctgccc ccccacgggg 1620
   cccccttcca cttccagctg agccccaggc tcccagagct cggccggaac tggagcctca 1680
   gccaggtcaa cgtgagccac gcgcgcctgc ggccgcgaca ccaggtcccc gaaggcctgc 1740
   accgcctcag cctgctgctc cgggactcgg ggcagccgcc ccagcagcgc gagcagcctc 1800
   tgaacgtgac cgtgtgccgc tgcggcaagg acggcgtctg cctgccgggg gccgcagcgc 1860
   tgctggcggg gggcacaggc ctcagcctgg gcgcactggt catcgtgctg gccagcgccc 1920
   tcctgctgct ggtgctggtc ctgctcgtgg cactccgggc gcggttctgg aagcagtctc 1980
   ggggcaaggg gctgctgcac ggcccccagg acgaccttcg agacaatgtc ctcaactacg 2040
   atgagcaagg aggcggggag gaggaccagg acgcctacga catcagccag ctgcgtcacc 2100
   cgacagcgct gagcctgcct ctgggaccgc cgccacttcg cagagatgcc ccgcagggcc 2160
   gcctgcaccc ccagccaccc cgagtgctgc ccaccagccc cctggacatc gccgacttca 2220
   tcaatgatgg cttggaggct gcagatagtg accccagtgt gccgccttac gacacagccc 2280
   tcatctatga ctacgagggt gacggctcgg tggcggggac gctgagctcc atcctgtcca 2340
   gccagggcga tgaggaccag gactacgact acctcagaga ctgggggccc cgcttcgccc 2400
   ggctggcaga catgtatggg cacccgtgcg ggttggagta cggggccaga tgggaccacc 2460
   aggccaggga gggtctttct cctggggcac tgctacccag acacagaggc cggacagcct 2520
   gaccctgggg cgcaactgga catgccactc cccggcctcg tggcagtgat ggcccctgca 2580
   gaggcagcct gaggtcaccg ggcccgaccc ccctgggcct ggggcagcct ccttcctgta 2640
   ggcgagggcc caagtctggg ggcagaacct gagtgtggat ggggcggcca ggaagaggcc 2700
   ccttcctgcc ggggtgggaa gagtttctct ccatcggccc catgcgggtc acctccctag 2760
   tcccaccttt gcctcctacc agtgaacctc atctttgtat gaaagacagc aacctcctgg 2820
   gtaaatctga atgaaaaacg tgctagtctc tttcatgcaa aaaaaaaaaa aaaaa      2875
<210> 44
   <211> 438
   <212> DNA
   <213> Artificial Sequence
<220>
   <223> Description of Artificial Sequence:primer
<220>
   <221> misc_feature
   <222> (1) .. (438) <223> 3' terminal sequence. ppar binding protein (PPARBP) gene.
<400> 44
   ttttttttgt gcttcttatg tttctctgtg ctgtattctg gaagtggtcg gataccatcg 60
   tctgagctgg gactattctg ataagatttc tctgctatgg aggagcctga ctcactttca 120
   ctgtccagat tctggggggt atatgctggt gacttactat ggctgggaga gccacgctca 180
   tgctttggag tggaaccact gatgagtgga gagccatagt ttttagaaga agccatttga 240
   ggcctaagcc cttctccact actttcccca ggtttctgca aagtcacttt ggctttaatg 300
   ctaggggagc ctccatcatg cttactgatg ataatttttg ccacacctgt gctccccaca 360
   ttttttgact ctgaggtctt cttaggaaga atccactgaa ctcccggagt gggaaacctt 420
   tngatttgtc tttatcac                                               438
<210> 45
   <211> 5809
   <212> DNA/RNA
   <213> Artificial Sequence
<220>
   <223> Description of Artificial Sequence:primer
<220>
   <221> misc_feature
   <222> (1) .. (5809)
   <223> ppar binding protein (PPARBP) gene.
<400> 45
   gggaagatgg cggcggcctc gagcaccctt ctcttcttgc cgccggggac ttcagattga 60
   tccttcccgg gaagagtagg gactgctggt gccctgcgtc ccgggatccc gagccaactt 120
   gtttcctccg ttagtggtgg ggaagggctt atccttttgt ggcggatcta gcttctcctc 180
   gccttcagga tgaaagctca gggggaaacc gaggagtcag aaaagctgag taagatgagt 240
   tctctcctgg aacggctcca tgcaaaattt aaccaaaata gaccctggag tgaaaccatt 300
   aagcttgtgc gtcaagtcat ggagaagagg gttgtgatga gttctggagg gcatcaacat 360
   ttggtcagct gtttggagac attgcagaag gctctcaaag taacatcttt accagcaatg 420
   actgatcgtt tggagtccat agcaagacag aatggactgg gctctcatct cagtgccagt 480
   ggcactgaat gttacatcac gtcagatatg ttctatgtgg aagtgcagtt agatcctgca 540
   ggacagcttt gtgatgtaaa agtggctcac catggggaga atcctgtgag ctgtccggag 600
   cttgtacagc agctaaggga aaaaaatttt gatgaatttt ctaagcacct taagggcctt 660
   gttaatctgt ataaccttcc aggggacaac aaactgaaga ctaaaatgta cttggctctc 720
   caatccttag aacaagatct ttctaaaatg gcaattatgt actggaaagc aactaatgct 780
   ggtcccttgg ataagattct tcatggaagt gttggctatc tcacaccaag gagtgggggt 840
   catttaatga acctgaagta ctatgtctct ccttctgacc tactggatga caagactgca 900
   tctcccatca ttttgcatga gaataatgtt tctcgatctt tgggcatgaa tgcatcagtg 960
   acaattgaag gaacatctgc tgtgtacaaa ctcccaattg caccattaat tatggggtca 1020
   catccagttg acaataaatg gaccccttcc ttctcctcaa tcaccagtgc caacagtgtt 1080
   gatcttcctg cctgtttctt cttgaaattt ccccagccaa tcccagtatc tagagcattt 1140
   gttcagaaac tgcagaactg cacaggaatt ccattgtttg aaactcaacc aacttatgca 1200
   cccctgtatg aactgatcac tcagtttgag ctatcaaagg accctgaccc catacctttg 1260
   aatcacaaca tgagatttta tgctgctctt cctggtcagc agcactgcta tttcctcaac 1320
   aaggatgctc ctcttccaga tggccgaagt ctacagggaa cccttgttag caaaatcacc 1380
   tttcagcacc ctggccgagt tcctcttatc ctaaatctga tcagacacca agtggcctat 1440
   aacaccctca ttggaagctg tgtcaaaaga actattctga aagaagattc tcctgggctt 1500
   ctccaatttg aagtgtgtcc tctctcagag tctcgtttca gcgtatcttt tcagcaccct 1560
   gtgaatgact ccctggtgtg tgtggtaatg gatgtgcagg actcaacaca tgtgagctgt 1620
   aaactctaca aagggctgtc ggatgcactg atctgcacag atgacttcat tgccaaagtt 1680
   gttcaaagat gtatgtccat ccctgtgacg atgagggcta ttcggaggaa agctgaaacc 1740
   attcaagccg acaccccagc actgtccctc attgcagaga cagttgaaga catggtgaaa 1800
   aagaacctgc ccccggctag cagcccaggg tatggcatga ccacaggcaa caacccaatg 1860
   agtggtacca ctacaccaac caacaccttt ccggggggtc ccattaccac cttgtttaat 1920
   atgagcatga gcatcaaaga tcggcatgag tcggtgggcc atggggagga cttcagcaag 1980
   gtgtctcaga acccaattct taccagtttg ttgcaaatca cagggaacgg ggggtctacc 2040
   attggctcga gtccgacccc tcctcatcac acgccgccac ctgtctcttc gatggccggc 2100
   aacaccaaga accacccgat gctcatgaac cttcttaaag ataatcctgc ccaggatttc 2160
   tcaacccttt atggaagcag ccctttagaa aggcagaact cctcttccgg ctcaccccgc 2220
   atggaaatat gctcggggag caacaagacc aagaaaaaga agtcatcaag attaccacct 2280
   gagaaaccaa agcaccagac tgaagatgac tttcagaggg agctattttc aatggatgtt 2340
   gactcacaga accctatctt tgatgtcaac atgacagctg acacgctgga tacgccacac 2400
   atcactccag ctccaagcca gtgtagcact cccccaacaa cttacccaca accagtacct 2460
   cacccccaac ccagtattca aaggatggtc cgactatcca gttcagacag cattggccca 2520
   gatgtaactg acatcctttc agacattgca gaagaagctt ctaaacttcc cagcactagt 2580
   gatgattgcc cagccattgg cacccctctt cgagattctt caagctctgg gcattctcag 2640
   agtaccctgt ttgactctga tgtctttcaa actaacaata atgaaaatcc atacactgat 2700
   ccagctgatc ttattgcaga tgctgctgga agccccagta gtgactctcc taccaatcat 2760
   ttttttcatg atggagtaga tttcaatcct gatttattga acagccagag ccaaagtggt 2820
   tttggagaag aatattttga tgaaagcagc caaagtgggg ataatgatga tttcaaagga 2880
   tttgcatctc aggcactaaa tactttgggg gtgccaatgc ttggaggtga taatggggag 2940
   accaagttta agggcaataa ccaagccgac acagttgatt tcagtattat ttcagtagcc 3000
   ggcaaagctt tagctcctgc agatcttatg gagcatcaca gtggtagtca gggtccttta 3060
   ctgaccactg gggacttagg gaaagaaaag actcaaaaga gggtaaagga aggcaatggc 3120
   accagtaata gtactctctc ggggcccgga ttagacagca aaccagggaa gcgcagtcgg 3180
   accccttcta atgatgggaa aagcaaagat aagcctccaa agcggaagaa ggcagacact 3240
   gagggaaagt ctccatctca tagttcttct aacagacctt ttaccccacc taccagtaca 3300
   ggtggatcta aatcgccagg cagtgcagga agatctcaga ctcccccagg tgttgccaca 3360
   ccacccattc ccaaaatcac tattcagatt cctaagggaa cagtgatggt gggcaagcct 3420
   tcctctcaca gtcagtatac cagcagtggt tctgtgtctt cctcaggcag caaaagccac 3480
   catagccatt cttcctcctc ttcctcatct gcttccacct cagggaagat gaaaagcagt 3540
   aaatcagaag gttcatcaag ttccaagtta agtagcagta tgtattctag ccaggggtct 3600
   tctggatcta gccagtccaa aaattcatcc cagtctgggg ggaagccagg ctcctctccc 3660
   ataaccaagc atggactgag cagtggctct agcagcacca agatgaaacc tcaaggaaag 3720
   ccatcatcac ttatgaatcc ttctttaagt aaaccaaaca tatccccttc tcattcaagg 3780
   ccacctggag gctctgacaa gcttgcctct ccaatgaagc ctgttcctgg aactcctcca 3840
   tcctctaaag ccaagtcccc tatcagttca ggttctggtg gttctcatat gtctggaact 3900
   agttcaagct ctggcatgaa gtcatcttca gggttaggat cctcaggctc gttgtcccag 3960
   aaaactcccc catcatctaa ttcctgtacg gcatcttcct cctccttttc ctcaagtggc 4020
   tcttccatgt catcctctca gaaccagcat gggagttcta aaggaaaatc tcccagcaga 4080
   aacaagaagc cgtccttgac agctgtcata gataaactga agcatggggt tgtcaccagt 4140
   ggccctgggg gtgaagaccc actggacggc cagatggggg tgagcacaaa ttcttccagc 4200
   catcctatgt cctccaaaca taacatgtca ggaggagagt ttcagggcaa gcgtgagaaa 4260
   agtgataaag acaaatcaaa ggtttccacc tccgggagtt cagtggattc ttctaagaag 4320
   acctcagagt caaaaaatgt ggggagcaca ggtgtggcaa aaattatcat cagtaagcat 4380
   gatggaggct cccctagcat taaagccaaa gtgactttgc agaaacctgg ggaaagtagt 4440
   ggagaagggc ttaggcctca aatggcttct tctaaaaact atggctctcc actcatcagt 4500
   ggttccactc caaagcatga gcgtggctct cccagccata gtaagtcacc agcatatacc 4560
   ccccagaatc tggacagtga aagtgagtca ggctcctcca tagcagagaa atcttatcag 4620
   aatagtccca gctcagacga tggtatccga ccacttccag aatacagcac agagaaacat 4680
   aagaagcaca aaaaggaaaa gaagaaagta aaagacaaag atagggaccg agaccgggac 4740
   aaagaccgag acaagaaaaa atctcatagc atcaagccag agagttggtc caaatcaccc 4800
   atctcttcag accagtcctt gtctatgaca agtaacacaa tcttatctgc agacagaccc 4860
   tcaaggctca gcccagactt tatgattggg gaggaagatg atgatcttat ggatgtggcc 4920
   ctgattggga attaggaacc ttatttccta aaagaaacag ggccagagga aaaaaaacta 4980
   ttgataagtt tataggcaaa ccaccataag gggtgagtca gacaggtctg atttggttaa 5040
   gaatcctaaa tggcatggct ttgacatcaa gctgggtgaa ttagaaaggc atatccagac 5100
   cctattaaag aaaccacagg gtttgattct ggttaccagg aagtcttctt tgttcctgtg 5160
   ccagaaagaa agttaaaata cttgcttaag aaagggaggg gggtgggagg ggtgtaggga 5220
   gagggaaggg agggaaacag ttttgtggga aatattcata tatattttct tctccctttt 5280
   tccattttta ggccatgttt taaactcatt ttagtgcatg tatatgaagg gctgggcaga 5340
   aaatgaaaaa gcaatacatt ccttgatgca tttgcatgaa ggttgttcaa ctttgtttga 5400
   ggtagttgtc cgtttgagtc atgggcaaat gaaggacttt ggtcattttg gacacttaag 5460
   taatgtttgg tgtctgtttc ttaggagtga ctgggggagg gaagattatt ttagctattt 5520
   atttgtaata ttttaaccct ttatctgttt gtttttatac agtgtttcgt tctaaatcta 5580
   tgaggtttag ggttcaaaat gatggaaggc cgaagagcaa ggcttatatg gtggtaggga 5640
   gcttatagct tgtgctaata ctgtagcatc aagcccaagc aaattagtca gagcccgcct 5700
   ttagagttaa atataataga aaaaccaaaa tgatattttt attttaggag ggtttaaata 5760
   gggttcagag atcataggaa tattaggagt tacctctctg tggaggtat 5809
<210> 46
   <211> 276
   <212> DNA
   <213> Artificial Sequence
<220>
   <223> Description of Artificial Sequence:primer
<220>
   <221> misc_feature
   <222> (1) .. (276) <223> 3' terminal sequence. cystatin a (stefin a) (CSTA) gene.
<400> 46
   tttttctttt nggaaaagaa tgcttcttta ttgatggtta tatttatcag caaggatcat 60
   gactcagtag ccagttgaag gaatcaganc actttgggta catgctgcta aaagcccgtc 120
   agctcgtcat ccttgttttt gtcaacctgg tatccagtaa gtaccaagtc ctcattttgt 180
   ccgggaagac ttttgaatac tttcaagtgc atatatttat tatcacctgc tcgtacctta 240
   atcgtagtaa tttgttccag caacaacttg agtttt 276
<210> 47
   <211> 265
   <212> DNA
   <213> Artificial Sequence
<220>
   <223> Description of Artificial Sequence:primer
<220>
   <221> misc_feature
   <222> (1) .. (265) <223> 5' terminal sequence. cystatin a (stefin a) (CSTA) gene.
<400> 47
   aaaactcaag ttgttgctgg aacaaattac tacattaagg tacgagcagg tgataataaa 60
   tatatgcact tgaaagtatt caaaagtctt cccggacaaa atgaggactt ggtacttact 120
   ggataccagg ttgacaaaaa caaggatgac gagctgacgg gcttttagca gcatgtaccc 180
   aaagtgttct gattccttca actggctact gagtcatgat ccttgctgat aaatataacc 240
   atcaataaag aagcattctt ttcca                                      265
<210> 48
   <211> 451
   <212> DNA/RNA
   <213> Artificial Sequence
<220>
   <223> Description of Artificial Sequence:primer
<220>
   <221> misc_feature
   <222> (1) .. (451)
   <223> cystatin a (stefin a) (CSTA) gene.
<400> 48
   acttccctgt tcactttggt tccagcatcc tgtccagcaa agaagcaatc agccaaaatg 60
   atacctggag gcttatctga ggccaaaccc gccactccag aaatccagga gattgttgat 120
   aaggttaaac cacagcttga agaaaaaaca aatgagactt atggaaaatt ggaagctgtg 180
   cagtataaaa ctcaagttgt tgctggaaca aattactaca ttaaggtacg agcaggtgat 240
   aataaatata tgcacttgaa agtattcaaa agtcttcccg gacaaaatga ggacttggta 300
   cttactggat accaggttga caaaaacaag gatgacgagc tgacgggctt ttagcagcat 360
   gtacccaaag tgttctgatt ccttcaactg gctactgagt catgatcctt gctgataaat 420
   ataaccatca ataaagaagc attcttttcc a                               451
<210> 49
   <211> 410
   <212> DNA
   <213> Artificial Sequence
<220>
   <223> Description of Artificial Sequence:primer
<220>
   <221> misc_feature
   <222> (1) .. (410)
   <223> 3' terminal sequence. signal peptide, cub domain, egf-like 2 (SCUBE2) gene.
<400> 49
   cacgagcact gcttagaact caagttcctg ggatcaaact tgtatcacac aggctggaga 60
   aggctgtgga gaaactgagt cctcccaggt ctcaaggtgg gtggagggag cctgcagggg 120
   tctccttccc tcccctcttg cctgttctgc ctggtcagag cctgcacacg agtgagaggg 180
   ctcccttaga gagggccggg ctagaggaag ctgaagtttc agaataagca gcttattctg 240
   tggcctcctt tccactacag actccttgag gaggagtaag accccagaag gacaggtgag 300
   tctcacctaa ggctgaccaa agtccagctc agccagcccg tgattcttat ccaagacatc 360
   cgccccacag cagtgaagaa gcngatgcca ctcaaaagcc attctcagtn            410
<210> 50
   <211> 474
   <212> DNA
   <213> Artificial Sequence
<220>
   <223> Description of Artificial Sequence:primer
<220>
   <221> misc_feature
   <222> (1) .. (474)
   <223> 5' terminal sequence. signal peptide, cub domain, egf-like 2 (SCUBE2) gene.
<400> 50
   atttttatag ataatacaga tattttggta aattgaactt ggtttttctt tcccagcatc 60
   gtggatgtag actgagaatg gctttgagtg gcatcagctt ctcactgctg tgggcggatg 120
   tcttggatag atcacgggct ggctgagctg gactttggtc agcctaggtg agactcacct 180
   gtccttctgg ggtcttactc ctcctcaagg agtctgtagt ggaaaggagg ccacagaata 240
   agctgcttat tctgaaactt cagcttcctc tagcccggcc ctctctaagg gagccctctg 300
   cactcgtgtg aggctctgac caggcagaac aggcaagagg ggagggaagg agacccctgc 360
   aggctccctc canccacctt gaagacctgg ggaggactca gtttctccca caagccttct 420
   ccagcctgtg tgatacaagt ttgatnccag gaacttgagt tctaagcagt gctc       474
<210> 51
   <211> 3737
   <212> DNA/RNA
   <213> Artificial Sequence
<220>
   <223> Description of Artificial Sequence:primer
<220>
   <221> misc_feature
   <222> (11)..(3737)
   <223> signal peptide, cub domain, egf-like 2 (SCUBE2) gene.
<400> 51
   ggcgtccgcg cacacctccc cgcgccgccg ccgccaccgc ccgcactccg ccgcctctgc 60
   ccgcaaccgc tgagccatcc atgggggtcg cgggccgcaa ccgtcccggg gcggcctggg 120
   cggtgctgct gctgctgctg ctgctgccgc cactgctgct gctggcgggg gccgtcccgc 180
   cgggtcgggg ccgtgccgcg gggccgcagg aggatgtaga tgagtgtgcc caagggctag 240
   atgactgcca tgccgacgcc ctgtgtcaga acacacccac ctcctacaag tgctcctgca 300
   agcctggcta ccaaggggaa ggcaggcagt gtgaggacat cgatgaatgt ggaaatgagc 360
   tcaatggagg ctgtgtccat gactgtttga atattccagg caattatcgt tgcacttgtt 420
   ttgatggctt catgttggct catgacggtc ataattgtct tgatgtggac gagtgcctgg 480
   agaacaatgg cggctgccag catacctgtg tcaacgtcat ggggagctat gagtgctgct 540
   gcaaggaggg gtttttcctg agtgacaatc agcacacctg cattcaccgc tcggaagagg 600
   gcctgagctg catgaataag gatcacggct gtagtcacat ctgcaaggag gccccaaggg 660
   gcagcgtcgc ctgtgagtgc aggcctggtt ttgagctggc caagaaccag agagactgca 720
   tcttgacctg taaccatggg aacggtgggt gccagcactc ctgtgacgat acagccgatg 780
   gcccagagtg cagctgccat ccacagtaca agatgcacac agatgggagg agctgccttg 840
   agcgagagga cactgtcctg gaggtgacag agagcaacac cacatcagtg gtggatgggg 900
   ataaacgggt gaaacggcgg ctgctcatgg aaacgtgtgc tgtcaacaat ggaggctgtg 960
   accgcacctg taaggatact tcgacaggtg tccactgcag ttgtcctgtt ggattcactc 1020
   tccagttgga tgggaagaca tgtaaagata ttgatgagtg ccagacccgc aatggaggtt 1080
   gtgatcattt ctgcaaaaac atcgtgggca gttttgactg cggctgcaag aaaggattta 1140
   aattattaac agatgagaag tcttgccaag atgtggatga gtgctctttg gataggacct 1200
   gtgaccacag ctgcatcaac caccctggca catttgcttg tgcttgcaac cgagggtaca 1260
   ccctgtatgg cttcacccac tgtggagaca ccaatgagtg cagcatcaac aacggaggct 1320
   gtcagcaggt ctgtgtgaac acagtgggca gctatgaatg ccagtgccac cctgggtaca 1380
   agctccactg gaataaaaaa gactgtgtgg aagtgaaggg gctcctgccc acaagtgtgt 1440
   caccccgtgt gtccctgcac tgcggtaaga gtggtggagg agacgggtgc ttcctcagat 1500
   gtcactctgg cattcacctc tcttcagatg tcaccaccat caggacaagt gtaaccttta 1560
   agctaaatga aggcaagtgt agtttgaaaa atgctgagct gtttcccgag ggtctgcgac 1620
   cagcactacc agagaagcac agctcagtaa aagagagctt ccgctacgta aaccttacat 1680
   gcagctctgg caagcaagtc ccaggagccc ctggccgacc aagcacccct aaggaaatgt 1740
   ttatcactgt tgagtttgag cttgaaacta accaaaagga ggtgacagct tcttgtgacc 1800
   tgagctgcat cgtaaagcga accgagaagc ggctccgtaa agccatccgc acgctcagaa 1860
   aggccgtcca cagggagcag tttcacctcc agctctcagg catgaacctc gacgtggcta 1920
   aaaagcctcc cagaacatct gaacgccagg cagagtcctg tggagtgggc cagggtcatg 1980
   cagaaaacca atgtgtcagt tgcagggctg ggacctatta tgatggagca cgagaacgct 2040
   gcattttatg tccaaatgga accttccaaa atgaggaagg acaaatgact tgtgaaccat 2100
   gcccaagacc aggaaattct ggggccctga agaccccaga agcttggaat atgtctgaat 2160
   gtggaggtct gtgtcaacct ggtgaatatt ctgcagatgg ctttgcacct tgccagctct 2220
   gtgccctggg cacgttccag cctgaagctg gtcgaacttc ctgcttcccc tgtggaggag 2280
   gccttgccac caaacatcag ggagctactt cctttcagga ctgtgaaacc agagttcaat 2340
   gttcacctgg acatttctac aacaccacca ctcaccgatg tattcgttgc ccagtgggaa 2400
   cataccagcc tgaatttgga aaaaataatt gtgtttcttg cccaggaaat actacgactg 2460
   actttgatgg ctccacaaac ataacccagt gtaaaaacag aagatgtgga ggggagctgg 2520
   gagatttcac tgggtacatt gaatccccaa actacccagg caattaccca gccaacaccg 2580
   agtgtacgtg gaccatcaac ccacccccca agcgccgcat cctgatcgtg gtccctgaga 2640
   tcttcctgcc catagaggac gactgtgggg actatctggt gatgcggaaa acctcttcat 2700
   ccaattctgt gacaacatat gaaacctgcc agacctacga acgccccatc gccttcacct 2760
   ccaggtcaaa gaagctgtgg attcagttca agtccaatga agggaacagc gctagagggt 2820
   tccaggtccc atacgtgaca tatgatgagg actaccagga actcattgaa gacatagttc 2880
   gagatggcag gctctatgca tctgagaacc atcaggaaat acttaaggat aagaaactta 2940
   tcaaggctct gtttgatgtc ctggcccatc cccagaacta tttcaagtac acagcccagg 3000
   agtcccgaga gatgtttcca agatcgttca tccgattgct acgttccaaa gtgtccaggt 3060
   ttttgagacc ttacaaatga ctcagcccac gtgccactca atacaaatgt tctgctatag 3120
   ggttggtggg acagagctgt cttccttctg catgtcagca cagtcgggta ttgctgcctc 3180
   ccgtatcagt gactcattag agttcaattt ttatagataa tacagatatt ttggtaaatt 3240
   gaacttggtt tttctttccc agcatcgtgg atgtagactg agaatggctt tgagtggcat 3300
   cagcttctca ctgctgtggg cggatgtctt ggatagatca cgggctggct gagctggact 3360
   ttggtcagcc taggtgagac tcacctgtcc ttctggggtc ttactcctcc tcaaggagtc 3420
   tgtagtggaa aggaggccac agaataagct gcttattctg aaacttcagc ttcctctagc 3480
   ccggccctct ctaagggagc cctctgcact cgtgtgcagg ctctgaccag gcagaacagg 3540
   caagagggga gggaaggaga cccctgcagg ctccctccac ccaccttgag acctgggagg 3600
   actcagtttc tccacagcct tctccagcct gtgtgataca agtttgatcc caggaacttg 3660
   agttctaagc agtgctcgtg aaaaaaaaaa gcagaaagaa ttagaaataa ataaaaacta 3720
   agcacttctg gagacat                                                3737
<210> 52
   <211> 572
   <212> DNA
   <213> Artificial Sequence
<220>
   <223> Description of Artificial Sequence:primer
<220>
   <221> misc_feature
   <222> (1) .. (572)
   <223> 5' terminal sequence. integrin, beta 3 (platelet glycoprotein IIIa, antigen CD61) (ITGB3) gene.
<400> 52
   accagcatct cccagttcat aatcacaacc cttcagattt gccttattgg cagctctact 60
   ctggaggttt gtttagaaga agtgtgtcac ccttaggcca gcaccatctc tttacctcct 120
   aattccacac cctcactcgc tgtagacatt tgctatgagc tggggatgtc tctcatgacc 180
   aaatgctttt cctcaaaggg agagagtgct attgtagagc cagaggtctg gccctatgct 240
   tccggcctcc tgtccctcat ccatagcacc tccacatacc tggccctgag ccttggtgtg 300
   ctgtatccat ccatggggct gattgtatgt accttctacc tcttggctgc cttgtgaagg 360
   aattattccc atgagttggc tgggaataag tgccaggatg gaatgatggg tcagctgtat 420
   cagcacgtgt ggcctgttct tctatgggtt ggacaacctc attgtaactc actctttaat 480
   ctgagaggcc acagcgcaat tttattttat ttttctcatg atgaggtttt cttaacttaa 540
   aagaacatgg atataaacat gctagcatta ta                               572
<210> 53
   <211> 3997
   <212> DNA/RNA
   <213> Artificial Sequence
<220>
   <223> Description of Artificial Sequence:primer
<220>
   <221> misc_feature
   <222> (1) .. (3997)
   <223> integrin, beta 3 (platelet glycoprotein IIIa, antigen CD61) (ITGB3) gene.
<400> 53
   gcgggaggcg gacgagatgc gagcgcggcc gcggccccgg ccgctctggg cgactgtgct 60
   ggcgctgggg gcgctggcgg gcgttggcgt aggagggccc aacatctgta ccacgcgagg 120
   tgtgagctcc tgccagcagt gcctggctgt gagccccatg tgtgcctggt gctctgatga 180
   ggccctgcct ctgggctcac ctcgctgtga cctgaaggag aatctgctga aggataactg 240
   tgccccagaa tccatcgagt tcccagtgag tgaggcccga gtactagagg acaggcccct 300
   cagcgacaag ggctctggag acagctccca ggtcactcaa gtcagtcccc agaggattgc 360
   actccggctc cggccagatg attcgaagaa tttctccatc caagtgcggc aggtggagga 420
   ttaccctgtg gacatctact acttgatgga cctgtcttac tccatgaagg atgatctgtg 480
   gagcatccag aacctgggta ccaagctggc cacccagatg cgaaagctca ccagtaacct 540
   gcggattggc ttcggggcat ttgtggacaa gcctgtgtca ccatacatgt atatctcccc 600
   accagaggcc ctcgaaaacc cctgctatga tatgaagacc acctgcttgc ccatgtttgg 660
   ctacaaacac gtgctgacgc taactgacca ggtgacccgc ttcaatgagg aagtgaagaa 720
   gcagagtgtg tcacggaacc gagatgcccc agagggtggc tttgatgcca tcatgcaggc 780
   tacagtctgt gatgaaaaga ttggctggag gaatgatgca tcccacttgc tggtgtttac 840
   cactgatgcc aagactcata tagcattgga cggaaggctg gcaggcattg tccagcctaa 900
   tgacgggcag tgtcatgttg gtagtgacaa tcattactct gcctccacta ccatggatta 960
   tccctctttg gggctgatga ctgagaagct atcccagaaa aacatcaatt tgatctttgc 1020
   agtgactgaa aatgtagtca atctctatca gaactatagt gagctcatcc cagggaccac 1080
   agttggggtt ctgtccatgg attccagcaa tgtcctccag ctcattgttg atgcttatgg 1140
   gaaaatccgt tctaaagtag agctggaagt gcgtgacctc cctgaagagt tgtctctatc 1200
   cttcaatgcc acctgcctca acaatgaggt catccctggc ctcaagtctt gtatgggact 1260
   caagattgga gacacggtga gcttcagcat tgaggccaag gtgcgaggct gtccccagga 1320
   gaaggagaag tcctttacca taaagcccgt gggcttcaag gacagcctga tcgtccaggt 1380
   cacctttgat tgtgactgtg cctgccaggc ccaagctgaa cctaatagcc atcgctgcaa 1440
   caatggcaat gggacctttg agtgtggggt atgccgttgt gggcctggct ggctgggatc 1500
   ccagtgtgag tgctcagagg aggactatcg cccttcccag caggacgaat gcagcccccg 1560
   ggagggtcag cccgtctgca gccagcgggg cgagtgcctc tgtggtcaat gtgtctgcca 1620
   cagcagtgac tttggcaaga tcacgggcaa gtactgcgag tgtgacgact tctcctgtgt 1680
   ccgctacaag ggggagatgt gctcaggcca tggccagtgc agctgtgggg actgcctgtg 1740
   tgactccgac tggaccggct actactgcaa ctgtaccacg cgtactgaca cctgcatgtc 1800
   cagcaatggg ctgctgtgca gcggccgcgg caagtgtgaa tgtggcagct gtgtctgtat 1860
   ccagccgggc tcctatgggg acacctgtga gaagtgcccc acctgcccag atgcctgcac 1920
   ctttaagaaa gaatgtgtgg agtgtaagaa gtttgaccgg gagccctaca tgaccgaaaa 1980
   tacctgcaac cgttactgcc gtgacgagat tgagtcagtg aaagagctta aggacactgg 2040
   caaggatgca gtgaattgta cctataagaa tgaggatgac tgtgtcgtca gattccagta 2100
   ctatgaagat tctagtggaa agtccatcct gtatgtggta gaagagccag agtgtcccaa 2160
   gggccctgac atcctggtgg tcctgctctc agtgatgggg gccattctgc tcattggcct 2220
   tgccgccctg ctcatctgga aactcctcat caccatccac gaccgaaaag aattcgctaa 2280
   atttgaggaa gaacgcgcca gagcaaaatg ggacacagcc aacaacccac tgtataaaga 2340
   ggccacgtct accttcacca atatcacgta ccggggcact taatgataag cagtcatcct 2400
   cagatcatta tcagcctgtg ccacgattgc aggagtccct gccatcatgt ttacagagga 2460
   cagtatttgt ggggagggat ttggggctca gagtggggta ggttgggaga atgtcagtat 2520
   gtggaagtgt gggtctgtgt gtgtgtatgt gggggtctgt gtgtttatgt gtgtgtgttg 2580
   tgtgtgggag tgtgtaattt aaaattgtga tgtgtcctga taagctgagc tccttagcct 2640
   ttgtcccaga atgcctcctg cagggattct tcctgcttag cttgagggtg actatggagc 2700
   tgagcaggtg ttcttcatta cctcagtgag aagccagctt tcctcatcag gccattgtcc 2760
   ctgaagagaa gggcagggct gaggcctctc attccagagg aagggacacc aagccttggc 2820
   tctaccctga gttcataaat ttatggttct caggcctgac tctcagcagc tatggtagga 2880
   actgctgggc ttggcagccc gggtcatctg tacctctgcc tcctttcccc tccctcaggc 2940
   cgaaggagga gtcagggaga gctgaactat tagagctgcc tgtgcctttt gccatcccct 3000
   caacccagct atggttctct cgcaagggaa gtccttgcaa gctaattctt tgacctgttg 3060
   ggagtgagga tgtctgggcc actcaggggt cattcatggc ctgggggatg taccagcatc 3120
   tcccagttca taatcacaac ccttcagatt tgccttattg gcagctctac tctggaggtt 3180
   tgtttagaag aagtgtgtca cccttaggcc agcaccatct ctttacctcc taattccaca 3240
   ccctcactgc tgtagacatt tgctatgagc tggggatgtc tctcatgacc aaatgctttt 3300
   cctcaaaggg agagagtgct attgtagagc cagaggtctg gccctatgct tccggcctcc 3360
   tgtccctcat ccatagcacc tccacatacc tggccctgag ccttggtgtg ctgtatccat 3420
   ccatggggct gattgtattt accttctacc tcttggctgc cttgtgaagg aattattccc 3480
   atgagttggc tgggaataag tgccaggatg gaatgatggg tcagttgtat cagcacgtgt 3540
   ggcctgttct tctatgggtt ggacaacctc attttaactc agtctttaat ctgagaggcc 3600
   acagtgcaat tttattttat ttttctcatg atgaggtttt cttaacttaa aagaacatgt 3660
   atataaacat gcttgcatta tatttgtaaa tttatgtgta tggcaaagaa ggagagcata 3720
   ggaaaccaca cagacttggg cagggtacag acactcccac ttggcatcat tcacagcaag 3780
   tcactggcca gtggctggat ctgtgagggg ctctctcatg atagaaggct atggggatag 3840
   atgtgtggac acattggacc tttcctgagg aagagggact gttcttttgt cccagaaaag 3900
   cagtggctcc attggtgttg acatacatcc aacattaaaa gccaccccca aatgcccaag 3960
   aaaaaaagaa agacttatca acatttgttc catgagg 3997
<210> 54
   <211> 591
   <212> DNA
   <213> Artificial Sequence
<220>
   <223> Description of Artificial Sequence:primer
<220>
   <221> misc_feature
   <222> (1) .. (591)
   <223> 5' terminal sequence. platelet/endothelial cell adhesion molecule (CD31 antigen) (PECAM1) gene.
<400> 54
   cttaccaact tttaaaaaca agtaaagttt tggagaatag taccaagaac tcaaatgatc 60
   ctgcggtatt caaagacaac cccactgaag acgtcgaata ccagtgtgtt gcagataatt 120
   gccattccca cgccaaaatg ttaagtgagg ttctgagggt gaaggtgata gccccggtgg 180
   atgaggtcca gatttctatc ctgtcaagta aggtggtgga gtctggagag gacattgtgc 240
   tgcaatgtgc tgtgaatgaa ggatctggtc ccatcaccta taagttttac agagaaaaag 300
   agggcaaacc cttctatcaa atgacctcaa atgccaccca ggcattttgg accaagcaga 360
   aggctaacaa ggaacaggag ggagagtatt actgcacagc cttcaacaga gccaaccacg 420
   cctccagtgt ccccagaagc aaaatactga cagtcagagt cattcttgcc ccatggaaga 480
   aaggacttat tgcagtggtt atcatcggag tgatcattgc tctcttgatc attgcggcca 540
   aatgttattt tctgaggaaa gccaaggcca agcagatgcc agtggaaatg t 591
<210> 55
   <211> 3189
   <212> DNA/RNA
   <213> Artificial Sequence
<220>
   <223> Description of Artificial Sequence:primer
<220>
   <221> misc_feature
   <222> (1) .. (3189)
   <223> platelet/endothelial cell adhesion molecule (CD31 antigen) (PECAM1) gene.
<400> 55
<210> 56
   <211> 463
   <212> DNA
   <213> Artificial Sequence
<220>
   <223> Description of Artificial Sequence:primer
<220>
   <221> misc feature
   <222> (1) .. (963)
   <223> 3' terminal sequence. antigen identified by monoclonal antibody ki-67 (MK167) gene.
<400> 56
   atcacaaaac taactttatt atatttttcc cagttcgatt tttctgtcaa atatcttcac 60
   tgtccctatg acttctggtt cttattttct tgacacacac attgtcctca gccttctttg 120
   gattttctgc acacctcttg acactccgcg ttactctctg cacagattcg ctctccaaag 180
   tgcttgctgc aggctggctt tttgtctttc ttgatctcag gcacatggag tctgaatttc 240
   ctgcttctcc tttccctttc tgattctgca tgagaacctt cgcactcttc tgccctccgc 300
   tctcctctgc caccttaggc tgggagctct cattctgtct agcagacctc aagcaccttt 360
   tgttctcact ggactttgtc tctaggtatg ggttttccgg gctccatcat ctggattctg 420
   aatggtccat ctctggggag gtcttcatgg gcttcttttc att                    463
<210> 57
   <211> 478
   <212> DNA
   <213> Artificial Sequence
<220>
   <223> Description of Artificial Sequence:primer
<220>
   <221> misc_feature
   <222> (1) .. (478)
   <223> 5' terminal sequence. antigen identified by monoclonal antibody ki-67 (MKI67) gene.
<400> 57
   nttcggcacc agcaaagatg gaagcgtcac gggaaccaag aggctgcgct gcatgccagc 60
   accagaggaa attgtggagg agctgccagc cagcaagaag cagagggttg ctcccagggc 120
   aagaggcaaa tcatccgaac ccgtggtcat catgaagaga agtttgagga cttctgcaaa 180
   aagaattgaa cctgcggaag agctgaacag caacgacatg aaaaccaaca aagaggaaca 240
   caaattacaa gactcagtcc ctgaaaataa gggaatatcc ctgcgctcca gaccgccaaa 300
   ataagactga ggcagaacag caaataactt gaggtctttg tattagcaga aagaatagaa 360
   ataaacagaa tgaaaagaag cccatgaaga cctccccaga gatgggacat tcagaatcca 420
   gatgatggag cccggaaacc catacctaga gacaaagtca gtgagaacaa aaggtgct   478
<210> 58
   <211> 12515
   <212> DNA/RNA
   <213> Artificial Sequence
<220>
   <223> Description of Artificial Sequence:primer
<220>
   <221> misc_feature
   <222> (1) .. (12515)
   <223> antigen identified by monoclonal antibody ki-67 (MKI67) gene.
<400> 58
   ctaccgggcg gaggtgagcg cggcgccggc tcctcctgcg gcggactttg ggtgcgactt 60
   gacgagcggt ggttcgacaa gtggccttgc gggccggatc gtcccagtgg aagagttgta 120
   aatttgcttc tggccttccc ctacggatta tacctggcct tcccctacgg attatactca 180
   acttactgtt tagaaaatgt ggcccacgag acgcctggtt actatcaaaa ggagcggggt 240
   cgacggtccc cactttcccc tgagcctcag cacctgcttg tttggaaggg gtattgaatg 300
   tgacatccgt atccagcttc ctgttgtgtc aaaacaacat tgcaaaattg aaatccatga 360
   gcaggaggca atattacata atttcagttc cacaaatcca acacaagtaa atgggtctgt 420
   tattgatgag cctgtacggc taaaacatgg agatgtaata actattattg atcgttcctt 480
   caggtatgaa aatgaaagtc ttcagaatgg aaggaagtca actgaatttc caagaaaaat 540
   acgtgaacag gagccagcac gtcgtgtctc aagatctagc ttctcttctg accctgatga 600
   gaaagctcaa gattccaagg cctattcaaa aatcactgaa ggaaaagttt caggaaatcc 660
   tcaggtacat atcaagaatg tcaaagaaga cagtaccgca gatgactcaa aagacagtgt 720
   tgctcaggga acaactaatg ttcattcctc agaacatgct ggacgtaatg gcagaaatgc 780
   agctgatccc atttctgggg attttaaaga aatttccagc gttaaattag tgagccgtta 840
   tggagaattg aagtctgttc ccactacaca atgtcttgac aatagcaaaa aaaatgaatc 900
   tcccttttgg aagctttatg agtcagtgaa gaaagagttg gatgtaaaat cacaaaaaga 960
   aaatgtccta cagtattgta gaaaatctgg attacaaact gattacgcaa cagagaaaga 1020
   aagtgctgat ggtttacagg gggagaccca actgttggtc tcgcgtaagt caagaccaaa 1080
   atctggtggg agcggccacg ctgtggcaga gcctgcttca cctgaacaag agcttgacca 1140
   gaacaagggg aagggaagag acgtggagtc tgttcagact cccagcaagg ctgtgggcgc 1200
   cagctttcct ctctatgagc cggctaaaat gaagacccct gtacaatatt cacagcaaca 1260
   aaattctcca caaaaacata agaacaaaga cctgtatact actggtagaa gagaatctgt 1320
   gaatctgggt aaaagtgaag gcttcaaggc tggtgataaa actcttactc ccaggaagct 1380
   ttcaactaga aatcgaacac cagctaaagt tgaagatgca gctgactctg ccactaagcc 1440
   agaaaatctc tcttccaaaa ccagaggaag tattcctaca gatgtggaag ttctgcctac 1500
   ggaaactgaa attcacaatg agccattttt aactctgtgg ctcactcaag ttgagaggaa 1560
   gatccaaaag gattccctca gcaagcctga gaaattgggc actacagctg gacagatgtg 1620
   ctctgggtta cctggtctta gttcagttga tatcaacaac tttggtgatt ccattaatga 1680
   gagtgaggga ataccttgga aaagaaggcg tgtgtccttt ggtgggcacc taagacctga 1740
   actatttgat gaaaacttgc ctcctaatac gcctctcaaa aggggagaag ccccaaccaa 1800
   aagaaagtct ctggtaatgc acactccacc tgtcctgaag aaaatcatca aggaacagcc 1860
   tcaaccatca ggaaaacaag agtcaggttc agaaatccat gtggaagtga aggcacaaag 1920
   cttggttata agccctccag ctcctagtcc taggaaaact ccagttgcca gtgatcaacg 1980
   ccgtaggtcc tgcaaaacag cccctgcttc cagcagcaaa tctcagacag aggttcctaa 2040
   gagaggagga gaaagagtgg caacctgcct tcaaaagaga gtgtctatca gccgaagtca 2100
   acatgatatt ttacagatga tatgttccaa aagaagaagt ggtgcttcgg aagcaaatct 2160
   gattgttgca aaatcatggg cagatgtagt aaaacttggt gcaaaacaaa cacaaactaa 2220
   agtcataaaa catggtcctc aaaggtcaat gaacaaaagg caaagaagac ctgctactcc 2280
   aaagaagcct gtgggcgaag ttcacagtca atttagtaca ggccacgcaa actctccttg 2340
   taccataata atagggaaag ctcatactga aaaagtacat gtgcctgctc gaccctacag 2400
   agtgctcaac aacttcattt ccaaccaaaa aatggacttt aaggaagatc tttcaggaat 2460
   agctgaaatg ttcaagaccc cagtgaagga gcaaccgcag ttgacaagca catgtcacat 2520
   cgctatttca aattcagaga atttgcttgg aaaacagttt caaggaactg attcaggaga 2580
   agaacctctg ctccccacct cagagagttt tggaggaaat gtgttcttca gtgcacagaa 2640
   tgcagcaaaa cagccatctg ataaatgctc tgcaagccct cccttaagac ggcagtgtat 2700
   tagagaaaat ggaaacgtag caaaaacgcc caggaacacc tacaaaatga cttctctgga 2760
   gacaaaaact tcagatactg agacagagcc ttcaaaaaca gtatccactg taaacaggtc 2820
   aggaaggtct acagagttca ggaatataca gaagctacct gtggaaagta agagtgaaga 2880
   aacaaataca gaaattgttg agtgcatcct aaaaagaggt cagaaggcaa cactactaca 2940
   acaaaggaga gaaggagaga tgaaggaaat agaaagacct tttgagacat ataaggaaaa 3000
   tattgaatta aaagaaaacg atgaaaagat gaaagcaatg aagagatcaa gaacttgggg 3060
   gcagaaatgt gcaccaatgt ctgacctgac agacctcaag agcttgcctg atacagaact 3120
   catgaaagac acggcacgtg gccagaatct cctccaaacc caagatcatg ccaaggcacc 3180
   aaagagtgag aaaggcaaaa tcactaaaat gccctgccag tcattacaac cagaaccaat 3240
   aaacacccca acacacacaa aacaacagtt gaaggcatcc ctggggaaag taggtgtgaa 3300
   agaagagctc ctagcagtcg gcaagttcac acggacgtca ggggagacca cgcacacgca 3360
   cagagagcca gcaggagatg gcaagagcat cagaacgttt aaggagtctc caaagcagat 3420
   cctggaccca gcagcccgtg taactggaat gaagaagtgg ccaagaacgc ctaaggaaga 3480
   ggcccagtca ctagaagacc tggctggctt caaagagctc ttccagacac caggtccctc 3540
   tgaggaatca atgactgatg agaaaactac caaaatagcc tgcaaatctc caccaccaga 3600
   atcagtggac actccaacaa gcacaaagca atggcctaag agaagtctca ggaaagcaga 3660
   tgtagaggaa gaattcttag cactcaggaa actaacacca tcagcaggga aagccatgct 3720
   tacgcccaaa ccagcaggag gtgatgagaa agacattaaa gcatttatgg gaactccagt 3780
   gcagaaactg gacctggcag gaactttacc tggcagcaaa agacagctac agactcctaa 3840
   ggaaaaggcc caggctctag aagacctggc tggctttaaa gagctcttcc agactcctgg 3900
   tcacaccgag gaattagtgg ctgctggtaa aaccactaaa ataccctgcg actctccaca 3960
   gtcagaccca gtggacaccc caacaagcac aaagcaacga cccaagagaa gtatcaggaa 4020
   agcagatgta gagggagaac tcttagcgtg caggaatcta atgccatcag caggcaaagc 4080
   catgcacacg cctaaaccat cagtaggtga agagaaagac atcatcatat ttgtgggaac 4140
   tccagtgcag aaactggacc tgacagagaa cttaaccggc agcaagagac ggccacaaac 4200
   tcctaaggaa gaggcccagg ctctggaaga cctgactggc tttaaagagc tcttccagac 4260
   ccctggtcat actgaagaag cagtggctgc tggcaaaact actaaaatgc cctgcgaatc 4320
   ttctccacca gaatcagcag acaccccaac aagcacaaga aggcagccca agacaccttt 4380
   ggagaaaagg gacgtacaga aggagctctc agccctgaag aagctcacac agacatcagg 4440
   ggaaaccaca cacacagata aagtaccagg aggtgaggat aaaagcatca acgcgtttag 4500
   ggaaactgca aaacagaaac tggacccagc agcaagtgta actggtagca agaggcaccc 4560
   aaaaactaag gaaaaggccc aacccctaga agacctggct ggctggaaag agctcttcca 4620
   gacaccagta tgcactgaca agcccacgac tcacgagaaa actaccaaaa tagcctgcag 4680
   atcacaacca gacccagtgg acacaccaac aagctccaag ccacagtcca agagaagtct 4740
   caggaaagtg gacgtagaag aagaattctt cgcactcagg aaacgaacac catcagcagg 4800
   caaagccatg cacacaccca aaccagcagt aagtggtgag aaaaacatct acgcatttat 4860
   gggaactcca gtgcagaaac tggacctgac agagaactta actggcagca agagacggct 4920
   acaaactcct aaggaaaagg cccaggctct agaagacctg gctggcttta aagagctctt 4980
   ccagacacga ggtcacactg aggaatcaat gactaacgat aaaactgcca aagtagcctg 5040
   caaatcttca caaccagacc tagacaaaaa cccagcaagc tccaagcgac ggctcaagac 5100
   atccctgggg aaagtgggcg tgaaagaaga gctcctagca gttggcaagc tcacacagac 5160
   atcaggagag actacacaca cacacacaga gccaacagga gatggtaaga gcatgaaagc 5220
   atttatggag tctccaaagc agatcttaga ctcagcagca agtctaactg gcagcaagag 5280
   gcagctgaga actcctaagg gaaagtctga agtccctgaa gacctggccg gcttcatcga 5340
   gctcttccag acaccaagtc acactaagga atcaatgact aatgaaaaaa ctaccaaagt 5400
   atcctacaga gcttcacagc cagacctagt ggacacccca acaagctcca agccacagcc 5460
   caagagaagt ctcaggaaag cagacactga agaagaattt ttagcattta ggaaacaaac 5520
   gccatcagca ggcaaagcca tgcacacacc caaaccagca gtaggtgaag agaaagacat 5580
   caacacgttt ttgggaactc cagtgcagaa actggaccag ccaggaaatt tacctggcag 5640
   caatagacgg ctacaaactc gtaaggaaaa ggcccaggct ctagaagaac tgactggctt 5700
   cagagagctt ttccagacac catgcactga taaccccaca gctgatgaga aaactaccaa 5760
   aaaaatactc tgcaaatctc cgcaatcaga cccagcggac accccaacaa acacaaagca 5820
   acggcccaag agaagcctca agaaagcaga cgtagaggaa gaatttttag cattcaggaa 5880
   actaacacca tcagcaggca aagccatgca cacgcctaaa gcagcagtag gtgaagagaa 5940
   agacatcaac acatttgtgg ggactccagt ggagaaactg gacctgctag gaaatttacc 6000
   tggcagcaag agacggccac aaactcctaa agaaaaggcc aaggctctag aagatctggc 6060
   tggcttcaaa gagctcttcc agacaccagg tcacactgag gaatcaatga ccgatgacaa 6120
   aatcacagaa gtatcctgca aatctccaca accagaccca gtcaaaaccc caacaagctc 6180
   caagcaacga ctcaagatat ccttggggaa agtaggtgtg aaagaagagg tcctaccagt 6240
   cggcaagctc acacagacgt cagggaagac cacacagaca cacagagaga cagcaggaga 6300
   tggaaagagc atcaaagcgt ttaaggaatc tgcaaagcag atgctggacc cagcaaacta 6360
   tggaactggg atggagaggt ggccaagaac acctaaggaa gaggcccaat cactagaaga 6420
   cctggccggc ttcaaagagc tcttccagac accagaccac actgaggaat caacaactga 6480
   tgacaaaact accaaaatag cctgcaaatc tccaccacca gaatcaatgg acactccaac 6540
   aagcacaagg aggcggccca aaacaccttt ggggaaaagg gatatagtgg aagagctctc 6600
   agccctgaag cagctcacac agaccacaca cacagacaaa gtaccaggag atgaggataa 6660
   aggcatcaac gtgttcaggg aaactgcaaa acagaaactg gacccagcag caagtgtaac 6720
   tggtagcaag aggcagccaa gaactcctaa gggaaaagcc caacccctag aagacttggc 6780
   tggcttgaaa gagctcttcc agacaccagt atgcactgac aagcccacga ctcacgagaa 6840
   aactaccaaa atagcctgca gatctccaca accagaccca gtgggtaccc caacaatctt 6900
   caagccacag tccaagagaa gtctcaggaa agcagacgta gaggaagaat ccttagcact 6960
   caggaaacga acaccatcag tagggaaagc tatggacaca cccaaaccag caggaggtga 7020
   tgagaaagac atgaaagcat ttatgggaac tccagtgcag aaattggacc tgccaggaaa 7080
   tttacctggc agcaaaagat ggccacaaac tcctaaggaa aaggcccagg ctctagaaga 7140
   cctggctggc ttcaaagagc tcttccagac accaggcact gacaagccca cgactgatga 7200
   gaaaactacc aaaatagcct gcaaatctcc acaaccagac ccagtggaca ccccagcaag 7260
   cacaaagcaa cggcccaaga gaaacctcag gaaagcagac gtagaggaag aatttttagc 7320
   actcaggaaa cgaacaccat cagcaggcaa agccatggac accccaaaac cagcagtaag 7380
   tgatgagaaa aatatcaaca catttgtgga aactccagtg cagaaactgg acctgctagg 7440
   aaatttacct ggcagcaaga gacagccaca gactcctaag gaaaaggctg aggctctaga 7500
   ggacctggtt ggcttcaaag aactcttcca gacaccaggt cacactgagg aatcaatgac 7560
   tgatgacaaa atcacagaag tatcctgtaa atctccacag ccagagtcat tcaaaacctc 7620
   aagaagctcc aagcaaaggc tcaagatacc cctggtgaaa gtggacatga aagaagagcc 7680
   cctagcagtc agcaagctca cacggacatc aggggagact acgcaaacac acacagagcc 7740
   aacaggagat agtaagagca tcaaagcgtt taaggagtct ccaaagcaga tcctggaccc 7800
   agcagcaagt gtaactggta gcaggaggca gctgagaact cgtaaggaaa aggcccgtgc 7860
   tctagaagac ctggttgact tcaaagagct cttctcagca ccaggtcaca ctgaagagtc 7920
   aatgactatt gacaaaaaca caaaaattcc ctgcaaatct cccccaccag aactaacaga 7980
   cactgccacg agcacaaaga gatgccccaa gacacgtccc aggaaagaag taaaagagga 8040
   gctctcagca gttgagaggc tcacgcaaac atcagggcaa agcacacaca cacacaaaga 8100
   accagcaagc ggtgatgagg gcatcaaagt attgaagcaa cgtgcaaaga agaaaccaaa 8160
   cccagtagaa gaggaaccca gcaggagaag gccaagagca cctaaggaaa aggcccaacc 8220
   cctggaagac ctggccggct tcacagagct ctctgaaaca tcaggtcaca ctcaggaatc 8280
   actgactgct ggcaaagcca ctaaaatacc ctgcgaatct cccccactag aagtggtaga 8340
   caccacagca agcacaaaga ggcatctcag gacacgtgtg cagaaggtac aagtaaaaga 8400
   agagccttca gcagtcaagt tcacacaaac atcaggggaa accacggatg cagacaaaga 8460
   accagcaggt gaagataaag gcatcaaagc attgaaggaa tctgcaaaac agacaccggc 8520
   tccagcagca agtgtaactg gcagcaggag acggccaaga gcacccaggg aaagtgccca 8580
   agccatagaa gacctagctg gcttcaaaga cccagcagca ggtcacactg aagaatcaat 8640
   gactgatgac aaaaccacta aaataccctg caaatcatca ccagaactag aagacaccgc 8700
   aacaagctca aagagacggc ccaggacacg tgcccagaaa gtagaagtga aggaggagct 8760
   gttagcagtt ggcaagctca cacaaacctc aggggagacc acgcacaccg acaaagagcc 8820
   ggtaggtgag ggcaaaggca cgaaagcatt taagcaacct gcaaagcgga acgtggacgc 8880
   agaagatgta attggcagca ggagacagcc aagagcacct aaggaaaagg cccaacccct 8940
   ggaagacctg gccagcttcc aagagctctc tcaaacacca ggccacactg aggaactggc 9000
   aaatggtgct gctgatagct ttacaagcgc tccaaagcaa acacctgaca gtggaaaacc 9060
   tctaaaaata tccagaagag ttcttcgggc ccctaaagta gaacccgtgg gagacgtggt 9120
   aagcaccaga gaccctgtaa aatcacaaag caaaagcaac acttccctgc ccccactgcc 9180
   cttcaagagg ggaggtggca aagatggaag cgtcacggga accaagaggc tgcgctgcat 9240
   gccagcacca gaggaaattg tggaggagct gccagccagc aagaagcaga gggttgctcc 9300
   cagggcaaga ggcaaatcat ccgaacccgt ggtcatcatg aagagaagtt tgaggacttc 9360
   tgcaaaaaga attgaacctg cggaagagct gaacagcaac gacatgaaaa ccaacaaaga 9420
   ggaacacaaa ttacaagact cggtccctga aaataaggga atatccctgc gctccagacg 9480
   ccaagataag actgaggcag aacagcaaat aactgaggtc tttgtattag cagaaagaat 9540
   agaaataaac agaaatgaaa agaagcccat gaagacctcc ccagagatgg acattcagaa 9600
   tccagatgat ggagcccgga aacccatacc tagagacaaa gtcactgaga acaaaaggtg 9660
   cttgaggtct gctagacaga atgagagctc ccagcctaag gtggcagagg agagcggagg 9720
   gcagaagagt gcgaaggttc tcatgcagaa tcagaaaggg aaaggagaag caggaaattc 9780
   agactccatg tgcctgagat caagaaagac aaaaagccag cctgcagcaa gcactttgga 9840
   gagcaaatct gtgcagagag taacgcggag tgtcaagagg tgtgcagaaa atccaaagaa 9900
   ggctgaggac aatgtgtgtg tcaagaaaat aacaaccaga agtcataggg acagtgaaga 9960
   tatttgacag aaaaatcgaa ctgggaaaaa tataataaag ttagttttgt gataagttct 10020
   agtgcagttt ttgtcataaa ttacaagtga attctgtaag taaggctgtc agtctgctta 10080
   agggaagaaa actttggatt tgctgggtct gaatcggctt cataaactcc actgggagca 10140
   ctgctgggct cctggactga gaatagttga acaccggggg ctttgtgaag gagtctgggc 10200
   caaggtttgc cctcagcttt gcagaatgaa gccttgaggt ctgtcaccac ccacagccac 10260
   cctacagcag ccttaactgt gacacttgcc acactgtgtc gtcgtttgtt tgcctatgtt 10320
   ctccagggca cggtggcagg aacaactatc ctcgtctgtc ccaacactga gcaggcactc 10380
   ggtaaacacg aatgaatgga taagcgcacg gatgaatgga gcttacaaga tctgtctttc 10440
   caatggccgg gggcatttgg tccccaaatt aaggctattg gacatctgca caggacagtc 10500
   ctatttttga tgtcctttcc tttctgaaaa taaagttttg tgctttggag aatgactcgt 10560
   gagcacatct ttagggacca agagtgactt tctgtaagga gtgactcgtg gcttgccttg 10620
   gtctcttggg aatacttttc taactagggt tgctctcacc tgagacattc tccacccgcg 10680
   gaatctcagg gtcccaggct gtgggccatc acgacctcaa actggctcct aatctccagc 10740
   tttcctgtca ttgaaagctt cggaagttta ctggctctgc tcccgcctgt tttctttctg 10800
   actctatctg gcagcccgat gccacccagt acaggaagtg acaccagtac tctgtaaagc 10860
   atcatcatcc ttggagagac tgagcactca gcaccttcag ccacgatttc aggatcgctt 10920
   ccttgtgagc cgctgcctcc gaaatctcct ttgaagccca gacatctttc tccagcttca 10980
   gacttgtaga tataactcgt tcatcttcat ttactttcca ctttgccccc tgtcctctct 11040
   gtgttcccca aatcagagaa tagcccgcca tcccccagat cacctgtctg gattcctccc 11100
   cattcaccca ccttgccagg tgcaggtgag gatggtgcac cagacagggt agctgtcccc 11160
   caaaatgtgc cctgtgcggg cagtgccctg tctccacgtt tgtttcccca gtgtctggcg 11220
   gggagccagg tgacatcata aatacttgct gaatgaatgc agaaatcagc ggtactgact 11280
   tgtactatat tggctgccat gatagggttc tcacagcgtc atccatgatc gtaagggaga 11340
   atgacattct gcttgaggga gggaatagaa aggggcaggg aggggacatc tgagggcttc 11400
   acagggctgc aaagggtaca gggattgcac cagggcagaa caggggaggg tgttcaagga 11460
   agagtggctc ttagcagagg cactttggaa ggtgtgaggc ataaatgctt ccttctacgt 11520
   aggccaacct caaaactttc agtaggaatg ttgctatgat caagttgttc taacacttta 11580
   gacttagtag taattatgaa cctcacatag aaaaatttca tccagccata tgcctgtgga 11640
   gtggaatatt ctgtttagta gaaaaatcct ttagagttca gctctaacca gaaatcttgc 11700
   tgaagtatgt cagcaccttt tctcaccctg gtaagtacag tatttcaaga gcacgctaag 11760
   ggtggttttc attttacagg gctgttgatg atgggttaaa aatgttcatt taagggctac 11820
   ccccgtgttt aatagatgaa caccacttct acacaaccct ccttggtact gggggaggga 11880
   gagatctgac aaatactgcc cattccccta ggctgactgg atttgagaac aaatacccac 11940
   ccatttccac catggtatgg taacttctct gagcttcagt ttccaagtga atttccatgt 12000
   aataggacat tcccattaaa tacaagctgt ttttactttt tcgcctccca gggcctgtgc 12060
   gatctggtcc cccagcctct cttgggcttt cttacactaa ctctgtacct accatctcct 12120
   gcctccctta ggcaggcacc tccaaccacc acacactccc tgctgttttc cctgcctgga 12180
   actttcccac cagccccacc aagatcattt catccagtcc tgagctcagc ttaagggagg 12240
   cttcttgcct gtgggttccc tcacccccat gcctgtcctc caggctgggg caggttctta 12300
   gtttgcctgg aattgttctg tacctctttg tagcacgtag tgttgtgaaa ctaagccact 12360
   aattgagttt ctggctcccc tcctggggtt gtaagttttg ttcattcatg agggccgact 12420
   gtatttcctg gttactgtat cccagtgacc agccacagga gatgtccaat aaagtatgtg 12480
   atgaaatggt cttaaaaaaa aaaaaaaaaa aaaaa                            12515
<210> 59
   <211> 416
   <212> DNA
   <213> Artificial Sequence
<220>
   <223> Description of Artificial Sequence:primer
<220>
   <221> misc_feature
   <222> (1) .. (416)
   <223> 3' terminal sequence. pre-b-cell colony-enhancing factor (PBEF) gene.
<400> 59
   aaggccatgt tttatttgct gattaatgga caaaaggcaa tgtaatttat tttcaagtat 60
   tttcttgaaa gtctgtgctc ataaaaatca tgaaaagttg gaaagactgt taaatcactg 120
   aaacttcaaa tatatcttac acaatcttgt ttgtacaaaa atacaagtta aatataaaca 180
   taaagcaatc atggtaattt tatgcaaatc tgttttatgt gatcatcagt tatatataaa 240
   agtttctcag ttctgttatt tgtgaaaaga tcaataccag attgaatgac tacctattgg 300
   caaagggccc taaaaagctt actttaagca ctcatctttt acatggttaa atgcatttcc 360
   taatttgaga tcacctaaac actggaaaag aaaaaaaatg aaagggcagt atgtcc     416
<210> 60
   <211> 500
   <212> DNA
   <213> Artificial Sequence
<220>
   <223> Description of Artificial Sequence:primer
<220>
   <221> misc_feature
   <222> (1) .. (500)
   <223> 5' terminal sequence. pre-b-cell colony-enhancing factor (PBEF) gene.
<400> 60
   atcacagtgg ccacaaattc tagagagcag aagaaaatat tggccaaata tttgttagaa 60
   acttctggta acttagatgg tctggaatac aagttacatg attttggcta cagaggagtc 120
   tcttcccaag agactgctgg cataggagca tctgctcact tggttaactt caaaggaaca 180
   gatacagtag caggacttgc tctaattaaa aaatattatg gaacgaaaga tcctgttcca 240
   ggctattctg ttccagcagc agaacacagt accataacag cttgggggaa agaccatgaa 300
   aaagatgctt ttgaacatat tgtaacacag ttttcatcag tgcctgtatc tgtggtcagc 360
   gatactatgg acatttataa tgcgtgtgag aaaatatggg gtgaagatct aagacattta 420
   atagtatcga gaagtacaca ggcaccacta ataatcagac ctgattctgg aaaccctctt 480
   gacactgtgt taaaggtttc                                             500
<210> 61
   <211> 2376
   <212> DNA/RNA
   <213> Artificial Sequence
<220>
   <223> Description of Artificial Sequence:primer
<220>
   <221> misc_feature
   <222> (1) .. (2376)
   <223> pre-b-cell colony-enhancing factor (PBEF) gene.
<400> 61
   cgcgcggccc ctgtcctccg gcccgagatg aatcctgcgg cagaagccga gttcaacatc 60
   ctcctggcca ccgactccta caaggttact cactataaac aatatccacc caacacaagc 120
   aaagtttatt cctactttga atgccgtgaa aagaagacag aaaactccaa attaaggaag 180
   gtgaaatatg aggaaacagt attttatggg ttgcagtaca ttcttaataa gtacttaaaa 240
   ggtaaagtag taaccaaaga gaaaatccag gaagccaaag atgtctacaa agaacatttc 300
   caagatgatg tctttaatga aaagggatgg aactacattc ttgagaagta tgatgggcat 360
   cttccaatag aaataaaagc tgttcctgag ggctttgtca ttcccagagg aaatgttctc 420
   ttcacggtgg aaaacacaga tccagagtgt tactggctta caaattggat tgagactatt 480
   cttgttcagt cctggtatcc aatcacagtg gccacaaatt ctagagagca gaagaaaata 540
   ttggccaaat atttgttaga aacttctggt aacttagatg gtctggaata caagttacat 600
   gattttggct acagaggagt ctcttcccaa gagactgctg gcataggagc atctgctcac 660
   ttggttaact tcaaaggaac agatacagta gcaggacttg ctctaattaa aaaatattat 720
   ggaacgaaag atcctgttcc aggctattct gttccagcag cagaacacag taccataaca 780
   gcttggggga aagaccatga aaaagatgct tttgaacata ttgtaacaca gttttcatca 840
   gtgcctgtat ctgtggtcag cgatagctat gacatttata atgcgtgtga gaaaatatgg 900
   ggtgaagatc taagacattt aatagtatcg agaagtacac aggcaccact aataatcaga 960
   cctgattctg gaaaccctct tgacactgtg ttaaaggttt tggagatttt aggtaagaag 1020
   tttcctgtta ctgagaactc aaagggttac aagttgctgc caccttatct tagagttatt 1080
   caaggggatg gagtagatat taatacctta caagagattg tagaaggcat gaaacaaaaa 1140
   atgtggagta ttgaaaatat tgccttcggt tctggtggag gtttgctaca gaagttgaca 1200
   agagatctct tgaattgttc cttcaagtgt agctatgttg taactaatgg ccttgggatt 1260
   aacgtcttca aggacccagt tgctgatccc aacaaaaggt ccaaaaaggg ccgattatct 1320
   ttacatagga cgccagcagg gaattttgtt acactggagg aaggaaaagg agaccttgag 1380
   gaatatggtc aggatcttct ccatactgtc ttcaagaatg gcaaggtgac aaaaagctat 1440
   tcatttgatg aaataagaaa aaatgcacag ctgaatattg aactggaagc agcacatcat 1500
   taggctttat gactgggtgt gtgttgtgtg tatgtaatac ataatgttta ttgtacagat 1560
   gtgtggggtt tgtgttttat gatacattac agccaaatta tttgttggtt tatggacata 1620
   ctgccctttc attttttttc ttttccagtg tttaggtgat ctcaaattag gaaatgcatt 1680
   taaccatgta aaagatgagt gctaaagtaa gctttttagg gccctttgcc aataggtagt 1740
   cattcaatct ggtattgatc ttttcacaaa taacagaact gagaaacttt tatatataac 1800
   tgatgatcac ataaaacaga tttgcataaa attaccatga ttgctttatg tttatattta 1860
   acttgtattt ttgtacaaac aagattgtgt aagatatatt tgaagtttca gtgatttaac 1920
   agtctttcca acttttcatg atttttatga gcacagactt tcaagaaaat acttgaaaat 1980
   aaattacatt gccttttgtc cattaatcag caaataaaac atggccttaa caaagttgtt 2040
   tgtgttattg tacaatttga aaattatgtc gggacatacc ctatagaatt actaacctta 2100
   ctgccccttg tagaatatgt attaatcatt ctacattaaa gaaaataatg gttcttactg 2160
   gaatgtctag gcactgtaca gttattatat atcttggttg ttgtattgta ccagtgaaat 2220
   gccaaatttg aaaggcctgt actgcaattt tatatgtcag agattgcctg tggctctaat 2280
   atgcacctca agattttaag gagataatgt ttttagagag aatttctgct tccactatag 2340
   aatatataca taaatgtaaa atacttacaa aagtgg                           2376
<210> 62
   <211> 456
   <212> DNA
   <213> Artificial Sequence
<220>
   <223> Description of Artificial Sequence:primer
<220>
   <221> misc_feature
   <222> (1) .. (456)
   <223> 3' terminal sequence. fatty acid desaturase 2 (FADS2) gene.

<400> 62
   ttttttttga gaataaaatt ccttatttta tttcaaaaaa tgtaggggtg gggaagtaac 60
   atgataaaca ttacgatcag ctccctatgg gttcattctg cctctgcggg ggtcgggggc 120
   atacagtagc tggggggcat gccattgcca tggcaaccca gatgcttaga tgcaggtccc 180
   tcctggctgc ttagagctgg ggggactagg cgccctcccc gaaagccccc attctgagtt 240
   gttggtgcct gcccttcccc tgaatctaag aactgattag tgggttagac tgcaacagca 300
   gctcaggatc ctcccaggga ctttccctcc ctcccctctt cacttggccc gtcccctcag 360
   cttaccagca cctccagccc ccacctcctc ctctttcttc agnttccacc ctggggtcct 420
   tcatgagggt accccttccc cagcccttca gggaag                           456
<210> 63
   <211> 523
   <212> DNA
   <213> Artificial Sequence
<220>
   <223> Description of Artificial Sequence:primer
<220>
   <221> misc_feature
   <222> (1) .. (523)
   <223> 5' terminal sequence. fatty acid desaturase 2 (FADS2) gene.
<400> 63
   ggacatcatc aggtccctga agaagtctgg gaagctgtgg ctggacgcct accttcacaa 60
   atgaagccac agcccccggg acactgtggg gaaggggtgc aggtggggtg atggccagag 120
   gaatgatggg cttttgttct gaggngtgtc cgagaggctg gtgtatgcac tgctcacgga 180
   ccccatgttg gatctttctc cctttctcct ctcctttttc tcttcacatc tcccccatag 240
   caccctgccc tcatgggacc tgccctccct cagccgtcag ccatcagcca tggccctccc 300
   agtgcctcct agccccttct tccaaggagc agagaggtgg ccaccggggg tgtnctngtc 360
   ctacctccac tctctgcccc taaagatggg aggagaccag cggtccatgg gtctggcctg 420
   tgagtctncc cttgcagctg ggtcattagg gatcaacccc gttttgtttt ttcaagatgn 480
   ttttgggggt tcataggggn aggtnctagt tgggnaaggg cct                  523
<210> 64
   <211> 3149
   <212> DNA/RNA
   <213> Artificial Sequence
<220>
   <223> Description of Artificial Sequence:primer
<220>
   <221> misc_feature
   <222> (1) .. (3149)
   <223> fatty acid desaturase 2 (FADS2) gene.
<400> 64
   agggggcgcg gtgggaggag taggagaaga caaaagccga aagcgaagag ggcccgggct 60
   gcacacaccg gctgggaggc agccgtctgt gcagcgagca gccggcgcgg ggaggccgca 120
   gtgcacgggg cgtcacagtc ggcaggcagc atggggaagg gagggaacca gggcgagggg 180
   gccgccgagc gcgaggtgtc ggtgcccacc ttcagctggg aggagattca gaagcataac 240
   ctgcgcaccg acaggtggct ggtcattgac cgcaaggttt acaacatcac caaatggtcc 300
   atccagcacc cgggggccca gcgggtcatc gggcactacg ctggagaaga tgcaacggat 360
   gccttccgcg ccttccaccc tgacctggaa ttcgtgggca agttcttgaa acccctgctg 420
   attggtgaac tggccccgga ggagcccagc caggaccacg gcaagaactc aaagatcact 480
   gaggacttcc gggccctgag gaagacggct gaggacatga acctgttcaa gaccaaccac 540
   gtgttcttcc tcctcctcct ggcccacatc atcgccctgg agagcattgc atggttcact 600
   gtcttttact ttggcaatgg ctggattcct accctcatca cggcctttgt ccttgctacc 660
   tctcaggccc aagctggatg gctgcaacat gattatggcc acctgtctgt ctacagaaaa 720
   cccaagtgga accaccttgt ccacaaattc gtcattggcc acttaaaggg tgcctctgcc 780
   aactggtgga atcatcgcca cttccagcac cacgccaagc ctaacatctt ccacaaggat 840
   cccgatgtga acatgctgca cgtgtttgtt ctgggcgaat ggcagcccat cgagtacggc 900
   aagaagaagc tgaaatacct gccctacaat caccagcacg aatacttctt cctgattggg 960
   ccgccgctgc tcatccccat gtatttccag taccagatca tcatgaccat gatcgtccat 1020
   aagaactggg tggacctggc ctgggccgtc agctactaca tccggttctt catcacctac 1080
   atccctttct acggcatcct gggagccctc cttttcctca acttcatcag gttcctggag 1140
   agccactggt ttgtgtgggt cacacagatg aatcacatcg tcatggagat tgaccaggag 1200
   gcctaccgtg actggttcag tagccagctg acagccacct gcaacgtgga gcagtccttc 1260
   ttcaacgact ggttcagtgg acaccttaac ttccagattg agcaccacct cttccccacc 1320
   atgccccggc acaacttaca caagatcgcc ccgctggtga agtctctatg tgccaagcat 1380
   ggcattgaat accaggagaa gccgctactg agggccctgc tggacatcat caggtccctg 1440
   aagaagtctg ggaagctgtg gctggacgcc taccttcaca aatgaagcca cagcccccgg 1500
   gacaccgtgg ggaaggggtg caggtggggt gatggccaga ggaatgatgg gcttttgttc 1560
   tgaggggtgt ccgagaggct ggtgtatgca ctgctcacgg accccatgtt ggatctttct 1620
   ccctttctcc tctccttttt ctcttcacat ctcccccata gcaccctgcc ctcatgggac 1680
   ctgccctccc tcagccgtca gccatcagcc atggccctcc cagtgcctcc tagccccttc 1740
   ttccaaggag cagagaggtg gccaccgggg gtggctctgt cctacctcca ctctctgccc 1800
   ctaaagatgg gaggagacca gcggtccatg ggtctggcct gtgagtctcc ccttgcagcc 1860
   tggtcactag gcatcacccc cgctttggtt cttcagatgc tcttggggtt cataggggca 1920
   ggtcctagtc gggcagggcc cctgaccctc ccggcctggc ttcactctcc ctgacggctg 1980
   ccattggtcc accctttcat agagaggcct gctttgttac aaagctcggg tctccctcct 2040
   gcagctcggt taagtacccg aggcctctct taagatgtcc agggccccag gcccgcgggc 2100
   acagccagcc caaaccttgg gccctggaag agtcctccac cccatcacta gagtgctctg 2160
   accctgggct ttcacgggcc ccattccacc gcctccccaa cttgagcctg tgaccttggg 2220
   accaaagggg gagtccctcg tctcttgtga ctcagcagag gcagtggcca cgttcaggga 2280
   ggggccggct ggcctggagg ctcagcccac cctccagctt ttcctcaggg tgtcctgagg 2340
   tccaagattc tggagcaatc tgacccttct ccaaaggctc tgttatcagc tgggcagtgc 2400
   cagccaatcc ctggccattt ggccccaggg gacgtgggcc ctgcaggctg caggagggca 2460
   ctggagctgg gaggtctcgt cccagccctc cccatctcgg ggctgctgtg tggacggcgc 2520
   tgcctcaggc actctcctgt ctgaacctgc ccttactgtg tttaacctgt tgctccagga 2580
   tgcattctga taggaggggg cggcagggct gggccttgtg acaatctgcc tttcaccaca 2640
   tggccttgcc tcggtggccc tgactgtcag ggagggccag ggaggcagag cgggagggag 2700
   tctcaggagg aggctgccct gaggggctgg ggagggggta cctcatgagg accagggtgg 2760
   agctgagaag aggaggaggt gggggctgga ggtgctggta gctgagggga cgggcaagtg 2820
   agaggggagg gagggaagtc ctgggaggat cctgagctgc tgttgcagtc taacccacta 2880
   atcagttctt agattcaggg gaagggcagg caccaacaac tcagaatggg ggctttcggg 2940
   gagggcgcct agtcccccca gctctaagca gccaggaggg acctgcatct aagcatctgg 3000
   gttgccatgg caatggcatg ccccccagct actgtatgcc cccgaccccc gcagaggcag 3060
   aatgaaccca tagggagctg atcgtaatgt ttatcatgtt acttccccac ccctacattt 3120
   tttgaaataa aataaggaat tttattctc                                    3149
<210> 65
   <211> 396
   <212> DNA
   <213> Artificial Sequence
<220>
   <223> Description of Artificial Sequence:primer
<220>
   <221> misc_feature
   <222> (1) .. (396)
   <223> 3' terminal sequence. homo sapiens transcribed sequence with weak similarity to protein ref:np_060265.1 (h.sapiens) hypothetical protein flj20378 [homo sapiens] (EST H29301) gene.
<400> 65
   tttttttttt aaatatataa ctatatttat tttgaatatt aaatagtttt taaattacaa 60
   gcaatttatt gaatcacact atgcatcaat atacagtaaa aatcttacaa tttaaaaatg 120
   tacacaattt aaactgaaag ttcattgact attatattgc catgaacctc ttctgcctgt 180
   gttaaagcac agcagggagg cctgggtggg tgtgacggcc ccctacgtcc tcctctgcag 240
   acggaatccg acggtggatc catacaccag ccccatgatc aagatcccgc tcggcccacg 300
   tagggcctga gtgcttaacc agcacgtgtc cacaccgggt caaactgatc ttcacagggc 360
   tgttggggaa ttcaacactg ttcaatggca tctttt                           396
<210> 66
   <211> 516
   <212> DNA
   <213> Artificial Sequence
<220>
   <223> Description of Artificial Sequence:primer
<220>
   <221> misc_feature
   <222> (1) .. (516)
   <223> 5' terminal sequence. homo sapiens transcribed sequence with weak similarity to protein ref:np_060265.1 (h.sapiens) hypothetical protein flj20378 [homo sapiens] (EST H29301) gene.
<400> 66
   attgccaaaa aagcatttta tacattgagt tggggggtag tggatcttag tgtggtgttg 60
   catggagggg cgagatttta tatttataat caacacgtgg gttaacatgt ttttttgaaa 120
   tccaagcaat acacaggaaa tttaagtaga ataaaaattg cagcccattt ttgaaatgtc 180
   agcatgtgct gtgttcagtt caggtttttg ttgtttgttt tgttattttt taactaataa 240
   gttggttatc agtggtgggt tttcaaaatg tacttgttct aataagttgt acaatgaact 300
   aaatcagtgg cattctctag ataatgtggg gggaaggtta gaatattttc tggccttcta 360
   tgggggtagc caccccagga atccaatctg gaattagtcc ctgttttggg tgggagtttg 420
   taccatttta aaccccataa ccaaaaataa tcntagtttt ccattcccct actagcncag 480
   gtgcggantt gtcccttttg nattgacccc ngtcca                           516
<210> 67
   <211> 490
   <212> DNA
   <213> Artificial Sequence
<220>
   <223> Description of Artificial Sequence:primer
<220>
   <221> misc_feature
   <222> (1) .. (490)
   <223> 3' terminal sequence. hypothetical protein flj10193 (FLJ10193) gene.
<400> 67
   tttttttttt tttgacagca gagcataagt ccttttaatt atgtgtttga aaaatgtcac 60
   aagtcaaaaa aggaacacaa ggcaggctcc ggctccctcc acccccgtga gagcccttgt 120
   ccatttcagc cttgcactca gaaagacccc gggggtcttg tagttccacg tgcttcatgt 180
   ttcgtggtat ctgtcagagc cttaaaacag gcccacccac tactgtgaaa tttcaaggaa 240
   ataactgatt cagttaaata acagtcccaa ggtagacctg ggtctcacag gtgaccaccc 300
   gttttaaatc cagaggcctt cttttctgtc caaagccact gaaatttgat ctcctccttc 360
   acacattccc gggnccccaa tatggccacc cacctttntg gacaggtggg ctaacagggt 420
   tttacattaa tgggnaggtt tggttaaaaa acatnttcca ccnaaccttt ttgccaaaga 480
   ggttagttgg                                                        490
<210> 68
   <211> 368
   <212> DNA
   <213> Artificial Sequence
<220>
   <223> Description of Artificial Sequence:primer
<220>
   <221> misc_feature
   <222> (1) .. (368)
   <223> 5' terminal sequence. hypothetical protein flj10193 (FLJ10193) gene.
<400> 68
   tctgcaaaag tacaagagcc tctgcatgtt cgaaatcccc aaggagtaga gtgaggctga 60
   cttccttaga aagaggggga agccaatggc ctgtctcccc actaccatcc ccaaacgctc 120
   cttggggcgt ggttcctgtg gaccccagct cagcacgtca agctgcaggg gcggggctcc 180
   tgtgctgctg cgcgcgcttc gnctgtgcgg gancagcgca gagcttggct gcgcgggggt 240
   tcctcgtgta gatccatatg tctagatgca taataactgg agtgcctgct cgtggaagtc 300
   agaatgctcc tgggaggctg cagagggngt ggaggactct tccctgcctc ttggggaagg 360
   ggccatct                                                          368
<210> 69
   <211> 2222
   <212> DNA/RNA
   <213> Artificial Sequence
<220>
   <223> Description of Artificial Sequence:primer
<220>
   <221> misc_feature
   <222> (1) .. (2222)
   <223> hypothetical protein flj10193 (FLJ10193) gene.
<400> 69
   gtagagtgcg cgacgctttt ggcgacccga cctctggcta acctaccccc ggagccatgg 60
   cctctgctgg ggtggcagcc gggcgacagg cggaggatgt attgccgcca acgtccgacc 120
   agccgctgcc tgacaccaag ccgctgccgc ctcctcagcc gccgccggtc cctgcgcctc 180
   aaccgcagca gtcgccggcg ccacggcctc agtcacctgc ccgcgcgagg gaggaagaga 240
   actactcctt tttacctttg gttcacaaca tcatcaaatg catggacaag gacagcccgg 300
   aggtccacca ggacctgaac gccctcaaaa gcaagttcca ggagatgcgc aagctcatca 360
   gcaccatgcc cggcatccac ctgagccccg aacagcagca gcagcagctg cagagcctcc 420
   gggagcaagt caggaccaag aatgagcttc tgcaaaagta caagagcctc tgcatgttcg 480
   aaatccccaa ggagtagagt gaggctgact tccttagaaa gagggggaag ccaatggcct 540
   gtctccccac taccatcccc aaacgctcct tggggcgtgg ttcctgtgga ccccagctca 600
   gctcgtcaag ctgcaggggc ggggctcctg tgctgctgcg cgcgcttcgc ctgtgcggga 660
   gccagcgcag agcttggctg cgccgggggt tcctcgtgta gatccatatg tctagatgca 720
   taataactgg agtgcctgct ggtggaagtc agaatgctcc tggaggctgc agagggggtg 780
   gaggactctc ccctgcctct ggggaggggg ccatctgctg cgcccggccc cactgacaga 840
   tctgaagagc acagtaggaa gggaggcggc tcctctttgc ttccttccct ctctctcctc 900
   ccacccccat aggatcagtg tgtaccaggt acacattgtt cctgttaaca gcagcttctt 960
   gaaacatttg catagaattc actggacgaa ttaagcctgc actcatatgg catagaattg 1020
   tgagagaatg ttttgaaagg ccagagggtg gcctttttcc ccaaacagtt tggttccttt 1080
   tatgtttgag ccagtgaagg gaactacgct ttgggggctt cagcctagag ccctgccagg 1140
   cagcccctgg ctccaggttc cctgcctcct agcgctctcc tcgccttcag ctcttgctcc 1200
   cttcctcgtt catcaccctc agtcagtgcc caagagtggc caaaccgctt cacatctgca 1260
   gtgcttcccc agggttgaca aggggccgtc ctttccacac aggccagaag aggtcttcag 1320
   gcgaaccgac cttccccctt ctggcatttc agattcccct tgctctggtt aaaaggtctt 1380
   tccctcgtgg cctttgcact tgcggcagca acgtgtacta cactgcagaa gggttcagta 1440
   tgcaccttgt gttgagagag aggcaaccct gggggccagt tcaggtggtc cccaaccata 1500
   agctaggtct gaaagttaca cagccaagtt tgagctctta aaagttgatg aacagcctca 1560
   tttccccagc ttccctgatt tcttccagat gggacgtttt atttgtgtgc tctcccttga 1620
   ctgtcagatt gaagtaagag cagttctctc cgttgcctct cgaggaggag gtgcgaagtc 1680
   ctggagtatt gtttgggtct cggaatgggc gcataacctg cgctgaccag tttaggggct 1740
   tagcagatgc ctgccagctg acctcgttgg caggagggtt gggtggagat gtttttagca 1800
   gagcttccat tagtgtagac ctgtagccac ctgtcagaag gtgggtggca tattggggac 1860
   ctgggaatgt gtgaaggagg agatcaaatt tcagtggctt tggacagaaa agaaggctct 1920
   ggatttaagc gggtggtcac ctgtgagacc aggtctacct tgggactgtt atttaactga 1980
   atcagttatt tccttgaaat ttcacagtag tgggtgggcc tgttttaagg ctctgacaga 2040
   taccacgaaa catgaagcac gtggaactac aagacccccg gggtctttct gagtgcaagg 2100
   ctgaaatgga caagggctcc tcacgggggt ggagggagcc ggagcctgcc ttgtgttcct 2160
   tttttgactt gtgacatttt tcaaacacat aattaaaagg acttatgctc tgctgtctca 2220
   gg                                                                2222
<210> 70
   <211> 214
   <212> DNA
   <213> Artificial Sequence
<220>
   <223> Description of Artificial Sequence:primer
<220>
   <221> misc_feature
   <222> (1) .. (214)
   <223> 3' terminal sequence. estrogen receptor 1 (ESR1) gene.
<400> 70
   tttagttgta attctttatt tgaacatcaa ataggttgag aaaattgttt acaggtgctc 60
   gagcatcccg ctggattctt tttcaaagtg caaaagaggt ttacaagtgt gtttcattaa 120
   acaaagcaaa gctgcgacaa aaccgagtca catcagtaat agtatgcatc ggcaaaaggg 180
   catattaatc catcaaacac aatttggcat ttga                             214
<210> 71
   <211> 520
   <212> DNA
   <213> Artificial Sequence
<220>
   <223> Description of Artificial Sequence:primer
<220>
   <221> misc_feature
   <222> (1) .. (520)
   <223> 5' terminal sequence. estrogen receptor 1 (ESR1) gene.
<400> 71
   tgaaaactta ctctcaactg gagcaaatga actttggtcc caaatatcca tcttttcagt 60
   agcgttaatt atgctctgtt tccaactgca tttcctttcc aattgaatta aagtgtggcc 120
   tcgtttttag tcatttaaaa ttgttttcta agtaattgct gcctctatta tggcacttca 180
   attttgcact gtcttttgag attcaagaaa aatttctatt cttttttttg catccaattg 240
   tgcctgaact tttaaaatat gtaaatgctg ccatgttcca aacccatcgt cagtgtgtgt 300
   gtttagagct gtcaccctag aaacaacata ttgtcccatg agcaggtgcc tgagacacag 360
   acccctttgc attcacagag aggtcattgg ttatagagac ttgaattaat aagtgacatt 420
   atgccagttt ctgttctctc acaggtgata aacaatgctt tttgtgcact acatactctt 480
   cagtgtagag ctcttgtttt atgggaaaag gctcaaatgc                       520
<210> 72
   <211> 6450
   <212> DNA/RNA
   <213> Artificial Sequence
<220>
   <223> Description of Artificial Sequence:primer
<220>
   <221> misc_feature
   <222> (1) .. (6450)
   <223> estrogen receptor 1 (ESR1) gene.
<400> 72
   gagttgtgcc tggagtgatg tttaagccaa tgtcagggca aggcaacagt ccctggccgt 60
   cctccagcac ctttgtaatg catatgagct cgggagacca gtacttaaag ttggaggccc 120
   gggagcccag gagctggcgg agggcgttcg tcctgggagc tgcacttgct ccgtcgggtc 180
   gccggcttca ccggaccgca ggctcccggg gcagggccgg ggccagagct cgcgtgtcgg 240
   cgggacatgc gctgcgtcgc ctctaacctc gggctgtgct ctttttccag gtggcccgcc 300
   ggtttctgag ccttctgccc tgcggggaca cggtctgcac cctgcccgcg gccacggacc 360
   atgaccatga ccctccacac caaagcatct gggatggccc tactgcatca gatccaaggg 420
   aacgagctgg agcccctgaa ccgtccgcag ctcaagatcc ccctggagcg gcccctgggc 480
   gaggtgtacc tggacagcag caagcccgcc gtgtacaact accccgaggg cgccgcctac 540
   gagttcaacg ccgcggccgc cgccaacgcg caggtctacg gtcagaccgg cctcccctac 600
   ggccccgggt ctgaggctgc ggcgttcggc tccaacggcc tggggggttt ccccccactc 660
   aacagcgtgt ctccgagccc gctgatgcta ctgcacccgc cgccgcagct gtcgcctttc 720
   ctgcagcccc acggccagca ggtgccctac tacctggaga acgagcccag cggctacacg 780
   gtgcgcgagg ccggcccgcc ggcattctac aggccaaatt cagataatcg acgccagggt 840
   ggcagagaaa gattggccag taccaatgac aagggaagta tggctatgga atctgccaag 900
   gagactcgct actgtgcagt gtgcaatgac tatgcttcag gctaccatta tggagtctgg 960
   tcctgtgagg gctgcaaggc cttcttcaag agaagtattc aaggacataa cgactatatg 1020
   tgtccagcca ccaaccagtg caccattgat aaaaacagga ggaagagctg ccaggcctgc 1080
   cggctccgca aatgctacga agtgggaatg atgaaaggtg ggatacgaaa agaccgaaga 1140
   ggagggagaa tgttgaaaca caagcgccag agagatgatg gggagggcag gggtgaagtg 1200
   gggtctgctg gagacatgag agctgccaac ctttggccaa gcccgctcat gatcaaacgc 1260
   tctaagaaga acagcctggc cttgtccctg acggccgacc agatggtcag tgccttgttg 1320
   gatgctgagc cccccatact ctattccgag tatgatccta ccagaccctt cagtgaagct 1380
   tcgatgatgg gcttactgac caacctggca gacagggagc tggttcacat gatcaactgg 1440
   gcgaagaggg tgccaggctt tgtggatttg accctccatg atcaggtcca ccttctagaa 1500
   tgtgcctggc tagagatcct gatgattggt ctcgtctggc gctccatgga gcacccagtg 1560
   aagctactgt ttgctcctaa cttgctcttg gacaggaacc agggaaaatg tgtagagggc 1620
   atggtggaga tcttcgacat gctgctggct acatcatctc ggttccgcat gatgaatctg 1680
   cagggagagg agtttgtgtg cctcaaatct attattttgc ttaattctgg agtgtacaca 1740
   tttctgtcca gcaccctgaa gtctctggaa gagaaggacc atatccaccg agtcctggac 1800
   aagatcacag acactttgat ccacctgatg gccaaggcag gcctgaccct gcagcagcag 1860
   caccagcggc tggcccagct cctcctcatc ctctcccaca tcaggcacat gagtaacaaa 1920
   ggcatggagc atctgtacag catgaagtgc aagaacgtgg tgcccctcta tgacctgctg 1980
   ctggagatgc tggacgccca ccgcctacat gcgcccacta gccgtggagg ggcatccgtg 2040
   gaggagacgg accaaagcca cttggccact gcgggctcta cttcatcgca ttccttgcaa 2100
   aagtattaca tcacggggga ggcagagggt ttccctgcca cagtctgaga gctccctggc 2160
   tcccacacgg ttcagataat ccctgctgca ttttaccctc atcatgcacc actttagcca 2220
   aattctgtct cctgcataca ctccggcatg catccaacac caatggcttt ctagatgagt 2280
   ggccattcat ttgcttgctc agttcttagt ggcacatctt ctgtcttctg ttgggaacag 2340
   ccaaagggat tccaaggcta aatctttgta acagctctct ttcccccttg ctatgttact 2400
   aagcgtgagg attcccgtag ctcttcacag ctgaactcag tctatgggtt ggggctcaga 2460
   taactctgtg catttaagct acttgtagag acccaggcct ggagagtaga cattttgcct 2520
   ctgataagca ctttttaaat ggctctaaga ataagccaca gcaaagaatt taaagtggct 2580
   cctttaattg gtgacttgga gaaagctagg tcaagggttt attatagcac cctcttgtat 2640
   tcctatggca atgcatcctt ttatgaaagt ggtacacctt aaagctttta tatgactgta 2700
   gcagagtatc tggtgattgt caattcactt ccccctatag gaatacaagg ggccacacag 2760
   ggaaggcaga tcccctagtt ggccaagact tattttaact tgatacactg cagattcaga 2820
   gtgtcctgaa gctctgcctc tggctttccg gtcatgggtt ccagttaatt catgcctccc 2880
   atggacctat ggagagcaac aagttgatct tagttaagtc tccctatatg agggataagt 2940
   tcctgatttt tgtttttatt tttgtgttac aaaagaaagc cctccctccc tgaacttgca 3000
   gtaaggtcag cttcaggacc tgttccagtg ggcactgtac ttggatcttc ccggcgtgtg 3060
   tgtgccttac acaggggtga actgttcact gtggtgatgc atgatgaggg taaatggtag 3120
   ttgaaaggag caggggccct ggtgttgcat ttagccctgg ggcatggagc tgaacagtac 3180
   ttgtgcagga ttgttgtggc tactagagaa caagagggaa agtagggcag aaactggata 3240
   cagttctgag cacagccaga cttgctcagg tggccctgca caggctgcag ctacctagga 3300
   acattccttg cagaccccgc attgcctttg ggggtgccct gggatccctg gggtagtcca 3360
   gctcttattc atttcccagc gtggccctgg ttggaagaag cagctgtcaa gttgtagaca 3420
   gctgtgttcc tacaattggc ccagcaccct ggggcacggg agaagggtgg ggaccgttgc 3480
   tgtcactact caggctgact ggggcctggt cagattacgt atgcccttgg tggtttagag 3540
   ataatccaaa atcagggttt ggtttgggga agaaaatcct cccccttcct cccccgcccc 3600
   gttccctacc gcctccactc ctgccagctc atttccttca atttcctttg acctataggc 3660
   taaaaaagaa aggctcattc cagccacagg gcagccttcc ctgggccttt gcttctctag 3720
   cacaattatg ggttacttcc tttttcttaa caaaaaagaa tgtttgattt cctctgggtg 3780
   accttattgt ctgtaattga aaccctattg agaggtgatg tctgtgttag ccaatgaccc 3840
   aggtagctgc tcgggcttct cttggtatgt cttgtttgga aaagtggatt tcattcattt 3900
   ctgattgtcc agttaagtga tcaccaaagg actgagaatc tgggagggca aaaaaaaaaa 3960
   aaaaagtttt tatgtgcact taaatttggg gacaatttta tgtatctgtg ttaaggatat 4020
   gcttaagaac ataattcttt tgttgctgtt tgtttaagaa gcaccttagt ttgtttaaga 4080
   agcaccttat atagtataat atatattttt ttgaaattac attgcttgtt tatcagacaa 4140
   ttgaatgtag taattctgtt ctggatttaa tttgactggg ttaacatgca aaaaccaagg 4200
   aaaaatattt agtttttttt tttttttttg tatacttttc aagctacctt gtcatgtata 4260
   cagtcattta tgcctaaagc ctggtgatta ttcatttaaa tgaagatcac atttcatatc 4320
   aacttttgta tccacagtag acaaaatagc actaatccag atgcctattg ttggatattg 4380
   aatgacagac aatcttatgt agcaaagatt atgcctgaaa aggaaaatta ttcagggcag 4440
   ctaattttgc ttttaccaaa atatcagtag taatattttt ggacagtagc taatgggtca 4500
   gtgggttctt tttaatgttt atacttagat tttcttttaa aaaaattaaa ataaaacaaa 4560
   aaaaatttct aggactagac gatgtaatac cagctaaagc caaacaatta tacagtggaa 4620
   ggttttacat tattcatcca atgtgtttct attcatgtta agatactact acatttgaag 4680
   tgggcagaga acatcagatg attgaaatgt tcgcccaggg gtctccagca actttggaaa 4740
   tctctttgta tttttacttg aagtgccact aatggacagc agatattttc tggctgatgt 4800
   tggtattggg tgtaggaaca tgatttaaaa aaaaaactct tgcctctgct ttcccccact 4860
   ctgaggcaag ttaaaatgta aaagatgtga tttatctggg gggctcaggt atggtgggga 4920
   agtggattca ggaatctggg gaatggcaaa tatattaaga agagtattga aagtatttgg 4980
   aggaaaatgg ttaattctgg gtgtgcacca aggttcagta gagtccactt ctgccctgga 5040
   gaccacaaat caactagctc catttacagc catttctaaa atggcagctt cagttctaga 5100
   gaagaaagaa caacatcagc agtaaagtcc atggaatagc tagtggtctg tgtttctttt 5160
   cgccattgcc tagcttgccg taatgattct ataatgccat catgcagcaa ttatgagagg 5220
   ctaggtcatc caaagagaag accctatcaa tgtaggttgc aaaatctaac ccctaaggaa 5280
   gtgcagtctt tgatttgatt tccctagtaa ccttgcagat atgtttaacc aagccatagc 5340
   ccatgccttt tgagggctga acaaataagg gacttactga taatttactt ttgatcacat 5400
   taaggtgttc tcaccttgaa atcttataca ctgaaatggc cattgattta ggccactggc 5460
   ttagagtact ccttcccctg catgacactg attacaaata ctttcctatt catactttcc 5520
   aattatgaga tggactgtgg gtactgggag tgatcactaa caccatagta atgtctaata 5580
   ttcacaggca gatctgcttg gggaagctag ttatgtgaaa ggcaaataaa gtcatacagt 5640
   agctcaaaag gcaaccataa ttctctttgg tgcaagtctt gggagcgtga tctagattac 5700
   actgcaccat tcccaagtta atcccctgaa aacttactct caactggagc aaatgaactt 5760
   tggtcccaaa tatccatctt ttcagtagcg ttaattatgc tctgtttcca actgcatttc 5820
   ctttccaatt gaattaaagt gtggcctcgt ttttagtcat ttaaaattgt tttctaagta 5880
   attgctgcct ctattatggc acttcaattt tgcactgtct tttgagattc aagaaaaatt 5940
   tctattcatt tttttgcatc caattgtgcc tgaactttta aaatatgtaa atgctgccat 6000
   gttccaaacc catcgtcagt gtgtgtgttt agagctgtgc accctagaaa caacatactt 6060
   gtcccatgag caggtgcctg agacacagac ccctttgcat tcacagagag gtcattggtt 6120
   atagagactt gaattaataa gtgacattat gccagtttct gttctctcac aggtgataaa 6180
   caatgctttt tgtgcactac atactcttca gtgtagagct cttgttttat gggaaaaggc 6240
   tcaaatgcca aattgtgttt gatggattaa tatgcccttt tgccgatgca tactattact 6300
   gatgtgactc ggttttgtcg cagctttgct ttgtttaatg aaacacactt gtaaacctct 6360
   tttgcacttt gaaaaagaat ccagcgggat gctcgagcac ctgtaaacaa ttttctcaac 6420
   ctatttgatg ttcaaataaa gaattaaact                                  6450
<210> 73
   <211> 205
   <212> DNA
   <213> Artificial Sequence
<220>
   <223> Description of Artificial Sequence:primer
<220>
   <221> misc_feature
   <222> (1) .. (205)
   <223> 3' terminal sequence. v-erb-b2 erythroblastic leukemia viral oncogene homolog 2, neuro/glioblastoma derived oncogene homolog (avian) (ERBB2) gene.
<400> 73
   ccccctgggt ctttatttca tctttaaaaa aacaaaacaa aaaaagtaaa aactaaacag 60
   aaaagcactc tgtacaaagc ctggatactg acaccattgc tgttccttcc tcatgggggg 120
   cagtactagg tttcagggac agtctctgaa tgggtcgctt ttgttcttag acactccctt 180
   agggccgctt cccctctcag gccag                                       205
<210> 74
   <211> 238
   <212> DNA
   <213> Artificial Sequence
<220>
   <223> Description of Artificial Sequence:primer
<220>
   <221> misc_feature
   <222> (1) .. (238)
   <223> 5' terminal sequence. v-erb-b2 erythroblastic leukemia viral oncogene homolog 2, neuro/glioblastoma derived oncogene homolog (avian) (ERBB2) gene.
<400> 74
   cctcgtcgcc tcgtgcgaag ccttagggaa gctggcctga gaggggaagc ggccctaagg 60
   gagtgtctaa gaacaaaagc gacccattca gagaccgtcc ctgaaaccta gtactgcccc 120
   ccatgaggaa ggaacagcaa tggtgtcagt atccaggcac tgtacagagt gcttttctgt 180
   ttagttttta ctttttttgt tttgtttttt taaagatgaa ataaagaccc agggggag   238
<210> 75
   <211> 4530
   <212> DNA/RNA
   <213> Artificial Sequence
<220>
   <223> Description of Artificial Sequence:primer
<220>
   <221> misc_feature
   <222> (1) .. (4530)
   <223> v-erb-b2 erythroblastic leukemia viral oncogene homolog 2, neuro/glioblastoma derived oncogene homolog (avian) (ERBB2) gene.
<400> 75
   aattctcgag ctcgtcgacc ggtcgacgag ctcgagggtc gacgagctcg agggcgcgcg 60
   cccggccccc acccctcgca gcaccccgcg ccccgcgccc tcccagccgg gtccagccgg 120
   agccatgggg ccggagccgc agtgagcacc atggagctgg cggccttgtg ccgctggggg 180
   ctcctcctcg ccctcttgcc ccccggagcc gcgagcaccc aagtgtgcac cggcacagac 240
   atgaagctgc ggctccctgc cagtcccgag acccacctgg acatgctccg ccacctctac 300
   cagggctgcc aggtggtgca gggaaacctg gaactcacct acctgcccac caatgccagc 360
   ctgtccttcc tgcaggatat ccaggaggtg cagggctacg tgctcatcgc tcacaaccaa 420
   gtgaggcagg tcccactgca gaggctgcgg attgtgcgag gcacccagct ctttgaggac 480
   aactatgccc tggccgtgct agacaatgga gacccgctga acaataccac ccctgtcaca 540
   ggggcctccc caggaggcct gcgggagctg cagcttcgaa gcctcacaga gatcttgaaa 600
   ggaggggtct tgatccagcg gaacccccag ctctgctacc aggacacgat tttgtggaag 660
   gacatcttcc acaagaacaa ccagctggct ctcacactga tagacaccaa ccgctctcgg 720
   gcctgccacc cctgttctcc gatgtgtaag ggctcccgct gctggggaga gagttctgag 780
   gattgtcaga gcctgacgcg cactgtctgt gccggtggct gtgcccgctg caaggggcca 840
   ctgcccactg actgctgcca tgagcagtgt gctgccggct gcacgggccc caagcactct 900
   gactgcctgg cctgcctcca cttcaaccac agtggcatct gtgagctgca ctgcccagcc 960
   ctggtcacct acaacacaga cacgtttgag tccatgccca atcccgaggg ccggtataca 1020
   ttcggcgcca gctgtgtgac tgcctgtccc tacaactacc tttctacgga cgtgggatcc 1080
   tgcaccctcg tctgccccct gcacaaccaa gaggtgacag cagaggatgg aacacagcgg 1140
   tgtgagaagt gcagcaagcc ctgtgcccga gtgtgctatg gtctgggcat ggagcacttg 1200
   cgagaggtga gggcagttac cagtgccaat atccaggagt ttgctggctg caagaagatc 1260
   tttgggagcc tggcatttct gccggagagc tttgatgggg acccagcctc caacactgcc 1320
   ccgctccagc cagagcagct ccaagtgttt gagactctgg aagagatcac aggttaccta 1380
   tacatctcag catggccgga cagcctgcct gacctcagcg tcttccagaa cctgcaagta 1440
   atccggggac gaattctgca caatggcgcc tactcgctga ccctgcaagg gctgggcatc 1500
   agctggctgg ggctgcgctc actgagggaa ctgggcagtg gactggccct catccaccat 1560
   aacacccacc tctgcttcgt gcacacggtg ccctgggacc agctctttcg gaacccgcac 1620
   caagctctgc tccacactgc caaccggcca gaggacgagt gtgtgggcga gggcctggcc 1680
   tgccaccagc tgtgcgcccg agggcactgc tggggtccag ggcccaccca gtgtgtcaac 1740
   tgcagccagt tccttcgggg ccaggagtgc gtggaggaat gccgagtact gcaggggctc 1800
   cccagggagt atgtgaatgc caggcactgt ttgccgtgcc accctgagtg tcagccccag 1860
   aatggctcag tgacctgttt tggaccggag gctgaccagt gtgtggcctg tgcccactat 1920
   aaggaccctc ccttctgcgt ggcccgctgc cccagcggtg tgaaacctga cctctcctac 1980
   atgcccatct ggaagtttcc agatgaggag ggcgcatgcc agccttgccc catcaactgc 2040
   acccactcct gtgtggacct ggatgacaag ggctgccccg ccgagcagag agccagccct 2100
   ctgacgtcca tcgtctctgc ggtggttggc attctgctgg tcgtggtctt gggggtggtc 2160
   tttgggatcc tcatcaagcg acggcagcag aagatccgga agtacacgat gcggagactg 2220
   ctgcaggaaa cggagctggt ggagccgctg acacctagcg gagcgatgcc caaccaggcg 2280
   cagatgcgga tcctgaaaga gacggagctg aggaaggtga aggtgcttgg atctggcgct 2340
   tttggcacag tctacaaggg catctggatc cctgatgggg agaatgtgaa aattccagtg 2400
   gccatcaaag tgttgaggga aaacacatcc cccaaagcca acaaagaaat cttagacgaa 2460
   gcatacgtga tggctggtgt gggctcccca tatgtctccc gccttctggg catctgcctg 2520
   acatccacgg tgcagctggt gacacagctt atgccctatg gctgcctctt agaccatgtc 2580
   cgggaaaacc gcggacgcct gggctcccag gacctgctga actggtgtat gcagattgcc 2640
   aaggggatga gctacctgga ggatgtgcgg ctcgtacaca gggacttggc cgctcggaac 2700
   gtgctggtca agagtcccaa ccatgtcaaa attacagact tcgggctggc tcggctgctg 2760
   gacattgacg agacagagta ccatgcagat gggggcaagg tgcccatcaa gtggatggcg 2820
   ctggagtcca ttctccgccg gcggttcacc caccagagtg atgtgtggag ttatggtgtg 2880
   actgtgtggg agctgatgac ttttggggcc aaaccttacg atgggatccc agcccgggag 2940
   atccctgacc tgctggaaaa gggggagcgg ctgccccagc cccccatctg caccattgat 3000
   gtctacatga tcatggtcaa atgttggatg attgactctg aatgtcggcc aagattccgg 3060
   gagttggtgt ctgaattctc ccgcatggcc agggaccccc agcgctttgt ggtcatccag 3120
   aatgaggact tgggcccagc cagtcccttg gacagcacct tctaccgctc actgctggag 3180
   gacgatgaca tgggggacct ggtggatgct gaggagtatc tggtacccca gcagggcttc 3240
   ttctgtccag accctgcccc gggcgctggg ggcatggtcc accacaggca ccgcagctca 3300
   tctaccagga gtggcggtgg ggacctgaca ctagggctgg agccctctga agaggaggcc 3360
   cccaggtctc cactggcacc ctccgaaggg gctggctccg atgtatttga tggtgacctg 3420
   ggaatggggg cagccaaggg gctgcaaagc ctccccacac atgaccccag ccctctacag 3480
   cggtacagtg aggaccccac agtacccctg ccctctgaga ctgatggcta cgttgccccc 3540
   ctgacctgca gcccccagcc tgaatatgtg aaccagccag atgttcggcc ccagccccct 3600
   tcgccccgag agggccctct gcctgctgcc cgacctgctg gtgccactct ggaaagggcc 3660
   aagactctct ccccagggaa gaatggggtc gtcaaagacg tttttgcctt tgggggtgcc 3720
   gtggagaacc ccgagtactt gacaccccag ggaggagctg cccctcagcc ccaccctcct 3780
   cctgccttca gcccagcctt cgacaacctc tattactggg accaggaccc accagagcgg 3840
   ggggctccac ccagcacctt caaagggaca cctacggcag agaacccaga gtacctgggt 3900
   ctggacgtgc cagtgtgaac cagaaggcca agtccgcaga agccctgatg tgtcctcagg 3960
   gagcagggaa ggcctgactt ctgctggcat caagaggtgg gagggccctc cgaccacttc 4020
   caggggaacc tgccatgcca ggaacctgtc ctaaggaacc ttccttcctg cttgagttcc 4080
   cagatggctg gaaggggtcc agcctcgttg gaagaggaac agcactgggg agtctttgtg 4140
   gattctgagg ccctgcccaa tgagactcta gggtccagtg gatgccacag cccagcttgg 4200
   ccctttcctt ccagatcctg ggtactgaaa gccttaggga agctggcctg agaggggaag 4260
   cggccctaag ggagtgtcta agaacaaaag cgacccattc agagactgtc cctgaaacct 4320
   agtactgccc cccatgagga aggaacagca atggtgtcag tatccaggct ttgtacagag 4380
   tgcttttctg tttagttttt actttttttg ttttgttttt ttaaagacga aataaagacc 4440
   caggggagaa tgggtgttgt atggggaggc aagtgtgggg ggtccttctc cacacccact 4500
   ttgtccattt gcaaatatat tttggaaaac                                  4530
<210> 76
   <211> 535
   <212> DNA
   <213> Artificial Sequence
<220>
   <223> Description of Artificial Sequence:primer
<220>
   <221> misc_feature
   <222> (1) .. (535)
   <223> 3' terminal sequence. v-erb-b2 erythroblastic leukemia viral oncogene homolog 2, neuro/glioblastoma derived oncogene homolog (avian) (ERBB2) gene.
<400> 76
   tttttttttt tttttttttt tttttttttt atttcatctt taaaaaaaca aaacaaaaaa 60
   agtaaaaact aaacagaaaa gcactctgta caaagcctgg atactgacac cattgctgtt 120
   ccttcctcat ggggggcagt actaggtttc agggacagtc tctgaatggg tcgcttttgt 180
   tcttagacac tcccttaggg ccgcttcccc tctcaggcca gcttccctaa ggctttcagt 240
   acccaggatc tggaaggaaa gggccaagct gggctgtggc atccactgga ccctagagtc 300
   tcattgggca gggctcagaa tccacaaaga ctccccagtg ctgttcctct tccaacgagg 360
   ctggacccct tccagccatc tgggaactca agcaggaaag aaggttcctt aggacaggtt 420
   cctggcatgg caggttcccc tggaaattgt cggagggccc ccccaactct tgatgccaac 480
   agaagtcagg cctttcctgg tccctgagga cacataaggg ttcttgggat ttggc      535
<210> 77
   <211> 544
   <212> DNA
   <213> Artificial Sequence
<220>
   <223> Description of Artificial Sequence:primer
<220>
   <221> misc_feature
   <222> (1) .. (544)
   <223> 5' terminal sequence. v-erb-b2 erythroblastic leukemia viral oncogene homolog 2, neuro/glioblastoma derived oncogene homolog (avian) (ERBB2) gene.
<400> 77
   agagtacctg ggtctggacg tgccagtgtg aaccagaagg ccaagtccgc agaagccctg 60
   atgtgtcctc agggagccgg gaaggcctga cttctgctgg catcaagagg tgggagggcc 120
   ctccgaccac ttccagggga acctgccatg ccaggaacct gtcctaagga accttccttc 180
   ctgcttgagt tcccagatgg ctggaagggg tccagcctcg ttggaagagg aacagcactg 240
   gggagtcttt gtggattctg aggccctgcc caatgagact ctagggtcca gtggatgcca 300
   cagcccagct tggccctttc cttccagatc ctgggtactg aaagccttag ggaagctggc 360
   ctgagagggg aagcggccct aagggagtgt ctaagaacaa aagcgaccca ttcagagact 420
   gtccctgaaa cctagtactg ccccccatga ggaaggaaca gcaatggtgt cagtatccag 480
   gctttgtaca gagtgctttt ctgtttagtt tttacttttt ttgttttgtg tttttaaaga 540
   tgaa                                                              544
<210> 78
   <211> 322
   <212> DNA
   <213> Artificial Sequence
<220>
   <223> Description of Artificial Sequence:primer
<220>
   <221> misc_feature
   <222> (1) .. (322)
   <223> 3' terminal sequence. gata binding protein 4 (GATA4) gene.
<400> 78
   tttgaggaat acagataaat ttattagtta aatactgatt ttccagccat ttcaccttaa 60
   gacaatgtta acaggtttgt gggttatgga gggtatacga gggggccttt ggaagaaaac 120
   aatgtaaatg atgattaaaa cagaatcttg gttcaaaggt attctctgct acagccagta 180
   ggattttgga gtgaggggtc tgggcgtgtg gggaggcgta gtaatgccac agtcagctac 240
   agctctgctg agaaagagga aggagtctcc ttgagctcca gcatcagggg cagaaacagc 300
   aatgtgcaga ggagaacgcg gc                                          322
<210> 79
   <211> 424
   <212> DNA
   <213> Artificial Sequence
<220>
   <223> Description of Artificial Sequence:primer
<220>
   <221> misc_feature
   <222> (1) .. (424)
   <223> 5' terminal sequence. gata binding protein 4 (GATA4) gene.
<400> 79
   gcccgaatga cggcatctgt ttgccatgta cctggatgtg acgggcccct ggggacaggc 60
   ccttgcccca tccatccgct tgaggcatgg caccgccatg catccctaat accaaatctg 120
   actccaaaac tgtggggtgt gacacacaag tgactgaaca cttcctgggg agctacaggg 180
   gcacttaacc caccacagcg cacctcatca aaatgcagct ggcaacttct cccccaggtg 240
   ccttccccct gctgccggcc tttgctcctt cacttccaac atctctcaaa ataaaaatcc 300
   ctcttcccgc tctgagcgat tcagctctgc ccgcagcttg tacatgtctc tcccctggca 360
   aaacaagagc tgggtagttt agccaaacgg caccccctcg agttcactgc agacccttcg 420
   ttca                                                              424
<210> 80
   <211> 2226
   <212> DNA/RNA
   <213> Artificial Sequence
<220>
   <223> Description of Artificial Sequence:primer
<220>
   <221> misc feature
   <222> (1)..(2226)
   <223> gata binding protein 4 (GATA4) gene.
<400> 80
   accaccaaaa attcaaattg ggattttccg gagtaaacaa gagcctagag ccctttgctc 60
   aatgctggat ttaatacgta tatattttta agcgagttgg ttttttcccc tttgattttt 120
   gatcttcgcg acagttcctc ccacgcatat tatcgttgtt gccgtcgttt tctctccccg 180
   cgtggctcct tgacctgcga gggagagaga ggacaccgaa gccgggagct cgcagggacc 240
   atgtatcaga gcttggccat ggccgccaac cacgggccgc cccccggtgc ctaccaggcg 300
   ggcggccccg gccccttcat gcacggcgcg ggcgccgcgt cctcgccagt ctacctgccc 360
   acaccgcggg tgccctcctc cgttctgggc ctgtcctacc tccagggcgg aggcgcgggc 420
   tctgcgtccg gaggcccctc gggcggcagc cccggtgggg ccgcgtctgg tgcggggccc 480
   gggacccagc agggcagccc gggatggagc caggcgggag cgaccggagc cgcttacacc 540
   ccgccgccgg tgtcgccgcg cttctccttc ccggggacca ccgggtccct ggcggcggcg 600
   gcggcggctg ccgccgcccg ggaagctgcg gcctacagca gtggcggcgg agcggcgggt 660
   gcgggcctgg cgggccgcga gcagtacggg cgcgccggct tcgcgggctc ctactccagc 720
   ccctacccgg cttacatggc cgacgtgggc gcgtcctggg ccgcagccgc cgccgcctcc 780
   gccggcccct tcgacagccc ggtcctgcac agcctgcccg gccgggccaa cccggccgcc 840
   cgacacccca atctcgatat gtttgacgac ttctcagaag gcagagagtg tgtcaactgt 900
   ggggctatgt ccaccccgct ctggaggcga gatgggacgg gtcactatct gtgcaacgcc 960
   tgtggcctct accacaagat gaacggcatc aaccggccgc tcatcaagcc tcagcgccgg 1020
   ctgtccgcct cccgccgagt gggcctctcc tgtgccaact gccagaccac caccaccacg 1080
   ctgtggcgcc gcaatgcgga gggcgagcct gtgtgcaatg cctgcggcct ctacatgaag 1140
   ctccacgggg tgcccaggcc tcttgcaatg cggaaagagg ggatccaaac cagaaaacgg 1200
   aagcccaaga acctgaataa atctaagaca ccagcagctc cttcaggcag tgagagcctt 1260
   cctcccgcca gcggtgcttc cagcaactcc agcaacgcca ccaccagcag cagcgaggag 1320
   atgcgtccca tcaagacgga gcctggcctg tcatctcact acgggcacag cagctccgtg 1380
   tcccagacgt tctcagtcag tgcgatgtct ggccatgggc cctccatcca ccctgtcctc 1440
   tcggccctga agctctcccc acaaggctat gcgtctcccg tcagccagtc tccacagacc 1500
   agctccaagc aggactcttg gaacagtctg gtcttggccg acagtcacgg ggacataatc 1560
   actgcgtaat cttccctctt ccctcctcaa attcctgcac ggacctggga cttggaggat 1620
   agcaaagaag gaggccctgg gctcccaggg gccggcctcc tctgcctggt aatgactcca 1680
   gaacaacaac tgggaagaaa cttgaagtcg acaatctggt taggggaagc gggtgttgga 1740
   ttttctcaga tgcctttaca cgctgatggg actggaggga gcccaccctt cagcacgagc 1800
   acactgcatc tctcctgtga gttggagact tctttcccaa gatgtccttg tcccctgcgt 1860
   tccccactgt ggcctagacc gtgggttttg cattgtgttt ctagcaccga aggatctgag 1920
   aacaagcgga gggccgggcc ctgggacccc tgctccagcc cgaatgacgg catctgtttg 1980
   ccatgtacct ggatgtgacg ggcccctggg gacaggccct tgccccatcc atccgcttga 2040
   ggcatggcac cgccctgcat ccctaatacc aaatctgact ccaaaactgt ggggtgtgac 2100
   acacaagtga ctgagcactt cctggggagc tacaggggca cttaacccac cacagcgcag 2160
   cctcatcaaa atgcagctgg caacttctcc cccaggtgcc ttccccctgc tgccggcctt 2220
   tgctcc                                                            2226
<210> 81
   <211> 513
   <212> DNA
   <213> Artificial Sequence
<220>
   <223> Description of Artificial Sequence:primer
<220>
   <221> misc_feature
   <222> (1)..(513)
   <223> 3' terminal sequence. lysyl oxidase (LOX) gene.
<400> 81
   gcgccgcagt tactgagcgc aggaacttct cccggcgctg tctggttctc cgcgcgcgag 60
   gcgagcttcg cggctctaga tgtcgagtag ccagcttgga accagtgacg ggcggtgggc 120
   ctggggcggc cagcggtgac tccagatgag ccggccgtcc gcgttcgccc gcgcggtgcg 180
   gttgtcgcgg atcagcagga tcggagtgcg gggctgctgg gcggaggcgt tggctgcacc 240
   agggacggcg gcgcctgggt cccggcggcg ctgaggctgg tactgtgagc ccaggctcag 300
   caagctgaac acctgcccgt tgttctccca ttggatctgc tggcgccagg ccgcggagcc 360
   gccgcgctca gcgcgggggg ctgctgttgg ccggcgcggt gaagggccca gtgcactagc 420
   gcgcaaactg caaaggcccg agcaggagca cgggtcaggc gacgcattca ttccttgttc 480
   cagattgacc ccgttcgaag aagacctggc tca                              513
<210> 82
   <211> 1946
   <212> DNA/RNA
   <213> Artificial Sequence
<220>
   <223> Description of Artificial Sequence:primer
<220>
   <221> misc_feature
   <222> (1)..(1946)
   <223> lysyl oxidase (LOX) gene.
<400> 82
   agacactgcc cgctctccgg gactccgcgc cgctccccgt tgccttccag gactgagaaa 60
   ggggaaaggg aagggtgcca cgtccgagca gccgccttga ctggggaagg gtctgaatcc 120
   cacccttggc attgcttggt ggagactgag atacccgtgc tccgctcgcc tccttggttg 180
   aagatttctc cttccctcac gtgatttgag ccccgttttt attttctgtg agccacgtcc 240
   tcctcgagcg gggtcaatct ggcaaaagga gtgatgcgct tcgcctggac cgtgctcctg 300
   ctcgggcctt tgcagctctg cgcgctagtg cactgcgccc ctcccgccgc cggccaacag 360
   cagcccccgc gcgagccgcc ggcggctccg ggcgcctggc gccagcagat ccaatgggag 420
   aacaacgggc aggtgttcag cttgctgagc ctgggctcac agtaccagcc tcagcgccgc 480
   cgggacccgg gcgccgccgt ccctggtgca gccaacgcct ccgcccagca gccccgcact 540
   ccgatcctgc tgatccgcga caaccgcacc gccgcggcgc gaacgcggac ggccggctca 600
   tctggagtca ccgctggccg ccccaggccc accgcccgtc actggttcca agctggctac 660
   tcgacatcta gagcccgcga agctggcgcc tcgcgcgcgg agaaccagac agcgccggga 720
   gaagttcctg cgctcagtaa cctgcggccg cccagccgcg tggacggcat ggtgggcgac 780
   gacccttaca acccctacaa gtactctgac gacaaccctt attacaacta ctacgatact 840
   tatgaaaggc ccagacctgg gggcaggtac cggcccggat acggcactgg ctacttccag 900
   tacggtctcc cagacctggt ggccgacccc tactacatcc aggcgtccac gtacgtgcag 960
   aagatgtcca tgtacaacct gagatgcgcg gcggaggaaa actgtctggc cagtacagca 1020
   tacagggcag atgtcagaga ttatgatcac agggtgctgc tcagatttcc ccaaagagtg 1080
   aaaaaccaag ggacatcaga tttcttaccc agccgaccaa gatattcctg ggaatggcac 1140
   agttgtcatc aacattacca cagtatggat gagtttagcc actatgacct gcttgatgcc 1200
   aacacccaga ggagagtggc tgaaggccac aaagcaagtt tctgtcttga agacacatcc 1260
   tgtgactatg gctaccacag gcgatttgca tgtactgcac acacacaggg attgagtcct 1320
   ggctgttatg atacctatgg tgcagacata gactgccagt ggattgatat tacagatgta 1380
   aaacctggaa actatatcct aaaggtcagt gtaaacccca gctacctggt tcctgaatct 1440
   gactatacca acaatgttgt gcgctgtgac attcgctaca caggacatca tgcgtatgcc 1500
   tcaggctgca caatttcacc gtattagaag gcaaagcaaa actcccaatg gataaatcag 1560
   tgcctggtgt tctgaagtgg gaaaaaatag actaacttca gtaggattta tgtattttga 1620
   aaaagagaac agaaaacaac aaaagaattt ttgtttggac tgttttcaat aacaaagcac 1680
   ataactggat tttgaacgct taagtcatca ttacttggga aatttttaat gtttattatt 1740
   tacatcactt tgtgaattaa cacagtgttt caattctgta attacatatt tgactctttc 1800
   aaagaaatcc aaatttctca tgttcctttt gaaattgtag tgcaaaatgg tcagtattat 1860
   ctaaatgaat gagccaaaat gactttgaac tgaaactttt ctaaagtgct ggaactttag 1920
   tgaaacataa taataatggg tttata                                      1946
<210> 83
   <211> 530
   <212> DNA
   <213> Artificial Sequence
<220>
   <223> Description of Artificial Sequence:primer
<220>
   <221> misc_feature
   <222> (1)..(530)
   <223> 3' terminal sequence. nuclear receptor subfamily 1, group d, member 1 (NR1D1) gene.
<400> 83
   ttttttcagc tgtgaactat tggatttgag acaggaacag aacaaatcag agggccaggg 60
   gagggttgtg ggggagacag agtggtttaa ataggggagg aggggaagtt cggtgatggg 120
   ggagggaggc aggtatttac aagaaggctc agggggccag agctcatctt ggaatatttt 180
   ataacaatat aaataagatt ctggtttgct tttccttttc gtctcgtaaa ggagagagaa 240
   gtgcagagtt cgattctgta caagggggca gcggcagaag gccgccgggc gggtcactgg 300
   gcgtccaccc ggaaggacag cagcttctcg gaatgcatgt tgttcagggt ccgcagtccg 360
   gcagcttgag cagcagcaag gtgaagcggg aagtctccaa gggccggttc ttcagcacca 420
   gagcccgaag aagcccgcag caggttctcc tggagctgct ccaccgaagc ggaattttcc 480
   atgcccgaag cggtctgccg agacaagcaa caccggcggt gaagaggccc            530
<210> 84
   <211> 497
   <212> DNA
   <213> Artificial Sequence
<220>
   <223> Description of Artificial Sequence:primer
<220>
   <221> misc_feature
   <222> (1)..(497)
   <223> 5' terminal sequence. nuclear receptor subfamily 1, group d, member 1 (NR1D1) gene.
<400> 84
   taccgaggag gagctgggcc tcttcaccgc ggtggtgctt gtctctgcag accgctcggg 60
   catggagaat tccgcttcgg tggagcagct ccaggagacg ctgctgcggc tcttcgggct 120
   ctggtgctga agaaccggcc cttggagact tcccgcttca ccaagctgct gctcaagctg 180
   ccggacctgc ggaccctgaa caacatgcat tccgagaagc tgctgtcctt ccgggtggac 240
   gcccagtgac ccgcccggcc ggccttctgc cgctgccccc ttgtacagaa tcgaactctg 300
   cacttctctc tcctttacga gacgaaaagg aaaagcaaac cagaatctta tttatattgt 360
   tataaaatat tccaagatga gcctctgatc cctgagcctt cttgtaaata cctgcctact 420
   tgccccatca ccgaacttcc cctcctcccc tatttaaacc actctgtctc ccccacaacc 480
   ctcccctggc cctctga                                                497
<210> 85
   <211> 2768
   <212> DNA/RNA
   <213> Artificial Sequence
<220>
   <223> Description of Artificial Sequence:primer
<220>
   <221> misc_feature
   <222> (1)..(2768)
   <223> nuclear receptor subfamily 1, group d, member 1 (NR1D1) gene.
<400> 85
   ccgaggcgct ccctgggatc acatggtacc tgctccagtg ccgcgtgcgg cccgggaacc 60
   ctgggctgct ggcgcctgcg cagagccctc tgtcccaggg aaaggctcgg gcaaaaggcg 120
   gctgagattg gcagagtgaa atattactgc cgagggaacg tagcagggca cacgtctcgc 180
   ctctttgcga ctcggtgccc cgtttctccc catcacctac ttacttcctg gttgcaacct 240
   ctcttcctct gggacttttg caccgggagc tccagattcg ctaccccgca gcgctgcgga 300
   gccggcaggc agaggcaccc cgtacactgc agagacccga ccctccttgc taccttctag 360
   ccagaactac tgcaggctga ttccccctac acactctctc tgctcttccc atgcaaagca 420
   gaactccgtt gcctcaacgt ccaacccttc tgcagggctg cagtccggcc accccaagac 480
   cttgctgcag ggtgcttcgg atcctgatcg tgagtcgcgg ggtccactcc ccgcccttag 540
   ccagtgccca gggggcaaca gcggcgatcg caacctctag tttgagtcaa ggtccagttt 600
   gaatgaccgc tctcagctgg tgaagacatg acgaccctgg actccaacaa caacacaggt 660
   ggcgtcatca cctacattgg ctccagtggc tcctccccaa gccgcaccag ccctgaatcc 720
   ctctatagtg acaactccaa tggcagcttc cagtccctga cccaaggctg tcccacctac 780
   ttcccaccat cccccactgg ctccctcacc caagacccgg ctcgctcctt tgggagcatt 840
   ccacccagcc tgagtgatga cggctcccct tcttcctcat cttcctcgtc gtcatcctcc 900
   tcctccttct ataatgggag cccccctggg agtctacaag tggccatgga ggacagcagc 960
   cgagtgtccc ccagcaagag caccagcaac atcaccaagc tgaatggcat ggtgttactg 1020
   tgtaaagtgt gtggggacgt tgcctcgggc ttccactacg gtgtgcacgc ctgcgagggc 1080
   tgcaagggct ttttccgtcg gagcatccag cagaacatcc agtacaaaag gtgtctgaag 1140
   aatgagaatt gctccatcgt ccgcatcaat cgcaaccgct gccagcaatg tcgcttcaag 1200
   aagtgtctct ctgtgggcat gtctcgagac gctgtgcgtt ttgggcgcat ccccaaacga 1260
   gagaagcagc ggatgcttgc tgagatgcag agtgccatga acctggccaa caaccagttg 1320
   agcagccagt gcccgctgga gacttcaccc acccagcacc ccaccccagg ccccatgggc 1380
   ccctcgccac cccctgctcc ggtcccctca cccctggtgg gcttctccca gtttccacaa 1440
   cagctgacgc ctcccagatc cccaagccct gagcccacag tggaggatgt gatatcccag 1500
   gtggcccggg cccatcgaga gatcttcacc tacgcccatg acaagctggg cagctcacct 1560
   ggcaacttca atgccaacca tgcatcaggt agccctccag ccaccacccc acatcgctgg 1620
   gaaaatcagg gctgcccacc tgcccccaat gacaacaaca ccttggctgc ccagcgtcat 1680
   aacgaggccc taaatggtct gcgccaggct ccctcctcct accctcccac ctggcctcct 1740
   ggccctgcac accacagctg ccaccagtcc aacagcaacg ggcaccgtct atgccccacc 1800
   cacgtgtatg cagccccaga aggcaaggca cctgccaaca gtccccggca gggcaactca 1860
   aagaatgttc tgctggcatg tcctatgaac atgtacccgc atggacgcag tgggcgaacg 1920
   gtgcaggaga tctgggagga tttctccatg agcttcacgc ccgctgtgcg ggaggtggta 1980
   gagtttgcca aacacatccc gggcttccgt gacctttctc agcatgacca agtcaccctg 2040
   cttaaggctg gcacctttga ggtgctgatg gtgcgctttg cttcgttgtt caacgtgaag 2100
   gaccagacag tgatgttcct aagccgcacc acctacagcc tgcaggagct tggtgccatg 2160
   ggcatgggag acctgctcag tgccatgttc gacttcagcg agaagctcaa ctccctggcg 2220
   cttaccgagg aggagctggg cctcttcacc gcggtggtgc ttgtctctgc agaccgctcg 2280
   ggcatggaga attccgcttc ggtggagcag ctccaggaga cgctgctgcg ggctcttcgg 2340
   gctctggtgc tgaagaaccg gcccttggag acttcccgct tcaccaagct gctgctcaag 2400
   ctgccggacc tgcggaccct gaacaacatg cattccgaga agctgctgtc cttccgggtg 2460
   gacgcccagt gacccgcccg gccggccttc tgccgctgcc cccttgtaca gaatcgaact 2520
   ctgcacttct ctctccttta cgagacgaaa aggaaaagca aaccagaatc ttatttatat 2580
   tgttataaaa tattccaaga tgagcctctg gccccctgag ccttcttgta aatacctgcc 2640
   tccctccccc atcaccgaac ttcccctcct cccctattta aaccactctg tctcccccac 2700
   aaccctcccc tggccctctg atttgttctg ttcctgtctc aaatccaata gttcacagct 2760
   gagctggg                                                          2768
<210> 86
   <211> 700
   <212> DNA
   <213> Artificial Sequence
<220>
   <223> Description of Artificial Sequence:primer
<220>
   <221> misc_feature
   <222> (1)..(700)
   <223> 5' terminal sequence. mitogen-activated protein kinase kinase 6, transcript variant 1 (MAP2K6) gene.
<400> 86
<210> 87
   <211> 2924
   <212> DNA/RNA
   <213> Artificial Sequence
<220>
   <223> Description of Artificial Sequence:primer
<220>
   <221> misc_feature
   <222> (1)..(2924)
   <223> mitogen-activated protein kinase kinase 6, transcript variant 1 (MAP2K6) gene.
<400> 87
   ggcttctggt tcggcccacc tctgaaggtt ccagaatcga tagtgaattc gtggttccaa 60
   gtttggagct tttagctgcc agccctggcc catcatgtag ctgcagcaca gccttcccta 120
   acgttgcaac tgggggaaaa atcactttcc agtctgtttt gcaaggtgtg catttccatc 180
   ttgattccct gaaagtccat ctgctgcatc ggtcaagaga aactccactt gcatgaagat 240
   tgcacgcctg cagcttgcat ctttgttgca aaactagcta cagaagagaa gcaaggcaaa 300
   gtcttttgtg ctcccctccc ccatcaaagg aaaggggaaa atgtctcagt cgaaaggcaa 360
   gaagcgaaac cctggcctta aaattccaaa agaagcattt gaacaacctc agaccagttc 420
   cacaccacct cgagatttag actccaaggc ttgcatttct attggaaatc agaactttga 480
   ggtgaaggca gatgacctgg agcctataat ggaactggga cgaggtgcgt acggggtggt 540
   ggagaagatg cggcacgtgc ccagcgggca gatcatggca gtgaagcgga tccgagccac 600
   agtaaatagc caggaacaga aacggctact gatggatttg gatatttcca tgaggacggt 660
   ggactgtcca ttcactgtca ccttttatgg cgcactgttt cgggagggtg atgtgtggat 720
   ctgcatggag ctcatggata catcactaga taaattctac aaacaagtta ttgataaagg 780
   ccagacaatt ccagaggaca tcttagggaa aatagcagtt tctattgtaa aagcattaga 840
   acatttacat agtaagctgt ctgtcattca cagagacgtc aagccttcta atgtactcat 900
   caatgctctc ggtcaagtga agatgtgcga ttttggaatc agtggctact tggtggactc 960
   tgttgctaaa acaattgatg caggttgcaa accatacatg gcccctgaaa gaataaaccc 1020
   agagctcaac cagaagggat acagtgtgaa gtctgacatt tggagtctgg gcatcacgat 1080
   gattgagttg gccatccttc gatttcccta tgattcatgg ggaactccat ttcagcagct 1140
   caaacaggtg gtagaggagc catcgccaca actcccagca gacaagttct ctgcagagtt 1200
   tgttgacttt acctcacagt gcttaaagaa gaattccaaa gaacggccta catacccaga 1260
   gctaatgcaa catccatttt tcaccctaca tgaatccaaa ggaacagatg tggcatcttt 1320
   tgtaaaactg attcttggag actaaaaagc agtggactta atcggttgac cctactgtgg 1380
   attggtgggt ttcggggtga agcaagttca ctacagcatc aatagaaagt catctttgag 1440
   ataatttaac cctgcctctc agagggtttt ctctcccaat tttcttttta ctccccctct 1500
   taagggggcc ttggaatcta tagtatagaa tgaactgtct agatggatga attatgataa 1560
   aggcttagga cttcaaaagg tgattaaata tttaatgatg tgtcatatga gtcctcaagc 1620
   ttctcagact tctcttattc tttacaaaat gaatgcattg gccctgacaa aaaggtgcta 1680
   cggtagtgat gaaattataa gtagatttgt agtttgtccc atttattatt ttaatattta 1740
   tgtttaagtg cttggttgaa aagattccat tttatacaag aagggagatt caaaaaaaaa 1800
   atataaggtt gggttagcaa tatttatagg gcttttattt tttaagttca attgtgtctg 1860
   tggtccagaa gaaattattt aatatgcatc tttgagaata ttataaaaat atcaaaaagg 1920
   agctcttctt gtgaaatgtc tgttccagct gttgtgactg ctgccatttt tggaaacatc 1980
   tgcccaatcc tgggtgatca ccacatcttt taggggaagt gacaagatgc tctggtcata 2040
   ctctttttcc caactttgga aaacataaaa atcactcata taacagctca aagagtaaaa 2100
   catttggttc ttctgacact tgtggtatag tattagtgga aagtgatttg taatatgatt 2160
   ttatatccac ctacctattc atctacctgt gtgtatgtgt gtgtttgtgt gtctatttgg 2220
   caattcacaa gtcctgccaa gtggtttcta tgagcatctc tgtttggtaa ggaggacaat 2280
   tgtcagtttt gagggggaca tgtgttaaat cacagaaaaa aatggtgcct tcttctgcgt 2340
   ttgtccctcc tgccatgtgt aagttgtaag gattgccttt gtagttaatg tactctttgg 2400
   ctttgtttgt ttgttttctt cttcagtgaa gcagccttac tattcataga agggctagaa 2460
   taggagaaaa tgaaaggtag tgagtaattc tttgataaga tgaggaaata atgggaaagg 2520
   ttgaattaat tcctgggcat ggactaccag atgaccacaa gttgcgttga ggccgcatct 2580
   ttcttcagca gcgtgcaata gctggctcct ctataggaga tgagcttcat tgggagttcc 2640
   tagcaagttg actaaacagc aaaagttctt tctcgtgggt aaatataccc acaggttcta 2700
   tgatttgtag ctctaggttt cttgatgatc aaggagtgaa gtaattgaca gggaaaatat 2760
   agacctatga taaataacca ggaagcattg cttttggaca aggaagaaca gagggttttg 2820
   attttaaaaa gaagaaaaaa aaaccttatt ttttctttct tggcctcaag ttcaatatgg 2880
   agaggattgc ttccctgaat cctctcttcc ttcccctttt agag                   2924
<210> 88
   <211> 501
   <212> DNA
   <213> Artificial Sequence
<220>
   <223> Description of Artificial Sequence:primer
<220>
   <221> misc_feature
   <222> (1)..(501)
   <223> 3' terminal sequence. integrin, alpha 2 (cd49b, alpha 2 subunit of vla-2 receptor) (ITGA2) gene.
<400> 88
   aaataaaatt ttgttggaat gaagcagcaa attctcacag ctgttaaaag aattggactc 60
   tggcatacct aactggagaa taccatgctg ttttttctat aaaccgctgg cattttatgc 120
   cttttttgtt tgttttgtgt tgtcaactac aaagtaattc tgtaaacagt acagatagcc 180
   ctggactgcc accacgggcc aggccagggt agtcaaggtt gtccatatgc acatgggcag 240
   ataacctgtt ttcctttgac atttttttcc aagtcaaatt cttggaggat tgtgtatttg 300
   tattatagga agtccagatc atagactttt aaaactaaaa gcatcactgc tgaactccag 360
   ctcagtcttc ccattttata atgaggactc tgaagtttat agaggtcaag gacttgtcca 420
   aagctttaga tatgtagtgt ctgtgccttt tcctcaagtt tccctagaga atgtgggggc 480
   tcagacagag aataaggtgc a                                          501
<210> 89
   <211> 648
   <212> DNA
   <213> Artificial Sequence
<220>
   <223> Description of Artificial Sequence:primer
<220>
   <221> misc_feature
   <222> (1)..(648)
   <223> 5' terminal sequence. integrin, alpha 2 (cd49b, alpha 2 subunit of vla-2 receptor) (ITGA2) gene.
<400> 89
   aaactaggta aaatttgttg ttggttcctt ttagaccacg gctgcccctt ccacacccca 60
   tcttgctcta atgatcaaaa catgcttgaa taactgagct tagagtatac ctcctatatg 120
   tccatttaag tcaggagagg gggcgatata gagactaagg cacaaaattt tgtttaaaac 180
   tcagaatata acatgtaaaa tcccatctgc tagaagccca tcctgtgcca gaggaaggaa 240
   aaggaggaaa tttcctttct cttttaggag gcacaacagt tctcttctag gatttgtttg 300
   gctgactggc agtaacctag tgaatttttg aaagatgagt aatttctttg gcaaccttcc 360
   tcctccctta ctgaaccact ctcccacctc ctggtggtac cattattata gaagccctct 420
   acagcctgac tttctctcca gcggtccaaa gttatcccct cctttacccc tcatccaaag 480
   ttcgcactcc ttcaggacag ctgctgtgca ttagatatta ggggggaaag tcatctgtnt 540
   aatttacaca cttgcatgaa ttactggata taactcctta actcagggag ctatgtcatt 600
   tagtgctaac aaagtagaaa aatagctcga gtgagttcta atggtgga             648
<210> 90
   <211> 5361
   <212> DNA/RNA
   <213> Artificial Sequence
<220>
   <223> Description of Artificial Sequence:primer
<220>
   <221> misc_feature
   <222> (1)..(5361)
   <223> integrin, alpha 2 (cd49b, alpha 2 subunit of vla-2 receptor) (ITGA2) gene.
<400> 90
   ctgcaaaccc agcgcaacta cggtcccccg gtcagaccca ggatggggcc agaacggaca 60
   ggggccgcgc cgctgccgct gctgctggtg ttagcgctca gtcaaggcat tttaaattgt 120
   tgtttggcct acaatgttgg tctcccagaa gcaaaaatat tttccggtcc ttcaagtgaa 180
   cagtttgggt atgcagtgca gcagtttata aatccaaaag gcaactggtt actggttggt 240
   tcaccctgga gtggctttcc tgagaaccga atgggagatg tgtataaatg tcctgttgac 300
   ctatccactg ccacatgtga aaaactaaat ttgcaaactt caacaagcat tccaaatgtt 360
   actgagatga aaaccaacat gagcctcggc ttgatcctca ccaggaacat gggaactgga 420
   ggttttctca catgtggtcc tctgtgggca cagcaatgtg ggaatcagta ttacacaacg 480
   ggtgtgtgtt ctgacatcag tcctgatttt cagctctcag ccagcttctc acctgcaact 540
   cagccctgcc cttccctcat agatgttgtg gttgtgtgtg atgaatcaaa tagtatttat 600
   ccttgggatg cagtaaagaa ttttttggaa aaatttgtac aaggccttga tataggcccc 660
   acaaagacac aggtggggtt aattcagtat gccaataatc caagagttgt gtttaacttg 720
   aacacatata aaaccaaaga agaaatgatt gtagcaacat cccagacatc ccaatatggt 780
   ggggacctca caaacacatt cggagcaatt caatatgcaa gaaaatatgc ctattcagca 840
   gcttctggtg ggcgacgaag tgctacgaaa gtaatggtag ttgtaactga cggtgaatca 900
   catgatggtt caatgttgaa agctgtgatt gatcaatgca accatgacaa tatactgagg 960
   tttggcatag cagttcttgg gtacttaaac agaaacgccc ttgatactaa aaatttaata 1020
   aaagaaataa aagcgatcgc tagtattcca acagaaagat actttttcaa tgtgtctgat 1080
   gaagcagctc tactagaaaa ggctgggaca ttaggagaac aaattttcag cattgaaggt 1140
   actgttcaag gaggagacaa ctttcagatg gaaatgtcac aagtgggatt cagtgcagat 1200
   tactcttctc aaaatgatat tctgatgctg ggtgcagtgg gagcttttgg ctggagtggg 1260
   accattgtcc agaagacatc tcatggccat ttgatctttc ctaaacaagc ctttgaccaa 1320
   attctgcagg acagaaatca cagttcatat ttaggttact ctgtggctgc aatttctact 1380
   ggagaaagca ctcactttgt tgctggtgct cctcgggcaa attataccgg ccagatagtg 1440
   ctatatagtg tgaatgagaa tggcaatatc acggttattc aggctcaccg aggtgaccag 1500
   attggctcct attttggtag tgtgctgtgt tcagttgatg tggataaaga caccattaca 1560
   gacgtgctct tggtaggtgc accaatgtac atgagtgacc taaagaaaga ggaaggaaga 1620
   gtctacctgt ttactatcaa aaagggcatt ttgggtcagc accaatttct tgaaggcccc 1680
   gagggcattg aaaacactcg atttggttca gcaattgcag ctctttcaga catcaacatg 1740
   gatggcttta atgatgtgat tgttggttca ccactagaaa atcagaattc tggagctgta 1800
   tacatttaca atggtcatca gggcactatc cgcacaaagt attcccagaa aatcttggga 1860
   tccgatggag cctttaggag ccatctccag tactttggga ggtccttgga tggctatgga 1920
   gatttaaatg gggattccat caccgatgtg tctattggtg cctttggaca agtggttcaa 1980
   ctctggtcac aaagtattgc tgatgtagct atagaagctt cattcacacc agaaaaaatc 2040
   actttggtca acaagaatgc tcagataatt ctcaaactct gcttcagtgc aaagttcaga 2100
   cctactaagc aaaacaatca agtggccatt gtatataaca tcacacttga tgcagatgga 2160
   ttttcatcca gagtaacctc cagggggtta tttaaagaaa acaatgaaag gtgcctgcag 2220
   aagaatatgg tagtaaatca agcacagagt tgccccgagc acatcattta tatacaggag 2280
   ccctctgatg ttgtcaactc tttggatttg cgtgtggaca tcagtctgga aaaccctggc 2340
   actagccctg cccttgaagc ctattctgag actgccaagg tcttcagtat tcctttccac 2400
   aaagactgtg gtgaggatgg actttgcatt tctgatctag tcctagatgt ccgacaaata 2460
   ccagctgctc aagaacaacc ctttattgtc agcaaccaaa acaaaaggtt aacattttca 2520
   gtaacactga aaaataaaag ggaaagtgca tacaacactg gaattgttgt tgatttttca 2580
   gaaaacttgt tttttgcatc attctcccta ccggttgatg ggacagaagt aacatgccag 2640
   gtggctgcat ctcagaagtc tgttgcctgc gatgtaggct accctgcttt aaagagagaa 2700
   caacaggtga cttttactat taactttgac ttcaatcttc aaaaccttca gaatcaggcg 2760
   tctctcagtt tccaagcctt aagtgaaagc caagaagaaa acaaggctga taatttggtc 2820
   aacctcaaaa ttcctctcct gtatgatgct gaaattcact taacaagatc taccaacata 2880
   aatttttatg aaatctcttc ggatgggaat gttccttcaa tcgtgcacag ttttgaagat 2940
   gttggtccaa aattcatctt ctccctgaag gtaacaacag gaagtgttcc agtaagcatg 3000
   gcaactgtaa tcatccacat ccctcagtat accaaagaaa agaacccact gatgtaccta 3060
   actggggtgc aaacagacaa ggctggtgac atcagttgta atgcagatat caatccactg 3120
   aaaataggac aaacatcttc ttctgtatct ttcaaaagtg aaaatttcag gcacaccaaa 3180
   gaattgaact gcagaactgc ttcctgtagt aatgttacct gctggttgaa agacgttcac 3240
   atgaaaggag aatactttgt taatgtgact accagaattt ggaacgggac tttcgcatca 3300
   tcaacgttcc agacagtaca gctaacggca gctgcagaaa tcaacaccta taaccctgag 3360
   atatatgtga ttgaagataa cactgttacg attcccctga tgataatgaa acctgatgag 3420
   aaagccgaag taccaacagg agttataata ggaagtataa ttgctggaat ccttttgctg 3480
   ttagctctgg ttgcaatttt atggaagctc ggcttcttca aaagaaaata tgaaaagatg 3540
   accaaaaatc cagatgagat tgatgagacc acagagctca gtagctgaac cagcagacct 3600
   acctgcagtg ggaaccggca gcatcccagc cagggtttgc tgtttgcgtg catggatttc 3660
   tttttaaatc ccatattttt tttatcatgt cgtaggtaaa ctaacctggt attttaagag 3720
   aaaactgcag gtcagtttgg atgaagaaat tgtggggggt gggggaggtg cggggggcag 3780
   gtagggaaat aatagggaaa atacctattt tatatgatgg gggaaaaaaa gtaatcttta 3840
   aactggctgg cccagagttt acattctaat ttgcattgtg tcagaaacat gaaatgcttc 3900
   caagcatgac aacttttaaa gaaaaatatg atactctcag attttaaggg ggaaaactgt 3960
   tctctttaaa atatttgtct ttaaacagca actacagaag tggaagtgct tgatatgtaa 4020
   gtacttccac ttgtgtatat tttaatgaat attgatgtta acaagagggg aaaacaaaac 4080
   acaggttttt tcaatttatg ctgctcatcc aaagttgcca cagatgatac ttccaagtga 4140
   taattttatt tataaactag gtaaaatttg ttgttggttc cttttatacc acggctgccc 4200
   cttccacacc ccatcttgct ctaatgatca aaacatgctt gaataactga gcttagagta 4260
   tacctcctat atgtccattt aagttaggag agggggcgat atagagacta aggcacaaaa 4320
   ttttgtttaa aactcagaat ataacattta tgtaaaatcc catctgctag aagcccatcc 4380
   tgtgccagag gaaggaaaag gaggaaattt cctttctctt ttaggaggca caacagttct 4440
   cttctaggat ttgtttggct gactggcagt aacctagtga atttttgaaa gatgagtaat 4500
   ttctttggca accttcctcc tcccttactg aaccactctc ccacctcctg gtggtaccat 4560
   tattatagaa gccctctaca gcctgacttt ctctccagcg gtccaaagtt atcccctcct 4620
   ttacccctca tccaaagttc ccactccttc aggacagctg ctgtgcatta gatattaggg 4680
   gggaaagtca tctgtttaat ttacacactt gcatgaatta ctgtatataa actccttaac 4740
   ttcagggagc tattttcatt tagtgctaaa caagtaagaa aaataagcta gagtgaattt 4800
   ctaaatgttg gaatgttatg ggatgtaaac aatgtaaagt aaaacactct caggatttca 4860
   ccagaagtta cagatgaggc actggaaacc accaccaaat tagcaggtgc accttctgtg 4920
   gctgtcttgt ttctgaagta ctttttcttc cacaagagtg aatttgacct aggcaagttt 4980
   gttcaaaagg tagatcctga gatgatttgg tcagattggg ataaggccca gcaatctgca 5040
   ttttaacaag caccccagtc actaggatgc agatggacca cactttgaga aacaccaccc 5100
   atttctactt tttgcacctt attttctctg ttcctgagcc cccacattct ctaggagaaa 5160
   cttagattaa aattcacaga cactacatat ctaaagcttt gacaagtcct tgacctctat 5220
   aaacttcaga gtcctcatta taaaatggga agactgagct ggagttcagc agtgatgctt 5280
   tttagtttta aaagtctatg atctgatctg gacttcctat aatacaaata cacaatcctc 5340
   caagaatttg acttggaaaa g                                           5361
<210> 91
   <211> 416
   <212> DNA
   <213> Artificial Sequence
<220>
   <221> misc_feature
   <222> (203)..(203)
   <223> n is a, c, g, or t
<400> 91
   catttataat ttttagtttt cttttctttt cttttctttt cttttttttt ttttttctga    60
   gacggagctt gctctgtcgc ccaggctgga gcgcagtggc aattctggac tcactgcaag   120
   ctctgccttc cgggttcacg ccattctcct gcctcagcct cccgagtagc tgggactaca   180
   ggcgcctgtc accacgcccg ganaagtttt tttggtataa tacttagttt tctttgtagc   240
   taacttagtc ttccaaagtc cttcaagtcc tctcagttgt gcttcccacc cagccagtca   300
   tcagataagg ctgttcttcc ctgttgctgc tgccctgcct gctttctggg acctgcttcc   360
   tgccttggga gttgggatcc ctccattttt gaactccagg ggccttcggt ttgttc       416
<210> 92
   <211> 436
   <212> DNA
   <213> Artificial Sequence
<400> 92
   atcttgagtc gacctcctat ctcattctgt aacctagaat gcctaactgt ctgggaatgc      60
   agcccagtag gtttcagcct cattgtcccc agcccctatt caagattcag ttgctctagt     120
   tcaaatgcct ctgacagttg tgcttcacca aacctagaga atgaacaaag cgaaggacct     180
   ggagttcaag aatggaggga tccaactcca aggcaggaag caggtccaga aagcaggcag     240
   ggcagcagca acagggaaga acagcttatc tgatgactgg ctgggtggga agcacaactg     300
   agaggacttg aaggactttg ggaagactta agttagctac aaagaaaact aagtattatt     360
   accaaaaaaa cttattccgg gcgtggttga cagggcgcct gttagttccc agctattcgg     420
   ggaggctgag gcaggg                                                     436
<210> 93
   <211> 4122
   <212> DNA/RNA
   <213> Artificial Sequence
<400> 93
   aggaggcgac agctgccagc cgaggaggcg cggcggagag gggactgcgg tcagctgcgt      60
   ccacttgggg ctgtgcggcg gtcccgcgcc cggcgatgtt cccgggcact ccctgagtag     120
   cggcagctta tcccccgccc gctagcccgc cctggtcccc ggctcgctcg ctggctggcg     180
   cggccccggc cccgctctgc gtcggccccg ccgcggtgga ggcgcgcgag ggggacgcgg    240
   ccggggatga gcggattgcg ggtgaactcg ccgccggggc cccgcgaagc cgtgagccgc    300
   tgcttttctc cgagtcgccg ccctgccctt ggatttgaga tcatgtccat ccacatcgtg     360
   gcgctgggga acgaggggga cacattccac caggacaacc agccgtcggg gcttatccgc     420
   acttacctgg ggagaagccc tctggtctcc ggggacgaga gcagcttgtt gctgaacgcg     480
   gccagcacgg tcgcgcgtcc ggtgttcacc gagtatcagg ccagtgcgtt tgggaatgtc     540
   aagctggtgg tccacgactg tcccgtctgg gacatatttg acagtgattg gtacacttct     600
   cgaaatctaa ttgggggcgc tgacatcatt gtgatcaaat acaacgttaa tgacaagttt     660
   tcattccatg aagtaaagga taattatatt ccagtgataa aaagagcatt aaattcagtt     720
   ccagtaatta ttgctgctgt tggtaccaga caaaatgaag agttaccttg tacatgccca     780
   ctatgtacct cagacagagg gagctgtgtt agtacaactg aagggatcca acttgcaaaa     840
   gaactaggag caacctatct tgaactccac agccttgatg acttctacat aggaaagtat     900
   tttggaggag tgttggagta ttttatgatt caagccttaa atcagaagac aagtgaaaaa     960
   atgaagaaaa gaaaaatgag caactccttt catggaatta gaccacctca acttgaacaa    1020
   ccagaaaaaa tgcctgtctt aaaggctgaa gcgtcacatt ataactctga cttaaataac    1080
   ttgctgttct gctgccagtg tgtggacgtg gtattttata accccgattt aaagaaagtt    1140
   gtagaggccc acaagatcgt tctctgcgct gtaagccatg ttttcatgct gcttttcaat    1200
   gtgaagagtc ccactgacat tcaggattcc agtatcatcc gaactaccca ggatcttttt    1260
   gctataaaca gagatactgc atttccaggt gctagccatg aatcttcagg caacccacca    1320
   ttacgagtca ttgttaaaga cgccctcttc tgttcttgtt tatcagacat ccttcgcttc    1380
   atttattcag gtgcttttca gtgggaagaa ttggaagaag atatcaggaa gaagttgaaa    1440
   gattctgggg atgtttcaaa tgtaatcgag aaagttaaat gcattttaaa aacaccagga    1500
   aagattaatt gcctaaggaa ttgcaaaacc tatcaagcca gaaaaccttt gtggttttat    1560
   aacacttccc tcaagttttt ccttaataag ccgatgcttg ccgatgttgt cttcgaaatt    1620
   caaggtacga cagtgccagc ccacagggcc atcctggtgg cccgttgtga agtgatggca    1680
   gccatgttta atggtaatta catggaagca aagagtgtcc tgattcccgt ttatggtgtt    1740
   tccaaagaga ctttcttgtc atttttagaa tacctgtaca cagactcctg ctgcccagct    1800
   ggcatattcc aggccatgtg tctcctgatc tgtgccgaga tgtaccaagt gtccagactg    1860
   cagcacatct gtgagctgtt catcattacc cagctgcaga gcatgccaag cagggaactg    1920
   gcatccatga accttgatat agttgacctg cttaaaaagg ccaagtttca ccactctgat    1980
   tgcctttcaa cctggctact tcatttcatt gctactaact acctcatctt cagtcaaaag    2040
   cctgaatttc aggatctttc agtggaagaa cgcagttttg ttgaaaagca cagatggccg    2100
   tcgaatatgt acttgaagca gcttgcggaa tacaggaagt atattcactc ccggaaatgt    2160
   cgttgcttag taatgtaacc tggagctttt atacactaca tttctttttt attattatga    2220
   agaatgggat acctccaggt tccagtaaaa ttcttctgac cgaaaccaat gtgggtgtta   2280
   gaaaaattac catatagctt aatatgttta ttagttctct ttggaaaaaa actaccactg   2340
   tggtcttaaa agggaacaaa atataccata ggctaaaact aaggctttca ctctagaatg   2400
   caaagctgtt ttgcagctgt tttcccttaa agatgtcctg ttgctttagt gatatttaga   2460
   cccctctcag ttaagaaatg cttagattaa aaaaaaaaaa ttacgtagga ttaatacaga   2520
   aatttaatca tgtctgatta attgctctat taaaataagg ggcatttaaa gacccagcat   2580
   aaccatttgt ataatgagaa atctagggga aaaccaatca gtccaacatg agattttagg   2640
   aatagaaatt tgccggccat ttggaaagtg aaatgccact tagttctcaa ttgatgacag   2700
   tgtttgaatc atcataaaaa aaatacctgc ttttcatctg gacaacccaa ttgagccact   2760
   ttatctcctt ttggcaatct gagtaggcgg ggaacctagg cagggctggc tttcttagcg   2820
   tgtaacttgt gtagcagcac agggcccaca cttagaagga ccccacactt ggttcaaggc   2880
   tctgctatag cggaaattct taataatgtt tgaagaaggg ccccatgatt tcattttgtg   2940
   ctgagccctc aaaattatgt ctgtttcgtg gtgggaaata tcctatgttt tcttgctcaa   3000
   acacctttct ctctgaaagc agaaaaaggc actgatataa agggaagaga aggaggctca   3060
   ccggagggaa gagaacatag tgaagattcc cgcctttggg gaggtctgga ccacccaggg   3120
   cctccactgc caccttggct ggcaagggag aaatgtgttg tgttgtctta gctttaaaac   3180
   agtcacagtt cttgctctat catagatgaa caaatacttt cttgatcatt ctgtaagacc   3240
   aggaggttgg taagagtgac taaccagcct aactttaata cacatgtata aagatgttca   3300
   cagagaaaga tgctctgtag agaatttgct accgaagttg gctcaagaat ttgtttttag   3360
   tgttatttac caagattagg acgtcagtgg cttaaattct ttgaattctt ttcaaggact   3420
   gcaagattat ttgataaaga gtagcatgaa tcttgtgctc taatattaca cagtaagttc   3480
   aaagaaagga tgtaagtcaa agacttgtta catagaggga aaatggactg ggatagagga   3540
   cagactgata gtttctttct ttcatatcac atgtatagag aaataattat atcagaaact   3600
   cacaaaccta gacatggaaa aacagattac tgtctattgt cagcatcatt ttcatctgta   3660
   agtcactact ggaatatatt tttcttttaa tttccagtga ctttagaata cacacagttt   3720
   ttccgacttt tcaaaaattt gattaaatgg ttttatagta taatattggg accccatacc   3780
   gttagccctt gtatgtatac caacactgcc aaagtaaaac attaggtcag gcatggtggc   3840
   tcaggcctgt aatcccagca ttttgggagg ctgaggcaag tggataactt gaggtcatga   3900
   gttcgaaacc agcctggcca aaacagtgaa accccgtctc tactaaaaat acaaaattag   3960
   ccagatgtgg tggcgcacac ctgtaatccc agctactcag gaagctgagg caggaaaatc   4020
   gcttgaacct gggaggtgga agttgcagtg agccgagatc gcaccactgc actccagcct   4080
   gggtgacaag agcgaaactc catcacaaaa aaaaaaaaaa aa                      4122
<210> 94
   <211> 393
   <212> DNA
   <213> Artificial Sequence
<220>
   <221> misc_feature
   <222> (28) ..(28)
   <223> n is a, c, g, or t
<220>
   <221> misc_feature
   <222> (58)..(58)
   <223> n is a, c, g, or t
<220>
   <221> misc_feature
   <222> (136)..(136)
   <223> n is a, c, g, or t
<220>
   <221> misc_feature
   <222> (147)..(147)
   <223> n is a, c, g, or t
<220>
   <221> misc_feature
   <222> (203)..(203)
   <223> n is a, c, g, or t
<220>
   <221> misc_feature
   <222> (322)..(322)
   <223> n is a, c, g, or t
<220>
   <221> misc_feature
   <222> (336)..(336)
   <223> n is a, c, g, or t
<220>
   <221> misc_feature
   <222> (341)..(341)
   <223> n is a, c, g, or t
<220>
   <221> misc_feature
   <222> (365)..(365)
   <223> n is a, c, g, or t
<220>
   <221> misc_feature
   <222> (375)..(375)
   <223> n is a, c, g, or t
<220>
   <221> misc_feature
   <222> (384)..(384)
   <223> n is a, c, g, or t
<220>
   <221> misc_feature
   <222> (390)..(390)
   <223> n is a, c, g, or t
<400> 94
   tccccaaggc tgccccatca caatgccngt gaagcttgac tggcagacac tgaggccnga     60
   agctgggggc tgcagggggt cactggctca cccggtcccc ccgtaatctg taaaacatac    120
   tgggtgaggg aggctnctgg aggaccngaa tctctccctt ctccaggcag tagtgaggca    180
   tatgcctgtt ggccttgggc canttaaaga tcattccagc cccagtgctg ttctctgaat    240
   tcttggggaa cacagggatg ggggctccta atgaggaccc cagaaactct gagctctcac    300
   aactttcaaa gacacttgcc tncctccttt gcccanacct ncaccattac agcatttgat    360
   cccanaagta agganggggc ggtnccattn cac                                 393
<210> 95
   <211> 2195
   <212> DNA/RNA
   <213> Artificial Sequence
<400> 95
   actctttctt cggctcgcga gctgagagga gcaggtagag gggcagaggc gggactgtcg     60
   tctgggggag ccgcccagga ggctcctcag gccgacccca gaccctggct ggccaggatg    120
   aagtatctcc ggcaccggcg gcccaatgcc accctcattc tggccatcgg cgctttcacc    180
   ctcctcctct tcagtctgct agtgtcacca cccacctgca aggtccagga gcagccaccg    240
   gcgatccccg aggccctggc ctggcccact ccacccaccc gcccagcccc ggccccgtgc    300
   catgccaaca cctctatggt cacccacccg gacttcgcca cgcagccgca gcacgttcag    360
   aacttcctcc tgtacagaca ctgccgccac tttcccctgc tgcaggacgt gcccccctct    420
   aagtgcgcgc agccggtctt cctgctgctg gtgatcaagt cctcccctag caactatgtg    480
   cgccgcgagc tgctgcggcg cacgtggggc cgcgagcgca aggtacgggg tttgcagctg    540
   cgcctcctct tcctggtggg cacagcctcc aacccgcacg aggcccgcaa ggtcaaccgg    600
   ctgctggagc tggaggcaca gactcacgga gacatcctgc agtgggactt ccacgactcc    660
   ttcttcaacc tcacgctcaa gcaggtcctg ttcttacagt ggcaggagac aaggtgcgcc    720
   aacgccagct tcgtgctcaa cggggatgat gacgtctttg cacacacaga caacatggtc    780
   ttctacctgc aggaccatga ccctggccgc cacctcttcg tggggcaact gatccaaaac    840
   gtgggcccca tccgggcttt ttggagcaag tactatgtgc cagaggtggt gactcagaat    900
   gagcggtacc caccctattg tgggggtggt ggcttcttgc tgtcccgctt cacggccgct    960
   gccctgcgcc gtgctgccca tgtcttggac atcttcccca ttgatgatgt cttcctgggt   1020
   atgtgtctgg agcttgaggg actgaagcct gcctcccaca gcggcatccg cacgtctggc   1080
   gtgcgggctc catcgcaaca cctgtcctcc tttgacccct gcttctaccg agacctgctg   1140
   ctggtgcacc gcttcctacc ttatgagatg ctgctcatgt gggatgcgct gaaccagccc   1200
   aacctcacct gcggcaatca gacacagatc tactgagtca gcatcagggt ccccagcctc   1260
   tgggctcctg tttccatagg aaggggcgac accttcctcc caggaagctg agacctttgt   1320
   ggtctgagca taagggagtg ccagggaagg tttgaggttt gatgagtgaa tattctggct   1380
   ggcgaactcc tacacatcct tcaaaaccca cctggtactg ttccagcatc ttccctggat   1440
   ggctggagga actccagaaa atatccatct tctttttgtg gctgctaatg gcagaagtgc   1500
   ctgtgctaga gttccaactg tggatgcatc cgtcccgttt gagtcaaagt cttacttccc   1560
   tgctctcacc tactcacaga cgggatgcta agcagtgcac ctgcagtggt ttaatggcag   1620
   ataagctccg tctgcagttc caggccagcc agaaactcct gtgtccacat agagctgacg   1680
   tgagaaatat ctttcagccc aggagagagg ggtcctgatc ttaacccttt cctgggtctc   1740
   agacaactca gaaggttggg gggataccag agaggtggtg gaataggacc gccccctcct   1800
   tacttgtggg atcaaatgct gtaatggtgg aggtgtgggc agaggaggga ggcaagtgtc   1860
   ctttgaaagt tgtgagagct cagagtttct ggggtcctca ttaggagccc ccatccctgt   1920
   gttccccaag aattcagaga acagcactgg ggctggaatg atctttaatg ggcccaaggc   1980
   caacaggcat atgcctcact actgcctgga gaagggagag attcaggtcc tccagcagcc   2040
   tccctcaccc agtatgtttt acagattacg gggggaccgg gtgagccagt gaccccctgc   2100
   agcccccagc ttcaggcctc agtgtctgcc agtcaagctt cacaggcatt gtgatggggc   2160
   agccttgggg aatataaaat tttgtgaaga cttgg                               2195
<210> 96
   <211> 306
   <212> DNA
   <213> Artificial Sequence
<220>
   <221> misc_feature
   <222> (232)..(232)
   <223> n is a, c, g, or t
<220>
   <221> misc_feature
   <222> (289)..(289)
   <223> n is a, c, g, or t
<400> 96
   attcaactct tctcggagga gcctggtgtt ggtgaagcag ttccggccag ctgtgtatgc     60
   gggtgaggtg gagcgccgct tcccagggtc cctagcagct gtagaccagg acgggcctcg    120
   ggagctacag ccagccctgc ccggctcagc gggggttgac agttgagctg tgtgccggcc    180
   tcgtggacca gcctgggctc tcgctggagg aagtggcttg caaggaggct tnggaggagt    240
   gtggctacca cttggccccc tctgatctgc gccgggtcgc cacatactng tcttgagttg    300
   ggactt                                                               306
<210> 97
   <211> 148
   <212> DNA
   <213> Artificial Sequence
<220>
   <221> misc_feature
   <222> (36)..(36)
   <223> n is a, c, g, or t
<400> 97
   ggagctatgc aagcctttat tggggtccgc ggggtntggg gtgagtggcc aagactggct     60
   ctgtctagaa ccctggagtc tcactggaga tccaggttgg gggccacctg gctgaggaac    120
   catgagacac caaagatgac gccgaggg                                       148
<210> 98
   <211> 840
   <212> DNA/RNA
   <213> Artificial Sequence
<400> 98
   gtcacgcgcc cgcccgaagg ctcctgtcgg gacagggcgc cgccccgtgt cggcccccgc     60
   ctgtccgggc gccgccatgg agcgcatcga gggggcgtcc gtgggccgct gcgccgcctc    120
   accctacctg cggccgctca cgctgcatta ccgccagaat ggtgcccaga agtcctggga    180
   cttcatgaag acgcatgaca gcgtgaccgt tctcttattc aactcttctc ggaggagcct    240
   ggtgttggtg aagcagttcc ggccagctgt gtatgcgggt gaggtggagc gccgcttccc    300
   agggtcccta gcagctgtag accaggacgg gcctcgggag ctacagccag ccctgcccgg    360
   ctcagcgggg gtgacagttg agctgtgtgc cggcctcgtg gaccagcctg ggctctcgct    420
   ggaggaagtg gcttgcaagg aggcttggga ggagtgtggc taccacttgg ccccctctga    480
   tctgcgccgg gtcgccacat actggtctgg agtgggactg actggctcca gacagaccat    540
   gttctacaca gaggtgacag atgcccagcg tagcggtcca ggtgggggcc tggtggagga    600
   gggtgagctc attgaggtgg tgcacctgcc cctggaaggc gcccaggcct ttgcagacga    660
   cccggacatc cccaagaccc tcggcgtcat ctttggtgtc tcatggttcc tcagccaggt    720
   ggcccccaac ctggatctcc agtgagactc cagggttcta gacagaggcc agtcttggcc    780
   actcacccca caccccgcgg accccaataa aggcttgcat agctccaaaa aaaaaaaaaa    840
<210> 99
   <211> 308
   <212> DNA
   <213> Artificial Sequence
<220>
   <221> misc_feature
   <222> (33)..(33)
   <223> n is a, c, g, or t
<220>
   <221> misc_feature
   <222> (35)..(35)
   <223> n is a, c, g, or t
<220>
   <221> misc_feature
   <222> (64)..(64)
   <223> n is a, c, g, or t
<220>
   <221> misc_feature
   <222> (70)..(70)
   <223> n is a, c, g, or t
<220>
   <221> misc_feature
   <222> (110)..(110)
   <223> n is a, c, g, or t
<220>
   <221> misc_feature
   <222> (147)..(147)
   <223> n is a, c, g, or t
<220>
   <221> misc_feature
   <222> (219)..(219)
   <223> n is a, c, g, or t
<220>
   <221> misc_feature
   <222> (234)..(234)
   <223> n is a, c, g, or t
<220>
   <221> misc_feature
   <222> (262)..(262)
   <223> n is a, c, g, or t
<220>
   <221> misc_feature
   <222> (267)..(267)
   <223> n is a, c, g, or t
<220>
   <221> misc_feature
   <222> (298)..(298)
   <223> n is a, c, g, or t
<400> 99
   ggctgcagcg gggtgagcgg cggcacggcc ggngngatcc tggagccatg gggctctgcg     60
   cgancgccan tcctggatgc gctggagaac ctgaccgccg aggagctcan gaagttcaag    120
   ctgaagctgc tgtcggtgcc gctgcgnagg ggctacgggc gcatcccgcg gggcgcgctg    180
   ctgtccatgg acgccttgga cctcaccgac aagctggtna gcttctacct gganacctac    240
   ggcgccaagt taaccgttaa cntgttncgc aaaatgggcc ttaaggaatt ggcccggnaa    300
   tttaaagg                                                             308
<210> 100
   <211> 459
   <212> DNA
   <213> Artificial Sequence
<220>
   <221> misc feature
   <222> (332)..(332)
   <223> n is a, c, g, or t
<220>
   <221> misc_feature
   <222> (404)..(404)
   <223> n is a, c, g, or t
<220>
   <221> misc_feature
   <222> (434)..(434)
   <223> n is a, c, g, or t
<220>
   <221> misc_feature
   <222> (452)..(452)
   <223> n is a, c, g, or t
<400> 100
   ggctcggccg tgaacatccc gtctgatgag ggccacatcc ccctgcacct ggcggcccag     60
   catggtcact atgatgtgtc tgagatgctg ctacagcacc agtctaaccc gtgcatggtg    120
   gacaactcgg ggaagacgcc cctgggacct ggcctgcgag ttcggccgcg ttggggtggt    180
   ccagctgctc ctcagcagca atatgtgtgc ggcgctgctg ggagccccgg ccgggagacg    240
   ccaccgaccc caacggcacc agccctttgc acctcgcagc taaaaacggc cacatcgaca    300
   tcatcaggta ggagccggta gcagggaggg cntcagcctt taggggttcc ccaggggttt    360
   cagcagcagc ctgggggttc aggggcacca gttttttggt tttnggggat aaggggcggg    420
   acaagggcag ggtnccaggt tccttggttc anggttttg                           459
<210> 101
   <211> 5761
   <212> DNA/RNA
   <213> Artificial Sequence
<220>
   <221> misc_feature
   <222> (66)..(66)
   <223> n is a, c, g, t or u
<400> 101
   ctgtgggagg cggccggtgc cgcggggccg ccgccgcctc tgagccgcgg ccgagcttca     60
   cggagncgca gccgcgtcgc tgcggccccg gccgcgcaat ggggaaggag caggagctgg    120
   tgcaggcggt gaaggcggag gacgtaggga ccgcgcagag gctgctgcag aggccgcggc    180
   ccgggaaggc caagctcctg ggttccacca agaagatcaa tgtcaacttc caggacccgg    240
   atgggttctc ggctctgcac catgcggccc tgaacggcaa cacggaattg atcagcctgc     300
   tgctggaggc ccaggccgct gtggacatca aggacaacaa aggcatgcgg ccgctgcact     360
   atgcggcctg gcagggccgg aaggagccca tgaagctggt gctgaaggcg ggctcggccg     420
   tgaacatccc gtctgatgag ggccacatcc ccctgcacct ggcggcccag catggtcact     480
   atgatgtgtc tgagatgctg ctacagcacc agtctaaccc gtgcatggtg gacaactcgg     540
   ggaagacgcc cctggacctg gcctgcgagt tcggccgcgt tggggtggtc cagctgctcc     600
   tcagcagcaa tatgtgtgcg gcgctgctgg agccccggcc gggagacgcc accgacccca     660
   acggcaccag ccctttgcac ctcgcagcta aaaacggcca catcgacatc atcaggctcc     720
   tcctccaagc cggcatcgac attaaccgcc agaccaagtc cggcacggcc ctgcacgagg     780
   ctgcgctctg cggaaagaca gaggtggtgc ggctgctgct ggatagcggg atcaatgccc     840
   acgtgaggaa cacctacagc cagacagccc tggacatcgt gcaccagttc accacgtccc     900
   aggccagcag ggagatcaag cagctgttgc gagaggcctc agcggccctg caggtccggg     960
   cgaccaagga ttattgcaac aattacgacc tgaccagcct caacgtgaag gcaggggaca    1020
   tcatcacagt cctcgagcag catccggatg gccggtggaa gggctgcatc catgacaacc    1080
   ggacgggcaa tgaccgggtg ggctacttcc cgtcctccct gggcgaggcc attgtcaagc    1140
   gagcaggttc ccgagcaggc actgaaccaa gcctgcccca gggaagcagc tcatcgggac    1200
   cctctgcacc cccagaggag atctgggtgc tgaggaagcc ttttgcaggt ggggaccgaa    1260
   gcggcagcat tagcggcatg gctggcggcc ggggcagcgg gggtcacgcc ctacacgcgg    1320
   gctctgaagg cgtcaagctc ctggcaacgg tgctttccca gaagtccgtc tctgagtccg    1380
   gcccggggga cagccccgcc aagcctccgg aaggctctgc aggtgtggcc cggtcccagc    1440
   ctccagtggc ccacgccggg caggtctatg ggagcagccc gcccaagaag ctggagccag     1500
   catcggaggg caagagctct gaggccgtga gccagtggct caccgcgttc cagctgcagc    1560
   tctacgcccc caacttcatc agcgccggct acgacctgcc caccatcagc cgcatgactc    1620
   ccgaggacct cacggccatt ggtgtcacca agccgggcca ccggaagaag atcgcggcag    1680
   agatcagcgg cctaagcatc cctgactggc tgcctgagca caaacccgct aacctggccg    1740
   tgtggctgtc catgatcggc ctggcccagt actacaaggt gttggtggac aatggctacg    1800
   agaacattga tttcatcacc gacatcacct gggaggacct gcaggagatc ggcatcacca    1860
   agctggggca ccagaagaag ctgatgctcg ctgtgaggaa gctggcagag ctgcagaagg    1920
   ctgaatacgc caagtatgag gggggccccc tgcgccggaa ggcgccccag tctcttgaag    1980
   tgatggccat cgagtcgccg cccccgcctg agcccacacc ggccgactgc cagtccccta    2040
   aaatgaccac cttccaggac agcgagctca gtgacgagct gcaggctgcc atgactggcc    2100
   cggctgaggt ggggcccacc actgagaagc cctccagcca cctgccaccc accccgaggg    2160
   ccaccacgcg gcaggactcc agcctgggtg gtcgggcacg gcacatgagc agctcgcagg    2220
   agctgctggg agatgggccc cctgggccca gcagccccat gtctcgaagc caggagtacc    2280
   tcctggatga gggccccgcc cccggcaccc cgcccaggga ggcccggccc ggccgccacg    2340
   gccacagcat caagagggcc agcgtgcccc ccgtgcctgg caagccacgg caggtcctcc    2400
   caccaggcac tagccacttc acgccccccc agacgcccac caaaacccga ccaggctctc    2460
   cccaggccct tgggggacct catggtccag ccccagctac ggccaaggtg aagcccaccc    2520
   cgcagctgct gccgccgaca gagcgcccca tgtcaccccg ctccctgcct cagtcaccga    2580
   cgcaccgcgg ctttgcctac gtgctgcccc agcccgtgga gggcgaggtg gggccggctg    2640
   ccccggggcc tgcgccccca cccgtgccga cggctgtgcc cacactgtgc ctgccccctg    2700
   aggccgacgc ggagccgggg cggcccaaga agcgggccca cagcctgaat cgctatgcgg    2760
   cgtccgacag cgagccggag cgggacgagc tgctggtgcc tgcggctgcc ggcccctatg    2820
   ccacggtcca gcggcgcgtg ggccgcagcc actcagtgag ggcgcccgca ggtgccgaca    2880
   agaacgtcaa ccgcagccag tcctttgccg tgcggccccg aaagaagggg cccccgccgc    2940
   ccccacccaa gcgctccagc tcggccctgg ctagtgccaa cctggcggat gagccggtgc    3000
   ctgacgccga gcctgaggat ggcctgctgg gggtccgggc acagtgccgg cgggccagtg    3060
   acctggccgg cagcgtggac acgggtagtg ccggcagtgt gaagagcatc gcggccatgc    3120
   tggagctgtc ctccattggg ggtgggggcc gggctgcccg caggcctcct gagggccacc    3180
   ccactccccg ccctgccagc ccagagccgg gccgggtggc caccgtgctg gcctcagtga    3240
   aacacaaaga ggccatcggg cctggcgggg aggtggtgaa ccggcgccgc acgctcagcg    3300
   ggccagtcac cggacttctg gccactgccc gccgggggcc tggggagtcg gcagacccag    3360
   gcccctttgt ggaggatggc actggccggc agcggcctcg gggtccctcc aagggcgagg    3420
   cgggtgtcga aggcccgccc ttggccaagg tggaagccag cgccacactc aagaggcgca    3480
   tccgggccaa gcagaaccag caggagaacg tcaagttcat cctgaccgag tctgacacgg    3540
   tcaagcgcag gcccaaggcc aaggagcggg aggccgggcc tgagccacca ccgccactgt    3600
   ccgtgtacca taatggcact ggcaccgtgc gccgccgacc ggcctcggag caggctgggc    3660
   ctccggagct gcctccaccg cccccgcctg ccgaaccccc gcccaccgac ctggcgcacc   3720
   tacccccatt gcccccgccc gagggcgaag cccggaagcc ggccaagccg cctgtctctc   3780
   ccaagcccgt cctgacgcag cctgtgccca agctccaggg ctcgcccaca cccacctcca   3840
   agaaggtgcc gctgccaggc cctggcagcc cagaggtgaa gcgcgcccac ggcacgccac   3900
   cgcccgtgtc tcccaagccg ccgccgccgc ccacagcgcc caagcccgtc aaggcggtcg   3960
   cggggctgcc ttcgggcagc gccggccctt cacccgcacc ctcgcccgcg cgacagccgc   4020
   ccgccgccct cgccaagccg cccggtacgc cgccctcgct gggcgccagc cccgccaagc   4080
   ccccgtcccc cggcgcgccc gcgctgcacg tgcccgccaa gcccccgcga gccgccgccg   4140
   ccgccgccgc cgccgccgcc gcgccccccg ccccgcccga aggcgcctcg ccaggggaca   4200
   gcgcccggca gaaactggag gagacaagcg cgtgcctggc cgcggcgctg caggcggtgg   4260
   aggagaagat ccggcaggag gacgcgcagg gcccgcgcga ctcggcggcg gaaaagagca   4320
   ctggcagcat cctggacgac atcggcagca tgttcgacga cctggccgac cagctggatg   4380
   ccatgctgga gtgaacgccg cctggccggg ccctcccgcg ccgcccgggc cctccccgca   4440
   cactgaccta tacctcagga tgggcgcgtc tgggcgcggc gcgagcggcc gggcagggcc   4500
   ctgcagaagc acaactccgg cccctggacc cgggccgggg cgcccaccgg ggaccgcctg   4560
   gccgggggct ccaagggctc taggcagacc ctgcgcccgc gggtcctccc cacctgctgc   4620
   tccccgtgca atacctgctg ggcctcctgc ccgcgcggga ggggagggcg ccccgggacc   4680
   agggatgggg cagcgcacag gcccgggccc agcacagaac tgcccatcgg gacgcccggc   4740
   cagccgctcg ggcgcagcac aagggacagg ggaccaaggt cagggccccc ctgccccacc   4800
   gcacccccag aatataagct atcaagagta ttaatttatt gggaatgagc tgaggcggat   4860
   ttccccagag aaacaaaaag gtataacttt aacaaatata tatttaaaga aagaaatatt   4920
   attgattcta tagaaaacca tttaccaact gaaaggacac gaagtctagc tccggacaaa   4980
   agctgttaga ggcccaggct gtctgcctgc ggtccttcct ccggccagag tggaggcccc   5040
   aggctgctag cacaggtgga aggctggggc ccgggggcgg gtgctctgcc gggggctcct    5100
   ccgtgcttct ctcctaagtt ctctgcgccc caagaactcg aggttgtgcc ttgctctcgg   5160
   cggccgtgtc ctcctcctgg cgtggctgcg tcccagggcg cgggtgggtt tggtgaatgt   5220
   gggtgtgtca gcgtggcagc cacgtggaag cagggcatgt agcaggccag gccacgggct   5280
   tgcagctcct ctgagggtgt gcctggcccc ctctccaggg gctgtgcagg cagcccatcc   5340
   accccagtgg agttcccacg gacgcttctg ctcatggtcc aaggctgtgt tgtgtggtca   5400
   ttctcgccct cccttttcct ctctcctcct gccccctcca agaagtactg actggtctcc    5460
   tctatcgtgt ttgctgatcc acagtgttgg gcacaacttt gacgtttctt aaaaaaaaaa   5520
   tttaaaaaat gccgaaaacc agcgattgtg gtgtggggcg ggtggaggcg gctggcggag   5580
   gtggcgtccc ggcgtgcagg gccccttcct gctgtctcac cccgtgtttc cctgcgagtg   5640
   gacgtgccca gctgtccccc caggcctctc ctcagagggc ggcacaggct gggctccagc   5700
   aggtgggcag gggaggtctg ggcaaggcgc tgccgtcggc tgtcaataaa cagcaggaaa   5760
   c                                                                   5761
<210> 102
   <211> 438
   <212> DNA
   <213> Artificial Sequence
<400> 102
   caaagtttaa ttcaatttta ttttccactt ttagtatttt tcaaattata caacatgcag     60
   tctgccagag tacccataca tcttcatttt agaacctaga agattaccaa aattttccgt    120
   gggccagagg agggtgactt ccagatcttt tgttacatgg actatagtac agcatcgtta    180
   ttgatataaa ccaccattct cccctcaaac cccccggaca agtttgtcca caattttttt    240
   aatgtgaaag ctactgtaca gatacataaa gcccagagaa cacacatctg cagtacacag    300
   gacacacttt acaaactaga catgtataat tctacagaat gccctattcc ccttatatct    360
   ggcacacaat gaacagtatt taaaactgga tacacaattt ttaaataagt aagcagactt    420
   catggagcgg cgccctcg                                                  438
<210> 103
   <211> 447
   <212> DNA
   <213> Artificial Sequence
<220>
   <221> misc_feature
   <222> (419)..(419)
   <223> n is a, c, g, or t
<220>
   <221> misc_feature
   <222> (436)..(436)
   <223> n is a, c, g, or t
<400> 103
   cgcgccgctc catgaagtct gcttacttat ttaaaaattg tgtatcagtt ttaaatactg     60
   ttcattgtgt gcagatataa ggggaatagg gcattctgta gaattataca tgtctagttt    120
   gtaaagtgtg tcctgtgtac tgcagatgtg tgttctctgg gctttatgta tctgtacagt    180
   agctttcaca ttaaaaaaat tgtggacaaa cttgtccggg gggtttgagg ggagaatggt    240
   ggtttatatc aataacgatg ctgtactata gtccatgtaa caaaagatct ggaagtcacc    300
   ctcctcctgg cccacggaaa attttggtaa tcttctaggt tctaaaatga agatgtatgg    360
   gtactcctgg cagactgcat gttgtaataa ttttgaaaaa tactaaaagt ggaaaattna    420
   attgaattta accttnggaa aaaaaaa                                        447
<210> 104
   <211> 494
   <212> DNA
   <213> Artificial Sequence
<220>
   <221> misc_feature
   <222> (359)..(359)
   <223> n is a, c, g, or t
<220>
   <221> misc_feature
   <222> (479)..(479)
   <223> n is a, c, g, or t
<220>
   <221> misc_feature
   <222> (485)..(485)
   <223> n is a, c, g, or t
<400> 104
   acagtaagaa aagaacttta ttgtttatta atgtttctgt gtaaaactta agcttttttt     60
   ttttttttta aagaaacacc accaaaaggg gattagctta gtccatccct tcctcagtca     120
   tcttcccacc ttcctccaaa tgttatccca gaacattctg gaggcaggga gaaggggagg    180
   cagctaatca gagtctgaga gcacgatgat ctcttctgga tcgcattgtg tggccacact    240
   tgtcttgcaa gtaccaggcc gaggaggatg tgaatggggg gtttgggaca gccgggctgg    300
   agaagggatg cagagggagc tggtcaccag gccatggctg ggagagtccc accctcgtng    360
   aaggacatca gcaactgggg ccaaggaagc caagggggaa ggttgggccg ggcagggtac    420
   atatcttttt cccattcttc tcatgcactg acctttgcct ttccacatag ctgtttccna    480
   atggncctga tcct                                                      494
<210> 105
   <211> 660
   <212> DNA
   <213> Artificial Sequence
<220>
   <221> misc_feature
   <222> (1)..(1)
   <223> n is a, c, g, or t
<220>
   <221> misc feature
   <222> (39)..(39)
   <223> n is a, c, g, or t
<220>
   <221> misc feature
   <222> (179)..(179)
   <223> n is a, c, g, or t
<220>
   <221> misc feature
   <222> (243)..(243)
   <223> n is a, c, g, or t
<220>
   <221> misc feature
   <222> (372)..(372)
   <223> n is a, c, g, or t
<220>
   <221> misc_feature
   <222> (387)..(387)
   <223> n is a, c, g, or t
<220>
   <221> misc_feature
   <222> (545)..(545)
   <223> n is a, c, g, or t
<220>
   <221> misc_feature
   <222> (599)..(599)
   <223> n is a, c, g, or t
<220>
   <221> misc_feature
   <222> (626)..(626)
   <223> n is a, c, g, or t
<220>
   <221> misc_feature
   <222> (647)..(647)
   <223> n is a, c, g, or t
<210> 106
   <211> 2549
   <212> DNA/RNA
   <213> Artificial Sequence
<220>
   <221> misc_feature
   <222> (2478)..(2478)
   <223> n is a, c, g, t or u
<220>
   <221> misc_feature
   <222> (2502)..(2502)
   <223> n is a, c, g, t or u
<400> 106
   gggaaccatg cgaggttctg agaattgcgg cgagggtcgc ctcgagagac ggtttctgag 60
   caggaattct gaaatcccca ccacttcctc cctccggggg atttgatccc ctatggccac 120
   cgctaacagc atcatcgtgc tggatgatga tgacgaagat gaagcagctg ctcagccagg 180
   gccctcccac ccactcccca atgcggcctc acctggggca gaagccccta gctcctctga 240
   gcctcatggg gccagaggaa gcagtagttc gggcggcaag aaatgctaca agctggagaa 300
   tgagaagctg ttcgaagagt tccttgaact ttgtaagatg cagacagcag accaccctga 360
   ggtggtccca ttcctctata accggcagca acgtgcccac tctctgtttt tggcctcggc 420
   ggagttctgc aacatcctct ctagggtcct gtctcgggcc cggagccggc cagccaagct 480
   ctatgtctac atcaatgagc tctgcactgt tctcaaggcc cactcagcca aaaagaagct 540
   gaacttggcc cctgccgcca ccacctccaa tgagccctct gggaataacc ctcccacaca 600
   cctctccttg gaccccacaa atgctgaaaa cactgcctct cagtctccaa ggacccgtgg 660
   ttcccggcgg cagatccagc gtttggagca gctgctggcg ctctatgtgg cagagatccg 720
   gcggctgcag gaaaaggagt tggatctctc agaattggat gacccagact ccgcatacct 780
   gcaggaggca cggttgaagc gtaagctgat ccgcctcttt gggcgactat gtgagctgaa 840
   agactgctct tcactgaccg gccgtgtcat agagcagcgc atcccctacc gtggcacccg 900
   ctacccagag gttaacaggc gcattgagcg gctcatcaac aagccagggc ctgatacctt 960
   ccctgactat ggggatgtgc ttcgggctgt agagaaggca gctgcccgac acagccttgg 1020
   cctcccccga cagcagctcc agctcatggc tcaggatgcc ttccgagatg tgggcatcag 1080
   gttacaggag cgacgtcacc tcgatctcat ctacaacttt ggctgccacc tcacagatga 1140
   ctataggcca ggcgttgacc ccgcactatc agatcctgtg ttggcccggc gccttcggga 1200
   aaaccggagt ttggccatga gtcggctgga tgaggtcatc tccaaatatg caatgttgca 1260
   agacaaaagt gaggagggcg agagaaaaaa gagaagagct cggctccaag gcacctcttc 1320
   ccactctgca gacacccccg aagcctcctt ggattctggt gagggcccta gtggaatggc 1380
   atcccagggg tgcccttctg cctccagagc tgagacagat gacgaagacg acgaggagag 1440
   tgatgaggaa gaggaggagg aggaggaaga agaagaggag gaggccacag attctgaaga 1500
   ggaggaggat ctggaacaga tgcaggaggg tcaggaggat gatgaagagg aggacgaaga   1560
   ggaagaagca gcagcaggta aagatggaga caagagcccc atgtcctcac tacagatctc   1620
   caatgaaaag aacctggaac ctggcaaaca gatcagcaga tcttcagggg agcagcaaaa   1680
   caaaggacgc atagtgtcac catcgttact gtcagaagaa cccctggccc cctccagcat   1740
   agatgctgaa agcaatggag aacagcctga ggagctgacc ctggaggaag aaagccctgt   1800
   gtctcagctc tttgagctag agattgaagc tttgcccctg gatacccctt cctctgtgga   1860
   gacggacatt tcctcttcca ggaagcaatc agaggagccc ttcaccactg tcttagagaa   1920
   tggagcaggc atggtctctt ctacttcctt caatggaggc gtctctcctc acaactgggg   1980
   agattctggt cccccctgca aaaaatctcg gaaggagaag aagcaaacag gatcagggcc   2040
   attaggaaac agctatgtgg aaaggcaaag gtcagtgcat gagaagaatg ggaaaaagat   2100
   atgtaccctg cccagcccac cttccccctt ggcttccttg gccccagttg ctgattcctc   2160
   cacgagggtg gactctccca gccatggcct ggtgaccagc tccctctgca tcccttctcc   2220
   agcccggctg tcccaaaccc cccattcaca gcctcctcgg cctggtactt gcaagacaag   2280
   tgtggccaca caatgcgatc cagaagagat catcgtgctc tcagactctg attagctgcc   2340
   tccccttctc cctgcctcca gaatgttctg ggataacatt tggaggaagg tgggaagcag   2400
   atgactgagg aagggatgga ctaagctaat ccccttttgg tggtgtttcc tttaaaaaaa   2460
   aaaaaaaaaa actccganaa agctttggac ttcttccgcc anaagttttg gtcaatctcc   2520
   caatcaaggt tgttcggctt tgtctacct                                      2549
<210> 107
   <211> 407
   <212> DNA
   <213> Artificial Sequence
<220>
   <221> misc_feature
   <222> (103)..(103)
   <223> n is a, c, g, or t
<220>
   <221> misc_feature
   <222> (106)..(106)
   <223> n is a, c, g, or t
<400> 107
   tcctggtttt tttattttta tttttttaac atccttgaag aagtcataat acatttaaaa    60
   catttgtctt tgtaagtaaa ctgaaacctc ctgccacctg tgntantagg gaaaactcga   120
   gctgggtagg accagaacca tatgagtggt agaaggagaa agatgcctgg gcctgggctg   180
   aacttaggtt gtggtctcag acctaggatc caggggccaa gtttccaagc ttgcccatca   240
   ggaaggtgga gcggttaaga actcatatgc taaatgccac ccagtgaagg ccaacagaga   300
   cctcactgcc cctgcttctg ggcagcagct atagtgacca ttggggccat cacaacgttt   360
   agacaggttt gtgcagcagc acattcctcc cctgggcctt tgactgg                 407
<210> 108
   <211> 2828
   <212> DNA/RNA
   <213> Artificial Sequence
<400> 108
   gcgctacggc ggacccggct gggcagttcc ttccccagaa ggagagattc ctctgccatg     60
   gagtcctacg atgtgatcgc caaccagcct gtcgtgatcg acaacggatc cggtgtgatt    120
   aaagctggtt ttgctggtga tcagatcccc aaatactgct ttccaaacta tgtgggccga    180
   cccaagcacg ttcgtgtcat ggcaggagcc cttgaaggcg acatcttcat tggccccaaa    240
   gctgaggagc accgagggct gctttcaatc cgctatccca tggagcatgg catcgtcaag    300
   gattggaacg acatggaacg catttggcaa tatgtctatt ctaaggacca gctgcagact    360
   ttctcagagg agcatcctgt gctcctgact gaggcgcctt taaacccacg aaaaaaccgg    420
   gaacgagctg ccgaagtttt cttcgagacc ttcaatgtgc ccgctctttt catctccatg    480
   caagctgtac tcagccttta cgctacaggc aggaccacag gggtggtgct ggattctggg     540
   gatggagtca cccatgctgt gcccatctat gagggctttg ccatgcccca ctccatcatg    600
   cgcatcgaca tcgcgggccg ggacgtctct cgcttcctgc gcctctacct gcgtaaggag    660
   ggctacgact tccactcatc ctctgagttt gagattgtca aggccataaa agaaagagcc    720
   tgttacctat ccataaaccc ccaaaaggat gagacgctag agacagagaa agctcagtac    780
   tacctgcctg atggcagcac cattgagatt ggtccttccc gattccgggc ccctgagttg    840
   ctcttcaggc cagatttgat tggagaggag agtgaaggca tccacgaggt cctggtgttc    900
   gccattcaga agtcagacat ggacctgcgg cgcacgcttt tctctaacat tgtcctctca    960
   ggaggctcta ccctgttcaa aggttttggt gacaggctcc tgagtgaagt gaagaaacta   1020
   gctccaaaag atgtgaagat caggatatct gcacctcagg agagactgta ttccacgtgg   1080
   attgggggct ccatccttgc ctccctggac acctttaaga agatgtgggt ctccaaaaag   1140
   gaatatgagg aagacggtgc ccgatccatc cacagaaaaa ccttctaatg tcgggacatc   1200
   atcttcacct ctctctgaag ttaactccac tttaaaactc gctttcttga gtcggagtgt   1260
   ttgcgaggaa ctgcctgtgt gtgagtgcgt gtgtggatat gagtgtgtgc gcacatgcga   1320
   gtgccgtgtg gccctgggac cctgggccca gaaaggacga tgaactaccc gcagtggtga   1380
   tgcctgaggc ctggggttga ccactaactg gctcctgaca gggaagagcg ctggcagagg   1440
   ctgtgctccc tcctcaggtg gcctctggct ggctgtgggg gactccgttt actaccacag   1500
   ggagacagag ggaggtaagc catcccccgg gagaccttgc tgctgaccat cctaggctgg  1560
   gctggcccac cctcaccccc acccccaggg tgccctgagg ccccaggcag ctgctgcctc   1620
   cactatcgat gcctcctgac tgcacactga ggactgggac tggggttgag ttctgtctgg   1680
   ttttgttgcc attttggttt gggaggctgg aaaagcaccc caagaagcta ttacagagac   1740
   tggagtcagg agagagcagg aggccctcat gttcaccagg gaacaggacc acaccggcca   1800
   ctgaaggagg gcaggagcag tcctccctct gaatggctgc agagttaatg ttcccagccc   1860
   agtccccttt cgggggcctt gggagagttt aaggcacctg ctggttccag gacctcgctt   1920
   tccatctgtt cttgttgcaa tgccatcttc aaaccgtttt atttattgaa gtgtttgttc   1980
   agttaggggc tggagagagg gagcttgctg cctcctgcct tgctacacta atgtttacag   2040
   cacctaagct tagcctccag ggccccacct ctcccagctg atggtgagct gacagtgtcc   2100
   acaggttcca ggaccatttg agattggaag ctacactcaa agacactccc accaggctct   2160
   ttctcccttt tcctcttctc actgccctgg aatcaacagg ctggttgctg gttagatttt   2220
   ctgaaacagg aggtaaaatt tttctttggc agaggcccct aagcaaggga ggggtgttgg   2280
   agagccagtg cccttaagac tggagaaagc tgcaatttac caagttgcct tttgccactg   2340
   tagctgacca ggggactagg ttgtagaggt gggaaggccc ctctgggctg atcttgtgcc   2400
   attcttgacc ttggacctgc ttggttaagg agggagtggg ccagaccaga gtgccaggag   2460
   ctaatggagc caggcctgac acctaggagt ggtccaaagc cttcagccta gatggtgcaa   2520
   agctggggcc agcctgtctt caccggcacc ctcacctgtg acaccaagac ccaccccaat   2580
   ccagacttca cacagtattc tcccccacgc cgtctatgac caaaggcccc tgccaggtgt   2640
   gggtccacag cagcaggtat gtgtgaaagc aacgtagcgc cccgcggact gcagtgcgct   2700
   taaccaactc acctcccttc tcttagccca agcctgtccc tcgcacagcc tcgcacaaac   2760
   cacattgcct ggtggggccc agtgtactga aataaagtcg ttccgataga cacgtcaaaa   2820
   aaaaaaaa                                                            2828
<210> 109
   <211> 528
   <212> DNA
   <213> Artificial Sequence
<220>
   <221> misc_feature
   <222> (4)..(4)
   <223> n is a, c, g, or t
<220>
   <221> misc_feature
   <222> (185)..(185)
   <223> n is a, c, g, or t
<220>
   <221> misc_feature
   <222> (359)..(359)
   <223> n is a, c, g, or t
<220>
   <221> misc_feature
   <222> (363)..(363)
   <223> n is a, c, g, or t
<220>
   <221> misc_feature
   <222> (366)..(366)
   <223> n is a, c, g, or t
<220>
   <221> misc_feature
   <222> (487)..(487)
   <223> n is a, c, g, or t
<220>
   <221> misc_feature
   <222> (503)..(503)
   <223> n is a, c, g, or t
<220>
   <221> misc_feature
   <222> (526)..(526)
   <223> n is a, c, g, or t
<400> 109
   cttngctctc ccagcggcaa ggagggggat gtctactctc cagcacgtgg cttcctctcc     60
   cactcccact tcttgtgctt cctctcccct ctgcccagcc cctgcctcgg cctcccccgt    120
   gggcctcccg ccccacccca acccccgtca cactcacaca aggttgacat cgtctgcctg    180
   tggtnccacg aacacaccaa gtttcaaatc ctttgttgct gccactgcct ctgtgacacc    240
   cccacaacag gggccagagg tggttggcac ccccagtccc ttgaaatccc ccagaagcag    300
   ctttcagagc ctctccttcc ccctcttcta catggagggg gaagaaaaag gattcaaang    360
   ganttncctg aggaaatgtt ggatgtggcc atgtttttga atgttttttt ttaaaatatt    420
   ttattactag cccacccatc aatttggaaa gatgaaattt gctcttactc ccataactga    480
   ttttaangtc cgaggcaaag ccnagttaaa aaaggaggta agtgtnac                 528
<210> 110
   <211> 472
   <212> DNA
   <213> Artificial Sequence
<220>
   <221> misc_feature
   <222> (27)..(27)
   <223> n is a, c, g, or t
<220>
   <221> misc_feature
   <222> (30)..(30)
   <223> n is a, c, g, or t
<220>
   <221> misc_feature
   <222> (32)..(32)
   <223> n is a, c, g, or t
<220>
   <221> misc_feature
   <222> (62)..(62)
   <223> n is a, c, g, or t
<220>
   <221> misc_feature
   <222> (77)..(77)
   <223> n is a, c, g, or t
<220>
   <221> misc_feature
   <222> (393)..(393)
   <223> n is a, c, g, or t
<220>
   <221> misc_feature
   <222> (403)..(403)
   <223> n is a, c, g, or t
<220>
   <221> misc_feature
   <222> (416)..(416)
   <223> n is a, c, g, or t
<220>
   <221> misc_feature
   <222> (424)..(424)
   <223> n is a, c, g, or t
<220>
   <221> misc_feature
   <222> (436)..(436)
   <223> n is a, c, g, or t
<220>
   <221> misc_feature
   <222> (451)..(451)
   <223> n is a, c, g, or t
<220>
   <221> misc_feature
   <222> (453)..(453)
   <223> n is a, c, g, or t
<400> 110
   aaccggaaga caccagtccg ggaaggnggn anagggtgca aatagttcta caaaccagtt     60
   gnacctgagc aaggtgnacc tccaagtgtg ggctcattag ggcaacatcc attcataaac    120
   caggaggtgg gccaggtgga agtaaaatct gagaagcttg acttcaagga cagagtccag    180
   ttcgaagatt gggtccctgg acaatatcac ccacgtccct ggcggaggaa ataaaaagat    240
   tgaaacccac aagctgacct tccgcgagaa cgccaaagcc aagacagacc acggggcgga    300
   gatcgtgtac aagtcgccag tggtgtctgg ggacacgtct ccacggcatc tcagcaatgt    360
   ctcctccacc ggcagcatcg acatggtaga ctngccccag ctngccacgc tagctnacga    420
   ggtntctgcc ttcctnggca agcagggttt ntnatcaagg cccttggggg gt            472
<210> 111
   <211> 3747
   <212> DNA/RNA
   <213> Artificial Sequence
<400> 111
   cctcccctgg ggaggctcgc gttcccgctg ctcgcgcctg ccgcccgccg gcctcaggaa 60
   cgcgccctct cgccgcgcgc gccctcgcag tcaccgccac ccaccagctc cggcaccaac 120
   agcagcgccg ctgccaccgc ccaccttctg ccgccgccac cacagccacc ttctcctcct 180
   ccgctgtcct ctcccgtcct cgcctctgtc gactatcagg tgaactttga accaggatgg 240
   ctgagccccg ccaggagttc gaagtgatgg aagatcacgc tgggacgtac gggttggggg 300
   acaggaaaga tcaggggggc tacaccatgc accaagacca agagggtgac acggacgctg 360
   gcctgaaaga atctcccctg cagaccccca ctgaggacgg atctgaggaa ccgggctctg 420
   aaacctctga tgctaagagc actccaacag cggaagatgt gacagcaccc ttagtggatg 480
   agggagctcc cggcaagcag gctgccgcgc agccccacac ggagatccca gaaggaacca 540
   cagctgaaga agcaggcatt ggagacaccc ccagcctgga agacgaagct gctggtcacg 600
   tgacccaaga gcctgaaagt ggtaaggtgg tccaggaagg cttcctccga gagccaggcc 660
   ccccaggtct gagccaccag ctcatgtccg gcatgcctgg ggctcccctc ctgcctgagg 720
   gccccagaga ggccacacgc caaccttcgg ggacaggacc tgaggacaca gagggcggcc 780
   gccacgcccc tgagctgctc aagcaccagc ttctaggaga cctgcaccag gaggggccgc 840
   cgctgaaggg ggcagggggc aaagagaggc cggggagcaa ggaggaggtg gatgaagacc 900
   gcgacgtcga tgagtcctcc ccccaagact cccctccctc caaggcctcc ccagcccaag 960
   atgggcggcc tccccagaca gccgccagag aagccaccag catcccaggc ttcccagcgg 1020
   agggtgccat ccccctccct gtggatttcc tctccaaagt ttccacagag atcccagcct 1080
   cagagcccga cgggcccagt gtagggcggg ccaaagggca ggatgccccc ctggagttca 1140
   cgtttcacgt ggaaatcaca cccaacgtgc agaaggagca ggcgcactcg gaggagcatt 1200
   tgggaagggc tgcatttcca ggggcccctg gagaggggcc agaggcccgg ggcccctctt 1260
   tgggagagga cacaaaagag gctgaccttc cagagccctc tgaaaagcag cctgctgctg 1320
   ctccgcgggg gaagcccgtc agccgggtcc ctcaactcaa agctcgcatg gtcagtaaaa 1380
   gcaaagacgg gactggaagc gatgacaaaa aagccaagac atccacacgt tcctctgcta 1440
   aaaccttgaa aaataggcct tgccttagcc ccaaactccc cactcctggt agctcagacc 1500
   ctctgatcca accctccagc cctgctgtgt gcccagagcc accttcctct cctaaacacg 1560
   tctcttctgt cacttcccga actggcagtt ctggagcaaa ggagatgaaa ctcaaggggg 1620
   ctgatggtaa aacgaagatc gccacaccgc ggggagcagc ccctccaggc cagaagggcc 1680
   aggccaacgc caccaggatt ccagcaaaaa ccccgcccgc tccaaagaca ccacccagct 1740
   ctggtgaacc tccaaaatca ggggatcgca gcggctacag cagccccggc tccccaggca 1800
   ctcccggcag ccgctcccgc accccgtccc ttccaacccc acccacccgg gagcccaaga 1860
   aggtggcagt ggtccgtact ccacccaagt cgccgtcttc cgccaagagc cgcctgcaga 1920
   cagcccccgt gcccatgcca gacctgaaga atgtcaagtc caagatcggc tccactgaga 1980
   acctgaagca ccagccggga ggcgggaagg tgcagataat taataagaag ctggatctta 2040
   gcaacgtcca gtccaagtgt ggctcaaagg ataatatcaa acacgtcccg ggaggcggca 2100
   gtgtgcaaat agtctacaaa ccagttgacc tgagcaaggt gacctccaag tgtggctcat 2160
   taggcaacat ccatcataaa ccaggaggtg gccaggtgga agtaaaatct gagaagcttg 2220
   acttcaagga cagagtccag tcgaagattg ggtccctgga caatatcacc cacgtccctg 2280
   gcggaggaaa taaaaagatt gaaacccaca agctgacctt ccgcgagaac gccaaagcca 2340
   agacagacca cggggcggag atcgtgtaca agtcgccagt ggtgtctggg gacacgtctc 2400
   cacggcatct cagcaatgtc tcctccaccg gcagcatcga catggtagac tcgccccagc 2460
   tcgccacgct agctgacgag gtgtctgcct ccctggccaa gcagggtttg tgatcaggcc   2520
   cctggggcgg tcaataattg tggagaggag agaatgagag agtgtggaaa aaaaaagaat   2580
   aatgacccgg cccccgccct ctgcccccag ctgctcctcg cagttcggtt aattggttaa   2640
   tcacttaacc tgcttttgtc actcggcttt ggctcgggac ttcaaaatca gtgatgggag   2700
   taagagcaaa tttcatcttt ccaaattgat gggtgggcta gtaataaaat atttaaaaaa   2760
   aaacattcaa aaacatggcc acatccaaca tttcctcagg caattccttt tgattctttt   2820
   ttcttccccc tccatgtaga agagggagaa ggagaggctc tgaaagctgc ttctggggga   2880
   tttcaaggga ctgggggtgc caaccacctc tggccctgtt gtgggggttg tcacagaggc   2940
   agtggcagca acaaaggatt tgaaaacttt ggtgtgttcg tggagccaca ggcagacgat   3000
   gtcaaccttg tgtgagtgtg acgggggttg gggtggggcg ggaggccacg ggggaggccg   3060
   aggcaggggc tgggcagagg ggaggaggaa gcacaagaag tgggagtggg agaggaagcc   3120
   acgtgctgga gagtagacat ccccctcctt gccgctggga gagccaaggc ctatgccacc   3180
   tgcagcgtct gagcggccgc ctgtccttgg tggccggggg tgggggcctg ctgtgggtca   3240
   gtgtgccacc ctctgcaggg cagcctgtgg gagaagggac agcgggttaa aaagagaagg   3300
   caagcctggc aggagggttg gcacttcgat gatgacctcc ttagaaagac tgaccttgat   3360
   gtcttgagag cgctggcctc ttcctccctc cctgcagggt agggcgcctg agcctaggcg   3420
   gttccctctg ctccacagaa accctgtttt attgagttct gaaggttgga actgctgcca   3480
   tgattttggc cactttgcag acctgggact ttagggctaa ccagttctct ttgtaaggac   3540
   ttgtgcctct tgggagacgt ccacccgttt ccaagcctgg gccactggca tctctggagt   3600
   gtgtgggggt ctgggaggca ggtcccgagc cccctgtcct tcccacggcc actgcagtca   3660
   ccccgtctgc gccgctgtgc tgttgtctgc cgtgagagcc caatcactgc ctatacccct   3720
   catcacacgt cacaatgtcc cgaattc                                       3747
<210> 112
   <211> 418
   <212> DNA
   <213> Artificial Sequence
<220>
   <221> misc_feature
   <222> (233)..(233)
   <223> n is a, c, g, or t
<220>
   <221> misc_feature
   <222> (272)..(272)
   <223> n is a, c, g, or t
<220>
   <221> misc_feature
   <222> (289)..(289)
   <223> n is a, c, g, or t
<220>
   <221> misc_feature
   <222> (356)..(356)
   <223> n is a, c, g, or t
<220>
   <221> misc_feature
   <222> (402)..(402)
   <223> n is a, c, g, or t

<220>
   <221> misc_feature
   <222> (412)..(412)
   <223> n is a, c, g, or t
<400> 112
   tttttttttt ttttgaagag gacagctaat atttattgag cactgataac acaagtatca     60
   tctgggtcaa aactccacaa taattcaatg tgatactatt actattccta tcttattgat    120
   acttgaagca tgaaaggcac taagttgtca gcatttacaa tgatggcaag tgacagagcg    180
   gcgtgaagga cggcagtgca gacctaagcc taatctgaat tgccattcta tgnaaatgac    240
   tggtgatgtt gtgtagtgta cccctgggca anagatggga aaaagtgant gctgggtgga    300
   catccaacaa gtctgcatga caatagcccc cctgtctcca gtctcctctc tgatanatga    360
   catcccccac aaaccacaag gagtggatct ctctggcatg anagcccaac tntgtggg      418
<210> 113
   <211> 667
   <212> DNA
   <213> Artificial Sequence
<400> 113
   gctatgacat gattacgaat ttaatacgac tcactatagg gaatttggcc ctcgaggcca     60
   agaattcggc acgaggctta ccaactttta aaaacaagta aagttttgga gaatagtacc    120
   aagaactcaa atgatcctgc ggtattcaaa gacaacccca ctgaagacgt cgaataccag    180
   tgtgttgcag ataattgcca ttcccacgcc aaaatgttaa gtgaggttct gagggtgaag    240
   gtgatagccc cggtggatga ggtccagatt tctatcctgt caagtaaggt ggtggagtct    300
   ggagaggaca ttgtgctgca atgtgctgtg aatgaaggat ctggtcccat cacctataag    360
   ttttacagag aaaaagaggg caaacccttc tatcaaatga cctcaaatgc cacccaggca    420
   ttttggacca agcagaaggc taacaaggaa caggagggag agtattactg cacagccttc    480
   aacagagcca accacgcctc cagtgtcccc agaagcaaaa tactgacagt cagagtcatt    540
   cttgccccat ggaagaaagg acttattgca gtggttatca tcggagtgat cattgctctc    600
   ttgatcattg cggccaaatg ttattttctg aggaaagcca aggccaagca gatgccagtg    660
   gaaatgt                                                              667
<210> 114
   <211> 700
   <212> DNA
   <213> Artificial Sequence
<400> 114
   aaacagctat gacatgatta cgaatttaat acgactcact atagggaatt tggccctcga     60
   ggccaagaat tcggcacgag gcctcgtgcc gcctcgtgcc gaagccttag ggaagctggc    120
   ctgagagggg aagcggccct aagggagtgt ctaagaacaa aagcgaccca ttcagagact    180
   gtccctgaaa cctagtactg ccccccggta atgactccaa cttattgata gtgttttatg    240
   ttcagataat gcccgatgac tttgtcatgc agctccaccg attttgagaa cgacagcgac    300
   ttccgtccca gccgtgccag gtgctgcctc agattcaggt tatgccgctc aattcgctgc    360
   gtatatcgct tgctgattac gtgcagcttt cccttcaggc gggattcata cagcggccag    420
   ccatccgtca tccatatcac cacgtcaaag ggtgacagca ggctcataag acgccccagc    480
   gtcgccatag tgcgttcacc gaatacgtgc gcaacaaccg tcttccggag actgtcatac    540
   gcgtaaaaca gccagcgctg gcgcgattta gccccgacat agccccactg ttcgtccatt    600
   tccgcgcaga cgatgacgtc actgcccggc tgtatgcgcg aggttaccga ctgcggcctg    660
   agttttttaa gtgacgtaaa atcgtgttga ggccaacgcc                           700
<210> 115
   <211> 658
   <212> DNA
   <213> Artificial Sequence
<400> 115
   cacaggaaac agctatgaca tgattacgaa tttaatacga ctcactatag ggaatttggc     60
   cctcgaggcc aagaattcgg cacgagacca gcatctccca gttcataatc acaacccttc    120
   agatttgcct tattggcagc tctactctgg aggtttgttt agaagaagtg tgtcaccctt    180
   aggccagcac catctcttta cctcctaatt ccacaccctc actcgctgta gacatttgct    240
   atgagctggg gatgtctctc atgaccaaat gcttttcctc aaagggagag agtgctattg    300
   tagagccaga ggtctggccc tatgcttccg gcctcctgtc cctcatccat agcacctcca    360
   catacctggc cctgagcctt ggtgtgctgt atccatccat ggggctgatt gtatgtacct    420
   tctacctctt ggctgccttg tgaaggaatt attcccatga gttggctggg aataagtgcc    480
   aggatggaat gatgggtcag ctgtatcagc acgtgtggcc tgttcttcta tgggttggac    540
   aacctcattg taactcactc tttaatctga gaggccacag cgcaatttta ttttattttt    600
   ctcatgatga ggttttctta acttaaaaga acatggatat aaacatgcta gcattata      658
<210> 116
   <211> 724
   <212> DNA
   <213> Artificial Sequence
<400> 116
   tgacctgatt acgccaagct tggcacgagg agcttttagc tgccagccct ggcccatcat     60
   gtagctgcag cacagccttc cctaacgttg caactggggg aaaaatcact ttccagtctg    120
   ttttgcaagg tgtgcatttc catcttgatt ccctgaaagt ccatctgctg catcggtcaa    180
   gagaaactcc acttgcatga agattgcacg cctgcagctt gcatctttgt tgcaaaacta    240
   gctacagaag agaagcaagg caaagtcttt tgtgctcccc tcccccatca aaggaaaggg    300
   gaaaatgtct cagtcgaaag gcaagaagcg aaaccctggc cttaaaattc caaaagaagc    360
   atttgaacaa cctcagacca gttccacacc acctcgagat ttagactcca aggcttgcat    420
   ttctattgga aatcagaact ttgaggtgaa ggcagatgac ctggagccta taatggaact    480
   gggacgaggt gcgtacgggg tggtggagaa gatgcggcac gtgcccagcg ggcagatcat    540
   ggcagtgaag cggatccgag ccacagtaaa tagccaggaa cagaaacggc tactgatgga    600
   tttggatatt tccatgagga cggtggactg tccattcact gtcacctttt atggcgcact    660
   gtttcgggag ggtgatatgt ggatctgcat ggagctcatg gatacatcac tagataaatt    720
   ctac                                                                 724
<210> 117
   <211> 1051
   <212> DNA
   <213> Artificial Sequence
<220>
   <221> misc_feature
   <222> (5)..(5)
   <223> n is a, c, g, or t
<220>
   <221> misc_feature
   <222> (15)..(15)
   <223> n is a, c, g, or t
<220>
   <221> misc_feature
   <222> (783)..(783)
   <223> n is a, c, g, or t
<220>
   <221> misc_feature
   <222> (794)..(794)
   <223> n is a, c, g, or t
<220>
   <221> misc_feature
   <222> (823)..(823)
   <223> n is a, c, g, or t
<220>
   <221> misc_feature
   <222> (829)..(829)
   <223> n is a, c, g, or t
<220>
   <221> misc_feature
   <222> (836)..(836)
   <223> n is a, c, g, or t
<220>
   <221> misc_feature
   <222> (843)..(843)
   <223> n is a, c, g, or t
<220>
   <221> misc_feature
   <222> (846)..(846)
   <223> n is a, c, g, or t
<220>
   <221> misc_feature
   <222> (856)..(856)
   <223> n is a, c, g, or t
<220>
   <221> misc_feature
   <222> (867)..(867)
   <223> n is a, c, g, or t
<220>
   <221> misc_feature
   <222> (893)..(893)
   <223> n is a, c, g, or t
<220>
   <221> misc_feature
   <222> (896)..(897)
   <223> n is a, c, g, or t
<220>
   <221> misc_feature
   <222> (907)..(907)
   <223> n is a, c, g, or t
<220>
   <221> misc_feature
   <222> (911)..(941)
   <223> n is a, c, g, or t
<220>
   <221> misc_feature
   <222> (943)..(947)
   <223> n is a, c, g, or t
<220>
   <221> misc_feature
   <222> (951)..(953)
   <223> n is a, c, g, or t
<220>
   <221> misc_feature
   <222> (957)..(957)
   <223> n is a, c, g, or t
<220>
   <221> misc_feature
   <222> (959)..(960)
   <223> n is a, c, g, or t
<220>
   <221> misc_feature
   <222> (962)..(962)
   <223> n is a, c, g, or t
<220>
   <221> misc_feature
   <222> (967)..(967)
   <223> n is a, c, g, or t
<220>
   <221> misc_feature
   <222> (974)..(981)
   <223> n is a, c, g, or t
<220>
   <221> misc_feature
   <222> (985)..(985)
   <223> n is a, c, g, or t
<220>
   <221> misc_feature
   <222> (989)..(990)
   <223> n is a, c, g, or t
<220>
   <221> misc_feature
   <222> (993)..(993)
   <223> n is a, c, g, or t
<220>
   <221> misc_feature
   <222> (1005)..(1007)
   <223> n is a, c, g, or t
<220>
   <221> misc_feature
   <222> (1009)..(1011)
   <223> n is a, c, g, or t
<220>
   <221> misc_feature
   <222> (1017)..(1017)
   <223> n is a, c, g, or t
<220>
   <221> misc_feature
   <222> (1020)..(1027)
   <223> n is a, c, g, or t
<220>
   <221> misc_feature
   <222> (1029)..(1035)
   <223> n is a, c, g, or t
<220>
   <221> misc_feature
   <222> (1037)..(1040)
   <223> n is a, c, g, or t
<220>
   <221> misc_feature
   <222> (1044)..(1046)
   <223> n is a, c, g, or t
<220>
   <221> misc_feature
   <222> (1050)..(1050)
   <223> n is a, c, g, or t
<400> 117
   attancgact actangggaa tttggccctc gaggccaaga attcggcacg agggcgggga     60
   gccggggcag acgtccgtag cgccccctcc cgaggaggtc gagccgggca gtggggtccg    120
   catcgtggtg gagtactgtg aaccctgcgg cttcgaggcg acctacctgg agctggccag    180
   tgctgtgaag gagcagtatc cgggcatcga gatcgagtcg cgcctcgggg gcacaggtgc    240
   ctttgagata gagataaatg gacagctggt gttctccaag ctggagaatg ggggctttcc    300
   ctatgagaaa gatctcattg aggccatccg aagagccagt aatggagaaa ccctagaaaa    360
   gatcaccaac agccgtcctc cctgcgtcat cctgtgactg cacaggactc tgggttcctg    420
   ctctgttctg gggtccaaac cttggtctcc ctttggtcct gctgggagct ccccctgcct    480
   ctttccccta cttagctcct tagcaaagag accctggcct ccactttgcc ctttgggtac    540
   aaagaaggaa tagaagattc cgtggccttg ggggcaggag agagacactc tccatgaaca    600
   cttctccagc cacctcatac ccccttccca gggtaagtgc ccacgaaagc ccagtccact    660
   cttcgcctcg gtaatacctg tctgatgcca cagattttat ttattctccc ctaacccagg    720
   gcaatgtcag ctattggcag taaagtggcg ctacaaacac taaaaaaaaa aaaaaaaatt    780
   tcntgggggc cccnaaagtt tattcctttt tagggagggt tanttttant tttggncact    840
   ggnccntctt ttttanaacg tcgggantgg gaaaaaccct ggggttaccc aantanntcc    900
   cccttgnaaa nnnnnnnnnn nnnnnnnnnn nnnnnnnnnn nannnnnaaa nnntttnann    960
   tntttcnaat tttnnnnnnn ntccntttnn ggnaatttgg ccccnnngnn naaaaanttn   1020
   nnnnnnngnn nnnnnannnn gggnnnaaan t                                  1051
<210> 118
   <211> 781
   <212> DNA/RNA
   <213> Artificial Sequence
<400> 118
   gtcacacccg gaagcagggg cccgagcgga gccggccgcg atgagcgggg agccggggca    60
   gacgtccgta gcgccccctc ccgaggaggt cgagccgggc agtggggtcc gcatcgtggt   120
   ggagtactgt gaaccctgcg gcttcgaggc gacctacctg gagctggcca gtgctgtgaa   180
   ggagcagtat ccgggcatcg agatcgagtc gcgcctcggg ggcacaggtg cctttgagat   240
   agagataaat ggacagctgg tgttctccaa gctggagaat gggggctttc cctatgagaa   300
   agatctcatt gaggccatcc gaagagccag taatggagaa accctagaaa agatcaccaa   360
   cagccgtcct ccctgcgtca tcctgtgact gcacaggact ctgggttcct gctctgttct   420
   ggggtccaaa ccttggtctc cctttggtcc tgctgggagc tccccctgcc tctttcccct   480
   acttagctcc ttagcaaaga gaccctggcc tccactttgc cctttgggta caaagaagga   540
   atagaagatt ccgtggcctt gggggcagga gagagacact ctccatgaac acttctccag   600
   ccacctcata cccccttccc agggtaagtg cccacgaaag cccagtccac tcttcgcctc   660
   ggtaatacct gtctgatgcc acagatttta tttattctcc cctaacccag ggcaatgtca   720
   gctattggca gtaaagtggc gctacaaaca ctaaaaaaaa aaaaaaaaaa aaaaaaaaaa   780
   a                                                                    781

## Description

### I - Field of the invention

The present invention relates to polynucleotide analysis and, in particular, to polynucleotide expression profiling of breast tumors and cancers using arrays of polynucleotides.

### II - Background

The *ERBB2* oncogene, also called *HER2* or *NEU*, is located in band q12 of chromosome 17. It codes for a 185-kDa transmembrane tyrosine kinase related to members of the ERBB family, which also includes epidermal growth factor receptor. *ERBB2* is amplified and overexpressed in 15-30% of breast cancers (1). Although its exact role in mammary oncogenesis remains unclear (2, 3, for reviews), the receptor is a clinically relevant target for the treatment of breast cancer for two reasons. First, *ERBB2* gene amplification and overexpression of *ERRB2* gene products have been associated in many studies with prognosis or response to anticancer therapies (4, 5, for reviews). Second, therapy based on a humanized monoclonal antibody (trastuzumab/Herceptin^{™}) aimed at reducing the aberrant expression of the receptor has shown benefits in metastatic breast cancer patients (6-8, for reviews). However, modifications of chemotherapy and hormonal therapy strategies based on ERBB2 status remain controversial. Furthermore, the clinical efficacy of trastuzumab is unexpectedly variable, implying that additional and/or alternate methods to accurately identify appropriate patients for treatment with ERBB2 antagonists may be warranted.

Currently, ERBB2 status is primarily determined by two different methods: fluorescence in situ hybridization (FISH), which reveals gene amplification, and immunohistochemistry (IHC), which detects the overexpressed ERBB2 protein (9-12, for recent reviews). FISH is a good method for ERBB2 testing but is technically more difficult to implement than IHC which is easier to perform but difficult to standardize (13). IHC is currently the only FDA-approved test for selection of patients for treatment with trastuzumab, and American Society for Clinical Oncology and National Comprehensive Cancer Network guidelines recommend the use of either FISH (PathVysion^{™}) or the HercepTest^{™}, a specific IHC test made by the Dako Corporation. This method includes a calibrated internal control to semi-quantitatively assess positive staining on a scale ranging from 0 (absence of ERBB2 protein overexpression) to 3+ (maximum of ERBB2 overexpression). Results are scored by a pathologist; interpretation is relatively straightforward in ERBB2-negative individuals (0-1+) and in patients who strongly overexpress the protein (3+); accurate scoring is however problematic for the intermediate level 2+. For cases scoring 2+ (10-15% of all breast cancers), the concordance with FISH is, at best, 25 %. Importantly, a proportion of 2+ cases are *bona fide* ERBB2-overexpressing tumors to which Herceptin treatment should be applied. Thus, universal, accurate, and standardized determination of ERBB2 status has not yet been achieved. The reliability of this determination will greatly influence the selection of the relevant cases and thus the clinical efficacy of Herceptin treatment. Moreover, the establishment of specific methods for patient selection for ERBB2 antagonists may serve as a paradigm for guiding clinical use of the new targeted approaches expected in the near future. It is thus important to further document the methods and parameters useful to assess ERBB2 status.

Moreover, preliminary reports suggest that clinical outcome may vary between patients with the same ERBB2 status and treatment, implying that other factors, in addition to ERBB2, may play a role in determining the level of sensitivity to trastuzumab. Additionally, it may be necessary to associate other targeted therapies to anti-ERBB2 treatment and identification of complementary or secondary targets may thus prove useful to guide selection of appropriate combination therapy. These secondary targets may contribute to activation of pathways associated with response to ERBB2 hyperactivity. Although the common pathways such as the RAS/MAPK pathway and other induced genes have been reported (14), ERBB2-associated signaling cascades have yet to be elucidated. Thus, accurate measurement of ERBB2 status as well as identification of associated molecular alterations are now intensively required.

The effect of surgery on proliferation of breast carcinomas, in particular those overexpressing HER2 oncoprotein, has been recently assessed(67). It has been found that residual breast carcinomas that had been surgically removed within 48 days after first surgery showed a significant increase in proliferation if they were erbb2-positive. Treatment of erbb2-positive tumour cells with trastuzumab before adding a growth stimulus abolished drainage-fluid-induced proliferation. This suggests that erbb2 overexpression by breast carcinoma cells has a role in post surgical stimulation of proliferation of breast carcinoma cells.

Emerging technologies may facilitate progress on both ERBB2 typing and target discovery. Among these, DNA microarrays are currently prominent; they provide massive parallel quantification of mRNA expression levels for thousands of genes in a sample (15, 16, for recent reviews). Several reports have shown that this technology can be used to improve the prognostic classification of breast cancers (17-24). In the present invention, 217 breast carcinomas have been analyzed using DNA microarrays containing -9.000 spotted cDNA clones. Our aim was to identify differences in gene expression patterns between ERBB2-negative and ERBB2-positive breast tumors. We have identified a series of 37 discriminator genes/mRNA/ESTs called "*ERBB2* gene expression signature" the expression of which was able to distinguish ERBB2-negative and positive samples. This signature was independently validated by correlative IHC and FISH analyses. Among the genes included in the signature were potential additional targets, such as *GATA4*.

Document WO 02/059271 (GENE LOGIC, INC.) discloses sequences deduced from the examination of tissue from breast carcinomas to identify genes differentially expressed between tumor biopsies and normal tissue. This document discloses also diagnostic and screening methods using said disclosed genes as well as solid supports comprising oligonucleotide arrays that are complementary to or hybridize to the differentially expressed genes.

Document WO 02/10436 (URAGEN CORPORATION) discloses polypeptides and nucleic acids encoding them, having properties related to stimulation of biochemical or physiological responses in a cell, a tissue, an organ or an organism. More particularly, the polypeptides are gene products of genes. Methods of use of these polypeptides and gene are also disclosed, encompassing diagnostic and prognostic assay procedures as well as methods of treating diverse pathological conditions.

### III - Summary of the invention

The present invention provides a gene expression signature that can accurately identify *ERBB2* alteration in breast tumors, as well as provide interesting clues to enhance current understanding of the role of ERBB2 in mammary oncogenesis. This signature contains genes that are neighbours of *ERBB2* on 17q12, and includes potential regulators and/or downstream effectors of ERBB2 (GATA4) and eventual targets (cadherin, integrins). It may be used both for breast tumor management in clinical settings and as a research tool in academic laboratories.

A first object of the invention is to provide a method for identifying ERBB2 alteration in breast tumors based on the analysis of the over or under expression of polynucleotide sequences in a breast tissue sample, said analysis comprising the detection of at least one, preferably at least two or three, polynucleotide sequence or complement thereof, selected in each of the predefined polynucleotide sequence sets consisting of:
Set 1 : SEQ ID NO. 73, 74, 75, 76, 77 (ERBB2)
Set 2 : SEQ ID NO. 28, 29, 30 (GRB7)
Set 3 : SEQ ID NO. 83, 84, 85 (NR1D1)
Set 4 : SEQ ID NO. 78, 79, 80 (GATA4)
Set 5 : SEQ ID NO. 41, 42, 43 (CDH15)
Set 6 . SEQ ID NO. 16, 17 (LTA)
Set 7 : SEQ ID NO. 86, 87, 116(MAP2K6)
Set 8 : SEQ ID NO. 54, 55, 113(PECAM1)
Set 9 : SEQ ID NO. 44, 45 (PPARBP)
Set 10 : SEQ ID NO. 33, 34, 35 (PPP1R1B)
Set 11 : SEQ ID NO. 39, 40 (RPL19)
Set 12 . SEQ ID NO. 4, 5, 6 (PSMB3)
Set 13 : SEQ ID NO. 10 (LOC148696)
Set 14 : SEQ ID NO. 12, 13(NOL3/loc283849)
Set 15 : SEQ ID NO. 14, 15 (ITGA2B)
Set 16 . SEQ ID NO. 18, 19 (NFKBIE)
Set 17 : SEQ ID NO. 22, 23 (PADI2)
Set 18 : SEQ ID NO. 24,25 (STAT3 or als2cr18)
Set 19 : SEQ ID NO. 26, 27 (OAS2)
Set 20 : SEQ ID NO. 36, 37, 38 (CDKL5)
Set 21 : SEQ ID NO. 46, 47, 48 (CSTA)
Set 22 . SEQ ID NO. 52, 53, 115 (ITGB3)
Set 23 : SEQ ID NO. 56, 57, 58 (MKI67 or KI67)
Set 24 : SEQ ID NO. 59, 60, 61 (PBEF)
Set 25 : SEQ ID NO. 62, 63, 64 (FADS2)
Set 26 : SEQ ID NO. 81, 82 (LOX)
Set 27 : SEQ ID NO. 88, 89, 90(ITGA2)
SET 28 : SEQ ID NO. 11 (ESTAA878915/NA or EST)
SET 29 : SEQ ID NO. 1, 2, 3 (JDP1)
SET 30 . SEQ ID NO. 7, 8, 9 (NAT1)
SET 31 : SEQ ID NO. 20, 21 (CELSR2)
SET 32 : SEQ ID NO. 31, 32 (ESTN33243 or EST)
SET 33 : SEQ ID NO. 49, 50, 51 (SCUBE2)
SET 34 : SEQ ID NO. 65, 66 (ESTH29301/NA or EST)
SET 35 : SEQ ID NO. 67, 68, 69 (FLJ10193)
SET 36 : SEQ ID NO. 70, 71, 72 (ESR1).

A disease, disorder, e.g., tumor or condition "associated with" an aberrant expression of a nucleic acid refers to a disease, disorder, e.g., tumor or condition in a subject which is caused by, contributed to by, or causative of an aberrant level of expression of a nucleic acid.

The term "subsequence" refers to any sequence corresponding to a part of said polynucleotide sequence, and which would be also suitable to perform the method of analysis according to the invention. A person skilled in the art may choose the position and length of a subsequence by applying routine experiments.

The over or under expression may be determined by any known method of the prior art such as disclosed in PCT patent application WO 02103320. It may comprise the detection of difference in the expression of the polynucleotide sequences according to the present invention in relation to at least one control. Said control comprises for example polynucleotide sequence(s) from sample of the same patient or from a pool of patients ERBB2+ or ERBB2-, or selected among reference sequence(s) which may be already known to be over or under expressed. The expression level of said control can be an average or an absolute value of the expression of reference polynucleotide sequences. These values may be processed in order to accentuate the difference relative to the expression of the polynucleotide sequences of the invention.

The analysis of the over or under expression of polynucleotide sequences can be carried out on sample such as biological material derived from breast tissue. The method according to the invention may be performed on sample from a patient or an animal (for example for veterinary application or preclinical trial).

By "Over or underexpression" of a polynucleotide sequence it is meant that overexpression of certain sequences are detected simultaneously to the underexpression of others sequences. "Simultaneously" means concurrent with or within a biologic or functionally relevant period of time during which the over expression of a sequence may be followed by the under expression of another sequence, or conversely, e.g., because both expressions are directly or indirectly correlated.

Table 1 hereafter displays among others the library of polynucleotide sequences of the present invention. Table 1 indicates the name of the gene with its gene symbol, its clone reference (Image or Ipsogen in italic)and for each gene the relevant sequence(s) defining the set (identifications numbers : SEQ ID NO.). The present invention defines the nucleotide sequences by the different sets but it may covers also a definition of the polynucleotide sequences by the name of the gene or fragments thereof.

**Table 1**

| Gene symbol | Clone Image Or *Ipsogen* | Name | Seq3' SEQ IDNO. | Seq5' SEQ IDNO. | Ref SEQ IDNO. |
|---|---|---|---|---|---|
| JDP1 | 120138 | j domain containing protein 1 | 1 | 2 | 3 |
| PSMB3 | 145275 | proteasome (prosome, macropain) subunit, beta type, 3 | 4 | 5 | 6 |
| NAT1 | 145894 | n-acetyltransferase 1 (arylamine n-acetyltransferase) | 7 | 8 | 9 |
| LOC 148696 | 1467504 | hypothetical protein loc148696 | 10 | | |
| ESTAA 878915 | 1493187 | sapiens, clone image:4831215, mrna | 11 | | |
| NOL3/ loc283849 | 150483 | nucleolar protein 3 (apoptosis repressor with card domain) | 12 | | 13 |
| ITGA2B | 1506558 | integrin, alpha 2b (platelet glycoprotein iib of iib/iiia complex, antigen cd41b) | 14 | | 15 |
| LTA | 1524491 | lymphotoxin alpha (tnf superfamily, member 1) | 16 | | 17 |
| NFKBIE | 1573311 | nuclear factor of kappa light polypeptide gene enhancer in b-cells inhibitor, epsilon | 18 | | 19 |
| CELSR2 | 175103 | cadherin, egf lag seven-pass g-type receptor 2 (flamingo homolog, drosophila) | 20 | | 21 |
| PADI2 | 180060 | peptidyl arginine deiminase, type ii | 22 | | 23 |
| STAT3 | 1950914 | signal transducer and activator of transcription 3 (acute-phase response factor) | 24 | | 25 |
| OAS2 | | 2'-5'-oligoadenylate synthetase 2, 69/71kDa, transcript variant 2 | | 26 | 27 |
| GRB7 | 236059 | growth factor receptor-bound protein 7 | 28 | 29 | 30 |
| EST N33243 | 270561 | sapiens cdna flj33383 fis, clone brace2006514. | 31 | 32 | |
| PPP 1R1B | 277173 | protein phosphatase 1, regulatory (inhibitor) subunit 1b (dopamine and camp regulated phosphoprotein, darpp-32) | 33 | 34 | 35 |
| CDKL5 | 301018 | cyclin-dependent kinase-like 5 | 36 | 37 | 38 |
| RPL19 | 321041 | ribosomal protein 119 | | 39 | 40 |
| CDH15 | 327684 | cadherin 15, m-cadherin (myotubule) | 41 | 42 | 43 |
| PPARBP | 33696 | ppar binding protein | 44 | | 45 |
| CSTA | 345957 | cystatin a (stefin a) | 46 | 47 | 48 |
| SCUBE2 | 346321 | signal peptide, cub domain, egf-like 2 | 49 | 50 | 51 |
| ITGB3 | *0000143* | integrin, beta 3 (platelet glycoprotein IIIa, antigen CD61) | | 52, 115 | 53 |
| PECAM1 | *0000133* | platelet/endothelial cell adhesion molecule (CD31 antigen) | | 54, 113 | 55 |
| MKI67 | 428545 | antigen identified by monoclonal antibody ki-67 | 56 | 57 | 58 |
| PBEF | 488548 | pre-b-cell colony-enhancing factor | 59 | 60 | 61 |
| FADS2 | 51069 | fatty acid desaturase 2 | 62 | 63 | 64 |
| EST H29301 | 52616 | homo sapiens transcribed sequence with weak similarity to protein ref:np_060265.1 (h.sapiens) hypothetical protein flj20378 [homo sapiens] | 65 | 66 | |
| FLJ 10193 | 52635 | hypothetical protein flj10193 | 67 | 68 | 69 |
| ESR1 | 725321 | estrogen receptor 1 | 70 | 71 | 72 |
| ERBB2 | 726223 | v-erb-b2 erythroblastic leukemia viral oncogene homolog 2, neuro/glioblastoma derived oncogene homolog (avian) | 73 | 74 | 75 |
| ERBB2 | 756253 | v-erb-b2 erythroblastic leukemia viral oncogene homolog 2, neuro/glioblastoma derived oncogene homolog (avian) | 76 | 77 | 75 |
| GATA4 | 781738 | gata binding protein 4 | 78 | 79 | 80 |
| LOX | 789069 | lysyl oxidase | 81 | | 82 |
| NR1D1 | 795330 | nuclear receptor subfamily 1, group d, member 1 | 83 | 84 | 85 |
| MAP2K6 | *0000170* | mitogen-activated protein kinasekinase 6, transcript variant1 | | 86, 116 | 87 |
| ITGA2 | 811740 | integrin, alpha 2 (cd49b, alpha 2 subunit of vla-2 receptor) | 88 | 89 | 90 |
| RHOBTB3 | 147138 | rho-related btb domain containing 3 | 91 | 92 | 93 |
| B3GNT3 | 150897 | udp-glcnac:betagal beta-1,3-n-acetylglucosaminyltransferase 3 | 94 | 95 | |
| NUDT14 | 152718 | nudix (nucleoside diphosphate linked moiety x)-type motif 14 | 96 | 97 | 98 |
| | 159538 | | | 99 | |
| CASKIN1 | 166862 | cask interacting protein 1 | 100 | | 101 |
| KIF5C | 278430 | kinesin family member 5c | 102 | 103 | |
| DAXX | 292042 | death-associated protein 6 | 104 | 105 | 106 |
| ACTR1A | 342342 | arp1 actin-related protein 1 homolog a, centractin alpha (yeast) | 107 | | 108 |
| MAPT | 50764 | microtubule-associated protein tau | 109 | 110 | 111 |
| | 52898 | | 112 | | |
| | *0000135* | | | 114 | |
| C170RF37 | *0000367* | chromosome 17 open reading frame 37 | | 117 | 118 |

The present invention provides a method where the differential gene expression corresponds to an alteration of *ERBB2* gene expression in breast tumor.

The detection of over or under expression of polynucleotide sequences according to the method of the invention may be carried out by FISH or IHC. It may be performed on nucleic acids from a breast tissue sample.

The invention relates more particularly to a method performed on DNA microarrays.

The invention relates particularly to a method for monitoring the treatment of a patient with a breast cancer comprising the implementation of the above methods on nucleic acids in a breast tissue sample from said patient.

Advantageously, the method is performed on patient scoring +2 with the HercepTest^{™}.

Also advantageously, the method is performed on patients to determine their need to be pre-treated with erbb2 antagonist, e.g., Herceptin^{™} (trastuzumab), before surgical removal of erbb2 positive primary breast tumors. Treatment with erbb2 inhibitor such as Herceptin^{™}before ablation could reduce tumor proliferation and metastatic risk stimulated by surgical resection.

The invention further relates to a polynucleotide library useful for the molecular characterization of a breast cancer consisting of the polynucleotide sequences defined in the method of the present invention.

Such polynucleotide library can be immobilized on a solid support, for example selected from the group comprising nylon membrane, nitrocellulose membrane, glass slide, glass beads, membranes on glass support or silicon chip.

A method according to the present invention comprises:
a) reacting nucleic acids sample with complement polynucleotide sequences of the selected predefined polynucleotide sequences of the invention, and
c) detecting the reaction product of step (a).

The polynucleotide sample is labeled, e.g., before reaction step (a), and the label of the polynucleotide sample may be selected from the group consisting of radioactive, colorimetric, enzymatic, molecular amplification, bioluminescent or fluorescent labels. For example, prefered label can be selected in the group comprising : biotine, digoxygenin.

The method of the invention may further comprise obtaining a control polynucleotide sample, reacting said control sample with a polynucleotide library of the invention, detecting a control sample reaction product and comparing the amount of said polynucleotide sample reaction product to the amount of said control sample reaction product.

By "Nucleic acids" is meant polynucleotides, e.g., isolated, such as deoxyribonucleic acid (DNA), and, where appropriate, ribonucleic acid (RNA). The term should also be understood to include, as equivalents, analogs of RNA or DNA made from nucleotide analogs, and, as applicable to the embodiment being described, single (sense or antisense) and double-stranded polynucleotides. ESTs, chromosomes, cDNAs, mRNAs, and rRNAs are representative examples of molecules that may be referred to as nucleic acids. DNA may be obtained from said nucleic acids sample and RNA may be obtained by transcription of said DNA. In addition, mRNA may be isolated from said nucleic acids sample and cDNA may be obtained by reverse transcription of said mRNA.

The present invention is useful for detecting, diagnosing, staging, or monitoring, breast cancer. It is particularly useful for predicting clinical outcome of breast cancer and/or predicting occurrence of metastatic relapse and/or determining the stage or aggressiveness of a breast disease in at least 50%, e.g., at least 55%, e.g., at least 60%, e.g., at least 65%, e.g., at least 70%-, e.g., at least 75%, e.g., at least 80%, e.g., at least 85%, e.g., at least 90%, e.g., at least 95%, e.g., 100% of the patients. The invention is also useful for selecting more appropriate doses and/or schedule of chemotherapeutics and/or biopharmaceuticals and/or radiation therapy to circumvent toxicities in a patient.

By "aggressiveness of a breast disease" is meant, e.g., cancer growth rate or potential to metastasise; a so-called "aggressive cancer" will grow or metastasise rapidly or significantly affect overall health status and quality of life.

By "predicting clinical outcome" is meant, e.g., the ability for a skilled artisan to classify patients into at least two classes good vs, poor prognosis showing significantly different long-term Metastasis Free Survival (MFS)

A method for treating a patient with a breast cancer is also disclosed. This method comprises i) implementing a method of analysing of differential gene expression profile according to the present invention on a sample from said patient, and ii) determining a treatment for this patient based on the analysis of differential gene expression profile obtained with said method. "treating" encompasses treating as well as ameliorating at least one symptom of the condition or disease.

The methods according to the present invention allows to achieve high specificity and sensitivity level of at least 80%, e.g., 85%, e.g., 90%, e.g., 93%, e.g., 95% e.g., 97%, e.g., 99%.

### IV - Description of the figures

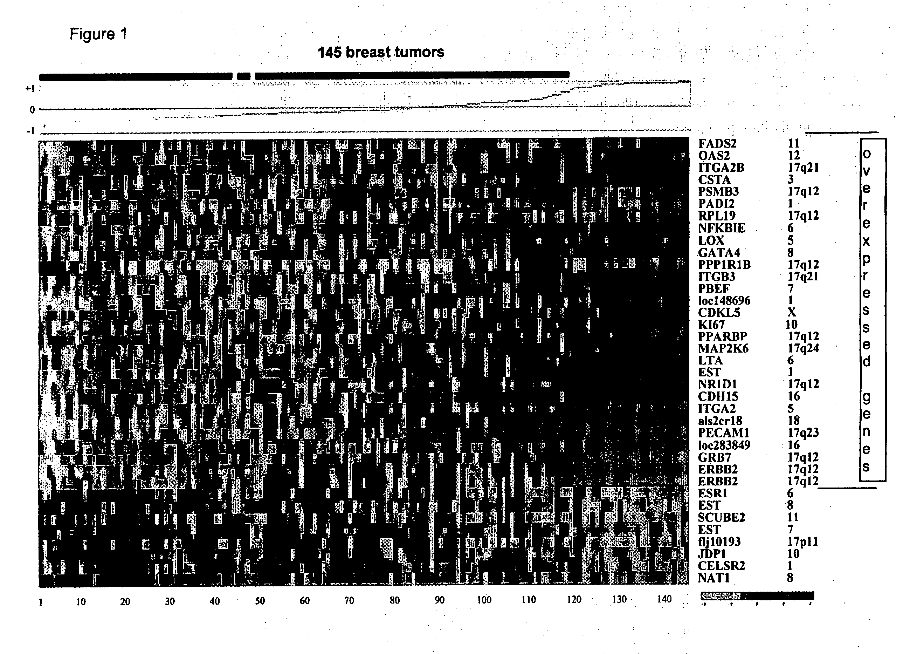

Figure 1 represents the supervised classification of 145 breast tumors using ERBB2 gene expression signature. Shown is the classification of the learning sample set (145 cases) by supervised analysis on the basis of 37 clones identified by iterative approach and defining our ERBB2 gene expression signature (GES). *Bottom panel:* Expression patterns of 37 cDNA clones in 145 samples. Each row represents a gene and each column represents a sample. Tumor samples are numbered from 1 to 145. Genes (right of panel) are referenced by their HUGO abbreviation as used in "Locus Link" (http://www.ncbi.nlm.nih.gov/LocusLink/) and their chromosomal location (including which arm for chromosome 17). EST (Expressed Sequenced Tag) is used for clones without similarity with known gene or protein. Samples are ordered according to the correlation of their expression profile with the average profile of the ERBB2-positive group and genes are ordered by their discriminating score. Each cell in the matrix represents the expression level of a transcript in a single sample relative to its median abundance across all samples and is depicted according to a color scale shown at the bottom. Red and green indicate expression levels respectively above and below the median. The magnitude of deviation from the median is represented by the color saturation. Grey indicates missing data. *Top panel*: The ERBB2 IHC status (HerceptTest) for each tumor sample is shown: white square indicates sample scored 3+ and black square indicates sample scored 0-1+.

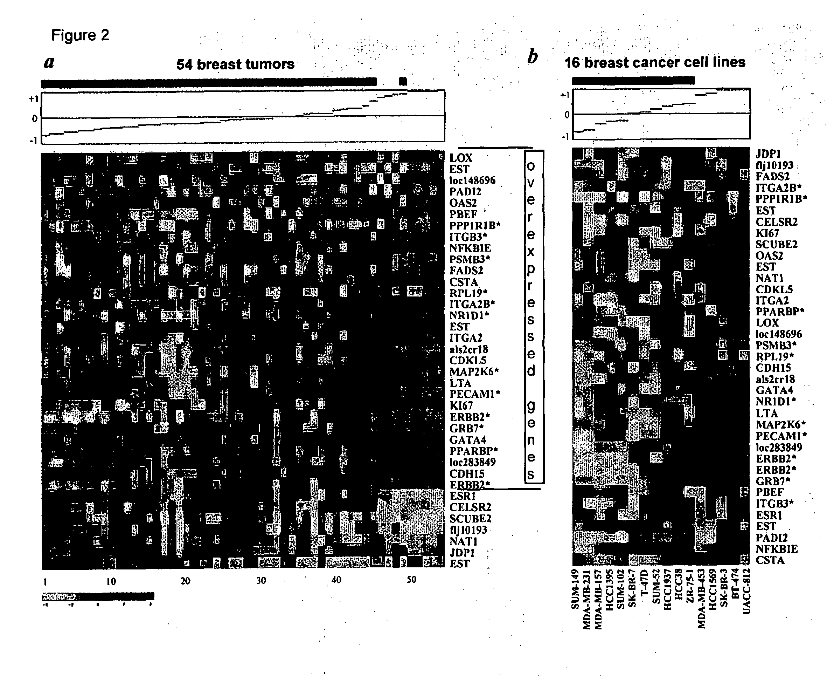

Figure 2 represents the validation of the ERBB2 gene expression signature. The ERBB2 gene expression signature according to the present invention (37 genes/ESTs) was used for classifying independent series of breast cancer samples.
   a, Same as for Figure 1, applied to the expression data of 54 additional breast cancers (validation set). Genes/ESTs located on 17q are marked with *.
   *b*, Same as for Figure 1, applied to the expression data of 16 breast cancer cell lines. *Top panel*: The ERBB2 status for each cell line is shown: white square indicates amplification and/or high mRNA expression of the *ERBB2* gene and black square indicates no amplification and no overexpression.

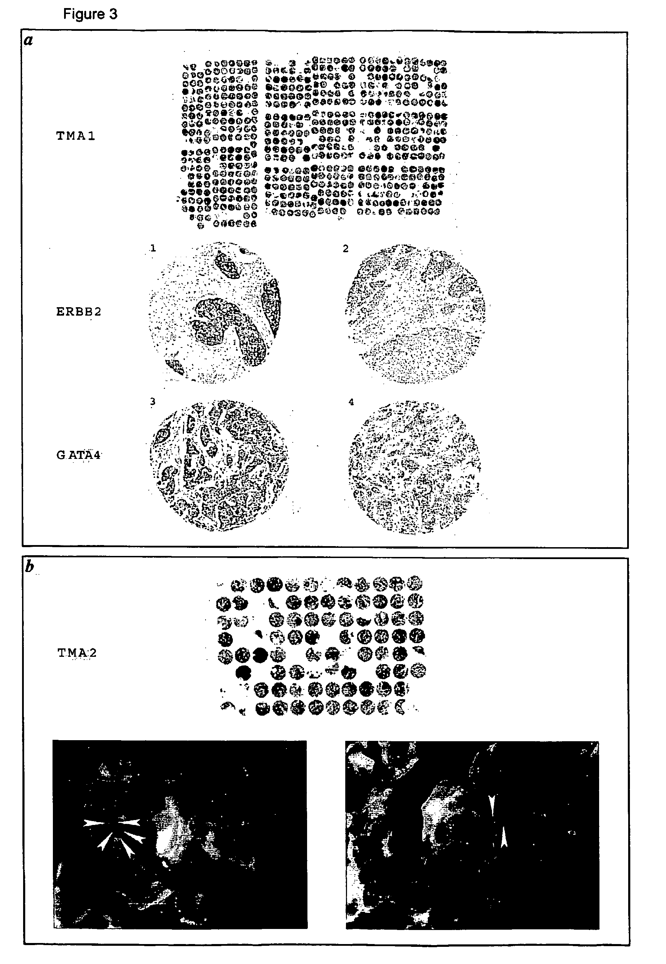

Figure 3. a. represents the Analysis of protein expression using immunohistochemistry on tissue microarray sections. On top, hematoxylin-eosin staining (H & E) of paraffin block section (25x30 mm) from TMA1 containing 552 tumors and control samples. Examples of IHC staining are shown below. Section 1 shows a sample with ERBB2 expression equal to 3+ and section 2 a sample with no detected ERBB2 expression. Section 3 shows a sample with GATA4 expression equal to Q = 300, and section 4 a sample with no GATA4 expression.
Figure 3. b. represents the analysis of ERBB2 gene copy number in breast tumors using fluorescence in situ hybridization on tissue microarray sections. Top panel, H & E of paraffin block section (25x30 mm) from TMA2 containing 94 tumors. Below, two sections of invasive breast carcinomas are shown, the first with ERBB2 amplification and the second with normal gene copy number. Red dots (arrows) represent ERBB2 copies and green dots represent centromere 17, on interphase chromosomes.

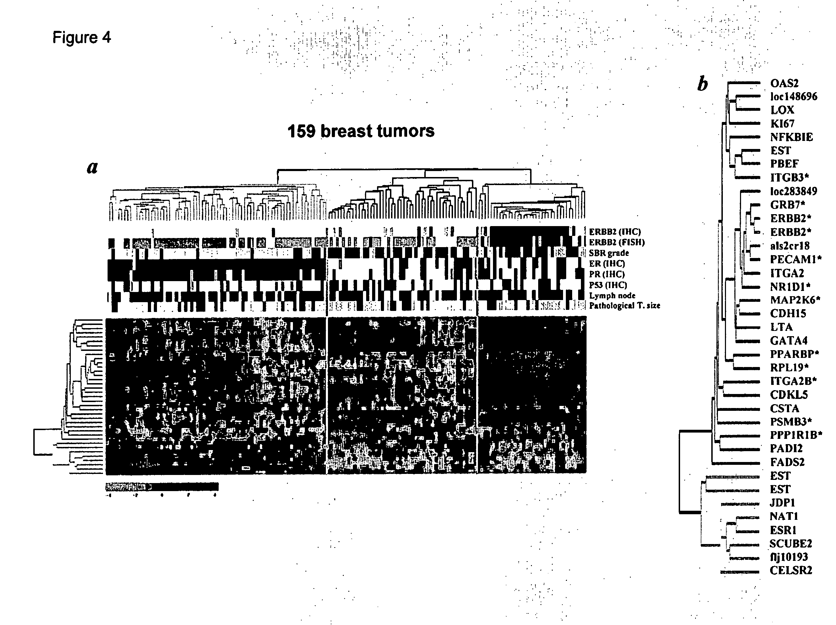

Figure 4 represents unsupervised hierarchical classification of 159 breast tumors using genes from the ERBB2 gene expression signature. *a*, Hierarchical clustering of 159 breast tumors and 37 clones from the ERBB2 gene expression signature. Each row represents a clone and each column represents a sample. Expression level of each gene in a single sample is relative to its median abundance across all samples and is depicted according to a color scale shown at the bottom. Red and green indicate expression levels respectively above and below the median. The magnitude of deviation from the median is represented by the color saturation. Grey indicates missing data. Dendrograms of samples (above data matrix) and genes (to the left of matrix) represent overall similarities in gene expression profiles. The orange vertical lines mark the subdivision into three main tumor groups; they are repesented in the branches of dendrogram in green (A), black (B) and red (C), respectively. The dendrogram of genes is zoomed in b. Between the dendrogram of samples and the data matrix are represented according to a grey color ladder relevant histoclinical data for the 159 tumors: ERBB2 IHC status (HercepTest: 0-1+, white; 2+, light grey; 3+, black; unavailable; dark grey), ERBB2 FISH status (negative, white; positive, black; unavailable, dark grey), SBR grade (1, white; 2, light grey; 3, black; unavailable, dark grey), ER, PR and P53 IHC status (negative, white; positive, black, unavailable, dark grey), axillary lymph node invasion (negative, white, positive, black), pathological size of tumors (pT1, white; pT2, light grey, pT3, black). *b*, Dendrogram of genes referenced by their HUGO abbreviation. Genes/ESTs located on 17q are marked with *. The "ERBB2 cluster" (red branches) and the "ER cluster" (green branches) respectively contain the *ERBB2* and *ESR1* genes.

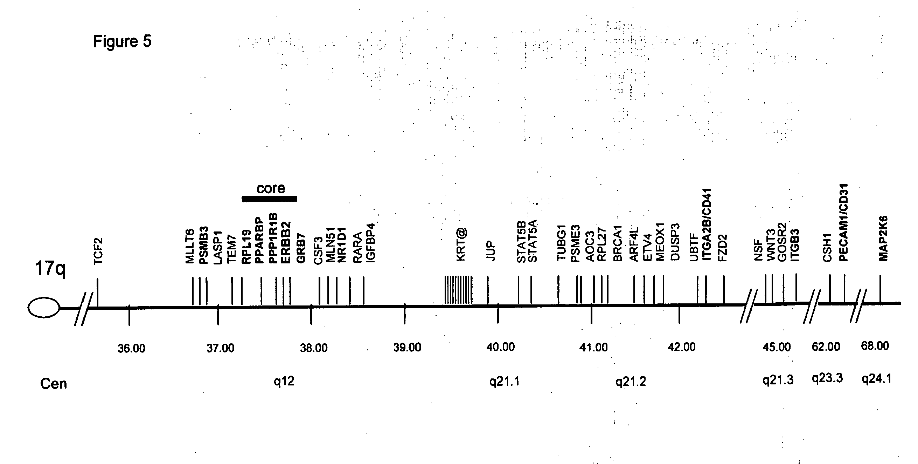

Figure 5 shows localization of genes from chromosome 17q12-24 region represented on the DNA microarray. Genes whose expression were upregulated in our ERBB2 breast cancer series as identified by supervised analysis of gene expression profiling using DNA microarrays are indicated in bold. The other genes indicated were represented on the microarray but were not found in the ERBB2 signature. The list of genes is not thorough for genes located outside 17q12. From several studies we can point to a "core" of genes almost always co-overexpressed with *ERBB2.* @ _{:} gene cluster.

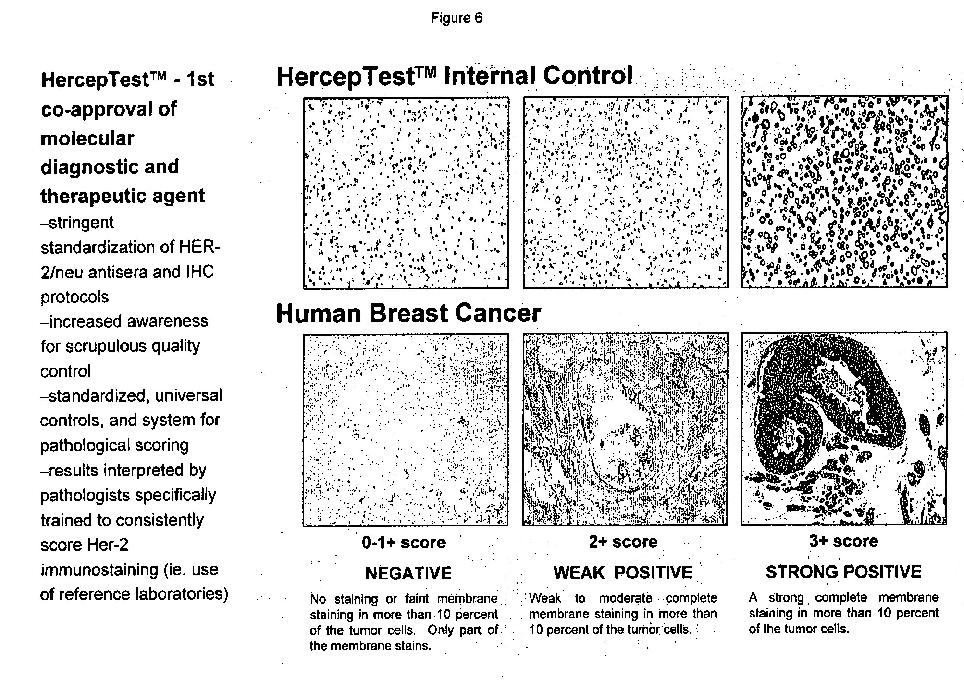

Figure 6 represents Herceptest^{™} assessing HER-2/neu Status in Patients.

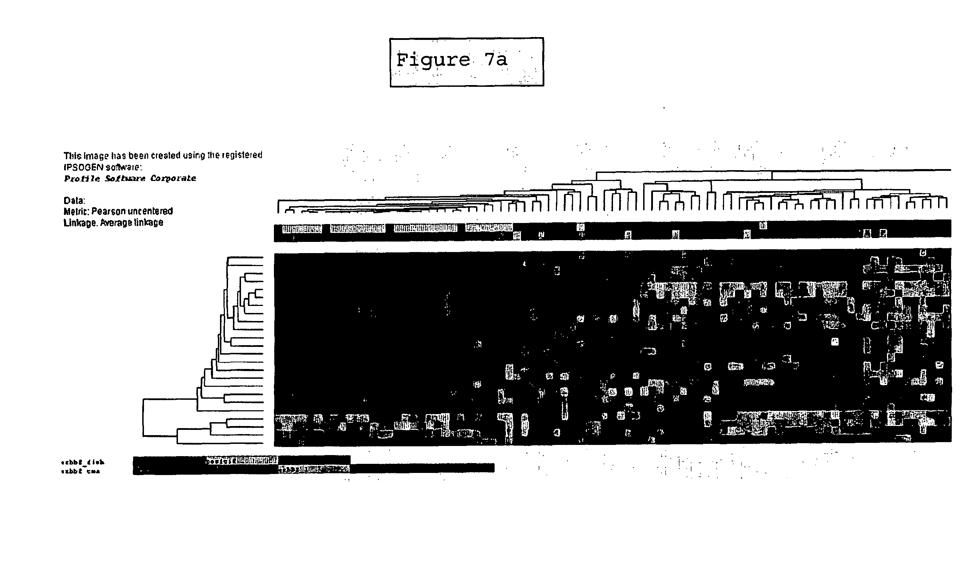

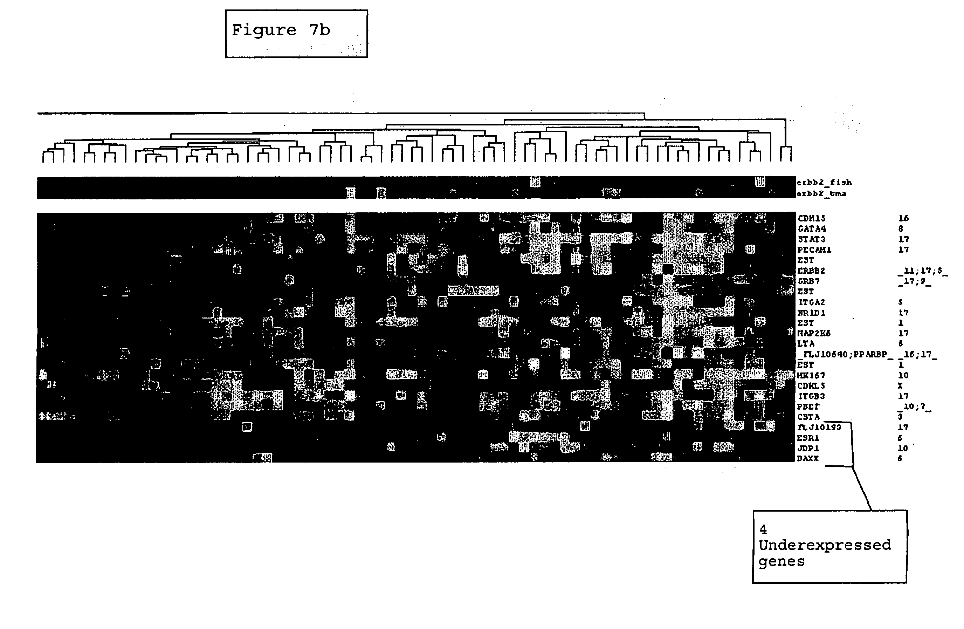

Figure 7 represents another unsupervised hierarchical classification of 159 breast tumors (split in two parts 7a and 7b due to figure lenght) on the basis of 24 clones identified by iterative approach and defining our ERBB2 gene expression signature (GES). Underexpressed genes are indicated, the others are overexpressed.

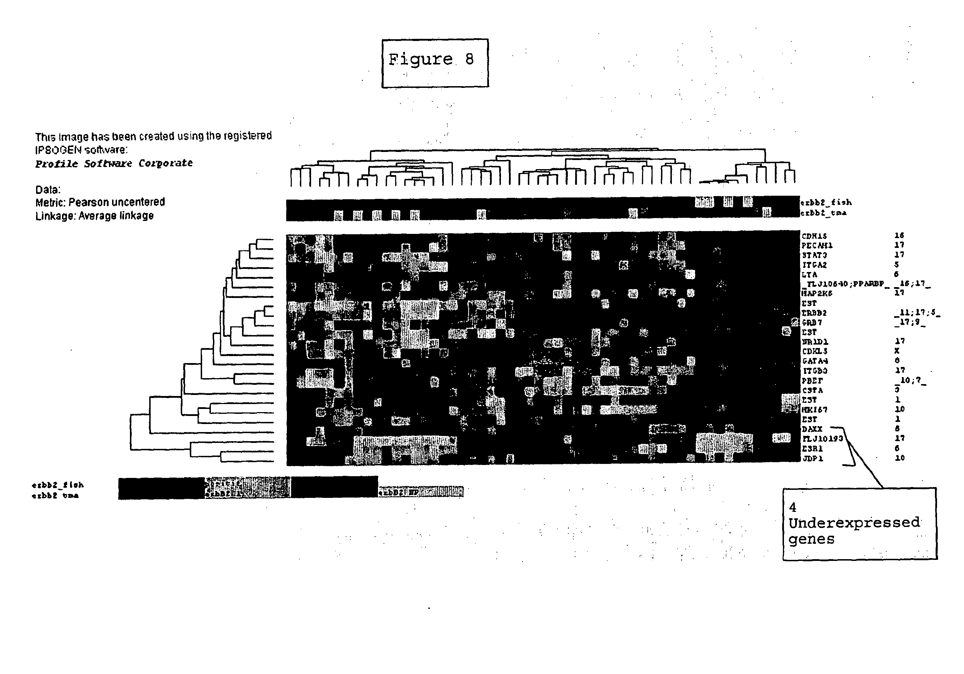

Figure 8 represents validation of the 24 clones (genes) signature presented on figure 7 on an independent set of 54 samples. Underexpressed genes are indicated, the others are overexpressed.

The row/colummn representation principle in Figs. 7 and 8 is as described for Fig 1.

### V - Detailed description of the invention

The present invention provides a set of genes that discriminate between ERBB2+ and ERBB2- breast tumors.

### V.1) Content of the signature

The identity of the discriminator genes gives insight in the underlying biological mechanisms associated with ERBB2 status and with the aggressive phenotype of ERBB2+ breast cancers. They also provide new diagnostic, prognostic and predictive factors, as well as new therapeutic targets.

Twenty-nine genes/ESTs were significantly overexpressed in ERBB2+ tumors. Their co-expression may indicate co-amplification (same chromosomal location), regulation by ERBB2, coregulation by common factors or association with unknown phenotypic feature of disease. In addition to *ERBB2* itself, there were 6 genes from region q12 of chromosome 17 in the signature (Figure 1); the 6 genes are all located within less than one megabase on either side of *ERBB2,* defining a small "core" region of co-expressed - probably co-amplified - genes (Figure 5). Overexpression of these genes with *ERBB2* may be associated with DNA amplification of the 17q12 amplicon; nevertheless, the functional affect of overabundant transcripts of these genes may impact on the clinical outcome in breast cancer patients. Indeed, this may be the case for example for *GRB7* or *PPARBP*. *GRB7*, a tyrosine kinase cytoplasmic adaptor substrate, has been implicated with different partners in integrin-mediated cell migration (33). *PPARBP* has been shown to downregulate P53-dependent apoptosis (34). Other genes from the microarray and located on 17q but further apart from *ERBB2* were not found in the signature except for *ITGA2B*/*CD41*, *ITGB3*/*CD61*, *PECAM1*/*CD31*, and *MAP2K6*. Overexpression of these genes may not be due to increased *ERBB2* gene copy number *per se* but may be triggered by intense ERBB2 signaling; it might also be due to the presence of other telomeric, 17q-associated amplicons (35, 36). *ITGA2*, whose gene is not on 17q, was also overexpressed in ERBB2+ tumors. There may be a other loci whose transcription is coordinately increased because the corresponding proteins belong to the same network. In total, four genes expressed in endothelial cells and platelets (encoding three integrins *ITGA2*, *ITGA2B*, *ITGB3*, and an adhesion molecule of the Ig family *PECAM1*) were overexpressed in ERBB2+ tumors (however, not all integrin genes from 17q present on the microarray were overexpressed since *ITGA3* was not). Collectively, these data suggest that neoangiogenesis and/or changes in blood vessel organization may play an important role in the pathogenesis of these tumors, and confirm that Herceptin and anti-cancer agents have an additive and/or synergistic activity. Other genes in the near vicinity of *ERBB2* locus may be co-amplified with *ERBB2* gene but may not be expressed due to the absence of an appropriate promoter or to repression. It is known that only a small proportion of genes from a given amplicon are overexpressed (37).

Other overexpressed genes were not located on chromosome arm 17q. CDH15, also called M-Cadherin or myotubule cadherin, is expressed in myoepithelial cells and may play a role in the muscle-like differentiation of these cells. It might suggest that ERBB2+ tumors have a certain degree of myoepithelial differentiation; alternatively they may be characterized by a high degree of dedifferentiation with appearance of new markers (this may also be true for other RNAs such as *PECAM1*). An interesting finding was *GATA4,* whose co-expression with ERBB2 was validated at the protein level. This gene codes for a transcription factor of the GATA family (38). It is expressed in adult vertebrate heart, gut epithelium, and gonads. *GATA4* is essential for cardiovascular development (39, 40), and regulate genes critical for myocardial differentiation and function. Likewise, ERBB2 is essential for heart development (41; reviewed in 42). Therefore ERBB2 may exert some of its downstream effects through GATA4 or alternatively, GATA4 may stimulate *ERBB2* gene transcription by positive feedback regulation. Incidentally, *MAP2K6* is also strongly expressed in cardiac muscle (43). The major adverse effect of Herceptin is cardiotoxicity (44). Investigation of the functional relationship between ERBB2, GATA4 and MAP2K6 may enhance current understanding of cardiotoxicities associated with ERBB2 antagonists, and contribute to design ways to circumvent this side-effect. Activation of GATA4 is thought to occur through RHO GTPases (45, 46), which are also central to the physiologic and pathophysiologic functions of integrins and cadherins (47, for review).

The works carried out under the present invention also shows ERBB2 and/or GATA4 variability, and ERBB2 and GATA4 co-variability may potentially serve as an inicator of patient risk shows a risk for cardiotoxicity by Herceptin treatment. Therefore, the present invention also relates to a method for determining the risk of averse cardiovascular secondary events for patients treated with Herceptin comprising the analysis of the differential expression GATA4 gene from a sample or cell line of said patient.

More particularly, this method comprises the detection of the over or under expression of at least one, preferably at least two or three, polynucleotide sequence, subsequence or complement thereof, selected in each of at least one predefined polynucleotide sequences sets consisting of:
Set 1 : SEQ ID NO. 73, 74, 75, 76, 77 (ERBB2)
Set 4 : SEQ ID NO. 78, 79, 80 (GATA4)

The *MKI67* gene encodes the proliferation marker Ki67/MIB1. It was upregulated in ERBB2+ samples, suggesting that ERBB2+ tumors are proliferative tumors. Immunohistochemical results on - 250 TMA1 tumors for ERBB2 and Ki67 stainings showed that expression of both proteins were correlated, confirming gene clustering at the protein level and in agreement with recent reports (48, 49). The overexpression of the *CSTA* gene, which encodes cystatin A, a cysteine protease inhibitor of the stefin family that acts as endogenous inhibitor of cathepsins, may be put in perspective with the finding of Oh et al. (14) on the downregulation of cathepsin D in ERBB2-transfected MCF-7 cells. Finally, the presence of genes encoding two structurally-related factors, lymphotoxin A (*LTA*) and preB-cell colony-enhancing factor (*PBEF*), and NFKBIE imply that specific immune and inflammatory mechanisms may be associated with ERBB2+ tumors.

Five genes with known function were downregulated in ERBB2-positive tumors. Interestingly, one of these was *ESR1,* which encodes estrogen receptor **_,** an important modulator hormone dependent mammary oncogenesis. It is recognized that most ERBB2-amplified tumors are ER-negative and resistant to hormone therapy (50-53). Moreover, an interplay between ERBB2 and ER pathways have been demonstrated (54). There were also *SCUBE2*, a gene encoding a secreted protein with an EGF-like domain (55), and *CELSR2,* which encodes a non-classical cadherin. These two molecules might have antagonistic regulatory roles of ERBB2 activities at the cell membrane. *SCUBE2* and *NAT1* were associated to *ESR1* in gene expression signature associated with ER positivity (24; data not shown).

### V.2) ERBB2 and microarrays

Several recent gene expression studies have adressed the issue of ERBB2 status and function in breast cancer. Most of them used cancer cell lines, and others included tissue samples.

An early large-scale study of the ERBB2 amplicon was done on 7 breast tumor cell lines by Kauraniemi et al. (30) using a custom-made cDNA microarray that included 217 clones from chromosome region 17q12. *ERBB2*, *GRB7*, *PPP1R1B* were consistently overexpressed when amplified, in conjunction with other genes that were not on our microarray. Willis et al. (56) used a commercially available oligonucleotide chip (Affymetrix GeneChip Hu35K) to study mRNA from 12 breast tumors and two cell lines also typed using comparative genomic hybridization. A total of 20 known genes showed significant overexpression in tumors with gains of region 17q12-23. These included *ERBB2*, *GRB7*, *PPARBP*, but also *MLLT6*, *KRT10* and *TUBG1* that were not identified in our signature. Wilson et al. (31) used a commercially available "breast specific" nylon microarray with -5.000 cDNAs to study cell lines and two sets of 5 ERBB2-positive and negative pooled breast tumors. Only few genes from 17q were among the upregulated genes; these included *RPL19* and *LASP1*. Dressman et al. (57) studied 34 tumors and established a gene expression signature specific of ERBB2+ samples that contained several 17q genes including *GRB7*, *NR1D1*, *PSMB3,* and *RPL19*. Sorlie et al. (24) have also defined ERBB2+ signature with five genes from 17q12, including *ERBB2*, *GRB7*

Genes located in the vicinity of *ERBB2* are frequently co-upregulated following DNA amplification. This phenomenon is less marked for genes located further apart from *ERBB2,* which may be included only when the amplification affects a large segment from the region. Some of the genes close to *ERBB2* did not appear in our signature whereas they were upregulated in other studies (i.e. *LASP1*, *MLLT6).* This may be due to a different proportion of tumors with variably-sized amplicons in the analyzed panels.

While amplification of region 17q12-21 affects *ERBB2* chromosomal neighbors, ERBB2 protein overexpression will affect downstream targets and possibly also upstream regulators via positive feedback regulatory mechanisms. Balance in cadherins and integrins and functional processes associated with cell-matrix adhesive systems seem particularly affected in ERBB2-positive tumors (31; this study). This suggests that ERBB2 oncogenic activity may be associated with cell motility, as has been proposed previously (58, 59). A recent study, using DNA microarrays from the Sanger center containing -6000 unique genes/ESTs, has described the transcriptional changes associated with a series of 61 genes following overexpression of a transfected *ERBB2* gene in an immortalized HB4a human mammary luminal epithelial cell line (60). Previously, several studies had identified genes whose transcription is affected by ERBB2 overexpression or amplification using differential screening (14, 61). Some of these genes are located near the *ERBB2* locus. Our GES shares no common gene with the list of Kumar-Sinha et al. (62) established in comparing cell lines including ERBB2-transfected cell line; however, a gene related to fatty acid biology, *FADS2*, is part of our signature. Tiwari et al. (63) reported a relationship between ERBB2, fatty acids and 2',5' oligoadenylate synthetases (*OAS2*), which is included in our "ERBB2 cluster". Peroxisome proliferator-activated receptors (PPARs) are known regulators of lipid metabolism; their trans-activating capacity depends on the recruitment of auxiliary proteins (64, for review. Modifications of fatty acid metabolism in ERBB2+ tumors may thus be associated with overexpression of PPARBP.

### V.3) ERBB2 signature and assessment of ERBB2 status

Alteration of ERBB2 is associated with poor prognosis (unfavorable clinical outcome with metastasis and death) and can be countered by a targeted therapy based on a humanized antibody, trastuzumab (Herceptin^{™}). Therefore, the determination of ERBB2 status is important in breast cancer management. Accurate quantitation of ERBB2 expression, however, has proved to be difficult in routine clinics since both IHC and FISH have limitations and may be influenced by many variables (9-13). As a consequence, there is still no consensus on the best method for assessing ERBB2 status. In routine practice, IHC, which more than FISH detects the actual target of Herceptin^{™}, is faster and more economic but highly dependent on fixative conditions, staining procedures, scoring system, quality controls and interlaboratory standardization. In addition, results are often difficult to interpret since a number of cases show only moderate overexpression of the protein and discrepancies in the results are subject to interobserver variability. FISH methods are quantitative and sensitive (65), but are also expensive, time-consuming and requires specialized expertise and equipment. Indeed, variable concordance between IHC and FISH, have led to the current practice of testing +2 HercepTest patients by both IHC and FISH to making a clinical decisions on whether to recommend treatment with anti-ERBB2 antagonists.

The work carried out for the present invention report the potential of DNA microarray-based gene expression profiling to establish ERBB2 status, and to identify among ERBB2 2+ cases those with gene amplification and those without. These observations need confirmation on larger series of tumors, notably for ERBB2 2+ samples.

In the near future, biochip specifically engineered to suit the needs and requirements of a hospital-based platform may be an interesting alternative method for establishing simultaneously in one test not only ERBB2 status but a series of parameters for breast cancer management, including predictive factors.

### VI - Material and methods

### VI.1) Breast carcinoma samples

Using DNA microarrays, we studied 217 breast cancer samples obtained from 210 women treated at the Institut Paoli-Calmettes between 1988 and 2001. Inclusion criteria of samples were: i) - sporadic primary localized breast cancer treated with surgery followed by adjuvant anthracyclin-based chemotherapy, ii) - tumor material quickly dissected and frozen in liquid nitrogen and stored at -160°C. Exclusion criteria included locally advanced or inflammatory or metastatic forms. The main characteristics of patients and tumors are listed in Table 2 hereafter.

**Table 2**

| Characteristic | | No (%)* |
|---|---|---|
| Age, years median (range) | | 53 (29, 83) |
| Histological type | | |
| | ductal | 166 (76) |
| | lobular | 25 (12) |
| | mixed | 12 (6) |
| | tubular | 4 (2) |
| | medullary | 3 (2) |
| | other | 4 (2) |

| Axillary lymph node status | | |
|---|---|---|
| | negative | 57 (26) |
| | positive | 160 (74) |

| Pathological tumor size | | |
|---|---|---|
| | pT1 | 59 (27) |
| | pT2 | 117 (54) |
| | pT3 | 41 (19) |

| SBR grade | | |
|---|---|---|
| | I | 32 (15) |
| | II | 99 (46) |
| | III | 85 (39) |

| Peritumoral vascular invasion | | |
|---|---|---|
| | absent | 115 (53) |
| | present | 101 (47) |

| ER status (IHC) | | |
|---|---|---|
| | negative | 72 (34) |
| | positive | 142 (66) |

| PR status (IHC) | | |
|---|---|---|
| | negative | 80 (38) |
| | positive | 130 (62) |

| ERBB2 status (IHC) | | |
|---|---|---|
| | 0-1+ | 162 (78) |
| | 2+ | 10 (4) |
| | 3+ | 37 (18) |

| P53 status (IHC) | | |
|---|---|---|
| | negative | 144 (69) |
| | positive | 65 (31) |

| ERBB2 status (FISH) | | |
|---|---|---|
| | negative | 38 (56) |
| | positive | 30 (44) |

| | | |
|---|---|---|
| *, % of evaluated cases | | |

Immunohistochemical parameters collected included estrogen receptor (ER), progesterone receptor (PR) and P53 status (positivity cut-off values of 1%), and ERBB2 status (0-3+ score as illustrated by the HercepTest kit scoring guidelines). All tumor sections were reviewed de *novo* by two pathologists (E. C-J., J. J.) prior to analysis and all samples contained more than 50% tumor cells. The series of 217 samples was divided in two sets: a first set of 163 samples, from which was derived, before supervised analysis, a "learning" set of 145 samples, and a second set of 54 samples designated "validation" set.

A consecutive series of 552 women with unilateral localized invasive breast carcinomas treated at the Institut Paoli-Calmettes between June 1981 and December 1999 was studied using a first TMA designated TMA1. Of the 552 cases studied, 257 were available for ERBB2, GATA4, ER and Ki67 staining. According to the WHO classification, there were 194 ductal, 26 lobular, 10 tubular, 3 medullary carcinomas and 24 other histological types. The average age at diagnosis was 59 years, median age 60, and range 25 to 91. A total of 135 tumors were associated with lymph node invasion, and 199 were positive for ER. A set of 94 tumors (chosen within tumors analyzed by DNA microarrays) was included in a second TMA designated TMA2.

### VI.2) Breast tumor cell lines

Except for SUM-52, SUM-102, and SUM-149 (a gift of S.P. Ethier, Ann Arbor, MI - http://www.cancer.med.umich.edu/breast cell/clines/clines.h tml), the breast cancer cell lines (BT-474, HCC38, HCC1395, HCC1569, HCC1937, MDA-MB-157, MDA-MB-231, MDA-MB-453, SK-BR-3, SK-BR-7, T-47D, UACC-812, and ZR-75-1) were obtained from ATCC (Rockville, MD; http://www.atcc.org/). All cell lines were grown according to the recommendations of the supplier.

### VI.3) RNA extraction

Total RNA was extracted from frozen tumor samples and cell lines by standard methods using guanidinium isothiocyanate solution and centrifugation on cesium chloride cushion, as previously described (25). RNA integrity was controlled by electrophoresis on agarose gels and by Agilent analysis (Bioanalyzer, Palo Alto, CA) before labeling.

### VI.4) Construction of DNA microarrays

PCR products from a total of 9038 Image clones, including 3910 expressed sequenced tags (EST) and 5125 known genes, were spotted on 12 x 8.5 cm² nylon filters with a Microgrid II robot (Biorobotics Apogent Discoveries, http://www.apogentdiscoveries.com/BioRobotics.asp). Several controls were included in the microarrays, such as poly(A)⁺ stretches, plant cDNAs, and PCR controls. Microarray spotting and hybridization processes were done as previously described (19).

### VI.5) DNA microarray data analysis and statistical methods

Hybridizations of microarrays membranes were done with radioactive [alpha-³³P]-dCTP-labeled probes made from 5 µg of total RNA from each sample according to described protocols (http:/tagc.univ-mrs.fr/pub/Cancer/). Membranes were then washed, exposed to phosphor-imaging plates and scanned with a FUJI BAS 1500 machine. Signal intensities were quantified with ArrayGauge software (Fuji, Düsseldorf, Germany), normalized for amount of spotted DNA (21) and the variability of experimental conditions using non-linear rank-based methods (26), then log-transformed. We first applied supervised analysis to identify the optimal set of genes which best discriminated between ERBB2-negative and positive breast cancer samples. The positivity cut-off of ERBB2 status was defined by protein expression using IHC (HercepTest^{™} kit): positive status was defined as 3+ and negative status as 0 or 1+. Analysis was done in two steps: the molecular signature was first derived through training on a set of 145 samples (learning set, including 116 ERBB2-negative and 29 ERBB2-positive samples); samples with ERBB2 status 2+ (n=10) or unavailable (n=8) were not included in the supervised analysis. It was then validated on the set of 54 samples (validation set, including 46 ERBB2-negative and 8 ERBB2-positive samples).

ProfileSoftware^{™} Corporate (Ipsogen, Marseille) was utilized for all analyses. This program uses a discriminating score (DS) (17) combined with iterative random permutation tests. The DS was calculated for each gene as DS=(M1-M2)/(S1+S2) where M1 and S1 respectively represent mean and standard deviation of expression levels of the gene in subgroup 1 (ERBB2-positive), and M2 and S2 in subgroup 2 (ERBB2-negative). Statistical confidence levels were estimated by bootstrap resampling as previously described (27) with a false positive rate of 2/10000. Briefly, approximately two-thirds (n=106) of the samples from the learning set (n=145) were randomly selected to include at least 20 ERBB2-positive cases. They were then submitted to supervised analysis described above. The process was repeated 30 times (30 randomly defined subgroups of 106 samples), thus generating 30 lists of genes. These lists were then compared and a gene was considered as discriminator if present in at least 25 gene-lists out of 30; allowing the identification of the most relevant genes, independent of the sample set used.

Unsupervised hierarchical clustering was applied to investigate relationships between samples and relationships between genes identified by supervised analysis. The hierarchical clustering was applied to data log-transformed and median-centred on genes using the *ProfileSoftware*^{™} Corporate program (Ipsogen, Marseille) (average linkage clustering using uncentered Pearson correlation as similarity metric) and results were displayed with the same program.

### VI.6) Construction of Tissue microarrays

Two TMA, TMA1 (552 samples) and TMA2 (94 samples), were prepared as described (28) with slight modifications (29). For each tumor, a representative tumor area was carefully selected by histopathological analysis of a hematoxylin-eosin stained section of a donor block. Core cylinders (one for each tumor for TMA2 and three for each tumor for TMA1) with a diameter of 0.6 mm for TMA1 and 2 mm for TMA2, were punched from this area and deposited into a recipient paraffin block using a specific arraying device (Beecher Instruments, Silver Spring, MD). In addition to tumor tissues, the recipient block also included normal breast and established breast tumor cell lines to serve as internal controls: BT-474 known to have four to eight-fold amplification of the *ERBB2* gene, and MCF-7, whose chromosomes 17 have each one copy of the *ERBB2* gene (30). Five-µm sections of the resulting array block were mounted onto glass slides and used for IHC (TMA1) and FISH (TMA2) analyses. The reliability of the method was assessed by comparison with conventional sections for the usual prognostic parameters (including estrogen receptor and ERBB2); the value of the kappa test was 0.95 (29).

### VI.7) Antibodies

The following antibodies were used for IHC: polyclonal antibody anti-ERBB2 (Dako-HercepTest^{™} , Copenhagen, Denmark), used strictly following the guidelines described by the manufacturer; goat polyclonal antibody anti-GATA4 (sc-1237, 1:50 dilution; Santa Cruz Biotechnology, Inc., Santa Cruz, CA), anti-MIB1/Ki67 (1:100 dilution, Dako), anti-ER (clone 6F11, 1:60 dilution, Novocastra Laboratories).

### VI.8) Immunohistochemistry

IHC was done on five-µm sections of TMA1. Briefly, tissues were deparaffinized in Histolemon (Carlo Erba Reagenti, Rodano, Italy) and rehydrated in graded alcohol. Antigen retrieval was done by incubation at 98°C in citrate buffer. Slides were transferred to a Dako autostainer, except for Dako-HercepTest^{™} where guidelines are imposed by the manufacturer. Staining was done at room temperature as follows: after washes in phosphate buffer, endogenous peroxidase activity was quenched by treatment with 0.1% H₂O₂, slides were pre-incubated with blocking serum (Dako Corporation) for 10 min, then incubated with the affinity-purified antibody for one hour. After washes, slides were incubated with biotinylated antibody against rabbit IgG for 20 min followed by streptadivin-conjugated peroxidase (Dako LSAB^{R}2 kit). Immunoreactive complexes were visualized with the peroxidase substrate, diaminobenzidine, counter-stained with hematoxylin, and coverslipped using Aquatex (Merck, Darmstadt, Germany) mounting solution. Slides were evaluated under a light microscope by three pathologists (E. C-J., J. H., J. J.).

Immunoreactivities for GATA4 and ER were classified by estimating the percentage (P) of tumor cells showing characteristic staining (from undetectable level or 0%, to homogenous staining or 100%) and by estimating the intensity (I) of staining (weak staining or 1, moderate staining or 2, strong staining or 3). Results were scored by multiplying the percentage of positive cells by the intensity, i.e. by the so-called quick score (Q) (Q = P x I; maximum = 300). For Ki67, only the percentage (P) of tumor cells was estimated, since intensity does not vary and for ERBB2, the status was defined using the Dako scale. Expression levels allowed to group tumors in two categories: no expression (Q = 0 for GATA4 and ER, P < 20 for Ki67, and 0/+ for ERBB2), and expression (Q > 0 for GATA4 and ER, P ≥ 20 for Ki67, and 2+/3+ for ERBB2). The average of the score of a minimum of two core biopsies was calculated for each case of TMA1.

### VI.9) ERBB2 gene amplification detected by FISH

FISH for *ERBB2* gene amplification was done on TMA2 using the Dako *ERBB2* FISH PharmDX^{™} Kit according to the manufacturer instructions. In brief, TMA2 sections were baked overnight at 55°C, deparaffinized in Histolemon (Carlo Erba Reagenti, Rodano, Italy), rehydrated in graded alcohol and washed in Dako wash buffer. Slides were pretreated by immersion in Dako pretreatment solution at 97°C for 10 min and cooled to room temperature. Slides were then washed in Dako wash buffer and immersed in Dako pepsin at room temperature for 10 min. Pepsin wasremoved with two changes of wash buffer. Slides were dehydrated in graded alcohol. Ten µl of HER2/CEN17 (centromere 17) Probe Mix (Dako) was added to the sample area of each section. Sections were coverslipped and the edges were sealed with rubber cement. Slides were placed on a flat metal surface and heated at 82°C for 5 min to codenature the probe and target DNA, and transferred to a preheated humidified hybridization chamber to hybridize the probe and DNA for 18 h at 45°C. After hybridization, the rubber cement and the coverslips were removed from the slides. Sections were washed in wash buffer at 65°C then at room temperature. Slides were dehydrated in graded alcohol and air-dried in the dark. Nuclei were counterstained with 15 µl of DAPI/antifade and coverslipped. Slides were stored at - 4°C in the dark for up to 7 days prior to analysis.

### VI.10) FISH scoring

Sections were examined with a fluorescent microscope (Zeiss-Axiophot) using the filter recommended by Dako. The invasive lesion selected for the TMA2 was easily localized under the microscope. Approximately forty malignant, non overlapping cell nuclei were scored for each case, and included and scored only if HER2 and CEN17 signals were clearly detected. A ratio of HER2/CEN17 was calculated for each specimen that met this inclusion criteria. *ERBB2* was considered as amplified when the FISH ratio HER2/CEN17 was >= 2.0. Each assay was read twice by two observers. Specimens were considered negative when less than 10% of tumor cells showed amplification of *ERBB2.*

### VI.11) Statistical analysis

Correlations between hierarchical clustering-based tumor groups and molecular and histoclinical parameters were investigated by using the Chi-2 test. All p-values were two-sided at the 5% level of significance. Distributions of molecular markers analyzed by TMA1 were compared using Fisher exact test.

### VII - Results

The mRNA expression profiles from 217 different human breast cancer samples and 16 breast cancer cell lines were determined with cDNA microarrays containing -9.000 spotted PCR products from known genes and ESTs. Analysis, both supervised and unsupervised, identified an ERBB2-specific gene expression signature (GES). To further validate this signature, studies were completed by FISH and IHC analyses on breast cancer tissue microarrays.

### VII.1) Identification and validation of an ERBB2 gene expression signature from tumor profiling

Supervised analysis was utilized to identify a gene expression signature correlated with ERBB2 status. It was applied to the mRNA expression profiles from 145 randomly chosen breast cancer samples (learning set) by comparing two subgroups defined by their ERBB2 status as determined by standard IHC: samples scoring 0 and 1+ (hereafter designated ERBB2-, 116 samples) were compared to samples scoring 3+ (ERBB2+, 29 samples). Cases with equivocal 2+ (n = 10) or unavailable (n=8) staining were excluded from analysis. To identify a molecular signature independent from the predefined subgroups of tumors identified by IHC, we iteratively defined several different subsets of samples and performed supervised analysis on each of these subsets independently. Thirty such iterations were done. The lists of genes identified as significant discriminators (these lists ranged from 80 to 274 clones) were then compared, revealing 37 clones present in at least 25 lists: these clones defined our ERBB2-specific gene expression signature (GES). All of the genes identified in this signature were tag-resequenced to confirm their identity.

Figure 1 shows the expression pattern of this signature in the 145 breast cancer samples in a color-coded matrix. Tumor samples are classified on the horizontal axis according to their correlation coefficients with the ERBB2+ group. As shown, the resulting discrimination between ERBB2+ and ERBB2- samples was successful . These 37 clones corresponded to 36 unique sequences representing 29 characterized genes (two different clones represented ERBB2) and 7 other sequences or ESTs. Twenty-nine were overexpressed and 8 were underexpressed in ERBB2+ samples. Their chromosomal location is listed in Figure 1.

Once identified on this set of 145 samples, we validated our ERBB2 GES in an independent set of 54 breast cancer samples (validation set). As shown in Figure 2a, classification of samples based on the GES successfully classified them according to ERBB2 IHC status with only 1 ERBB2-negative sample misplaced in the ERBB2+ group.

### VII.2) Comparative analysis of ERBB2 gene expression signature of human breast tissues to breast cancer cell lines

We profiled on the Ipsogen *DiscoveryChip* a series of 16 breast cancer cell lines that included 5 cell lines (BT-474, HCC1569, MDA-MB-453, SK-BR-3 and UACC-812) known to have amplification and/or high mRNA expression of the *ERBB2* gene (30, 31). Importantly, ERBB2 GES successfully separated ERBB2+ and ERBB2- cell lines (Figure 2b), further validating the discriminator potential of the signature.

Collectively, these analyses demonstrated that the ERBB2 gene expression signature according to the invention correctly classified breast tumors and cell lines consistent with ERBB2 status evaluated with standard procedure (Herceptest™, Dako Corporation).

### VII.3) Analysis of breast tumor samples using tissue microarrays

Significant discriminator genes were further validated by immunohistochemical analysis of their corresponding proteins (Figure 3a). A total of - 250 cases from TMA1 were available for the study of ERBB2, ER, GATA4 and Ki67. In ERBB2 GES, *ERBB2*, *GATA4* and *Ki67* genes were overexpressed and *ESR1* was underexpressed in ERBB2+ samples. These correlations were confirmed at the protein level: overexpression of ERBB2 protein was significantly associated with an upregulation of GATA4 (p<0.001), Ki67 (p<0.025), and with negativity of ER (p<0.0001) (Table 3 hereunder).

**Table 3**

| | ERBB2 (0-1+) n (%) | ERBB2 (2-3+) n (%) | p-value* |
|---|---|---|---|
| GATA4 | | | |
| negative | 169 (90%) | 18 (10%) | |
| positive | 50 (71%) | 20 (29%) | <0.001 |

| Ki67 | | | |
|---|---|---|---|
| <20 | 151 (88%) | 21 (12%) | |
| >=20 | 59 (78%) | 17 (22%) | <0.025 |

| ER | | | |
|---|---|---|---|
| negative | 27 (60%) | 18 (40%) | |
| positive | 179 (90%) | 20 (10%) | <0.0001 |

| | | | |
|---|---|---|---|
| * Fisher exact test | | | |

We found 40% of ERBB2-positive tumors in ER-negative tumors but only 10% in ER-positive tumors.

A total of 68 (72%) of the 94 samples included in TMA2 were available for FISH analysis of *ERBB2* locus. Examples of results are shown in Figure 3b. Of the 68 cases, 30 displayed *ERBB2* amplification whereas 38 were not amplified.

### VII.4) Classification of breast tumors using ERBB2 gene expression signature

Previous supervised analyses did not include the breast cancer samples scored 2+ for ERBB2 IHC. We reclassified these cases with all 145 samples previously analyzed - which included the 68 cases with available FISH ERBB2 data - by using hierarchical clustering program based on ERBB2 GES. Results are displayed in Figure 4 that highlights clusters of correlated genes across clusters of correlated samples (n=159, learrning set, 2+ samples, and 4 samples with unavailable ERBB2 status). The first large gene cluster contained 29 genes/ESTs, including *ERBB2* (it was designated "ERBB2 cluster"). The second gene cluster was globally anticorrelated with the previous one: it contained 8 genes/ESTs, including *ESR1* that codes for estrogen receptor _ (it was designated "ER cluster").

Despite significant transcriptional heterogeneity between tumors for these genes, the combined expression patterns defined at least three clusters of tumors, designated A, B and C. Group A (73 cases, in green) displayed an overexpression of the "ER cluster" and an underexpression of the "ERBB2 cluster" overall compared to groups B and C. Conversely, the "ERBB2 cluster" and the "ER cluster" were upregulated and downregulated in group C samples (36 cases, in red) overall, as compared to other groups. Finally, group B (50 cases, in black) displayed an intermediate profile with heterogenous expression of the "ERBB2 cluster" and underexpression of the "ER cluster".

Correlations of tumor groups as defined by hierarchical clustering with ERBB2 status were analyzed. As expected, group C strongly differed from the other groups with respect to ERBB2 protein expression since 93% of all ERBB2 3+ samples were located in this group. In group C, 77% of samples scored 3+, 9% 2+ and 14% 0-1+; in contrast, in groups A and B, these rates were 0% and 5% (3+), 3% and 10% (2+), and 97% and 85% (0-1+) (p<0.0001, Chi-2 test, A *vs* B *vs* C groups), respectively. As expected, there was also a strong correlation between tumor groups and FISH status with most of the FISH positive cases clustered in group C (p<0.0001, Chi-2 test, A vs B vs C groups). ERBB2 FISH information and IHC status were both available in 64 cases out of 159. Interestingly, the three 2+ tumors located in group C displayed ERBB2 amplification (FISH positive), while the seven 2+ tumors included in group A (2 cases) and group B (5 cases) had no amplification (FISH negative). These results shows that our ERBB2 GES could separate FISH-positive and FISH-negative ERBB2 2+ tumors, providing more specific information than FISH with respect to ERBB2 IHC status (HercepTest^{™}). Indeed, the correlation between GES groups (C samples vs A+B samples) and FISH result (negative *vs* positive) provided a sensitivity of 90% and a specificity of 88% (concordance in 89% of cases). In comparison, the correlation between IHC-based grouping (0-1+ vs 2-3+) and FISH status showed an equal sensitivity of 90% but a weaker specificity of 76% (concordance in 82% of cases) (Table 4 hereunder).

**Table 4**

| | | FISH status | | Total* |
|---|---|---|---|---|
| | | negative | positive | |
| GES groups | A+B | 30 | 3*** | 33 |
| | C | 4 | 27 | 31 |
| Total* | | 34 | 30 | 64 |
| | | | | |
| IHC status** | negative | 26 | 3*** | 29 |
| | positive | 8 | 27 | 35 |
| Total* | | 34 | 30 | 64 |

| | | | | |
|---|---|---|---|---|
| *, considering 64 tumors with data available for IHC, FISH et GES-based grouping; **, negative: 0-1+ and positive, 2-3+; ***, two samples are probably false-positive FISH results. | | | | |

Sensitivity was even probably better for the two comparisons; as shown in Figure 4, two samples located in groups A and B and IHC-negative for ERBB2 were FISH-positive; reviewing of the corresponding sections revealed in fact the presence of intra-ductal carcinoma in one case and abundant necrosis in the other case, both of which might have lead to false positive FISH results. Verification using real-time quantitative PCR definitely demonstrated absence of ERBB2 amplification (not shown). Taken into account the two samples with false-positive FISH results, the error rate was 5 out of 64 (with 4 false-positive and 1 false-negative) for correlation between our classification and FISH, whereas it was 9 out of 64 for correlation between standard IHC and FISH.

### VII.5) Correlation with histoclinical parameters

We searched for correlations between tumor groups and relevant molecular and histoclinical parameters of samples. As expected (32) our GES-based grouping correlated with SBR grade and hormone receptor status, further, albeit indirectly, validating our classification. Group C did not contain grade 1 samples; 44% of samples were grade 2 and 56% were grade 3. In groups A+B, 15% of samples were grade 1, 48% were grade 2 and 37% were grade 3 (p=0.02, Chi-2 test). In group C, samples were likely to be ER-negative (59%), compared with 27% in groups A+B (p=0.001, Chi-2 test). Similarly, although not significant, correlation was found for PR status (p=0.07, Chi-2 test). No correlation was found with pathological size of tumors, axillary lymph node status and P53 IHC status.

### References:

1. Slamon DJ, Clark GM, Wong SG, Levin WJ, Ullrich A, McGuire WL: Human breast cancer: correlation of relapse and survival with amplification of the HER-2/neu oncogene. Science 1987, 235, 177-182.
2. Eccles SA: The role of c-erbB-2/HER2/neu in breast cancer progression and metastasis. J Mammary Gland Biol Neoplasia 2001, 6:393-406.
3. Holbro T, Civenni G, Hynes NE: The ErbB receptors and their role in cancer progression. Exp Cell Res 2003, 284:99-110.
4. Ross JS, Fletcher JA: The HER-2/neu oncogene: prognostic factor, predictive factor and target for therapy. Semin Cancer Biol 1999, 9:125-138.
5. Hayes DF, Thor AD: c-erbB-2 in breast cancer: development of a clinically useful marker. Semin Oncol 2002, 29:231-245.
6. Slamon DJ: Herceptin((R)): increasing survival in metastatic breast cancer. Eur J Oncol Nurs 2000, 4:24-29.
7. Horton J: Trastuzumab use in breast cancer: clinical issues. Cancer Control 2002, 9:499-507.
8. Leyland-Jones B: Trastuzumab: hopes and realities. Lancet Oncol 2002, 3:137-144.
9. Di Leo A, Dowsett M, Horten B, Penault-Llorca F: Current status of HER2 testing. Oncology 2002, 63 Suppl 1:25-32.
10. Rampaul RS, Pinder SE, Gullick WJ, Robertson JF, Ellis IO: HER-2 in breast cancer--methods of detection, clinical significance and future prospects for treatment. Crit Rev Oncol Hematol 2002, 43:231-244.
11. Bilous M, Dowsett M, Hanna W, Isola J, Lebeau A, Moreno A, Penault-Llorca F, Ruschoff J, Tomasic G, Van De Vijver M: Current Perspectives on HER2 Testing: A Review of National Testing Guidelines. Mod Pathol 2003, 16:173-182.
12. Zarbo RJ, Hammond ME: Conference summary, Strategic Science symposium. Her-2/neu testing of breast cancer patients in clinical practice. Arch Pathol Lab Med 2003, 127:549-553.
13. Pauletti G, Dandekar S, Rong H, Ramos L, Peng H, Seshadri R, Slamon DJ: Assessment of methods for tissue-based detection of the HER-2/neu alteration in human breast cancer: a direct comparison of fluorescence in situ hybridization and immunohistochemistry. J Clin Oncol 2000, 18:3651-3664.
14. Oh JJ, Grosshans DR, Wong SG, Slamon DJ: Identification of differentially expressed genes associated with HER-2/neu overexpression in human breast cancer cells. Nucleic Acids Res 1999, 27:4008-4017.
15. Bertucci F, Viens P, Hingamp P, Nasser V, Houlgatte R, Birnbaum D: Breast cancer revisited using DNA array-based gene expression profiling. Int J Cancer 2003, 103: 565-571
16. Bertucci F, Viens P, Tagett R, Nguyen C, Houlgatte R, Birnbaum D. DNA arrays in clinical oncology: promises and challenges. Lab Invest 2003, 83:305-316.
17. Golub TR, Slonim DK, Tamayo P, Huard C, Gaasenbeek M, Mesirov JP, Coller H, Loh ML, Downing JR, Caligiuri MA, Bloomfield CD, Lander ES: Molecular classification of cancer: class discovery and class prediction by gene expression monitoring. Science 1999, 286:531-537.
18. Perou CM, Sorlie T, Eisen MB, van de Rijn M, Jeffrey SS, Rees CA, Pollack JR, Ross DT, Johnsen H, Akslen LA, Fluge 0, Pergamenschikov A, Williams C, Zhu SX, Lonning PE, Borresen-Dale AL, Brown PO, Botstein D. Molecular portraits of human breast tumors. Nature 2000, 406:747-752
19. Bertucci F, Houlgatte R, Benziane A, Granjeaud S, Adelaide J, Tagett R, Loriod B, Jacquemier J, Viens P, Jordan B, Birnbaum D Nguyen C: Expression profiling in primary breast carcinomas using arrays of candidate genes. Hum Mol Genet 2000, 9:2981-2991
20. Sorlie T, Perou CM, Tibshirani R, Aas T, Geisler S, Johnsen H, Hastie T, Eisen MB, van de Rijn M, Jeffrey SS, Thorsen T, Quist H, Matese JC, Brown PO, Botstein D, Eystein Lonning P, Borresen-Dale AL. Gene expression patterns of breast carcinomas distinguish tumor subclasses with clinical implications. Proc Natl Acad Sci USA 2001; 98: 10869-10874.
21. Bertucci F, Nasser V, Granjeaud S, Eisinger F, Adelaide J, Tagett R, Loriod B, Giaconia A, Benziane A, Devilard E, Jacquemier J, Viens P, Nguyen C, Birnbaum D, Houlgatte R: Gene expression profiles of poor prognosis primary breast cancer correlate with survival. Hum Mol Genet 2002, 11: 863-872
22. Van't Veer LJ, Dai H, van de Vijver M, He YD, Hart AA, Mao M, Peterse HL, van der Kooy K, Marton MJ, Witteveen AT, Schreiber GJ, Kerkhoven RM, Roberts C, Linsley PS, Bernards R, Friend SH: Gene expression profiling predicts clinical outcome of breast cancer. Nature 2002, 415:530-535
23. van de Vijver MJ, He YD, van't Veer LJ, Dai H, Hart AA, Voskuil DW, Schreiber GJ, Peterse JL, Roberts C, Marton MJ, Parrish M, Atsma D, Witteveen A, Glas A, Delahaye L, van der Velde T, Bartelink H, Rodenhuis S, Rutgers ET, Friend SH, Bernards R: A gene-expression signature as a predictor of survival in breast cancer. N Engl J Med 2002, 347:1999-2009
24. Sorlie T, Tibshirani R, Parker J, Hastie T, Marron JS, Nobel A, Deng S, Johnsen H, Pesich R, Geisler S, Demeter J, Perou CM, Lonning PE, Brown PO, Borresen-Dale AL, Botstein D: Repeated observation of breast tumor subtypes in independent gene expression data sets. Proc Natl Acad Sci USA 2003, 100:8418-8423.
25. Theillet C, Adelaïde J, Louason G, Bonnet-Dorion F, Jacquemier J, Adnane J, Longy M, Katsaros D, Sismondi P, Gaudray P, Birnbaum D: FGFRI and PLAT genes and DNA amplification at 8p12 in breast and ovarian cancers. Genes Chromosomes Cancer 1993, 7:219-226.
26. Sabatti C, Karsten SL, Geschwind DH: Thresholding rules for recovering a sparse signal from microarray experiments. Math Biosci 2002, 176:17-34.
27. Magrangeas F, Nasser V, Avet-Loiseau H, Loriod B, Decaux 0, Granjeaud S, Bertucci F, Birnbaum D, Nguyen C, Harousseau JL, Bataille R, Houlgatte R, Minvielle S: Gene expression profiling of multiple myeloma reveals molecular portraits in relation to the pathogenesis of the disease. Blood 2003101:4998-5006.
28. Richter J, Wagner U, Kononen J, Fijan A, Bruderer J, Schmid U, Ackerman D, Maurer R, Alund G, Knönagel H, Rist M, Wilber K, Anabitarte M, Hering F, Hardmeier T, Schönenberger A, Flury R, Jäger P, Fehr JL, Schrami P, Moch H, Mihatsch MJ, Gasser T, Kallioniemi OP, Sauter G: High-throughput tissue microarray analysis of cyclin E gene amplification and overexpression in urinary bladder cancer. Am J Pathol 2000, 157:787-794.
29. Ginestier C, Charafe-Jauffret E, Bertucci F, Eisinger F, Geneix J, Bechlian D, Conte N, Adelaide J, Toiron Y, Nguyen C, Viens P, Mozziconacci MJ, Houlgatte R, Birnbaum D, Jacquemier J: Distinct and complementary information provided by use of tissue and DNA microarrays in the study of breast tumor markers. Am J Pathol 2002, 161:1223-1233
30. Kauraniemi P, Bärlund M, Monni O, Kallioniemi A: New amplified and highly expressed genes discovered in the ERBB2 amplicon in breast cancer by cDNA microarray. Cancer Res 2001, 61:8235-8240.
31. Wilson KS, Roberts H, Leek R, Harris AL, Geradts J: Differential gene expression patterns in HER2/neu- positive and -negative breast cancer cell lines and tissues. Am J Pathol 2002, 161:1171-1185
32. Revillion F, Bonneterre J, Peyrat JP: ERBB2 oncogene in human breast cancer and its clinical significance. Eur J Cancer 1998, 34:791-808.
33. Shen TL, Han DC, Guan JL: Association of Grb7 with phosphoinositides and its role in the regulation of cell migration. J Biol Chem 2002, 277:29069-29077
34. Frade R, Balbo M, Barel M: RB18A regulates p53-dependent apoptosis. Oncogene 2002, 21:861-866.
35. Andersen CL, Monni O Wagner U, Kononen J, Barlund M, Bucher C, Haas P, Nocito A, Bissig H, Sauter G, Kallioniemi A: High-throughput copy number analysis of 17q23 in 3520 tissue specimens by fluorescence in situ hybridization to tissue microarrays. Am J Pathol 2002, 161:73-79.
36. Hyman E, Kauraniemi P, Hautaniemi S, Wolf M, Mousses S, Rozenblum E, Ringner M, Sauter G, Monni O, Elkahloun A, Kallioniemi OP, Kallioniemi A: Impact of DNA amplification on gene expression patterns in breast cancer. Cancer Res 2002, 62:6240-6245.
37. Platzer P, Upender MB, Wislon K, Willis J, Lutterbaugh J, Nosrati A, Willson JKV, mack D, Ried T, Markowitz S: Silence of chromosomal amplifications in colon cancer. Cancer Res 2002, 62:1134-1138.
38. Patient RK, McGhee JD. The GATA family (vertebrates and invertebrates). Curr Opin Genet Dev 2002, 12:416-422.
39. Kuo CT, Morrisey EE, Anandappa R, Sigrist K, Lu MM, Parmacek MS, Soudais C, Leiden JM. GATA4 transcription factor is required for ventral morphogenesis and heart tube formation. Genes Dev 1997, 11:1048-1060.
40. Molkentin JD, Lin Q, Duncan SA, Olson EN. Requirement of the transcription factor GATA4 for heart tube formation and ventral morphogenesis. Genes Dev 1997,11:1061-1072.
41. Lee KF, Simon H, Chen H, Bates B, Hung MC, Hauser C. Requirement for neuregulin receptor erbB2 in neural and cardiac development. Nature 1995, 378:394-398.
42. Garratt AN, Ozcelik C, Birchmeier C: ErbB2 pathways in heart and neural diseases. Trends Cardiovasc Med 2003, 13:80-86.
43. Han J, Lee JD, Jiang Y, Li Z, Feng L, Ulevitch RJ: Characterization of the structure and function of a novel MAP kinase kinase (MKK6). J Biol Chem 1996, 271:2886-2891.
44. Schneider JW, Chang AY, Garratt A. Trastuzumab cardiotoxicity: Speculations regarding pathophysiology and targets for further study. Semin Oncol 2002, 29:22-28.
45. Charron F, Tsimiklis G, Arcand M, Robitaille L, Liang Q, Molkentin JD, Meloche S, Nemer M: Tissue-specific GATA factors are transcriptional effectors of the small GTPase RhoA. Genes Dev 2001, 15:2702-2719.
46. Yanazume T, Hasegawa K, Wada H, Morimoto T, Abe M, Kawamura T, Sasayama S: Rho/ROCK pathway contributes to the activation of extracellular signal-regulated kinase/GATA-4 during myocardial cell hypertrophy. J Biol Chem 2002, 277:8618-2865.
47. Arthur WT, Noren NK, Burridge K: Regulation of Rho family GTPases by cell-cell and cell-matrix adhesion. Biol Res 2002, 35:239-246.
48. Korsching E, Packeisen J, Agelopoulos K, Eisenacher M, Voss R, Isola J, van Diest PJ, Brandt B, Boecker W, Buerger H: Cytogenetic alterations and cytokeratin expression patterns in breast cancer: integrating a new model of breast differentiation into cytogenetic pathways of breast carcinogenesis. Lab Invest 2002, 82:1525-1533.
49. Callagy G, Cattaneo E, Daigo Y, Happerfield L, Bobrow LG, Pharoah PD, Caldas C: Molecular classification of breast carcinomas using tissue microarrays. Diagn Mol Pathol 2003, 12:27-34.
50. Berns EM, Klijn JG, van Staveren IL, Portengen H, Noordegraaf E, Foekens JA: Prevalence of amplification of the oncogenes c-myc, HER2/neu, and int-2 in one thousand human breast tumors: correlation with steroid receptors. Eur J Cancer 1992, 28:697-700.
51. Keshgegian AA: ErbB-2 oncoprotein overexpression in breast carcinoma: inverse correlation with biochemically- and immunohistochemically-determined hormone receptors. Breast Cancer Res Treat 1995, 35:201-210.
52. Carlomagno C, Perrone F, Gallo C, De Laurentiis M, Lauria R, Morabito A, Pettinato G, Panico L, D'Antonio A, Bianco AR, De Placido S: c-erb B2 overexpression decreases the benefit of adjuvant tamoxifen in early-stage breast cancer without axillary lymph node metastases. J Clin Oncol 1996, 14:2702-2708.
53. Konecny G, Pauletti G, Pegram M, Untch M, Dandekar S, Aguilar Z, Wilson C, Rong HM, Bauerfeind I, Felber M, Wang HJ, Beryt M, Seshadri R, Hepp H, Slamon DJ: Quantitative association between HER-2/neu and steroid hormone receptors in hormone receptor-positive primary breast cancer. J Natl Cancer Inst 2003, 95:142-153.
54. Pietras RJ, Arboleda J, Reese DM, Wongvipat N, Pegram MD, Ramos L, Gorman CM, Parker MG, Sliwkowski MX, Slamon DJ: HER-2 tyrosine kinase pathway targets estrogen receptor and promotes hormone-independent growth in human breast cancer cells. Oncogene 1995, 10:2435-2446.
55. Yang RB, Ng CK, Wasserman SM, Colman SD, Shenoy S, Mehraban F, Komuves LG, Tomlinson JE, Topper JN: Identification of a novel family of cell-surface proteins expressed in human vascular endothelium. J Biol Chem 2002, 277:46364-46373.
56. Willis S, Hutchins AM, Hammet F, Ciciulla J, Soo WK, White D, van der Spek P, Henderson MA, Gish K, Venter DJ, Armes JE: Detailed gene copy number and RNA expression analysis of the 17q12-23 region in primary breast cancers. Genes Chromosomes Cancer 2003, 36:382-392
57. Dressman MA, Baras A, Malinowski R, Alvis LB, Kwon I, Walz TM, Polymeropoulos MH: Gene expression profiling detects gene amplification and differentiates tumor types in breast cancer. Cancer Res 2003, 63:2194-2199
58. Tan M, Yao J, Yu D: Overexpression of the c-erbB-2 gene enhanced intrinsic metastasis potential in human breast cancer cells without increasing their transformation abilities. Cancer Res 1997, 57:1199-1205.
59. Spencer KS, Graus-Porta D, Leng J, Hynes NE, Klemke RL: ErbB2 is necessary for induction of carcinoma cell invasion by ErbB family receptor tyrosine kinases. J Cell Biol 2000, 148:385-397.
60. Mackay A, Jones C, Dexter T, la Silva R, Bulmer K, Jones A, Simpson P, Harris RA, Jat PS, Neville AM, Reis LFL, Lakhani SR, O'Hare MJ: cDNA microarray analysis of genes associated with ERBB2 (HER2/neu) overexpression in humna mammary luminal epithelial cells. Oncogene 2003, 22:2680-2688
61. Tomasetto C, Regnier C, Moog-Lutz C, Mattei MG, Chenard MP, Lidereau R, Basset P, Rio MC: Identification of four novel human genes amplified and overexpressed in breast carcinoma and localized to the q11-q21.3 region of chromosome 17. Genomics 1995, 28:367-376.
62. Kumar-Sinha C, Woods Ignatoski K, Lippman ME, Ethier SP, Chinnaiyan AM: Transcriptome analysis of HER2 reveals a molecular connection to fatty acid synthesis. Cancer Res 2003, 63:132-139.
63. Tiwari RK, Mukhopadhyay B, Telang NT, Osborne MP: Modulation of gene expression by selected fatty acids in human breast cancer cells. Anticancer Res 1991, 11:1383-1388.
64. Gilde AJ, Van Bilsen M: Peroxisome proliferator-activated receptors (PPARS): regulators of gene expression in heart and skeletal muscle. Acta Physiol Scand 2003, 178:425-434.
65. Press MF, Slamon DJ, Flom KJ, Park J, Zhou JY, Bernstein L: Evaluation of HER-2/neu gene amplification and overexpression: comparison of frequently used assay methods in a molecularly characterized cohort of breast cancer specimens. J Clin Oncol 2002, 20:3095-3105.
66. van de Vijver M: Emerging technologies for HER2 testing. Oncology 2002, 63 Suppl 1:33-38.
67. Tagliabuea E, Agrestib R, Carcangiuc ML, Ghirellia C, Morellid D, Campiglioa M, Martelc M, Giovanazzib R, Grecob M, Balsarie A and Ménard S : Role of HER2 in wound-induced breast carcinoma proliferation The Lancet Volume 362, Issue 9383 , Pages 527-533

## Claims

1. Method for identifying ERBB2 alteration in breast tumors based on the analysis of the over or under expression of polynucleotide sequences in a breast tissue sample said analysis comprising the detection of at least one, preferably at least two or three, polynucleotide sequence or complement thereof, selected in each of the predefined polynucleotide sequence sets consisting of:
Set 1 : SEQ ID NO. 73, 74, 75, 76, 77 (ERBB2)
Set 2 . SEQ ID NO. 28, 29, 30 (GRB7)
Set 3 : SEQ ID NO. 83, 84, 85 (NR1D1)
Set 4 : SEQ ID NO. 78, 79, 80 (GATA4)
Set 5 : SEQ ID NO. 41, 42, 43 (CDH15)
Set 6 : SEQ ID NO. 16, 17 (LTA)
Set 7 : SEQ ID NO. 86, 87, 116(MAP2K6)
Set 8 : SEQ ID NO. 54, 55, 113(PECAM1)
Set 9 : SEQ ID NO. 44, 45 (PPARBP)
Set 10 : SEQ ID NO. 33, 34, 35 (PPP1R1B)
Set 11 : SEQ ID NO. 39, 40 (RPL19)
Set 12 : SEQ ID NO. 4, 5, 6 (PSMB3)
Set 13 : SEQ ID NO. 10 (LOC148696)
Set 14 : SEQ ID NO. 12, 13(NOL3/loc283849)
Set 15 : SEQ ID NO. 14, 15 (ITGA2B)
Set 16 : SEQ ID NO. 18, 19 (NFKBIE)
Set 17 : SEQ ID NO. 22, 23 (PADI2)
Set 18 : SEQ ID NO. 24,25 (STAT3 or als2cr18)
Set 19 : SEQ ID NO. 26, 27 (OAS2)
Set 20 : SEQ ID NO. 36, 37, 38 (CDKL5)
Set 21 : SEQ ID NO. 46, 47, 48 (CSTA)
Set 22 : SEQ ID NO. 52, 53, 115 (ITGB3)
Set 23 : SEQ ID NO. 56, 57, 58 (MKI67 or KI67)
Set 24 : SEQ ID NO. 59, 60, 61 (PBEF)
Set 25 : SEQ ID NO. 62, 63, 64 (FADS2)
Set 26 : SEQ ID NO. 81, 82 (LOX)
Set 27 : SEQ ID NO. 88, 89, 90(ITGA2)
SET 28 : SEQ ID NO. 11 (ESTAA878915/NA or EST)
SET 29 : SEQ ID NO. 1, 2, 3 (JDP1)
SET 30 . SEQ ID NO. 7, 8, 9 (NAT1)
SET 31 : SEQ ID NO. 20, 21 (CELSR2)
SET 32 : SEQ ID NO. 31, 32 (ESTN33243 or EST)
SET 33 : SEQ ID NO. 49, 50, 51 (SCUBE2)
SET 34 : SEQ ID NO. 65, 66 (ESTH29301/NA or EST)
SET 35 : SEQ ID NO. 67, 68, 69 (FLJ10193)
SET 36 : SEQ ID NO. 70, 71, 72 (ESR1).

2. A method according to claim 1, wherein said detection of expression of polynucleotide sequences is carried out by FISH or IHC.

3. A method according to any of claim 1 or 2, wherein said detection of expression of polynucleotide sequences is performed on DNA microarrays.

4. A method according to claim 2 or 3 comprising:
a) reacting nucleic acids sample with complement polynucleotide sequences of said selected predefined polynucleotide sequences sets, and
b) detecting the reaction product of step (a).

5. The method according to Claim 4, wherein said nucleic acids sample is labelled before reaction step (a).

6. The method according to claim 5, wherein the label of the polynucleotide sample is selected from the group consisting of radioactive, colorimetric, enzymatic, molecular amplification, bioluminescent or fluorescent labels.

7. The method according to any of claims 4 to 6 further comprising obtaining a control polynucleotide sample, reacting said control sample with said complement polynucleotide sequence, detecting a control sample reaction product and comparing the amount of said polynucleotide sample reaction product to the amount of said control sample reaction product.

8. The method according to any of claims 4 to 7, wherein the nucleic acids sample is cDNA, RNA or mRNA.

9. The method of claim 8, wherein DNA is obtained from said nucleic acids sample and RNA is obtained by transcription of said DNA.

10. The method of claim 8, wherein mRNA is isolated from said nucleic acids sample and cDNA is obtained by reverse transcription of said mRNA.

11. The method according to any of claims 4 to 10, wherein said reaction step is performed by hybridising the nucleic acids sample with said complement polynucleotide sequences.

12. Use of a method according to any of claims 1 to 11, wherein said method is used for detecting, diagnosing, staging, or monitoring breast cancer.

13. The use according to claim 12, wherein said method is used to follow up the stage or aggressiveness of a breast cancer.

14. The use according to claims 12 for monitoring the treatment of a patient with a breast cancer comprising the implementation of the method of claim 1 on nucleic acids in a breast tissue sample of the patient.

15. The use according to claim 14, wherein said patient is scoring 2+ with the HercepTest^{™}.

16. The use according to claim 14 or 15, wherein said monitoring relates to the clinical efficacy of Herceptin treatment.

17. Use of the method of claim 1 for determining a treatment for the patient an animal with a breast cancer based on the analysis of differential gene expression profile obtained with said method.

18. A polynucleotide library useful for the molecular characterization of a breast cancer consisting of the polynucleotide sequences defined in claim 1.

19. A polynucleotide library according to claim 18 immobilized on a solid support.

20. A polynucleotide library according to claim 19, wherein the support is selected from the group comprising nylon membrane, nitrocellulose membrane, glass slide, glass beads, membranes on glass support or silicon chip.

## Patentansprüche

1. Verfahren zum Identifizieren einer ERBB2-Veränderung in Brusttumoren auf Basis der Analyse der Über- oder Unterexpression von Polynukleotidsequenzen in einer Brustgewebeprobe, wobei die Analyse den Nachweis von mindestens einer, vorzugsweise mindestens zwei oder drei Polynukleotidsequenzen oder Komplemente davon umfasst, die aus jedem der vorher definierten Polynukleotidsequenzensätze ausgewählt sind, die aus Folgendem bestehen:
Satz 1: SEQ ID Nr. 73, 74, 75, 76, 77 (ERBB2)
Satz 2: SEQ ID Nr. 28, 29, 30 (GRB7)
Satz 3: SEQ ID Nr. 83, 84, 85 (NR1D1)
Satz 4: SEQ ID Nr. 78, 79, 80 (GATA4)
Satz 5: SEQ ID Nr. 41, 42, 43 (CDH15)
Satz 6: SEQ ID Nr. 16, 17 (LTA)
Satz 7: SEQ ID Nr. 86, 87, 116 (MAP2K6)
Satz 8: SEQ ID Nr. 54, 55, 113 (PECAM1)
Satz 9: SEQ ID Nr. 44, 45 (PPARBP)
Satz 10: SEQ ID Nr. 33, 34, 35 (PPP1R1B)
Satz 11: SEQ ID Nr. 39, 40 (RPL19)
Satz 12: SEQ ID Nr. 4, 5, 6 (PSMB3)
Satz 13: SEQ ID Nr. 10 (LOC148696)
Satz 14: SEQ ID Nr. 12, 13 (NOL3/loc283849)
Satz 15: SEQ ID Nr. 14, 15 (ITGA2B)
Satz 16: SEQ ID Nr. 18, 19 (NFKBIE)
Satz 17: SEQ ID Nr. 22, 23 (PADI2)
Satz 18: SEQ ID Nr. 24, 25 (STAT3 oder als2cr18)
Satz 19: SEQ ID Nr. 26, 27 (OAS2)
Satz 20: SEQ ID Nr. 36, 37, 38 (CDKL5)
Satz 21: SEQ ID Nr. 46, 47, 48 (CSTA)
Satz 22: SEQ ID Nr. 52, 53, 115 (ITGB3)
Satz 23: SEQ ID Nr. 56, 57, 58 (MKI67 oder KI67)
Satz 24: SEQ ID Nr. 59, 60, 61 (PBEF)
Satz 25: SEQ ID Nr. 62, 63, 64 (FADS2)
Satz 26: SEQ ID Nr. 81, 82 (LOX)
Satz 27: SEQ ID Nr. 88, 89, 90 (ITGA2)
SATZ 28: SEQ ID Nr. 11 (ESTAA878915/NA oder EST)
SATZ 29: SEQ ID Nr. 1, 2, 3 (JDP1)
SATZ 30: SEQ ID Nr. 7, 8, 9 (NAT1)
SATZ 31: SEQ ID Nr. 20, 21 (CELSR2)
SATZ 32: SEQ ID Nr. 31, 32 (ESTN33243 oder EST)
SATZ 33: SEQ ID Nr. 49, 50, 51 (SCUBE2)
SATZ 34: SEQ ID Nr. 65, 66 (ESTH29301/NA oder EST)
SATZ 35: SEQ ID Nr. 67, 68, 69 (FLJ10193)
SATZ 36: SEQ ID Nr. 70, 71, 72 (ESR1).

2. Verfahren nach Anspruch 1, wobei der Nachweis der Expression der Polynukleotidsequenzen mittels FISH oder IHC durchgeführt wird.

3. Verfahren nach einem der Ansprüche 1 oder 2, wobei der Nachweis der Expression der Polynukleotidsequenzen auf DNA-Mikroarrays durchgeführt wird.

4. Verfahren nach Anspruch 2 oder 3, das Folgendes umfasst:
a) Reagieren einer Nukleinsäurenprobe mit komplementären Polynukleotidsequenzen der ausgewählten vorher definierten Polynukleotidsequenzensätze und
b) Nachweisen des Reaktionsprodukts von Schritt (a).

5. Verfahren nach Anspruch 4, wobei die Nukleinsäurenprobe vor Reaktionsschritt (a) markiert wird.

6. Verfahren nach Anspruch 5, wobei der Marker der Polynukleotidprobe aus der Gruppe bestehend aus radioaktiven, kolorimetrischen, enzymatischen, molekularen Amplifikations-, biolumineszenten oder fluoreszierenden Markern ausgewählt ist.

7. Verfahren nach einem der Ansprüche 4 bis 6, das weiterhin das Erhalten einer Kontrollpolynukleotidprobe, Reagieren der Kontrollprobe mit der komplementären Polynukleotidsequenz, Nachweisen eines Kontrollprobenreaktionsprodukts und Vergleichen der Menge des Polynukleotidprobenreaktionsprodukts mit der Menge des Kontrollprobenreaktionsprodukts umfasst.

8. Verfahren nach einem der Ansprüche 4 bis 7, wobei die Nukleinsäurenprobe cDNA, RNA oder mRNA ist.

9. Verfahren nach Anspruch 8, wobei DNA aus der Nukleinsäurenprobe erhalten wird und RNA mittels Transkription der DNA erhalten wird.

10. Verfahren nach Anspruch 8, wobei mRNA aus der Nukleinsäurenprobe isoliert wird und cDNA mittels Umkehrtranskriptase der mRNA erhalten wird.

11. Verfahren nach einem der Ansprüche 4 bis 10, wobei der Reaktionsschritt durch Hybridisieren der Nukleinsäurenprobe mit der komplementären Polynukleotidprobe durchgeführt wird.

12. Verwendung eines Verfahrens nach einem der Ansprüche 1 bis 11, wobei das Verfahren zum Nachweisen, Diagnostizieren, Staging oder Überwachen eines Brustkarzinoms verwendet wird.

13. Verwendung nach Anspruch 12, wobei das Verfahren zum Weiterverfolgen des Stadiums oder der Aggressivität eines Brustkarzinoms verwendet wird.

14. Verwendung nach Anspruch 12 zum Überwachen der Behandlung eines Patienten mit einem Brustkarzinom, die die Anwendung des Verfahrens nach Anspruch 1 auf Nukleinsäuren in einer Brustgewebeprobe des Patienten umfasst.

15. Verwendung nach Anspruch 14, wobei der Patient beim HercepTest^{™} einen Score von 2+ hat.

16. Verwendung nach Anspruch 14 oder 15, wobei das Überwachen die klinische Wirksamkeit von Herceptin-Behandlung betrifft.

17. Verwendung des Verfahrens nach Anspruch 1 zum Bestimmen einer Behandlung für den Patienten mit einem Brustkarzinom auf Basis der Analyse eines Differentialgenexpressionsprofils, das mit dem Verfahren erhalten wurde.

18. Polynukleotidbibliothek, die zur molekularen Charakterisierung eines Brustkarzinoms geeignet ist und aus den in Anspruch 1 definierten Polynukleotidsequenzen besteht.

19. Polynukleotidbibliothek nach Anspruch 18, die auf einem festen Träger immobilisiert ist.

20. Polynukleotidbibliothek nach Anspruch 19, wobei der Träger aus der Gruppe umfassend Nylonmembran, Nitrocellulosemembran, Glasobjektträger, Glasperlen, Membranen auf einem Glasträger oder Siliziumchip ausgewählt ist.

## Revendications

1. Procédé d'identification d'une altération ERBB2 dans des tumeurs du sein basé sur l'analyse de la sur-expression ou de la sous-expression de séquences polynucléotidiques dans un échantillon de tissu du sein, ladite analyse comprenant la détection d'au moins une, de préférence d'au moins deux ou trois, séquence polynucléotidique ou de son complément, choisie dans chacun des jeux de séquences polynucléotidiques prédéfinis consistant en les :
jeu 1 : SEQ ID NO : 73, 74, 75, 76, 77 (ERBB2)
jeu 2 : SEQ ID NO : 28, 29, 30 (GRB7)
jeu 3 : SEQ ID NO : 83, 84, 85 (NR1D1)
jeu 4 : SEQ ID NO : 78, 79, 80 (GATA4)
jeu 5 : SEQ ID NO : 41, 42, 43 (CDH15)
jeu 6 : SEQ ID NO : 16, 17 (LTA)
jeu 7 : SEQ ID NO : 86, 87, 116 (MAP2K6)
jeu 8 : SEQ ID NO : 54, 55, 113 (PECAM1)
jeu 9 : SEQ ID NO : 44, 45 (PPARBP)
jeu 10 : SEQ ID NO : 33, 34, 35 (PPP1R1B)
jeu 11 : SEQ ID NO : 39, 40 (RPL19)
jeu 12 : SEQ ID NO : 4, 5, 6 (PSMB3)
jeu 13 : SEQ ID NO : 10 (LOC148696)
jeu 14 : SEQ ID NO : 12, 13 (NOL3/loc283849)
jeu 15 : SEQ ID NO : 14, 15 (ITGA2B)
jeu 16 : SEQ ID NO : 18, 19 (NFKBIE)
jeu 17 : SEQ ID NO : 22, 23 (PADI2)
jeu 18 : SEQ ID NO : 24, 25 (STAT3 ou als2cr18)
jeu 19 : SEQ ID NO : 26, 27 (OAS2)
jeu 20 : SEQ ID NO : 36, 37, 38 (CDKL5)
jeu 21 : SEQ ID NO : 46, 47, 48 (CSTA)
jeu 22 : SEQ ID NO : 52, 53, 115 (ITGB3)
jeu 23 : SEQ ID NO : 56, 57, 58 (MKI67 ou KI67)
jeu 24 : SEQ ID NO : 59, 60, 61 (PBEF)
jeu 25 : SEQ ID NO : 62, 63, 64 (FADS2)
jeu 26 : SEQ ID NO : 81, 82 (LOX)
jeu 27 : SEQ ID NO : 88, 89, 90 (ITGA2)
jeu 28 : SEQ ID NO : 11 (ESTAA878915/NA ou EST)
jeu 29 SEQ ID NO : 1, 2, 3 (JDP1)
jeu 30 : SEQ ID NO : 7, 8, 9 (NAT1)
jeu 31 : SEQ ID NO : 20, 21 (CELSR2)
jeu 32 : SEQ ID NO : 31, 32 (ESTN33243 ou EST)
jeu 33 : SEQ ID NO : 49, 50, 51 (SCUBE2)
jeu 34 : SEQ ID NO : 65, 66, (ESTH29301/NA ou EST)
jeu 35 : SEQ ID NO : 67, 68, 69 (FLJ10193)
jeu 36 : SEQ ID NO : 70, 71, 72 (ESR1).

2. Procédé selon la revendication 1, dans lequel ladite détection de l'expression de séquences polynucléotidiques est exécutée par FISH ou IHC.

3. Procédé selon l'une quelconque des revendications 1 ou 2, dans lequel ladite détection de l'expression de séquences polynucléotidiques est effectuée sur des puces à ADN.

4. Procédé selon la revendication 2 ou 3, comprenant :
a) la réaction d'un échantillon d'acides nucléiques avec des séquences polynucléotidiques complémentaires desdits jeux de séquences polynucléotidiques prédéfinis choisis, et
b) la détection du produit réactionnel de l'étape (a).

5. Procédé selon la revendication 4, dans lequel ledit échantillon d'acides nucléiques est marqué avant l'étape réactionnelle (a).

6. Procédé selon la revendication 5, dans lequel le marqueur de l'échantillon de polynucléotide est choisi dans le groupe constitué par des marqueurs radioactifs, colorimétriques, enzymatiques, d'amplification moléculaire, bioluminescents ou fluorescents.

7. Procédé selon l'une quelconque des revendications 4 à 6, comprenant en outre l'obtention d'un échantillon de polynucléotide de contrôle, la réaction dudit échantillon de contrôle avec ladite séquence polynucléotidique complémentaire, la détection d'un produit réactionnel de l'échantillon de contrôle et la comparaison de la quantité dudit produit réactionnel de l'échantillon de polynucléotide avec la quantité dudit produit réactionnel de l'échantillon de contrôle.

8. Procédé selon l'une quelconque des revendications 4 à 7, dans lequel l'échantillon d'acides nucléiques est l'ADNc, l'ARN ou l'ARNm.

9. Procédé selon la revendication 8, dans lequel l'ADN est obtenu à partir de l'échantillon d'acides nucléiques et l'ARN est obtenu par transcription dudit ADN.

10. Procédé selon la revendication 8, dans lequel l'ARNm est isolé à partir dudit échantillon d'acides nucléiques et l'ADNc est obtenu par transcription inverse dudit ARNm.

11. Procédé selon l'une quelconque des revendications 4 à 10, dans lequel ladite étape réactionnelle est effectuée par hybridation de l'échantillon d'acides nucléiques aux dites séquences polynucléotidiques complémentaires.

12. Utilisation du procédé selon l'une quelconque des revendications 1 à 11, dans laquelle ledit procédé est utilisé pour détecter, diagnostiquer, évaluer le stade, ou surveiller un cancer du sein.

13. Utilisation selon la revendication 12, dans laquelle ledit procédé est utilisé pour suivre le stade ou l'agressivité d'un cancer du sein.

14. Utilisation selon la revendication 12 pour surveiller le traitement d'une patiente avec un cancer du sein, comprenant la mise en oeuvre du procédé selon la revendication 1 sur des acides nucléiques dans un échantillon de tissu du sein de la patiente.

15. Utilisation selon la revendication 14, dans laquelle ladite patiente obtient un résultat de 2+ avec le dispositif HercepTest^{™}.

16. Utilisation selon la revendication 14 ou 15, dans laquelle ladite surveillance concerne l'efficacité clinique du traitement par Herceptin.

17. Utilisation selon la revendication 1 pour déterminer un traitement pour la patiente avec un cancer du sein basé sur l'analyse du profil d'expression de gène différentiel obtenu avec ledit procédé.

18. Bibliothèque de polynucléotides utile pour la caractérisation moléculaire d'un cancer du sein, constituée par les séquences polynucléotidiques définies dans la revendication 1.

19. Bibliothèque de polynucléotides selon la revendication 18, immobilisée sur un support solide.

20. Bibliothèque de polynucléotide selon la revendication 19, dans laquelle le support est choisi dans le groupe comprenant une membrane de nylon, une membrane de nitrocellulose, une lamelle de verre, des billes de verre, des membranes sur un support de verre ou une puce de silicium.
